# EUROPEAN PATENT SPECIFICATION

(11) **EP 3 272 871 B1**
(45) Date of publication and mention of the grant of the patent: **15.01.2020**
(21) Application number: 17188792.0
(22) Date of filing: 29.03.2010
(51) Int. Cl.: C12Q 1/6886, C07K 14/47, G01N 33/574, C12N 9/02

(54) **ABERRANT MITOCHONDRIAL DNA, ASSOCIATED FUSION TRANSCRIPTS AND TRANSLATION PRODUCTS AND HYBRIDIZATION PROBES THEREFOR**
ABERRANTE MITOCHONDRIALE DNA, ENTSPRECHENDE FUSIONSTRANSKRIPTE SOWIE ÜBERSETZUNGSPRODUKTE UND HYBRIDISIERUNGSSONDEN DAFÜR
ADN MITOCHONDRIAL ABERRANT, TRANSCRIPTION DE FUSION ASSOCIÉES, PRODUITS DE TRANSLATION ET SONDES D'HYBRIDATION ASSOCIÉES

(30) Priority: 27.03.2009 WO PCT/CA2009/000351
(43) Date of publication of application: 24.01.2018
(62) Divisional of application: 10761136.0
(73) Proprietor: MDNA Life Sciences Inc., Wilmington, DE 19801 (US)
(72) Inventor: Parr, Ryan, Thunder Bay, Ontario P7G 1J4 (CA); Dakubo, Gabriel, Thunder Bay, Ontario P7J 1H7 (CA); Harbottle, Andrew, Newcastle Upon Tyne Tyne and Wear NE1 3DW (GB); Reguly, Brian, Vancouver, British Columbia V5Z 1R3 (CA); Creed, Jennifer, Broomfield, CO 80023 (US); Robinson, Kerry, Thunder Bay ON P7A 3S9 (CA); Klein, Daniel, Thunder Bay, Ontario P7E 5S8 (CA)
(74) Representative: Nederlandsch Octrooibureau

(56) References cited:
- WO-A1-2009/117811
- US-B1- 6 218 117
- ANNA M CZARNECKA ET AL: "Mitochondrial NADH-dehydrogenase subunit 3 (ND3) polymorphism (A10398G) and sporadic breast cancer in Poland", BREAST CANCER RESEARCH AND TREATMENT, KLUWER ACADEMIC PUBLISHERS, BO, vol. 121, no. 2, 6 March 2009 (2009-03-06) , pages 511-518, XP019814339, ISSN: 1573-7217
- JENNIFER MAKI ET AL: "Mitochondrial genome deletion aids in the identification of false- and true-negative prostate needle core biopsy specimens", AMERICAN JOURNAL OF CLINICAL PATHOLOGY, AMERICAN SOCIETY FOR CLINICAL PATHOLOGY, US, vol. 129, no. 1, 1 January 2008 (2008-01-01), pages 57-66, XP008132881, ISSN: 0002-9173, DOI: 10.1309/UJJTH4HFEPWAQ78Q
- ZHU WEIZHU ET AL: "Large-scale mitochondrial DNA deletion mutations and nuclear genome instability in human breast cancer", CANCER DETECTION AND PREVENTION, ELSEVIER SCIENCE, vol. 28, no. 2, 1 January 2004 (2004-01-01), pages 119-126, XP002524596, ISSN: 0361-090X, DOI: 10.1016/J.CDP.2003.11.008
- G. GASPARRE ET AL: "Disruptive mitochondrial DNA mutations in complex I subunits are markers of oncocytic phenotype in thyroid tumors", PROCEEDINGS OF THE NATIONAL ACADEMY OF SCIENCES, vol. 104, no. 21, 22 May 2007 (2007-05-22) , pages 9001-9006, XP055044227, ISSN: 0027-8424, DOI: 10.1073/pnas.0703056104

## Description

### FIELD OF THE INVENTION

The present invention relates to the field of mitochondrial genomics and proteomics. In one aspect, the invention relates to the identification and use of mitochondrial genome fusion transcripts and translation products, as well as probes that hybridize thereto.

### BACKGROUND OF THE INVENTION

### Mitochondrial Genome

The mitochondrial genome is a compact yet critical sequence of nucleic acids. Mitochondrial DNA, or "mtDNA", comprises a small genome of 16,569 nucleic acid base pairs (bp) (Anderson et al., 1981; Andrews et al., 1999) in contrast to the immense nuclear genome of 3.3 billion bp (haploid). Its genetic complement is substantially smaller than that of its nuclear cell mate (0.0005%). However, individual cells carry anywhere from 10³ to 10⁴ mitochondria depending on specific cellular functions (Singh and Modica-Napolitano 2002). Communication or chemical signalling routinely occurs between the nuclear and mitochondrial genomes (Sherratt et al., 1997). Moreover, specific nuclear components are responsible for the maintenance and integrity of mitochondrial sequences (Croteau et al., 1999). All mtDNA genomes in a given individual are identical due to the clonal expansion of mitochondria within the ovum, once fertilization has occurred. However mutagenic events can induce sequence diversity reflected as somatic mutations. These mutations may accumulate in different tissues throughout the body in a condition known as heteroplasmy.

### Mitochondrial Proteome

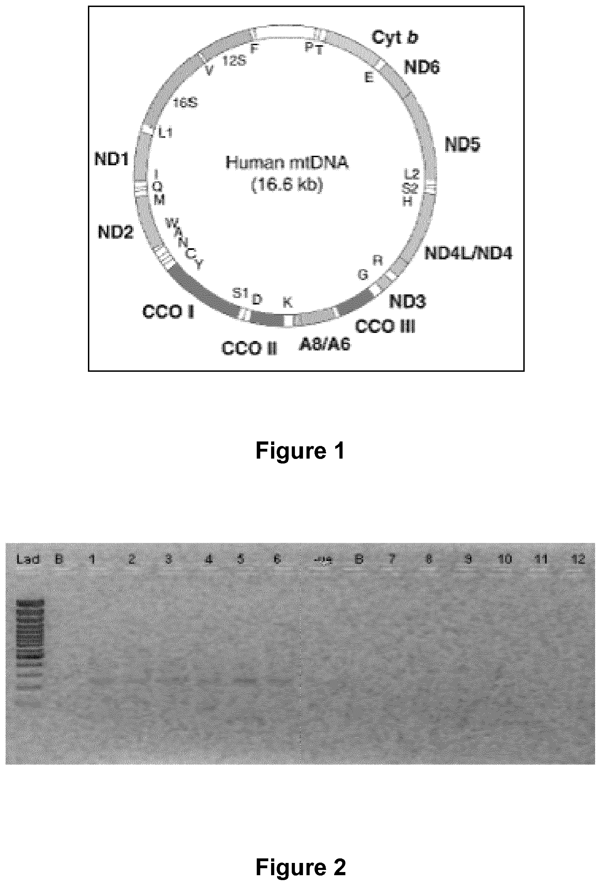

About 3,000 nuclear genes are required to construct, operate and maintain mitochondria, with only thirty-seven of these coded by the mitochondrial genome, indicating heavy mitochondrial dependence on nuclear loci. The mitochondrial genome codes for a complement of 24 genes, including 2 rRNAs and 22 tRNAs that ensure correct translation of the remaining 13 genes which are vital to electron transport (see Figure 1). The mitochondrial genome is dependent on seventy nuclear encoded proteins to accomplish the oxidation and reduction reactions necessary for this vital function, in addition to the thirteen polypeptides supplied by the mitochondrial genome. Both nuclear and mitochondrial proteins form complexes spanning the inner mitochondrial membrane and collectively generate 80-90% of the chemical fuel adenosine triphosphate, or ATP, required for cellular metabolism. In addition to energy production, mitochondria play a central role in other metabolic pathways as well. A critical function of the mitochondria is mediation of cell death, or apoptosis (see Green and Kroemer, 2005). Essentially, there are signal pathways which permeabilize the outer mitochondrial membrane, or in addition, the inner mitochondrial membrane as well. When particular mitochondrial proteins are released into the cytosol, non-reversible cell death is set in motion. This process highlights the multi-functional role that some mitochondrial proteins have. These multi-tasking proteins suggest that there are other mitochondrial proteins as well which may have alternate functions.

### Mitochondrial Fusion Transcriptome/Proteome

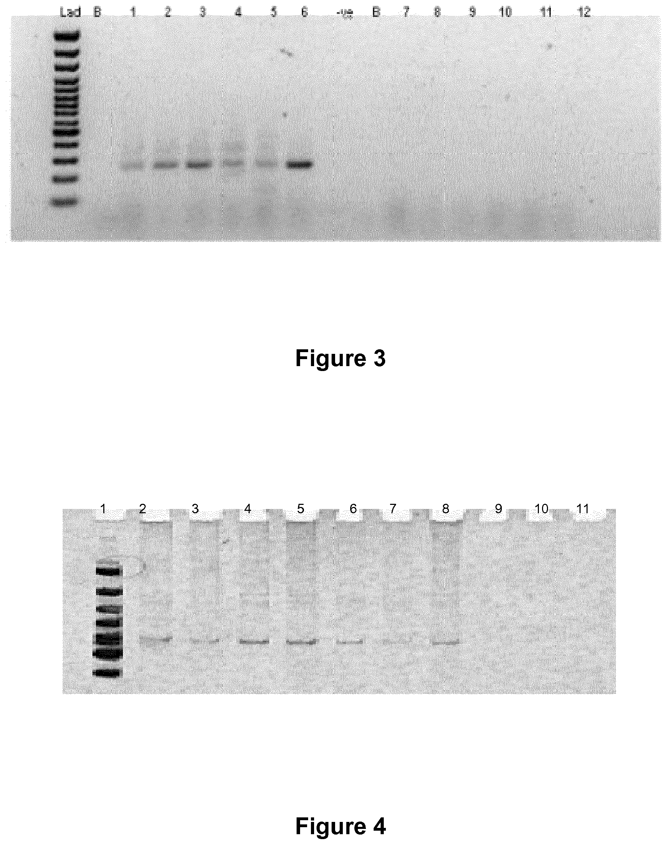

The mitochondrial genome is unusual in that it is a circular, intron-less DNA molecule. The genome is interspersed with repeat motifs which flank specific lengths of sequences. Sequences between these repeats are prone to deletion under circumstances which are not well understood. Given the number of repeats in the mitochondrial genome, there are many possible deletions. The best known example is the 4977 "common deletion." This deletion has been associated with several purported conditions and diseases and is thought to increase in frequency with aging (Dai et al., 2004; Ro et al., 2003; Barron et al., 2001; Lewis et al., 2000; Muller-Hocker, 1998; Porteous et al., 1998) (Figure 4). The current thinking in the field of mitochondrial genomics is that mitochondrial deletions are merely deleterious by-products of damage to the mitochondrial genome by such agents as reactive oxygen species and UVR. (Krishnan et al 2008, Nature Genetics). Further, though it is recognized that high levels of mtDNA deletions can have severe consequences on the cell's ability to produce energy in the form of ATP as a result of missing gene sequences necessary for cellular respiration, it is not anticipated that these deleted mitochondrial molecules may be a component of downstream pathways, have an intended functional role, and possibly may be more aptly viewed as alternate natural forms of the recognized genes of the mitochondria.

The sequence dynamics of mtDNA are important diagnostic tools. Mutations in mtDNA are often preliminary indicators of developing disease. For example, it has been demonstrated that point mutations in the mitochondrial genome are characteristic of tumour foci in the prostate. This trend also extends to normal appearing tissue both adjacent to and distant from tumour tissue (Parr et al. 2006). This suggests that mitochondrial mutations occur early in the malignant transformation pathway.

For example, the frequency of a 3.4kb mitochondrial deletion has excellent utility in discriminating between benign and malignant prostate tissues (Maki et al. 2008). Furthermore, an investigation of the disease associated deletions and the novel sequences, created through reclosure of the molecule identifies many open reading frames, suggesting the possibility of unique mitochondrial fusion proteins.

Mitochondrial fusion transcripts have been reported previously in the literature, first in soybeans (Morgens et al. 1984) and then later in two patients with Kearns-Sayre Syndrome, a rare neuromuscular disorder (Nakase et al 1990). Importantly, these transcripts were not found to have (or investigated regarding) association with any human cancers.

### Nuclear Fusion Proteome

There is important nuclear precedence for fusion proteins and their resulting effects on cancer. Nuclear *MLL* gene partner translocations are well established in correlation with high risk acute leukemia and therapy-related acute myeloid leukemias following treatment with agents that target topoisomerase II (Libura et al., 2005). Currently, around 50 translocations of the human *MLL* gene are known to be associated with these cancers (Meyer et al., 2005). Break points for these mutations, whether partial tandem duplications or translocations, for the majority of these events, occur within nuclear specific repetitive motifs such as *Alu* I. Most of these mutations are reciprocal translocations (84%) and include about 40 different genes (Libura et al. 2005).

There are known functional chimeric proteins created from some of these rearrangements which affect the course of malignant disease. For example, murine cells which express the protein from *MLL-ENL* accelerate the prevalence of chromosome abnormalities in cells which survive exposure to etoposide (Eguchi et al., 2006). Of particular interest is *MLL-SMAP1* and the reciprocal *SMAP1-MLL. SMAP1* binds calcium and as such participates in cell signalling and trafficking.

Mitochondrial fusion proteins may be assumed to have similar attributes to nuclear fusion proteins, especially since mitochondria and mitochondrial proteins play similar roles in signalling and apoptosis.

### SUMMARY OF THE INVENTION

An object of the present invention to provide aberrant mitochondrial DNA, associated fusion transcripts and translation products and hybridization probes therefor.

In accordance with an aspect of the invention, there is provided an isolated mitochondrial fusion transcript associated with cancer, wherein the transcript comprises a nucleic acid sequence as set forth in SEQ ID NO:20.

In accordance with another aspect of the invention, there is provided an isolated mtDNA encoding a fusion transcript of the invention.

In accordance with another aspect of the invention, there is provided a hybridization probe having a nucleic acid sequence complementary to at least a portion of a mitochondrial fusion transcript or an mtDNA of the invention.

In accordance with another aspect of the invention, there is provided a method of detecting a cancer in a mammal, the method comprising assaying a tissue sample from the mammal for the presence of at least one mitochondrial fusion transcript associated with cancer by hybridizing the sample with at least one hybridization probe having a nucleic acid sequence complementary to at least a portion of a mitochondrial fusion transcript according to the invention.

In accordance with another aspect of the invention, there is provided a method of detecting a cancer in a mammal, the method comprising assaying a tissue sample from the mammal for the presence of at least one aberrant mtDNA associated with cancer by hybridizing the sample with at least one hybridization probe having a nucleic acid sequence complementary to at least a portion of an mtDNA according to the invention.

In accordance with another aspect of the invention, there is provided a kit for conducting an assay for detecting the presence of a cancer in a mammal, said kit comprising at least one hybridization probe complementary to a fusion transcript or an mtDNA of the invention.

In accordance with another aspect of the invention, there is provided a mitochondrial fusion protein, the protein having an amino acid sequence resulting from the translation of a mitochondrial fusion transcript of the invention.

In accordance with another aspect of the invention, there is provided a method of detecting a cancer in a mammal, the method comprising assaying a tissue sample from the mammal for the presence of at least one mitochondrial fusion protein, the protein having an amino acid sequence resulting from the translation of a mitochondrial fusion transcript according to the invention.

### BRIEF DESCRIPTION OF THE DRAWINGS

The embodiments of the invention will now be described by way of example only with reference to the appended drawings wherein:
Figure 1 is an illustration showing mitochondrial protein coding genes.
Figure 2 shows polyadenalated fusion transcripts in prostate samples invoked by the loss of the 3.4kb deletion.
Figure 3 shows polyadenalated fusion transcripts in prostate samples invoked by the loss of the 4977kb common deletion.
Figure 4 shows polyadenalated fusion transcripts in breast samples invoked by the loss of the 3.4 kb segment from the mtgenome.
Figures 5a and 5b show an example of a mitochondrial DNA region before and after splicing of genes.
Figures 6a to 6g illustrate the results for transcripts 2, 3, 8, 9, 10, 11 and 12 of the invention in the identification of colorectal cancer tumours.
Figures 7a to 7d illustrate the results for transcripts 6, 8, 10 and 20 of the disclosure in the identification of lung cancer tumours.
Figures 8a to 8j illustrate the results for transcripts 6, 10, 11, 14, 15, 16 and 20 of the disclosure in the identification of melanomas.
Figures 9a to 9h illustrate the results for transcripts 1, 2, 3, 6, 11, 12, 15 and 20 of the invention in the identification of ovarian cancer.
Figures 10 to 18 illustrate the results for transcripts 2, 3, 4, 11, 12, 13, 15, 16 and 20 of the invention in the identification of testicular cancer.
Figure 19 illustrates the SDS PAGE gel of cytosolic and mitochondrial fractions of RWPE1 and WPE1-NA22 celll lines conducted during the fusion protein discovery phase.
Figure 20a illustrates the identified protein of fusion transcript P0026 based on the peptides ILYMTDEVNDPSLTIK and STPYECGFDPMSP.
Figure 20b illustrates the wild-type CO2 protein identified in mitochondrial NA22 cell line gel slice 5 of Figure 19 after searching the Human (SwissProt) database.
Figure 21a illustrates the identified protein of fusion transcript P0062 based on the peptides KGPNVVGPYGLLQPFADAMK, YDQLMHLLWK and LITTQQWLIK.
Figure 21b illustrates the identified peptides of ND1 identified in gel slice 5 of Figure 19 after searching the Human (SwissProt) database.
Figure 22 illustrates the identified protein of fusion transcript P0064 based on the peptides KGPNVVGPYGLLQPFADAMK and WAIIEEFTK.
Figure 23a illustrates the identified protein of fusion transcript P0176 based on the peptides KGPNVVGPYGLLQPFADAMK, VFSWLATLHGSNMK and VLMVEEPSMNLEWLYGCPPPYHTFEEPVYMK.
Figure 23b illustrates the wild-type CO1 protein identified in mitochondrial NA22 cell line gel slice 4 of Figure 19 after searching the Human (SwissProt) database.
Figures 24a to 24d illustrate the results of quantitative measurements of fusion transcripts P0026, P0062, P0064 and P0176, respectively.

### DETAILED DESCRIPTION OF THE INVENTION

The present invention provides novel mitochondrial fusion transcripts, the parent mutated mtDNA molecules, and the resulting translation products that are useful for predicting, diagnosing and/or monitoring cancer. The invention further provides hybridization probes for the detection of fusion transcripts and associated mtDNA molecules and the use of such probes.

### Definitions

Unless defined otherwise, all technical and scientific terms used herein have the same meaning as commonly understood by one of ordinary skill in the art to which this invention belongs.

The terms "comprise", "comprises", "comprised" or "comprising" may be used in the present description. As used herein (including the specification and/or the claims), these terms are to be interpreted as specifying the presence of the stated features, integers, steps or components, but not as precluding the presence of one or more other feature, integer, step, component or a group thereof as would be apparent to persons having ordinary skill in the relevant art.

As used herein, "aberration" or "mutation" encompasses any modification in the wild type mitochondrial DNA sequence that results in a fusion transcript and includes, without limitation, insertions, translocations, deletions, duplications, recombinations, rearrangements or combinations thereof.

As defined herein, "biological sample" refers to a tissue or bodily fluid containing cells from which a molecule of interest can be obtained. For example, the biological sample can be derived from tissue such as prostate, breast, colorectal, lung and skin, or from blood, saliva, cerebral spinal fluid, sputa, urine, mucous, synovial fluid, peritoneal fluid, amniotic fluid and the like. The biological sample may be a surgical specimen or a biopsy specimen. The biological sample can be used either directly as obtained from the source or following a pre-treatment to modify the character of the sample. Thus, the biological sample can be pre-treated prior to use by, for example, preparing plasma or serum from blood, disrupting cells, preparing liquids from solid materials, diluting viscous fluids, filtering liquids, distilling liquids, concentrating liquids, inactivating interfering components, adding reagents, and the like.

A "continuous" transcript is a fusion transcript that keeps the reading frame from the beginning to the end of both spliced genes. An "end" transcript is a fusion transcript that results in a premature termination codon before the original termination codon of a second spliced gene.

As used herein, "mitochondrial DNA" or "mtDNA" is DNA present in mitochondria.

As used herein, the expression "mitochondrial fusion protein" or "fusion protein" refers to a peptide product produced by the transcription and translation of a mutated mitochondrial DNA, wherein such mutations comprise deletions or other "large-scale" mitochondrial DNA rearrangements. In addition, or alternatively, an in-frame protein may be translated from alternate initiation and termination codons within that sequence.

As used herein, the expression "mitochondrial fusion transcript" or "fusion transcript" refers to an RNA transcription product produced as a result of the transcription of a mutated mitochondrial DNA sequence wherein such mutations may comprise mitochondrial deletions and other large-scale mitochondrial DNA rearrangements.

As used herein, the expression "mitochondrial translation product" or "translation product" refers to any amino acid chain derived from a mitochondrial fusion transcript including peptides, polypeptides and proteins. It will be understood that "mitochondrial translation products" comprise "fusion proteins", as defined above.

### Computer Analysis and Sequence Targetting

As discussed above, mitochondrial fusion transcripts have been reported in soybeans (Morgens et al. 1984) and in humans suffering from a rare neuromuscular disorder (Nakase et al 1990). Fusion transcripts associated with human cancer have not, however, been described.

Using the knowledge gained from mapping the large-scale deletions of the human mitochondrial genome associated with cancer, the observation of high frequencies of these deletions, and the evidence in another organism and another disease type of trancriptionally active mutated mtDNA molecules, the present inventors hypothesized that such deletions may have importance beyond the DNA molecule and the damage and repair processes as it relates to cancer. To test this hypothesis computer analysis of the mitochondrial genome was conducted, specific for repeat elements, which suggested many potential deletion sites. Following this initial step of identifying unique repeats in the mitochondrial sequence having non-adjacent or non-tandem locations, a filter was then applied to identify those repeats that upon initiating a deletion event in the DNA molecule would then likely reclose or religate to produce a fused DNA sequence having an open reading frame (ORF) and thus capable of being transcribed by the mitochondrial transcription machinery. A subset of 18 of these molecules were then selected for targetting to investigate whether: they existed in the natural biological state of humans; they were polyadenylated and thus expected to proceed to protein synthesis; they had relevance to malignancy. Results from these investigations proved positive for all three queries and are described hereinafter.

### Genomic Mutations

Mitochondrial DNA (mtDNA) dynamics are an important diagnostic tool. Mutations in mtDNA are often preliminary indicators of developing disease and may act as biomarkers indicative of risk factors associated with disease onset. According to the present invention, mutations in the mitochondrial genome result in the generation of fusion transcripts associated with cancer. Thus, the use of mtDNA encoding such transcripts and probes directed thereto for the detection, diagnosis and monitoring of cancer is provided.

One of skill in the art will appreciate that the mtDNA molecules for use in the methods of the present invention may be derived through the isolation of naturally-occurring mutants or may be based on the complementary sequence of any of the fusion transcripts described herein. Exemplary mtDNA sequences and fusion transcripts are disclosed in Applicant's co-pending U.S. Application No. 61/040,616 and published PCT application no. PCT/CA2009/000351 (published as WO 2009/117811).

### Detection of Mutant Genomic Sequences

Mutant mtDNA sequences according to the present invention may comprise any modification that results in the generation of a fusion transcript. Non-limiting examples of such modifications include insertions, translocations, deletions, duplications, recombinations, rearrangements or combinations thereof. While the modification or change can vary greatly in size from only a few bases to several kilobases, preferably the modification results in a substantive deletion or other large-scale genomic aberration.

Extraction of DNA to detect the presence of such mutations may take place using art-recognized methods, followed by amplification of all or a region of the mitochondrial genome, and may include sequencing of the mitochondrial genome, as described in Current Protocols in Molecular Biology.

The step of detecting the mutations can be selected from any technique known in the art. For example, analyzing mtDNA can comprise sequencing the mtDNA, amplifying mtDNA by PCR, Southern, Northern, Western South-Western blot hybridizations, denaturing HPLC, hybridization to microarrays, biochips or gene chips, molecular marker analysis, biosensors, melting temperature profiling or a combination of any of the above.

Any suitable means to sequence mitochondrial DNA may be used. Preferably, mtDNA is amplified by PCR prior to sequencing. The method of PCR is well known in the art and may be performed as described in Mullis and Faloona, 1987, Methods Enzymol., 155: 335. PCR products can be sequenced directly or cloned into a vector which is then placed into a bacterial host. Examples of DNA sequencing methods are found in Brumley, R. L. Jr. and Smith, L.M., 1991, Rapid DNA sequencing by horizontal ultrathin gel electrophoresis, Nucleic Acids Res. 19:4121-4126 and Luckey, J.A., et al, 1993, High speed DNA sequencing by capillary gel electrophoresis, Methods Enzymol. 218: 154-172. The combined use of PCR and sequencing of mtDNA is described in Hopgood, R., et al, 1992, Strategies for automated sequencing of human mtDNA directly from PCR products, Biotechniques 13:82-92 and Tanaka, M. et al, 1996, Automated sequencing of mtDNA, Methods Enzymol. 264: 407-421.

Methods of selecting appropriate sequences for preparing various primers are also known in the art. For example, the primer can be prepared using conventional solid-phase synthesis using commercially available equipment, such as that available from Applied Biosystems USA Inc. (Foster City, California), DuPont, (Wilmington, Del.), or Milligen (Bedford, Mass.).

According to an aspect of the invention, to determine candidate genomic sequences, a junction point of a sequence deletion is first identified. Sequence deletions are primarily identified by direct and indirect repetitive elements which flank the sequence to be deleted at the 5' and 3' end. The removal of a section of the nucleotides from the genome followed by the ligation of the genome results in the creation of a novel junction point.

Upon identification of the junction point, the nucleotides of the genes flanking the junction point are determined in order to identify a spliced gene. Typically the spliced gene comprises the initiation codon from the first gene and the termination codon of the second gene, and may be expressed as a continuous transcript, i.e. one that keeps the reading frame from the beginning to the end of both spliced genes. Some known mitochondrial deletions discovered to have an open reading frame (ORF) when the rearranged sequences are rejoined at the splice site are provided in Table 1.

Exemplary mtDNA molecules for use in the methods of the present invention are provided below. These mtDNAs are based on modifications of the known mitochondrial genome (SEQ ID NO: 1) and have been assigned a fusion or "FUS" designation, wherein A:B represents the junction point between the last mitochondrial nucleotide of the first spliced gene and the first mitochondrial nucleotide of the second spliced gene. The identification of the spliced genes is provided in parentheses followed by the corresponding sequence identifier. Where provided below, (AltMet) and (OrigMet) refer to alternate and original translation start sites, respectively.
FUS 8469:13447 (AltMet) (ATP synthase F0 subunit 8 to NADH dehydrogenase subunit) (SEQ ID No: 2)
FUS 10744:14124 (NADH dehydrogenase subunit 4L (ND4L) to NADH dehydrogenase subunit 5 (ND5)) (SEQ ID No: 3)
FUS 7974:15496 (Cytochrome c oxidase subunit II (COII) to Cytochrome b (Cytb)) (SEQ ID No: 4)
FUS 7992:15730 (Cytochrome c oxidase subunit II (COII) to Cytochrome b (Cytb)) (SEQ ID No: 5)
FUS 8210:15339 (Cytochrome c oxidase subunit II (COII) to Cytochrome b (Cytb)) (SEQ ID No: 6)
FUS 8828:14896 (ATP synthase F0 subunit 6 (ATPase6) to Cytochrome b (Cytb)) (SEQ ID No: 7)
FUS 10665:14856 (NADH dehydrogenase subunit 4L (ND4L) to Cytochrome b (Cytb)) (SEQ ID No: 8)
FUS 6075:13799 (Cytochrome c oxidase subunit I (COI) to NADH de hydrogenase subunit 5 (ND5)) (SEQ ID No: 9)
FUS 6325:13989 (Cytochrome c oxidase subunit I (COI) to NADH dehydrogenase subunit 5 (ND5)) (SEQ ID No: 10)
FUS 7438:13476 (Cytochrome c oxidase subunit I (COI) to NADH dehydrogenase subunit 5 (ND5)) (SEQ ID No: 11)
FUS 7775:13532 (Cytochrome c oxidase subunit II (COII) to NADH dehydrogenase subunit 5 (ND5)) (SEQ ID No: 12)
FUS 8213:13991 (Cytochrome c oxidase subunit II (COII) to NADH dehydrogenase subunit 5 (ND5)) (SEQ ID No: 13)
FUS 9191:12909 (ATP synthase F0 subunit 6 (ATPase6) to NADH dehydrogenase subunit 5 (ND5)) (SEQ ID No: 14)
FUS 9574:12972 (Cytochrome c oxidase subunit III (COIII) to NADH dehydrogenase subunit 5 (ND5)) (SEQ ID No: 15)
FUS 10367:12829 (NADH dehydrogenase subunit 3 (ND3) to NADH dehydrogenase subunit 5 (ND5)) (SEQ ID No: 16)
FUS 11232:13980 (NADH dehydrogenase subunit 4 (ND4) to NADH dehydrogenase subunit 5 (ND5) (SEQ ID No: 17)
FUS 8469:13447 (OrigMet) (ATP synthase F0 subunit 8 to NADH dehydrogenase subunit) (SEQ ID No: 18)
FUS 9144:13816 ((ATP synthase F0 subunit 6 (ATPase6) to NADH dehydrogenase subunit 5 (ND5)) (SEQ ID No: 54)

The present invention also provides the use of variants or fragments of these sequences for predicting, diagnosing and/or monitoring cancer.

"Variant", as used herein, refers to a nucleic acid differing from an mtDNA sequence of the present invention, but retaining essential properties thereof. Generally, variants are overall closely similar, and, in many regions, identical to a select mtDNA sequence. Specifically, the variants of the present invention comprise at least one of the nucleotides of the junction point of the spliced genes, and may further comprise one or more nucleotides adjacent thereto. In one embodiment of the invention, the variant sequence is at least 80%, 85%, 90%, 95%, 96%, 97%, 98% or 99% identical to any one of the mtDNA sequences of the invention, or the complementary strand thereto.

In the present invention, "fragment" refers to a short nucleic acid sequence which is a portion of that contained in the disclosed genomic sequences, or the complementary strand thereto. This portion includes at least one of the nucleotides comprising the junction point of the spliced genes, and may further comprise one or more nucleotides adjacent thereto. The fragments of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A fragment "at least 20 nt in length," for example, is intended to include 20 or more contiguous bases of any one of the mtDNA sequences listed above. In this context "about" includes the particularly recited value, a value larger or smaller by several (5, 4, 3, 2, or 1) nucleotides, at either terminus or at both termini. These fragments have uses that include, but are not limited to, as diagnostic probes and primers as discussed herein. Of course, larger fragments (e.g., 50, 150, 500, 600, 2000 nucleotides) are also contemplated.

Thus, in specific embodiments of the invention, the mtDNA sequences are selected from the group consisting of:
SEQ ID NO: 2 (FUS 8469:13447; AltMet)
SEQ ID NO: 3 (FUS 10744:14124)
SEQ ID NO: 4 (FUS 7974:15496)
SEQ ID NO: 5 (FUS 7992:15730)
SEQ ID NO: 6 (FUS 8210:15339)
SEQ ID NO: 7 (FUS 8828:14896)
SEQ ID NO: 8 (FUS 10665:14856)
SEQ ID NO: 9 (FUS 6075:13799)
SEQ ID NO: 10 (FUS 6325:13989)
SEQ ID NO: 11 (FUS 7438:13476)
SEQ ID NO: 12 (FUS 7775:13532)
SEQ ID NO: 13 (FUS 8213:13991)
SEQ ID NO: 14 (FUS 9191:12909)
SEQ ID NO: 15 (FUS 9574:12972)
SEQ ID NO: 16 (FUS 10367:12829)
SEQ ID NO: 17 (FUS 11232:13980)
SEQ ID NO: 18 (FUS 8469:13447; OrigMet)
SEQ ID NO: 54 (FUS 9144:13816),
- and fragments or variants thereof.

### Probes

Another aspect of the invention is to provide a hybridization probe capable of recognizing an aberrant mtDNA sequence of the invention. As used herein, the term "probe" refers to an oligonucleotide which forms a duplex structure with a sequence in the target nucleic acid, due to complementarity of at least one sequence in the probe with a sequence in the target region. The probe may be labeled, according to methods known in the art.

Once aberrant mtDNA associated with particular disease is identified, hybridization of mtDNA to, for example, an array of oligonucleotides can be used to identify particular mutations, however, any known method of hybridization may be used.

As with the primers of the present invention, probes may be generated directly against exemplary mtDNA fusion molecules of the invention, or to a fragment or variant thereof. For instance, the sequences set forth in SEQ ID NOs: 2-18 and 54 and those disclosed in Table 1 can be used to design primers or probes that will detect a nucleic acid sequence comprising a fusion sequence of interest. As would be understood by those of skill in the art, primers or probes which hybridize to these nucleic acid molecules may do so under highly stringent hybridization conditions or lower stringency conditions, such conditions known to those skilled in the art and found, for example, in Current Protocols in Molecular Biology (John Wiley & Sons, New York (1989)), 6.3.1-6.3.6.

In specific embodiments of the invention, the probes of the invention contain a sequence complementary to at least a portion of the aberrant mtDNA comprising the junction point of the spliced genes. This portion includes at least one of the nucleotides involved in the junction point A:B, and may further comprise one or more nucleotides adjacent thereto. In this regard, the present invention encompasses any suitable targeting mechanism that will select an mtDNA molecule using the nucleotides involved and/or adjacent to the junction point A:B.

Various types of probes known in the art are contemplated by the present invention. For example, the probe may be a hybridization probe, the binding of which to a target nucleotide sequence can be detected using a general DNA binding dye such as ethidium bromide, SYBR® Green, SYBR® Gold and the like. Alternatively, the probe can incorporate one or more detectable labels. Detectable labels are molecules or moieties a property or characteristic of which can be detected directly or indirectly and are chosen such that the ability of the probe to hybridize with its target sequence is not affected. Methods of labelling nucleic acid sequences are well-known in the art (see, for example, Ausubel et al., (1997 & updates) Current Protocols in Molecular Biology, Wiley & Sons, New York).

Labels suitable for use with the probes of the present invention include those that can be directly detected, such as radioisotopes, fluorophores, chemiluminophores, enzymes, colloidal particles, fluorescent microparticles, and the like. One skilled in the art will understand that directly detectable labels may require additional components, such as substrates, triggering reagents, light, and the like to enable detection of the label. The present invention also contemplates the use of labels that are detected indirectly.

The probes of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A probe of "at least 20 nt in length," for example, is intended to include 20 or more contiguous bases that are complementary to an mtDNA sequence of the invention. Of course, larger probes (e.g., 50, 150, 500, 600, 2000 nucleotides) may be preferable.

The probes of the invention will also hybridize to nucleic acid molecules in biological samples, thereby enabling the methods of the invention. Accordingly, in one aspect of the invention, there is provided a hybridization probe for use in the detection of cancer, wherein the probe is complementary to at least a portion of an aberrant mtDNA molecule. In another aspect the present invention provides probes and a use of (or a method of using) such probes for the detection of colorectal cancer, lung cancer, breast cancer, ovarian cancer, testicular, cancer, prostate cancer and/or melanoma skin cancer.

### Assays

Measuring the level of aberrant mtDNA in a biological sample can determine the presence of one or more cancers in a subject. The present invention, therefore, encompasses methods for predicting, diagnosing or monitoring cancer, comprising obtaining one or more biological samples, extracting mtDNA from the samples, and assaying the samples for aberrant mtDNA by: quantifying the amount of one or more aberrant mtDNA sequences in the sample and comparing the quantity detected with a reference value. As would be understood by those of skill in the art, the reference value is based on whether the method seeks to predict, diagnose or monitor cancer. Accordingly, the reference value may relate to mtDNA data collected from one or more known non-cancerous biological samples, from one or more known cancerous biological samples, and/or from one or more biological samples taken over time.

In one aspect, the invention provides a method of detecting cancer in a mammal, the method comprising assaying a tissue sample from the mammal for the presence of an aberrant mitochondrial DNA described above. The present invention also provides for methods comprising assaying a tissue sample from the mammal by hybridizing the sample with at least one hybridization probe. The probe may be generated against a mutant mitochondrial DNA sequence of the invention as described herein.

In another aspect, the invention provides a method as above, wherein the assay comprises:
a) conducting a hybridization reaction using at least one of the probes to allow the at least one probe to hybridize to a complementary aberrant mitochondrial DNA sequence;
b) quantifying the amount of the at least one aberrant mitochondrial DNA sequence in the sample by quantifying the amount of the mitochondrial DNA hybridized to the at least one probe; and,
c) comparing the amount of the mitochondrial DNA in the sample to at least one known reference value.

Also included in the present invention are methods for predicting, diagnosing or monitoring cancer comprising diagnostic imaging assays as described below. The diagnostic assays of the invention can be readily adapted for high-throughput. High-throughput assays provide the advantage of processing many samples simultaneously and significantly decrease the time required to screen a large number of samples. The present invention, therefore, contemplates the use of the nucleotides of the present invention in high-throughput screening or assays to detect and/or quantitate target nucleotide sequences in a plurality of test samples.

### Fusion Transcripts

The present invention further provides the identification of fusion transcripts and associated hybridization probes useful in methods for predicting, diagnosing and/or monitoring cancer. One of skill in the art will appreciate that such molecules may be derived through the isolation of naturally-occurring transcripts or, alternatively, by the recombinant expression of mtDNAs isolated according to the methods of the invention. As discussed, such mtDNAs typically comprise a spliced gene having the initiation codon from the first gene and the termination codon of the second gene. Accordingly, fusion transcripts derived therefrom comprise a junction point associated with the spliced genes.

### Detection of Fusion Transcripts

Naturally occurring fusion transcripts can be extracted from a biological sample and identified according to any suitable method known in the art, or may be conducted according to the methods described in the examples. In one embodiment of the invention, stable polyadenylated fusion transcripts are identified using Oligo(dT) primers that target transcripts with poly-A tails, followed by RT-PCR using primer pairs designed against the target transcript.

The following exemplary fusion transcripts were detected using such methods and found useful in predicting, diagnosing and/or monitoring cancer as indicated in the examples. Likewise, fusion transcripts derived from the ORF sequences identified in Table 1 may be useful in predicting, diagnosing and/or monitoring cancer.
SEQ ID NO: 19 (Transcript 1; 8469:13447; AltMet)
SEQ ID NO: 20 (Transcript 2;10744:14124)
SEQ ID NO: 21 (Transcript 3;7974:15496)
SEQ ID NO: 22 (Transcript 4;7992:15730)
SEQ ID NO: 23 (Transcript 5;8210:15339)
SEQ ID NO: 24 (Transcript 6;8828:14896)
SEQ ID NO: 25 (Transcript 7;10665:14856)
SEQ ID NO: 26 (Transcript 8;6075:13799)
SEQ ID NO: 27 (Transcript 9;6325:13989)
SEQ ID NO: 28 (Transcript 10;7438:13476)
SEQ ID NO: 29 (Transcript 11;7775:13532)
SEQ ID NO: 30 (Transcript 12;8213:13991)
SEQ ID NO: 31 (Transcript 14;9191:12909)
SEQ ID NO: 32 (Transcript 15;9574:12972)
SEQ ID NO: 33 (Transcript 16;10367:12829)
SEQ ID NO: 34 (Transcript 17;11232:13980)
SEQ ID NO: 35 (Transcript 20;8469:13447; OrigMet)
SEQ ID NO: 53 (Transcript 13; 9144:13816)

Fusion transcripts can also be produced by recombinant techniques known in the art. Typically this involves transformation (including transfection, transduction, or infection) of a suitable host cell with an expression vector comprising an mtDNA sequence of interest.

Variants or fragments of the fusion transcripts identified herein are also provided. Such sequences may adhere to the size limitations and percent identities described above with respect to genomic variants and fragments, or as determined suitable by a skilled technician.

### Probes

Once a fusion transcript has been characterized, primers or probes can be developed to target the transcript in a biological sample. Such primers and probes may be prepared using any known method (as described above) or as set out in the examples provided below. A probe may, for example, be generated for the fusion transcript, and detection technologies, such as QuantiGene 2.0™ by Panomics™, used to detect the presence of the transcript in a sample. Primers and probes may be generated directly against exemplary fusion transcripts of the invention, or to a fragment or variant thereof. For instance, the sequences set forth in SEQ ID NOs: 19-35 and 53, as well as those disclosed in Table 1, can be used to design probes that will detect a nucleic acid sequence comprising a fusion sequence of interest.

As would be understood by those skilled in the art, probes designed to hybridize to the fusion transcripts of the invention contain a sequence complementary to at least a portion of the transcript expressing the junction point of the spliced genes. This portion includes at least one of the nucleotides complementary to the expressed junction point, and may further comprise one or more complementary nucleotides adjacent thereto. In this regard, the present invention encompasses any suitable targeting mechanism that will select a fusion transcript that uses the nucleotides involved and adjacent to the junction point of the spliced genes.

Various types of probes and methods of labelling known in the art are contemplated for the preparation of transcript probes. Such types and methods have been described above with respect to the detection of genomic sequences. The transcript probes of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A probe of "at least 20 nt in length," for example, is intended to include 20 or more contiguous bases that are complementary to an mtDNA sequence of the invention. Of course, larger probes (e.g., 50, 150, 500, 600, 2000 nucleotides) may be preferable.

In one aspect, the invention provides a hybridization probe for use in the detection of cancer, wherein the probe is complementary to at least a portion of a mitochondrial fusion transcript provided above.

In another aspect, the present invention provides probes and a use of (or a method of using) such probes for the detection of colorectal cancer, lung cancer, breast cancer, ovarian cancer, testicular cancer, prostate cancer or melanoma skin cancer.

### Assays

Measuring the level of mitochondrial fusion transcripts in a biological sample can determine the presence of one or more cancers in a subject. The present invention, therefore, provides methods for predicting, diagnosing or monitoring cancer, comprising obtaining one or more biological samples, extracting mitochondrial RNA from the samples, and assaying the samples for fusion transcripts by: quantifying the amount of one or more fusion transcripts in the sample and comparing the quantity detected with a reference value. As would be understood by those of skill in the art, the reference value is based on whether the method seeks to predict, diagnose or monitor cancer. Accordingly, the reference value may relate to transcript data collected from one or more known non-cancerous biological samples, from one or more known cancerous biological samples, and/or from one or more biological samples taken over time.

In one aspect, the invention provides a method of detecting a cancer in a mammal, the method comprising assaying a tissue sample from said mammal for the presence of at least one fusion transcript of the invention by hybridizing said sample with at least one hybridization probe having a nucleic acid sequence complementary to at least a portion of the mitochondrial fusion transcript.

In another aspect, the invention provides a method as above, wherein the assay comprises:
a) conducting a hybridization reaction using at least one of the above-noted probes to allow the at least one probe to hybridize to a complementary mitochondrial fusion transcript;
b) quantifying the amount of the at least one mitochondrial fusion transcript in the sample by quantifying the amount of the transcript hybridized to the at least one probe; and,
c) comparing the amount of the mitochondrial fusion transcript in the sample to at least one known reference value.

As discussed above, the diagnostic assays of the invention may also comprise diagnostic imaging methods as described herein and can be readily adapted for high-throughput. The present invention, therefore, contemplates the use of the fusion transcripts and associated probes of the present invention in high-throughput screening or assays to detect and/or quantitate target nucleotide sequences in a plurality of test samples.

### Translation Products

To date, mitochondrial fusion proteins have not been detected or isolated. However, the levels of mitochondrial fusion transcripts observed from the examples provided below and the indications that they are polyadenylated provide further evidence supporting the existence of such mitochondrial fusion proteins. Accordingly, the present invention provides the identification of fusion proteins for predicting, diagnosing, and/or monitoring of cancer.

Fusion proteins contemplated for use in the disclosed methods may be derived through the isolation of naturally-occurring polypeptides or through gene expression. Such polypeptides can be prepared by methods known in the art, such as purification from cell extracts or the use of recombinant techniques.

Putative protein sequences corresponding to transcripts 1-17 and 20 are provided below along with their respective sequence identifier. The putative protein sequence corresponding to transcript 2 is herein contemplated for use in the methods of the present invention.
SEQ ID NO: 36 (Transcript 1)
SEQ ID NO: 37 (Transcript 2)
SEQ ID NO: 38 (Transcript 3)
SEQ ID NO: 39 (Transcript 4)
SEQ ID NO: 40 (Transcript 5)
SEQ ID NO: 41 (Transcript 6)
SEQ ID NO: 42 (Transcript 7)
SEQ ID NO: 43 (Transcript 8)
SEQ ID NO: 44 (Transcript 9)
SEQ ID NO: 45 (Transcript 10)
SEQ ID NO: 46 (Transcript 11)
SEQ ID NO: 47 (Transcript 12)
SEQ ID NO: 48 (Transcript 14)
SEQ ID NO: 49 (Transcript 15)
SEQ ID NO: 50 (Transcript 16)
SEQ ID NO: 51 (Transcript 17)
SEQ ID NO: 52 (Transcript 20)
SEQ ID NO: 55 (Transcript 13)

### Detection of Fusion Proteins

Fusion proteins of the invention can be recovered and purified from a biological sample by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, hydrophobic charge interaction chromatography and lectin chromatography. Most preferably, high performance liquid chromatography ("HPLC") is employed for purification.

Assaying fusion protein levels in a biological sample can occur using a variety of techniques. For example, protein expression in tissues can be studied with classical immunohistological methods (Jalkanen et al., J. Cell. Biol. 101:976-985 (1985); Jalkanen, M., et al., J. Cell. Biol. 105:3087-3096 (1987)). Other methods useful for detecting protein expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (<125> I, <121> I), carbon (<14> C), sulfur (<35> S), tritium (<3> H), indium (<112> In), and technetium (<99m> Tc), and fluorescent labels, such as fluorescein and rhodamine, and biotin.

The polypeptides of the invention can also be produced by recombinant techniques known in the art. Typically this involves transformation (including transfection, transduction, or infection) of a suitable host cell with an expression vector comprising a polynucleotide encoding the protein or polypeptide of interest.

### Antibodies

Protein specific antibodies for use in the assays of the present invention can be raised against the wild-type or expressed mitochondrial fusion proteins of the invention or an antigenic polypeptide fragment thereof, which may be presented together with a carrier protein, such as an albumin, to an animal system (such as rabbit or mouse) or, if it is long enough (at least about 25 amino acids), without a carrier.

As used herein, the term "antibody" (Ab) or "monoclonal antibody" (Mab) is meant to include intact molecules as well as antibody fragments, or antigen-binding fragments, thereof (such as, for example, Fab and F(ab')2 fragments) which are capable of specifically binding to, or having "specificity to", a mitochondrial fusion protein. Fab and F(ab')2 fragments lack the Fc fragment of intact antibody, clear more rapidly from the circulation, and may have less non-specific tissue binding of an intact antibody (Wahl et al., J. Nucl. Med. 24:316-325 (1983)). Thus, these fragments are preferred.

The antibodies of the present disclosure may be prepared by any of a variety of methods. For example, cells expressing the mitochondrial fusion protein or an antigenic fragment thereof can be administered to an animal in order to induce the production of sera containing polyclonal antibodies. In one method, a preparation of mitochondrial fusion protein is prepared and purified to render it substantially free of natural contaminants. Such a preparation is then introduced into an animal in order to produce polyclonal antisera of greater specific activity.

In a related method, the antibodies of the present disclosure are monoclonal antibodies. Such monoclonal antibodies can be prepared using hybridoma technology (Kohler et al., Nature 256:495 (1975); Kohler et al., Eur. J. Immunol. 6:511 (1976); Kohler et al., Eur. J. Immunol. 6:292 (1976); Hammerling et al., in: Monoclonal Antibodies and T-Cell Hybridomas, Elsevier, N.Y., (1981) pp. 563-681). In general, such procedures involve immunizing an animal (preferably a mouse) with a mitochondrial fusion protein antigen or with a mitochondrial fusion protein-expressing cell.

The present disclosure comprises immunological assays using antibodies or antigen-binding fragments having specificity to the fusion proteins described herein. Such immunological assays may be facilitated by kits containing the antibodies or antigen-binding fragments along with any other necessary reagents, test strips, materials, instructions etc.

### Assays

Measuring the level of a translation product such as a fusion protein in a biological sample can determine the presence of one or more cancers in a subject. The present invention, therefore, provides methods for predicting, diagnosing or monitoring cancer, comprising obtaining one or more biological samples, extracting mitochondrial fusion proteins from the samples, and assaying the samples for such molecules by: quantifying the amount of one or more molecules in the sample and comparing the quantity detected with a reference value. As would be understood by those of skill in the art, the reference value is based on whether the method seeks to predict, diagnose or monitor cancer. Accordingly, the reference value may relate to protein data collected from one or more known non-cancerous biological samples, from one or more known cancerous biological samples, and/or from one or more biological samples taken over time.

Techniques for quantifying proteins in a sample are well known in the art and include, for instance, classical immunohistological methods (Jalkanen et al., J. Cell. Biol. 101:976-985 (1985 ); Jalkanen, M., et al., J. Cell. Biol. 105:3087-3096 (1987)). Additional methods useful for detecting protein expression include immunoassays such as the radioimmunoassay (RIA) and the enzyme linked immunosorbent assay (ELISA).

In one aspect, the invention provides a method of detecting a cancer in a mammal, the method comprising assaying a tissue sample from the mammal for the presence of at least one mitochondrial fusion protein. In another aspect, the present invention provides for the detection of mitochondrial fusion proteins in the diagnosis of colorectal cancer, lung cancer, breast cancer, ovarian cancer, testicular cancer, prostate cancer and/or melanoma skin cancer.

### Diagnostic Imaging

### Diagnostic Devices

The disclosure includes diagnostic devices such as biochips, gene chips or microarrays used to diagnose specific diseases or identify specific mutations. All sequenced mitochondrial genomes are assessed to create a consenus structure of the base pair arrangement and are assigned a prohibiting index for proportion of base pair deletions and mutations associated with a particular disease or disorder. The diagnostic arrangement is then used to create biochips, gene chips, or microarrays.

Once sequences associated with particular diseases, disease states or disorders are identified, hybridization of a mitochondrial nucleotide sample to an array of oligonucleotides can be used to identify particular mutations. Any known method of hybridization may be used. Preferably, an array is used, which has oligonucleotide probes matching the wild type or mutated region, and a control probe. Commercially available arrays such as microarrays or gene chips are suitable. These arrays contain thousands of matched and control pairs of probes on a slide or microchip, and are capable of sequencing the entire genome very quickly. Review articles describing the use of microarrays in genome and DNA sequence analysis are available on-line.

### Microarray

Polynucleotide arrays provide a high throughput technique that can assay a large number of polynucleotides in a sample comprising one or more target nucleic acid sequences. The arrays of the disclosure are useful for gene expression analysis, diagnosis of disease and prognosis of disease (e.g., monitoring a patient's response to therapy, and the like).

Any combination of the polynucleotide sequences of mtDNA indicative of disease, or disease progression are used for the construction of a microarray.

The target nucleic acid samples to be analyzed using a microarray are derived from any human tissue or fluid which contains adequate amounts of mtDNA, as previously described. The target nucleic acid samples are contacted with polynucleotide members under hybridization conditions sufficient to produce a hybridization pattern of complementary nucleic acid members/target complexes.

### Construction of a Microarray

The microarray comprises a plurality of unique polynucleotides attached to one surface of a solid support, wherein each of the polynucleotides is attached to the surface of the solid support in a non-identical preselected region. Each associated sample on the array comprises a polynucleotide composition, of known identity, usually of known sequence, as described in greater detail below. Any conceivable substrate may be employed in the disclosure.

The array is constructed using any known means. The nucleic acid members may be produced using established techniques such as polymerase chain reaction (PCR) and reverse transcription (RT). These methods are similar to those currently known in the art (see e.g. PCR Strategies, Michael A. Innis (Editor), et al. (1995) and PCR: Introduction to Biotechniques Series, C. R. Newton, A. Graham (1997)). Amplified polynucleotides are purified by methods well known in the art (e.g., column purification). A polynucleotide is considered pure when it has been isolated so as to be substantially free of primers and incomplete products produced during the synthesis of the desired polynucleotide. Preferably, a purified polynucleotide will also be substantially free of contaminants which may hinder or otherwise mask the binding activity of the molecule.

In the arrays of the disclosure, the polynucleotide compositions are stably associated with the surface of a solid support, wherein the support may be a flexible or rigid solid support.

Any solid support to which a nucleic acid member may be attached may be used in the disclosure. Examples of suitable solid support materials include, but are not limited to, silicates such as glass and silica gel, cellulose and nitrocellulose papers, nylon, polystyrene, polymethacrylate, latex, rubber, and fluorocarbon resins such as TEFLON™.

The solid support material may be used in a wide variety of shapes including, but not limited to slides and beads. Slides provide several functional advantages and thus are a preferred form of solid support. Due to their flat surface, probe and hybridization reagents are minimized using glass slides. Slides also enable the targeted application of reagents, are easy to keep at a constant temperature, are easy to wash and facilitate the direct visualization of RNA and/or DNA immobilized on the solid support. Removal of RNA and/or DNA immobilized on the solid support is also facilitated using slides.

The particular material selected as the solid support is not essential to the disclosure, as long as it provides the described function. Normally, those who make or use the disclosure will select the best commercially available material based upon the economics of cost and availability, the expected application requirements of the final product, and the demands of the overall manufacturing process.

Numerous methods are used for attachment of the nucleic acid members of the invention to the substrate (a process referred as spotting). For example, polynucleotides are attached using the techniques of, for example U.S. Pat. No. 5,807,522 for teaching methods of polymer attachment. Alternatively, spotting is carried out using contact printing technology.

The amount of polynucleotide present in each composition will be sufficient to provide for adequate hybridization and detection of target polynucleotide sequences during the assay in which the array is employed. Generally, the amount of each nucleic acid member stably associated with the solid support of the array is at least about 0.1 ng, preferably at least about 0.5 ng and more preferably at least about 1 ng, where the amount may be as high as 1000 ng or higher, but will usually not exceed about 20 ng.

Control polynucleotides may be spotted on the array and used as target expression control polynucleotides and mismatch control nucleotides to monitor non-specific binding or cross-hybridization to a polynucleotide in the sample other than the target to which the probe is directed. Mismatch probes thus indicate whether a hybridization is specific or not. For example, if the target is present the perfectly matched probes should be consistently brighter than the mismatched probes. In addition, if all central mismatches are present, the mismatch probes are used to detect a mutation.

### Target Preparation

The targets for the microarrays, may be derived from one or more biological samples. It may be desirable to amplify the target nucleic acid sample prior to hybridization. One of skill in the art will appreciate that whatever amplification method is used, if a quantitative result is desired, care must be taken to use a method that maintains or controls for the relative frequencies of the amplified polynucleotides. Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. The high density array may then include probes specific to the internal standard for quantification of the amplified polynucleotide. Detailed protocols for quantitative PCR are provided in PCR Protocols, A Guide to Methods and Applications, Innis et al., Academic Press, Inc. N.Y., (1990). Other suitable amplification methods include, but are not limited to polymerase chain reaction (PCR) (Innis, et al., PCR Protocols. A guide to Methods and Application. Academic Press, Inc. San Diego, (1990)), ligase chain reaction (LCR) (see Wu and Wallace, Genomics, 4: 560 (1989), Landegren, etal., Science, 241: 1077 (1988) and Barringer, etal., Gene, 89: 117 (1990), transcription amplification (Kwoh, et al., Proc. Natl. Acad. Sci. USA, 86: 1173 (1989)), and self-sustained sequence replication (Guatelli, et al., Proc. Nat. Acad. Sci. USA, 87: 1874 (1990)).

The invention provides for labeled target or labeled probe as described above. For the microarrays, any analytically detectable marker that is attached to or incorporated into a molecule may be used in the invention. An analytically detectable marker refers to any molecule, moiety or atom which is analytically detected and quantified. Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., fluorescein, texas red, rhodamine, green fluorescent protein, and the like), radiolabels (e.g., 3H, 1251, 35S, 14C, or 32P), enzymes (e.g., horseradish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,277,437; 4,275,149; and 4,366,241.

Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

The labels may be incorporated by any of a number of means well known to those of skill in the art. However, in a preferred embodiment, the label is simultaneously incorporated during the amplification step in the preparation of the sample polynucleotides. Thus, for example, polymerase chain reaction (PCR) with labeled primers or labeled nucleotides will provide a labeled amplification product. In a preferred embodiment, transcription amplification, as described above, using a labeled nucleotide (e.g. fluorescein-labeled UTP and/or CTP) incorporates a label into the transcribed polynucleotides. Alternatively, a label may be added directly to the original polynucleotide sample (e.g., mRNA, polyA mRNA, cDNA, etc.) or to the amplification product after the amplification is completed. Means of attaching labels to polynucleotides are well known to those of skill in the art and include, for example nick translation or end-labeling (e.g. with a labeled RNA) by kinasing of the polynucleotide and subsequent attachment (ligation) of a polynucleotide linker joining the sample polynucleotide to a label (e.g., a fluorophore).

In a preferred embodiment, the target will include one or more control molecules which hybridize to control probes on the microarray to normalize signals generated from the microarray. Labeled normalization targets are polynucleotide sequences that are perfectly complementary to control oligonucleotides that are spotted onto the microarray as described above. The signals obtained from the normalization controls after hybridization provide a control for variations in hybridization conditions, label intensity, "reading" efficiency and other factors that may cause the signal of a perfect hybridization to vary between arrays.

### Image Acquisition and Data Analysis

Following hybridization and any washing step(s) and/or subsequent treatments of a conventional nature, the resultant hybridization pattern is detected. In detecting or visualizing the hybridization pattern, the intensity or signal value of the label will be not only be detected but quantified, by which is meant that the signal from each spot of the hybridization will be measured and compared to a unit value corresponding to the signal emitted by a known number of end labeled target polynucleotides to obtain a count or absolute value of the copy number of each end-labeled target that is hybridized to a particular spot on the array in the hybridization pattern.

Methods for analyzing the data collected from hybridization to arrays are well known in the art. For example, where detection of hybridization involves a fluorescent label, data analysis can include the steps of determining fluorescent intensity as a function of substrate position from the data collected, removing outliers, i.e., data deviating from a predetermined statistical distribution, and calculating the relative binding affinity of the test polynucleotides from the remaining data. The resulting data is displayed as an image with the intensity in each region varying according to the binding affinity between associated oligonucleotides and/or polynucleotides and the test polynucleotides.

### Diagnostic Tests

Following detection or visualization, the hybridization pattern is used to determine quantitative information about the genetic profile of the labeled target polynucleotide sample that was contacted with the array to generate the hybridization pattern, as well as the state or condition of the tissue, fluid, organs, cell, etc. from which the sample was derived. In this regard, the invention further provides for diagnostic tests for detecting cancer. The invention also provides for monitoring a patient's condition. According to the method of the invention, the presence of cancer is detected by obtaining a biological sample from a patient. A test sample comprising nucleic acid is prepared from the biological sample. The nucleic acid extracted from the sample is hybridized to an array comprising a solid substrate and a plurality of nucleic acid members, wherein each member is indicative of the presence of disease or a predisposition to cancer. According to this diagnostic test, hybridization of the sample comprising nucleic acid to one or more nucleic acid members on the array is indicative of cancer or a predisposition to a cancer.

### Diagnostic Monitoring

The methods of the present invention may further comprise the step of recommending a monitoring regime or course of therapy based on the outcome of one or more assays. This allows clinicians to practice personalized medicine; e.g. cancer therapy, by monitoring the progression of the patient's cancer (such as by recognizing when an initial or subsequent mutation occurs) or treatment (such as by recognizing when a mutation is stabilized).

With knowledge of the boundaries of the sequence variation in hand, the information can be used to diagnose a pre-cancerous condition or existing cancer condition. Further, by quantitating the amount of aberrant mtDNA in successive samples over time, the progression of a cancer condition can be monitored. For example, data provided by assaying the patient's tissues at one point in time to detect a first set of mutations from wild-type could be compared against data provided from a subsequent assay, to determine if changes in the aberration have occurred.

Where a mutation is found in an individual who has not yet developed symptoms of cancer, the mutation may be indicative of a genetic susceptibility to develop a cancer condition. A determination of susceptibility to disease or diagnosis of its presence can further be evaluated on a qualitative basis based on information concerning the prevalence, if any, of the cancer condition in the patient's family history and the presence of other risk factors, such as exposure to environmental factors and whether the patient's cells also carry a mutation of another sort.

### Biological Sample

The present invention provides for diagnostic tests which involve obtaining or collecting one or more biological samples. In the context of the present invention, "biological sample" refers to a tissue or bodily fluid containing cells from which mtDNA, mtRNA and translation products or fusion proteins can be obtained. For example, the biological sample can be derived from tissue including, but not limited to, skin, lung, breast, prostate, nervous, muscle, heart, stomach, colon, rectal tissue and the like; or from blood, saliva, cerebral spinal fluid, sputa, urine, mucous, synovial fluid, peritoneal fluid, amniotic fluid and the like. The biological sample may be obtained from a cancerous or non-cancerous tissue and may be, but is not limited to, a surgical specimen or a biopsy specimen.

The biological sample can be used either directly as obtained from the source or following a pre-treatment to modify the character of the sample. Thus, the biological sample can be pre-treated prior to use by, for example, preparing plasma or serum from blood, disrupting cells, preparing liquids from solid materials, diluting viscous fluids, filtering liquids, distilling liquids, concentrating liquids, inactivating interfering components, adding reagents, and the like.

One skilled in the art will understand that more than one sample type may be assayed at a single time (i.e. for the detection of more than one cancer). Furthermore, where a course of collections are required, for example, for the monitoring of cancer over time, a given sample may be diagnosed alone or together with other samples taken throughout a test period. In this regard, biological samples may be taken once only, or at regular intervals such as biweekly, monthly, semi-annually or annually.

### Kits

The present invention provides diagnostic/screening kits for detecting cancer in a clinical environment. Such kits may include one or more sampling means, in combination with one or more probes according to the present invention. Alternatively, or in addition thereto, the kit may comprise means for detecting a translation product of the invention.

The kits can optionally include reagents required to conduct a diagnostic assay, such as buffers, salts, detection reagents, and the like. Other components, such as buffers and solutions for the isolation and/or treatment of a biological sample, may also be included in the kit. One or more of the components of the kit may be lyophilised and the kit may further comprise reagents suitable for the reconstitution of the lyophilised components.

Where appropriate, the kit may also contain reaction vessels, mixing vessels and other components that facilitate the preparation of the test sample. The kit may also optionally include instructions for use, which may be provided in paper form or in computer-readable form, such as a disc, CD, DVD or the like.

In one embodiment of the invention there is provided a kit for diagnosing cancer comprising sampling means and a hybridization probe of the invention.

In another embodiment, the kits of the present invention may comprise an immunological assay. In such case, the kits may comprise antibodies or antigen-binding fragments having specificity towards the fusion proteins described herein. It will be understood that various other reagents, test strips etc. required for such immunological assay will be contained in the kits as will the required instructions to users.

### EXAMPLES

Various aspects of the invention will be described by illustration using the following examples. The examples provided herein serve only to illustrate certain specific embodiments of the invention and are not intended to limit the scope of the invention in any way.

### Example 1: Detection of Mitochondrial Fusion Transcripts

The mitochondrial 4977 "common deletion" and a 3.4kb deletion previously identified by the present Applicant in PCT application no. PCT/CA2007/001711 (published under number WO 2009/039601) result in unique open reading frames having active transcripts as identified by oligo-dT selection in prostate tissue (Figures 2 and 3). Examination of breast tissue samples also reveals the presence of a stable polyadenylated fusion transcript resulting from the 3.4kb deletion (Figure 4).

### Reverse transcriptase-PCR protocol for deletion transcript detection

### RNA isolation cDNA synthesis

Total RNA was isolated from snap frozen prostate and breast tissue samples (both malignant and normal samples adjacent to tumours) using the Aurum™ Total RNA Fatty and Fibrous Tissue kit (Bio-Rad, Hercules, CA) following the manufacturer's instructions. Since in this experiment, genomic DNA contamination was to be avoided, a DNase I treatment step was included, using methods as commonly known in the art. RNA quantity and quality were determined with an ND-1000 spectrophotometer (NanoDrop® technologies). From a starting material of about 100g, total RNA concentrations varied from 100 - 1000ng/ul with a 260/280 ratio between 1.89 - 2.10. RNA concentrations were adjusted to 100ng/ul and 2ul of each template were used for first strand DNA synthesis with SuperScript™ First-Strand Synthesis System for RT-PCR (Invitrogen) following the manufacturer's instructions. In order to identify stable polyadenylated fusion transcripts, Oligo(dT) primers that target transcripts with poly-A tails were used.

### PCR

Real time PCR was performed using 5ul of each cDNA template with the iQ™ SYBR® Green Supermix (Bio-Rad, Hercules, CA) on DNA Engine Opticon® 2 Continuous Fluorescence Detection System (Bio-Rad, Hercules, CA). The primer pairs targeting the 4977bp deletion are; 8416F 5'- CCTTACACTATTCCTCATCAC- 3', 13637R 5'- TGACCTGTTAGGGTGAGAAG - 3', and those for the 3.4 kb deletion are; ND4LF 5'- TCGCTCACACCTCATATCCTC -3', ND5R 5'-TGTGATTAGGAGTAGGGTTAGG -3'. The reaction cocktail included: 2X SYBR® Green Supermix (100mM KCL, 40mM Tris-HCI, pH 8.4, 0.4mM of each dNTP [dATP, dCTP, dGTP, and dTTP], iTaq™ DNA polymerase, 50 units/ml, 6mM MgCl2, SYBR® Green 1, 20nM flourescein, and stabilizers), 250nM each of primers, and ddH₂O. PCR cycling parameters were as follows; (1) 95°C for 2 min, (2) 95°C for 30 sec, (3) 55°C (for the 4977bp deletion) and 63°C (for the 3.4 kb deletion) for 30 sec , (4) 72°C for 45 sec, (5) plate read, followed by 39 cycles of steps 3 to 5, and final incubation at 4°C. Apart from cycling threshold and melting curve analysis, samples were run on agarose gels for specific visualization of amplification products (see Figures 2 to 4).

Figure 2 is an agarose gel showing polyadenalated fusion transcripts in prostate samples invoked by the loss of 3.4kb from the mitochondrial genome. Legend for Figure 2: B-blank, Lanes 1-6 transcripts detected in cDNA; lanes 7-12 no reverse transcriptase (RT) controls for samples in lanes 1-6.

Figure 3 shows polyadenalated fusion transcripts in prostate samples invoked by the loss of the 4977kb common deletion. Legend for Figure 3: B-blank, Lanes 1-6 transcripts detected in cDNA; lanes 7-12 no RT controls for samples in lanes 1-6.

Figure 4 shows polyadenalated fusion transcripts in breast samples invoked by the loss of 3.4kb from the mtgenome. Legend for Figure 4: Lanes 2-8 transcripts from breast cDNAs; lane 9 negative (water) control; lanes 10 and 11, negative, no RT, controls for samples in lanes 2 and 3.

These results demonstrate the existence of stable mitochondrial fusion transcripts.

### Example 2: Identification and Targetting of Fusion Products

Various hybridization probes were designed to detect, and further demonstrate the presence of novel transcripts resulting from mutated mitochondrial genomes, such as the 3.4kb deletion. For this purpose, a single-plex branched DNA platform for quantitative gene expression analysis (QuantiGene 2.0™, Panomics™) was utilized. The specific deletions and sequences listed in this example are based on their relative positions with the entire mtDNA genome, which is recited in SEQ ID NO: 1. The nucleic acid sequences of the four transcripts to which the probes were designed in this example are identified herein as follows: Transcript 1 (SEQ ID NO: 19), Transcript 2 (SEQ ID NO: 20), Transcript 3 (SEQ ID NO: 21) and Transcript 4 (SEQ ID NO: 22).

An example of a continuous transcript from the 3.4kb mitochondrial genome deletion occurs with the genes ND4L (NADH dehydrogenase subunit 4L) and ND5 (NADH dehydrogenase subunit 5). A probe having a complementary sequence to SEQ ID NO: 20, was used to detect transcript 2. The repetitive elements occur at positions 10745-10754 in ND4L and 14124-14133 in ND5.

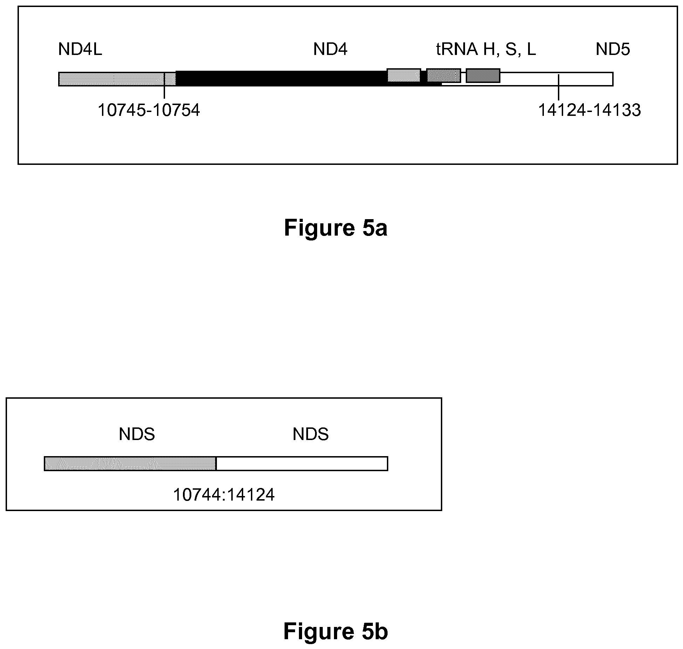

The 3.4kb deletion results in the removal of the 3' end of ND4L, the full ND4 gene, tRNA histidine, tRNA serine2, tRNA leucine2, and the majority of the 5' end of ND5 (see Figure 5a), resulting in a gene splice of ND4L and ND5 with a junction point of 10744(ND4L):14124(ND5) (Figure 5b).

By starting at the original initiation codon of the first gene, ND4L, the amino acid sequence was translated until a termination codon occurs. In this example the termination codon is the original termination codon of ND5. Therefore, despite splicing two genes together, the reading frame is kept intact resulting in a hypothetical or predicted transcript that is 100 amino acids (or 300 bp) in length. This fusion protein transcript product is identified herein as SEQ ID NO: 37. The nucleotide sequence (SEQ ID NO: 3) encoding such protein corresponds to the mitochondrial genome positions of 10470-10744:14124-14148. SEQ ID NO: 3 is the complementary DNA sequence to the RNA transcript (SEQ ID NO: 20) detected in the manner described above.

Similarly, transcript 1 is a fusion transcript between ATPase 8 and ND5 associated with positions 8469:13447 (SEQ ID NO: 19). Transcripts 3 and 4 (SEQ ID NO: 21 and SEQ ID NO: 22, respectively) are fusion transcripts between COII and Cytb associated with nucleotide positions 7974:15496 and 7992:15730 respectively. Table 3 provides a summary of the relationships between the various sequences used in this example. Table 3 includes the detected fusion transcript, the DNA sequence complementary to the fusion transcript detected and hypothetical translation products for each transcript.

### Example 3: Application to Prostate Cancer

Using the four fusion transcripts, i.e. transcripts 1 to 4, discussed above, two prostate tissue samples from one patient were analyzed to assess the quantitative difference of the novel predicted fusion transcripts. The results of the experiment are provided in Table 2 below, wherein "Homog 1" refers to the homogenate of frozen prostate tumour tissue from a patient and "Homog 2" refers to the homogenate of frozen normal prostate tissue adjacent to the tumour of the patient. These samples were processed according to the manufacturer's protocol (*QuantiGene*® *Sample Processing Kit for Fresh or Frozen Animal Tissues; and QuantiGene*® *2.0 Reagent System User Manual)* starting with 25.8 mg of Homog 1 and 28.9 mg of Homog 2 (the assay setup is shown in Tables 5a and 5b).

Clearly demonstrated is an increased presence of mitochondrial fusion transcripts in prostate cancer tissue compared to normal adjacent prostate tissue. The fusion transcript is present in the normal tissue, although at much lower levels. The relative luminescence units (RLU) generated by hybridization of a probe to a target transcript are directly proportional to the abundance of each transcript. Table 2 also indicates the coefficients of variation, CV, expressed as a percentage, of the readings taken for the samples. The CV comprises the Standard deviation divided by the average of the values. The significance of such stably transcribed mitochondrial gene products in cancer tissue has implications in disease evolution and progression.

### Example 4: Application to Breast Cancer

Using the same protocol from Example 3 but focusing only on Transcript 2, the novel fusion transcript associated with the 3.4kb mtgenome deletion, analyses were conducted on two samples of breast tumour tissue and two samples of tumour-free tissues adjacent to those tumours, as well as three samples of prostate tumour tissue, one sample comprising adjacent tumour-free tissue. Results for this example are provided in Table 4. The prostate tumour tissue sample having a corresponding normal tissue section demonstrated a similar pattern to the prostate sample analyzed in Example 3 in that the tumour tissue had approximately 2 times the amount of the fusion transcript than did the normal adjacent tissue. The breast tumour samples demonstrated a marked increase in the fusion transcript levels when compared to the adjacent non-tumour tissues. A 1:100 dilution of the homogenate was used for this analysis as it performed most reproducibly in the experiment cited in Example 3.

Thus, the above discussed results illustrate the application of the transcripts of the invention in the detection of tumours of both prostate and breast tissue.

### Example 5: Application to Colorectal Cancer

This study sought to determine the effectiveness of several transcripts of the invention in detecting colorectal cancer. A total of 19 samples were prepared comprising nine control (benign) tissue samples (samples 1 to 9) and ten tumour (malignant) tissue samples (samples 10 to 19). The samples were homogenized according to the manufacturer's recommendations (Quantigene® Sample Processing Kit for Fresh or Frozen Animal Tissues; and Quantigene 2.0 Reagent System User Manual). Seven target transcripts and one housekeeper transcript were prepared in the manner as outlined above in previous examples. The characteristics of the transcripts are summarized as follows:

**Table 7: Characteristics of Breast Cancer Transcripts**

| **Transcript ID** | **Junction Site** | **Gene Junction** |
|---|---|---|
| 2 | 10744:14124 | ND4L:ND5 |
| 3 | 7974:15496 | COII:Cytb |
| 10 | 7438:13476 | COI:ND5 |
| 11 | 7775:13532 | COII:ND5 |
| 12 | 8213:13991 | COII:ND5 |
| Peptidylpropyl isomerase B (PPIB) ("housekeeper") | N/A | N/A |

It is noted that transcripts 2 and 3 are the same as those discussed above with respect to Examples 3 and 4.

Homogenates were prepared using approximately 25mg of tissue from OCT blocks and diluted 1:1 for transcripts 2 and 4, and 1:8 for transcripts 10 and 11. The quantity of the transcripts was measured in Relative Luminenscence Units RLU on a Glomax™ Multi Detection System (Promega). All samples were assayed in triplicate for each transcript. Background measurements (no template) were done in triplicate as well. The analysis accounted for background by subtracting the lower limit from the RLU values for the samples. Input RNA was accounted for by using the formula log₂ a RLU - log₂ h RLU where a is the target fusion transcript and h is the housekeeper transcript.

The analysis of the data comprised the following steps:
a) Establish CV's (coefficients of variation) for triplicate assays; acceptable if ≤ 15%.
b) Establish average RLU value for triplicate assays of target fusion transcript(a) and housekeeper transcript (h).
c) Establish lower limit from triplicate value of background RLU (I).
d) Subtract lower limit (I) from (a).
e) Calculate log2 a RLU - log2 h RLU.

### Summary of Results:

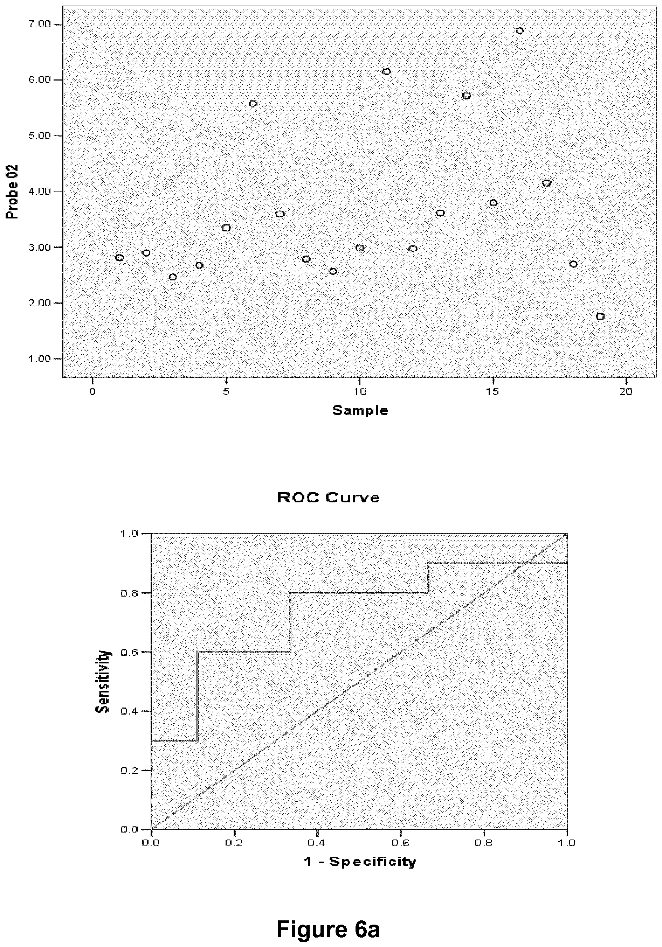

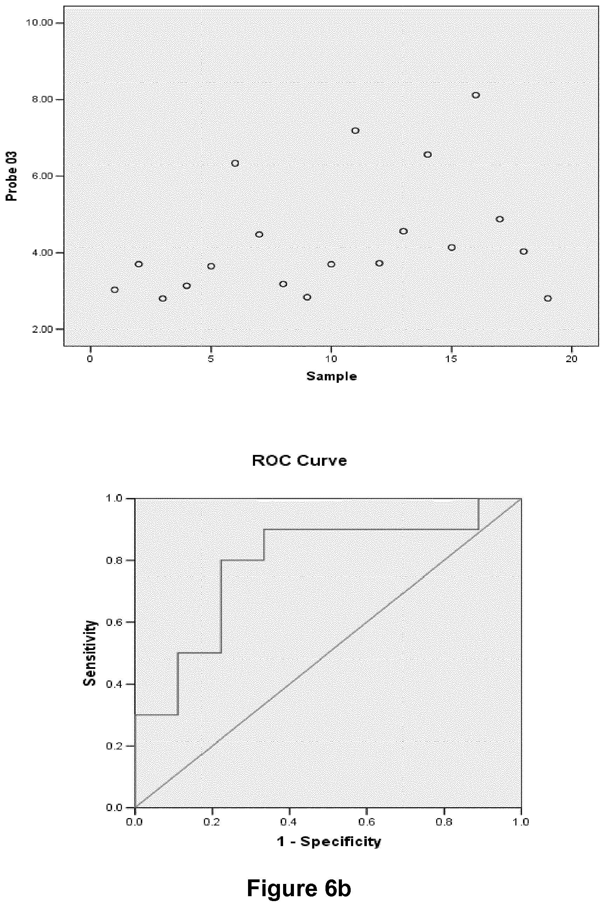

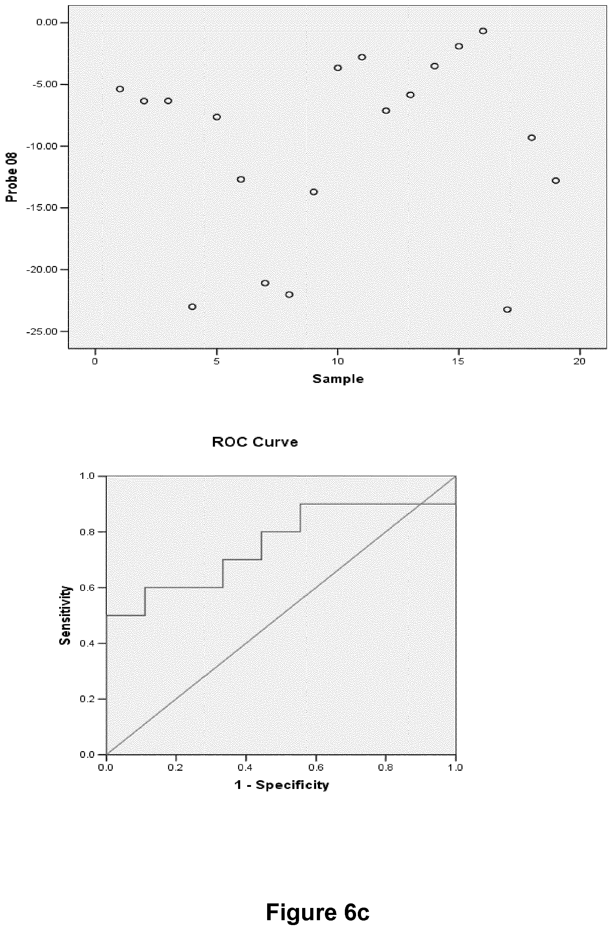

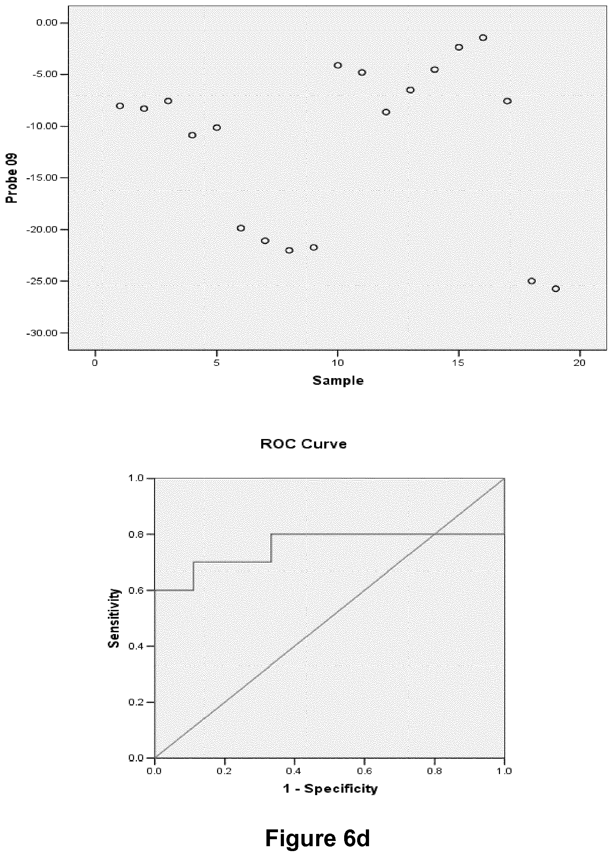

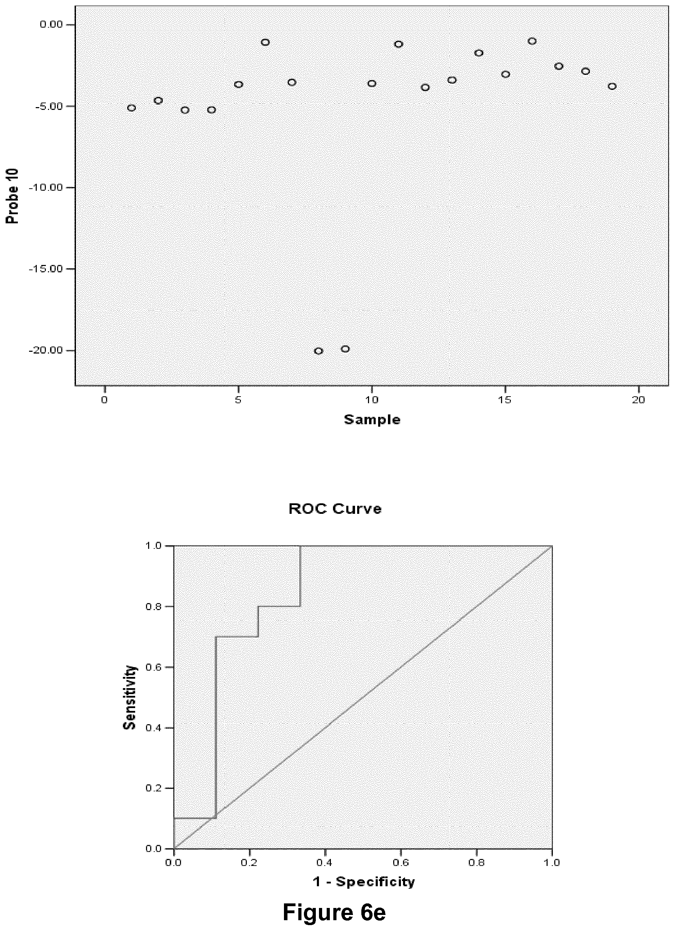

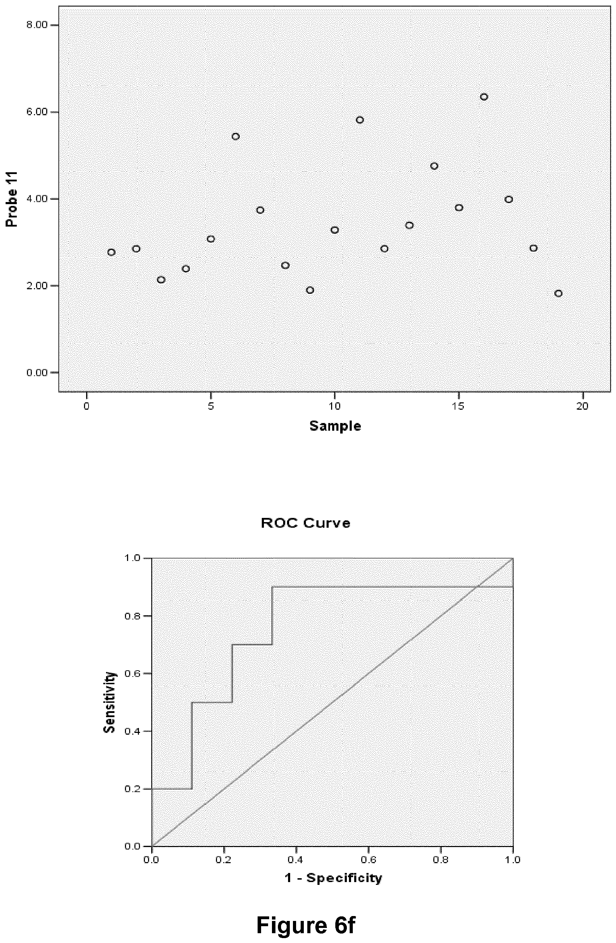

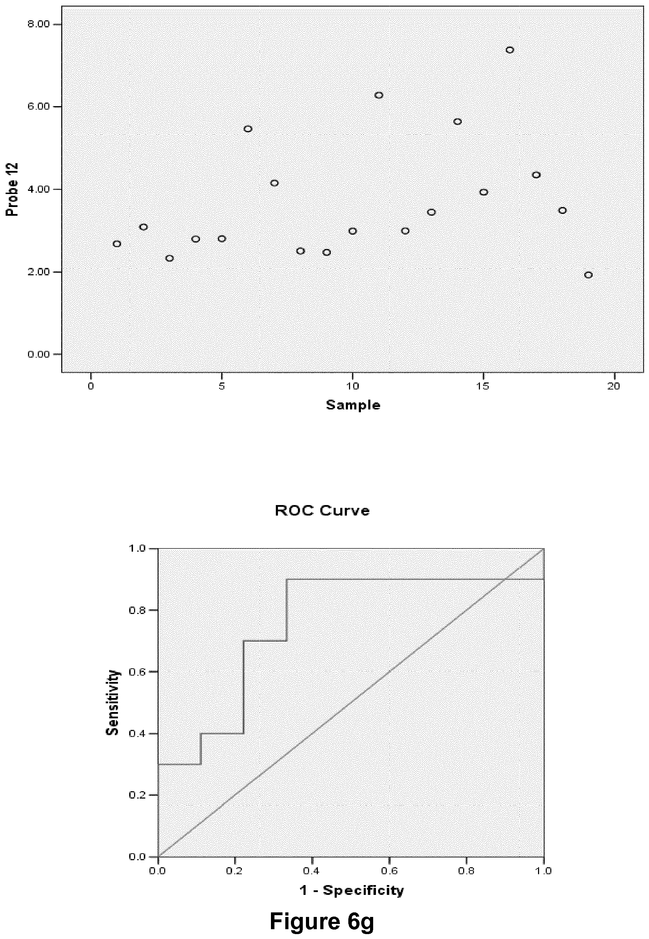

The results of the above analysis are illustrated in Figures 6a to 6g, which comprise plots of the log₂ a RLU - log₂ h RLU against sample number. Also illustrated are the respective ROC (Receiver Operating Characteristic) curves determined from the results for each transcript.
Transcript 2: There exists a statistically significant difference between the means (p<0.10) of the normal and malignant groups (p>0.09), using a cutoff value of 3.6129 as demonstrated by the ROC curve results in a sensitivity of 60% and specificity of 89% and the area under the curve is 0.73 indicating fair test accuracy. The threshold value chosen may be adjusted to increase either the specificity or sensitivity of the test for a particular application.
Transcript 3: There exists a statistically significant difference between the means (p<0.05) of the normal and malignant groups (p=0.03), using a cutoff value of 4.0813 as demonstrated by the ROC curve results in a sensitivity of 60% and specificity of 78% and the area under the curve is 0.79 indicating fair to good test accuracy. The threshold value chosen may be adjusted to increase either the specificity or sensitivity of the test for a particular application.
Transcript 8: There exists a statistically significant difference between the means (p<0.1) of the normal and malignant groups (p=0.06). Using a cutoff value of -6.0975 as demonstrated by the ROC curve results in a sensitivity of 60% and specificity of 89% and the area under the curve is 0.76 indicating fair test accuracy. The threshold value chosen may be adjusted to increase either the specificity or sensitivity of the test for a particular application.
Transcript 9: There exists a statistically significant difference between the means (p<0.1) of the normal and malignant groups (p=0.06). Using a cutoff value of -7.5555 as demonstrated by the ROC curve results in a sensitivity of 60% and specificity of 89% and the area under the curve is 0.76 indicating fair to good test accuracy. The threshold value chosen may be adjusted to increase either the specificity or sensitivity of the test for a particular application.
Transcript 10: There is a statistically significant difference between the means (p≤0.01) of the normal and malignant groups (p=0.01). Using a cutoff value of -3.8272as demonstrated by the ROC curve results in a sensitivity of 90% and specificity of 67% and the area under the curve is 0.84, indicating good test accuracy. The threshold value chosen may be adjusted to increase either the specificity or sensitivity of the test for a particular application.
Transcript 11: There exists a statistically significant difference between the means (p<0.1) of the normal and malignant groups (p=0.06), using a cutoff value of 3.1753 as demonstrated by the ROC curve results in a sensitivity of 70% and specificity of 78% and the area under the curve is 0.76 indicating fair to good test accuracy. The threshold value chosen may be adjusted to increase either the specificity or sensitivity of the test for a particular application.
Transcript 12: There exists a statistically significant difference between the means (p<0.1) of the normal and malignant groups (p=0.06), using a cut-off value of 3.2626 as demonstrated by the ROC curve results in a sensitivity of 70% and specificity of 78% and the area under the curve is 0.76 indicating fair to good test accuracy. The threshold value chosen may be adjusted to increase either the specificity or sensitivity of the test for a particular application.

### Conclusions:

The above results illustrate the utility of transcripts 2, 3, 8, 9, 10, 11, and 12 in the detection of colorectal cancer and in distinguishing malignant from normal colorectal tissue. As indicated above, transcripts 2 and 3 were also found to have utility in the detection of prostate cancer. Transcript 2 was also found to have utility in the detection of breast cancer. Transcript 11 was also found to have utility in the detection of melanoma skin cancer. Transcript 10 was also found to have utility in the detection of lung cancer and melanoma. Transcript 8 was also found to have utility in the detection of lung cancer. Any of the 7 transcripts listed may be used individually or in combination as a tool for the detection of characterization of colorectal cancer in a clinical setting.

### Example 6: Application to Lung Cancer

This study sought to determine the effectiveness of several transcripts of the disclosure in the detection of lung cancer. As in Example 5, nine control (benign) tissue samples (samples 1 to 9) and ten tumour (malignant) tissue samples (samples 10 to 19) were homogenized according to the manufacturer's recommendations (Quantigene® Sample Processing Kit for Fresh or Frozen Animal Tissues; and Quantigene 2.0 Reagent System User Manual). Homogenates were diluted 1:8 and the quantity of 4 target transcripts and 1 housekeeper transcript was measured in Relative Luminenscence Units RLU on a Glomax™ Multi Detection System (Promega). All samples were assayed in triplicate for each transcript. Background measurements (no template) were done in triplicate as well.

The following transcripts were prepared for this example:

**Table 8: Characteristics of Lung Cancer Transcripts**

| **Transcript ID** | **Junction Site** | **Gene Junction** |
|---|---|---|
| 6 | 8828:14896 | ATPase6:Cytb |
| 8 | 6075:13799 | COI:ND5 |
| 10 | 7438:13476 | COI:ND5 |
| 20 | 8469:13447 | ATPase8:ND5 |
| Peptidylpropyl isomerase B (PPIB) ("housekeeper") | N/A | N/A |

The tissue samples used in this example had the following characteristics:

**Table 9: Characteristics of Lung Cancer Samples**

| **Sample** | **Malignant** | **Comments (source of tissue)** |
|---|---|---|
| 1 | NO | interstitial lung disease |
| 2 | NO | emphysema |
| 3 | NO | aneurysm |
| 4 | NO | bronchopneumonia, COPD |
| 5 | NO | malignant neoplasm in liver, origin unknown, calcified granulomas in lung |
| 6 | NO | 12 hours post mortem, mild emphysema |
| 7 | NO | 12 hours post mortem, large B cell lymphoma, pulmonary edema, pneumonia |
| 8 | NO | pneumonia, edema, alveolar damage |
| 9 | NO | congestion and edema |
| 10 | YES | adenocarcinoma, non-small cell |
| 11 | YES | small cell |
| 12 | YES | squamous cell carcinoma, NSC, emphysema |
| 13 | YES | adenocarcinoma, lung cancer, nsc, metastatic |
| 14 | YES | squamous cell carcinoma, non-small cell |
| 15 | YES | mixed squamous and adenocarcinoma |
| 16 | YES | non-small cell carcinoma, squamous |
| 17 | YES | small cell carcinoma |
| 18 | YES | adenocarcinoma, lung cancer, nsc |
| 19 | YES | adenocarcinoma, lung cancer, nsc, metastatic |

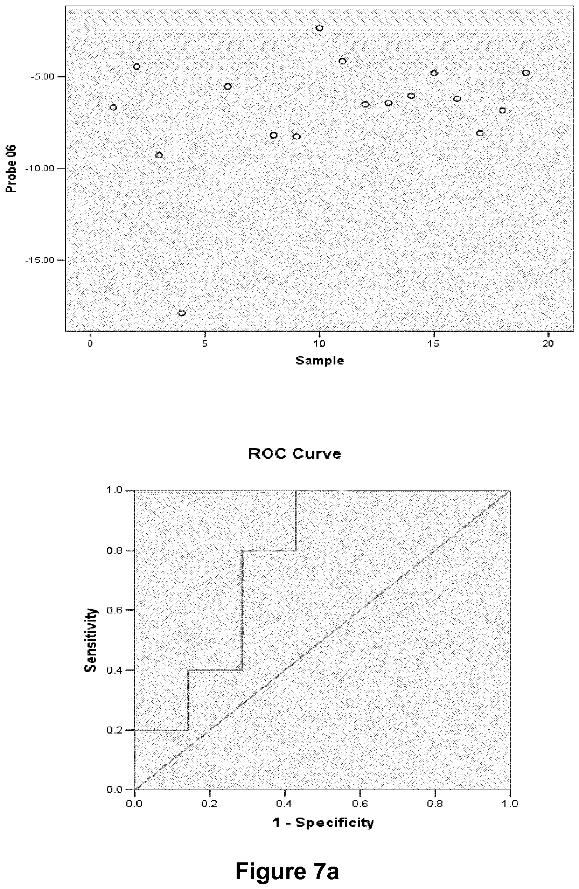

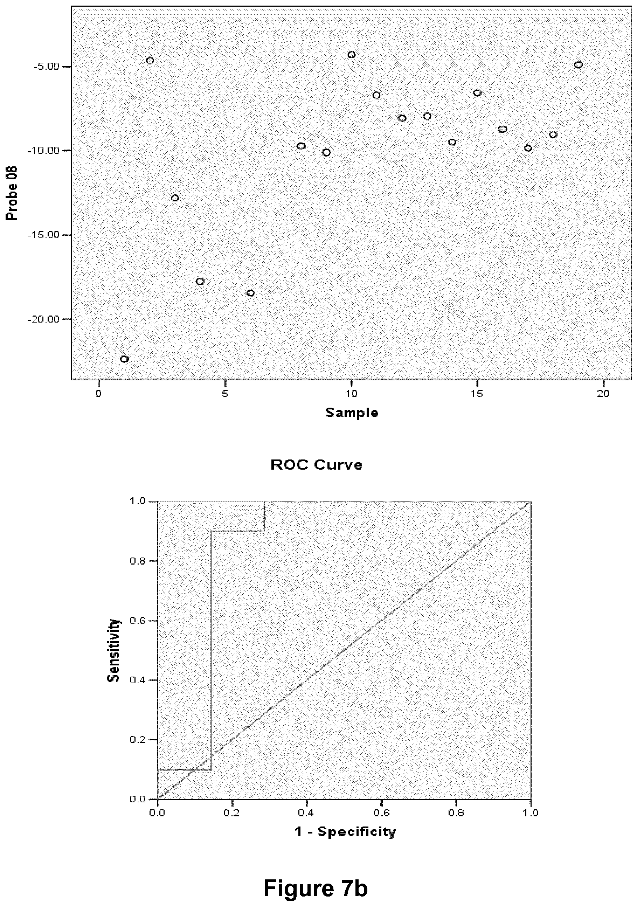

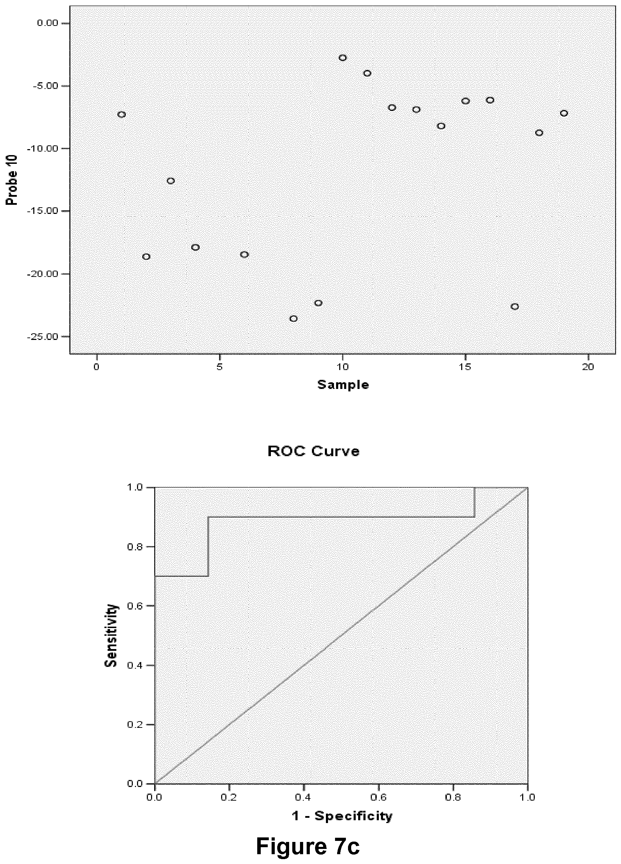

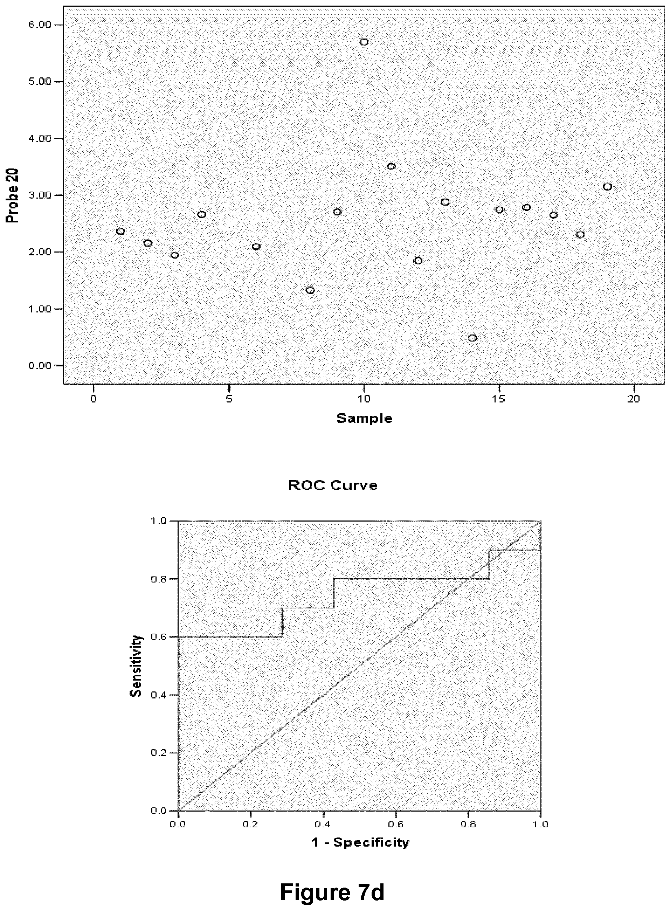

The analysis of data was performed according to the method described in Example 5. The results are illustrated in figures 7a, 7b, 7c and 7d.

### Summary of Results:

Transcript 6: There exists a statistically significant difference between the means (p<0.1) of the normal (benign) and malignant groups (p=0.06), using a cutoff value of -6.5691as demonstrated by the ROC curve results in a sensitivity of 80% and specificity of 71% and the area under the curve is 0.77, indicating fair test accuracy. The threshold value chosen may be adjusted to increase either the specificity or sensitivity of the test for a particular application.
Transcript 8: The difference between the means of the normal and malignant groups is statistically significant, p<0.05 (p=0.02). Using a cutoff value of -9.6166 as demonstrated by the ROC curve results in a sensitivity of 90% and specificity of 86% and the area under the curve is 0.86 indicating good test accuracy. The threshold value chosen may be adjusted to increase either the specificity or sensitivity of the test for a particular application.
Transcript 10: The difference between the means of the normal and malignant groups is statistically significant, p≤0.01 (p=0.01). Using a cutoff value of -10.6717 as demonstrated by the ROC curve results in a sensitivity of 90% and specificity of 86% and the area under the curve is 0.89 indicating good test accuracy. The threshold value chosen may be adjusted to increase either the specificity or sensitivity of the test for a particular application.
Transcript 20: The difference between the means of the normal and malignant groups is statistically significant, p≤0.1 (p=0.1). Using a cutoff value of 2.5071 as demonstrated by the ROC curve results in a sensitivity of 70% and specificity of 71% and the area under the curve is 0.74 indicating fair test accuracy. The threshold value chosen may be adjusted to increase either the specificity or sensitivity of the test for a particular application.

### Conclusions:

The results from example 6 illustrate the utility of transcripts 6, 8, 10, and 20 of the invention in the detection of lung cancer tumours and the distinction between malignant and normal lung tissues. Any of these three transcripts may be used for the detection or characterization of lung cancer in a clinical setting.

### Example 7: Application to Melanoma

This study sought to determine the effectiveness of several transcripts of the disclosure in the detection of melanomas. In this study a total of 14 samples were used, comprising five control (benign) tissue samples and nine malignant tissue samples. All samples were formalin fixed, paraffin embedded (FFPE). The FFPE tissue samples were sectioned into tubes and homogenized according to the manufacturer's recommendations (Quantigene® 2.0 Sample Processing Kit for FFPE Samples; and Quantigene 2.0 Reagent System User Manual) such that each sample approximated 20 microns prior to homogenization. Homogenates were diluted 1:4 and the quantity of 7 target transcripts and 1 housekeeper transcript was measured in Relative Luminenscence Units RLU on a Glomax™ Multi Detection System (Promega). All samples were assayed in triplicate for each transcript. Background measurements (no template) were done in triplicate as well.

The 14 tissue samples used in this example had the following characteristics:

**Table 10: Characteristics of Melanoma Cancer Samples**

| **Sample** | **Malignant** | **Comments (source of tissue)** |
|---|---|---|
| 1 | NO | breast reduction tissue (skin) |
| 2 | NO | breast reduction tissue (skin) |
| 3 | NO | breast reduction tissue (skin) |
| 4 | NO | breast reduction tissue (skin) |
| 5 | NO | breast reduction tissue (skin) |
| 6 | YES | lentigo maligna, (melanoma in situ) invasive melanoma not present |
| 7 | YES | invasive malignant melanoma |
| 8 | YES | nodular melanoma, pT3b, associated features of lentigo maligna |
| 9 | YES | residual superficial spreading invasive malignant melanoma, Clark's level II |
| 10 | YES | superficial spreading malignant melanoma, Clark's Level II |
| 11 | YES | nodular malignant melanoma, Clark's level IV |
| 12 | YES | superficial spreading malignant melanoma in situ, no evidence of invasion |
| 13 | YES | superficial spreading malignant melanoma, Clark's level II, focally present vertical phase |
| 14 | YES | superficial spreading malignant melanoma in situ, Clark's level I |

The following transcripts were prepared for this example:

**Table 11: Characteristics of Melanoma Cancer Transcripts**

| **Transcript ID** | **Junction Site** | **Gene Junction** |
|---|---|---|
| 6 | 8828:4896 | ATPase6:Cytb |
| 10 | 7438:13476 | COI:ND5 |
| 11 | 7775:13532 | COII:ND5 |
| 14 | 9191:12909 | ATPase6:ND5 |
| 15 | 9574:12972 | COIII:ND5 |
| 16 | 10367:12829 | ND3:ND5 |
| 20 | 8469:13447 | ATPase8:ND5 |
| Peptidylpropyl isomerase B (PPIB) ("housekeeper") | N/A | N/A |

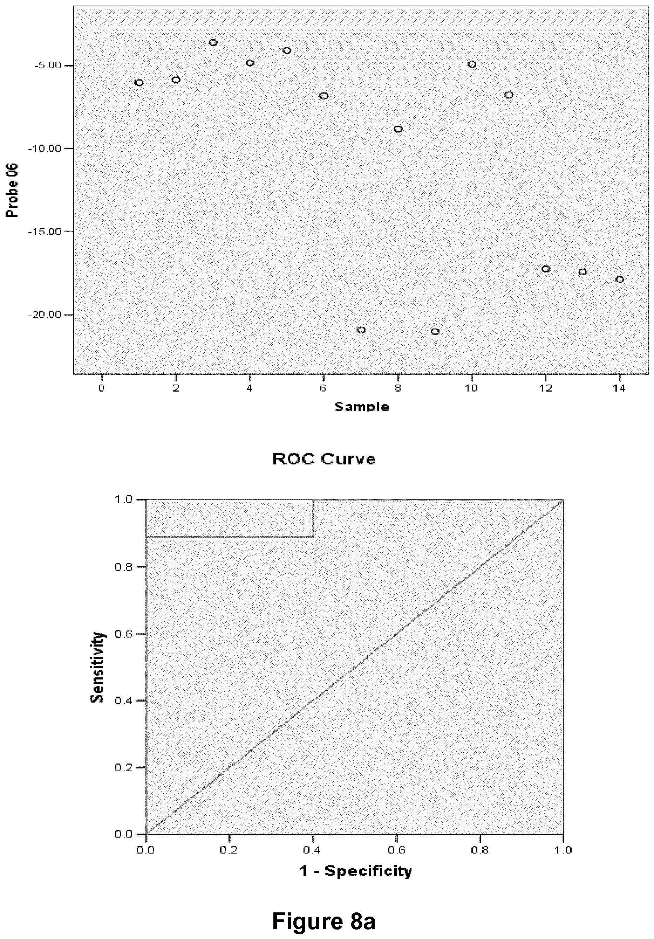

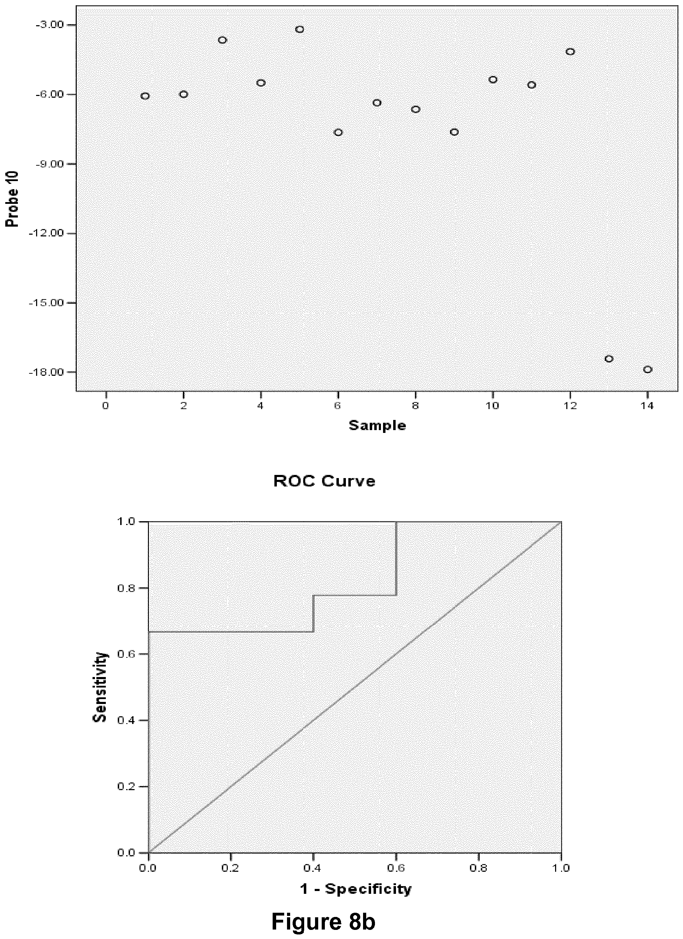

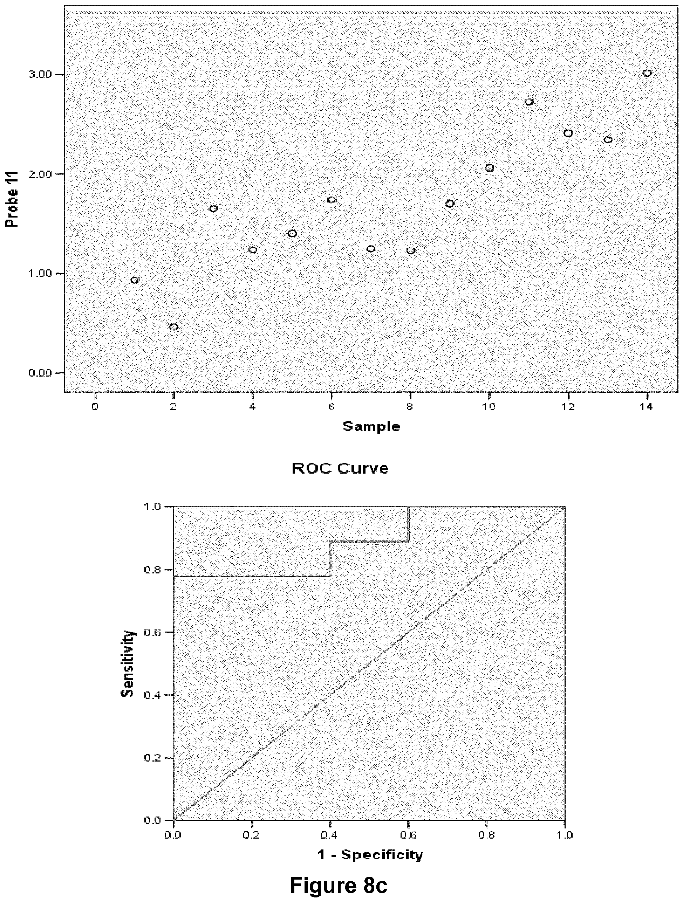

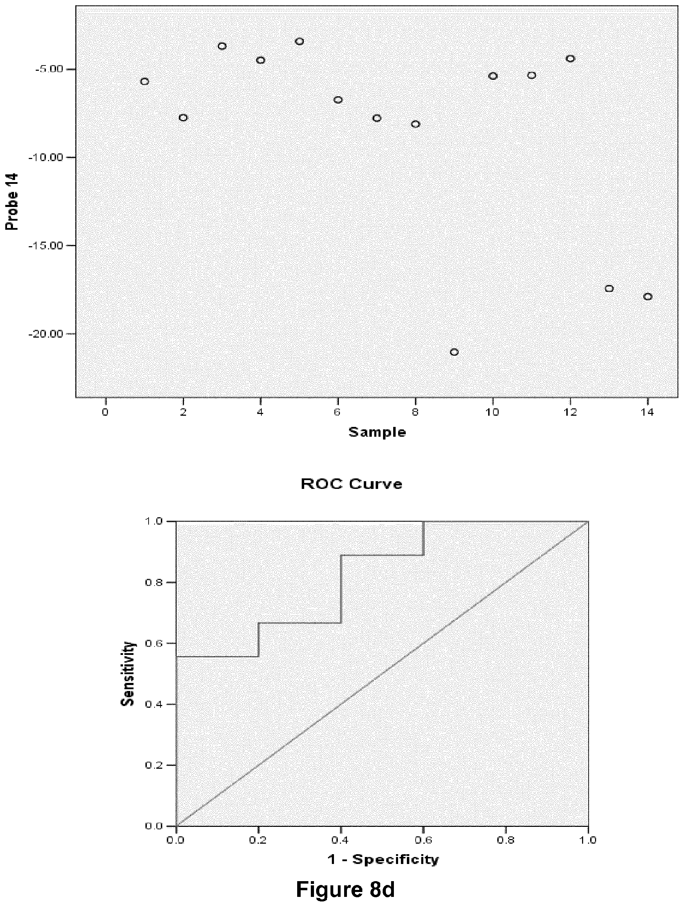

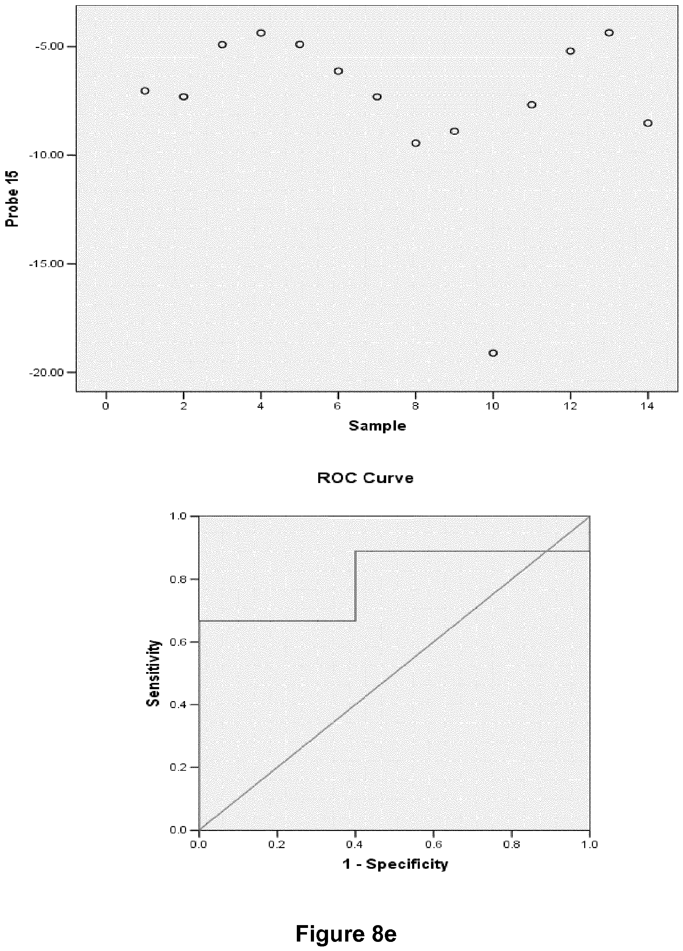

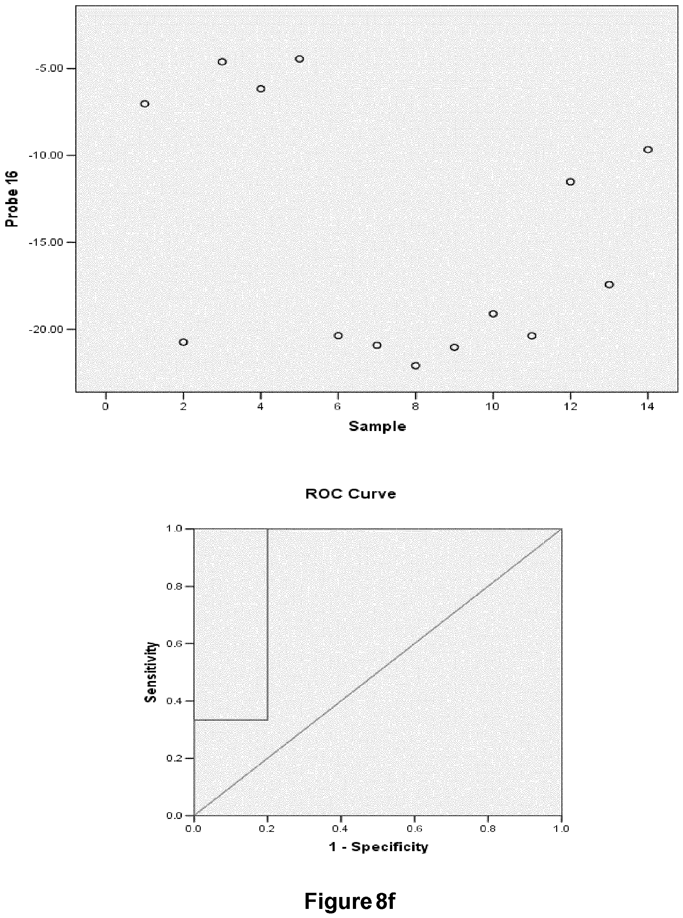

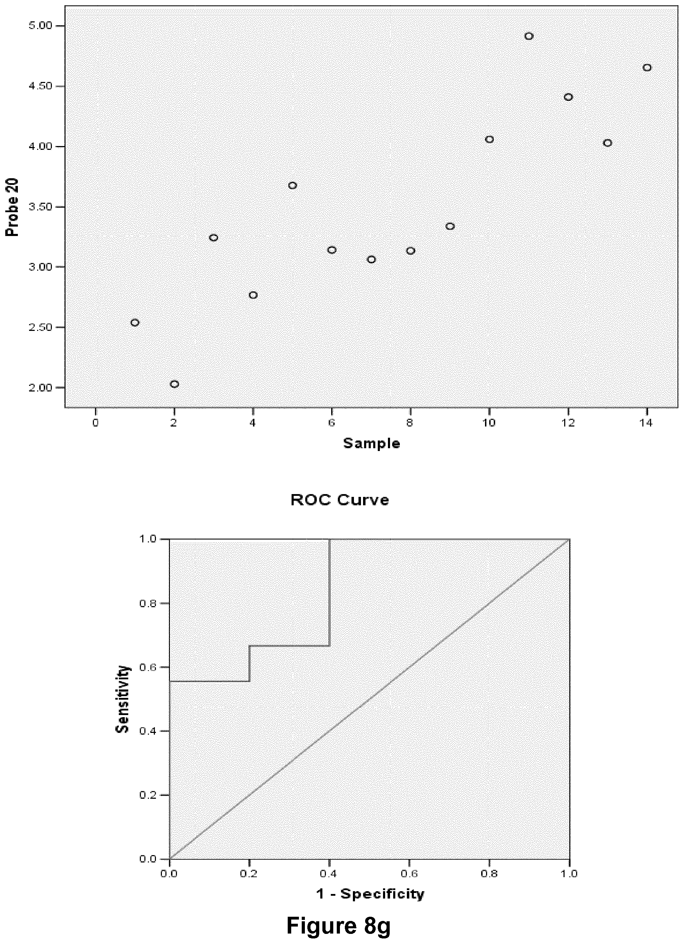

As indicated, transcripts 10 and 11 were also used in Example 5. The analysis of data was performed according to the method described in Example 5. The results are illustrated in figures 8a -8g.

### Summary of Results:

Transcript 6: There exists a statistically significant difference between the means (p≤0.01) of the normal and malignant groups (p=0.01). Further, using a cutoff value of -5.9531 as demonstrated by the ROC curve results in a sensitivity of 89% and specificity of 80% and the area under the curve is 0.96, indicating very good test accuracy. The threshold value chosen may be adjusted to increase either the specificity or sensitivity of the test for a particular application.
Transcript 10: There exists a statistically significant difference between the means (p≤0.05) of the normal and malignant groups (p=0.05), using a cutoff value of -4.7572as demonstrated by the ROC curve results in a sensitivity of 89% and specificity of 40% and the area under the curve is 0.82, indicating good test accuracy. The threshold value chosen may be adjusted to increase either the specificity or sensitivity of the test for a particular application.
Transcript 11: There exists a statistically significant difference between the means (p<0.05) of the normal and malignant groups (p=0.02). Further, using a cutoff value of 1.6762 as demonstrated by the ROC curve results in a sensitivity of 78% and specificity of 100% and the area under the curve is 0.89, indicating good test accuracy. The threshold value chosen may be adjusted to increase either the specificity or sensitivity of the test for a particular application.
Transcript 14: There exists a statistically significant difference between the means (p≤0.05) of the normal and malignant groups (p=0.05). Further, using a cutoff value of -4.9118 as demonstrated by the ROC curve results in a sensitivity of 89% and specificity of 60% and the area under the curve is 0.82, indicating good test accuracy. The threshold value chosen may be adjusted to increase either the specificity or sensitivity of the test for a particular application.
Transcript 15: There exists a statistically significant difference between the means (p<0.1) of the normal and malignant groups (p=0.07), using a cutoff value of -7.3107as demonstrated by the ROC curve results in a sensitivity of 100% and specificity of 67% and the area under the curve is 0.80, indicating good test accuracy. The threshold value chosen may be adjusted to increase either the specificity or sensitivity of the test for a particular application.
Transcript 16: There exists a statistically significant difference between the means (p<0.05) of the normal and malignant groups (p=0.03). Further, using a cutoff value of -10.5963as demonstrated by the ROC curve results in a sensitivity of 89% and specificity of 80% and the area under the curve is 0.878, indicating good test accuracy. The threshold value chosen may be adjusted to increase either the specificity or sensitivity of the test for a particular application.
Transcript 20: There exists a statistically significant difference between the means (p<0.05) of the normal and malignant groups (p=0.04). Further, using a cutoff value of -8.3543as demonstrated by the ROC curve results in a sensitivity of 100% and specificity of 80% and the area under the curve is 0.89, indicating good test accuracy. The threshold value chosen may be adjusted to increase either the specificity or sensitivity of the test for a particular application.

### Conclusions:

The results from example 7 illustrate the utility of transcripts 6, 10, 11, 14, 15, 16 and 20 of the invention in the detection of malignant melanomas. As indicated above, transcripts 10 and 11 were also found have utility in detecting colorectal cancer while transcript 6 has utility in the detection of lung cancer. A transcript summary by disease is provided at Table 6.

### Example 8: Application to Ovarian Cancer

This study sought to determine the effectiveness of several transcripts of the invention in detecting ovarian cancer. A total of 20 samples were prepared comprising ten control (benign) tissue samples (samples 1 to 10) and ten tumour (malignant) tissue samples (samples 11 to 20). The samples were homogenized according to the manufacturer's recommendations (Quantigene® Sample Processing Kit for Fresh or Frozen Animal Tissues; and Quantigene 2.0 Reagent System User Manual). Eight target transcripts and one housekeeper transcript were prepared in the manner as outlined above in previous examples.

The 20 tissue samples used in this example had the following characteristics:

**Table 12: Characteristics of Ovarian Cancer Samples**

| **Sample** | **Diagnosis** | **Comments** |
|---|---|---|
| 1 | Normal | follicular cyst |
| 2 | Normal | fibroma |
| 3 | Normal | No pathological change in ovaries |
| 4 | Normal | follicular cysts |
| 5 | Normal | cellular fibroma |
| 6 | Normal | benign follicular and simple cysts |
| 7 | Normal | leiomyomata, corpora albicantia |
| 8 | Normal | copora albicantia and an epithelial inclusions cysts |
| 9 | Normal | corpora albicantia |
| 10 | Normal | corpora albicantia, surface inclusion cysts, follicullar cysts |
| 11 | Malignant | high grade poorly differentiated papillary serous carcinoma involving omentum |
| 12 | Malignant | endometrioid adenocarcinoma, well to moderately differentiated with focal serous differentiation |
| 13 | Malignant | papillary serous carcinoma |
| 14 | Malignant | mixed epithelial carcinoma predominantly papillary serous carcinoma |
| 15 | Malignant | High grade: serous carcinoma, papillary and solid growth patterns |
| 16 | Malignant | High Grade (3/3) Papillary serous carcinoma |
| 17 | Malignant | papillary serous carcinoma, high nuclear grade |
| 18 | Malignant | Papillary serous cystadenocarcinomas Grade:III |
| 19 | Malignant | poorly differentiated papillary serous carcinoma |
| 20 | Malignant | Well-differentiated adnocarcinoma, Endometrioid type, Grade 1 |

The characteristics of the transcripts are summarized as follows:

**Table 13: Characteristics of Ovarian Cancer Transcripts**

| **Transcript ID** | **Junction Site** | **Gene Junction** |
|---|---|---|
| 1 | 8469:13447 | ATPase8:ND5 |
| 2 | 10744:14124 | ND4L:ND5 |
| 3 | 7974:15496 | COII:Cytb |
| 6 | 8828:14896 | ATPase6:Cytb |
| 11 | 7775:13532 | COII:ND5 |
| 12 | 8213:13991 | COII:ND5 |
| 15 | 9574:12972 | COIII:ND5 |
| 20 | 8469:13447 | ATPase8:ND5 |
| Ribosomal Protein Large PO (LRP) Housekeeper | N/A | N/A |

It is noted that transcripts 1, 2, 3, 6, 11, 12, 15 and 20 are the same as those discussed above with respect to Examples 3-7.

Homogenates were prepared using approximately 25mg of frozen tissue and diluted 1:4. The quantity of the transcripts was measured in Relative Luminenscence Units RLU on a Glomax™ Multi Detection System (Promega). All samples were assayed in triplicate for each transcript. Background measurements (no template) were done in triplicate as well. The analysis accounted for background by subtracting the lower limit from the RLU values for the samples. Input RNA was accounted for by using the formula log₂ a RLU - log₂ h RLU where a is the target fusion transcript and h is the housekeeper transcript.

The analysis of the data comprised the following steps:
a) Establish CV's (coefficients of variation) for triplicate assays; acceptable if ≤ 15%.
b) Establish average RLU value for triplicate assays of target fusion transcript(a) and housekeeper transcript (h).
c) Establish lower limit from triplicate value of background RLU (I).
d) Subtract lower limit (I) from (a).
e) Calculate log₂ a RLU - log₂ *h* RLU.

### Summary of Results:

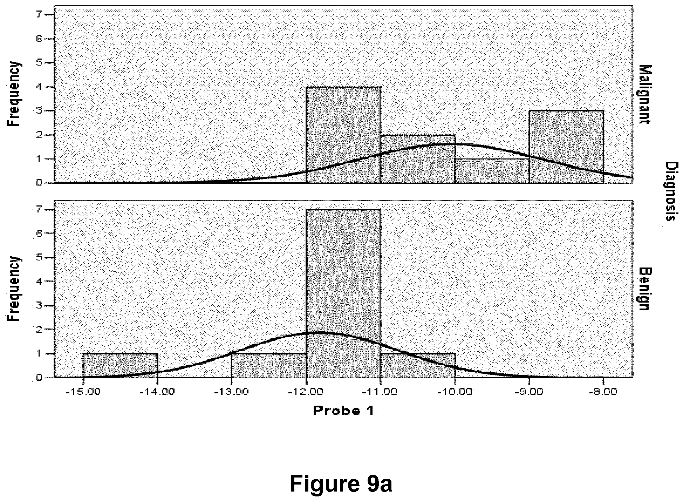

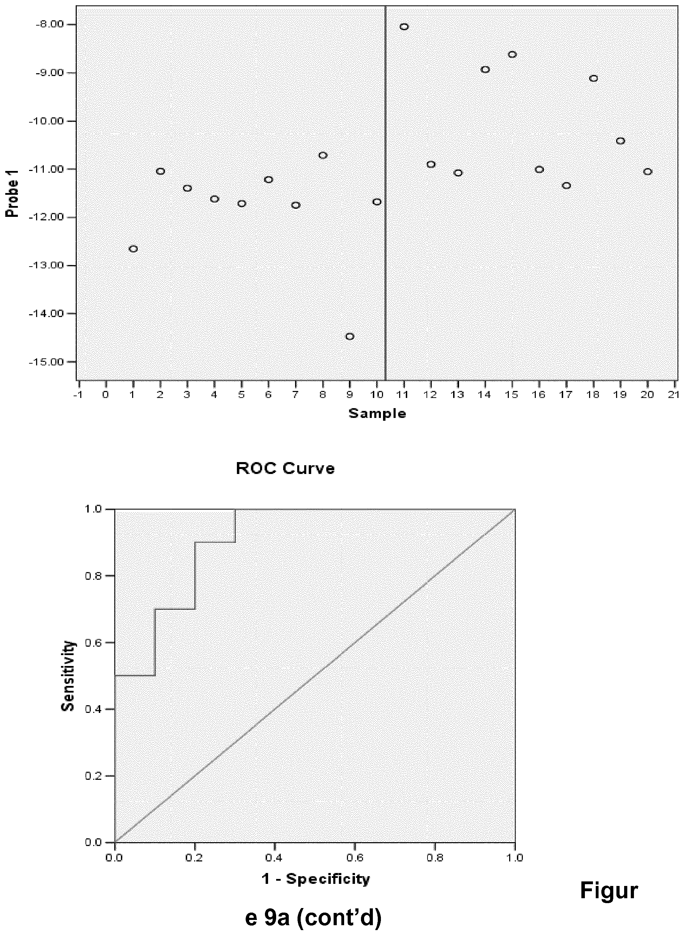

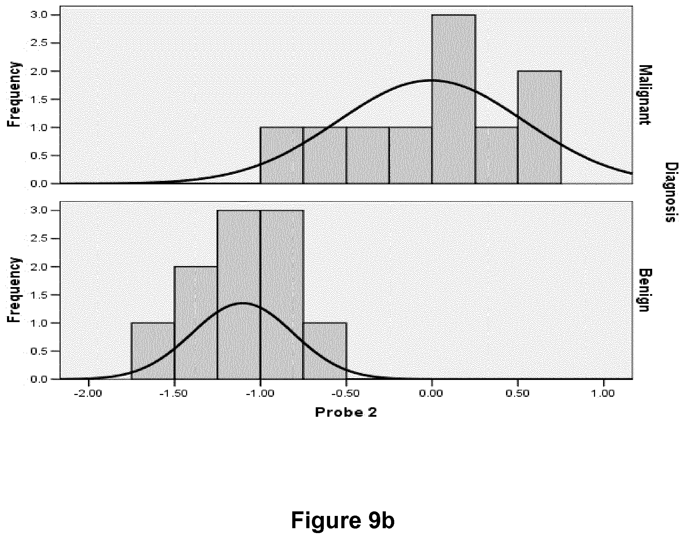

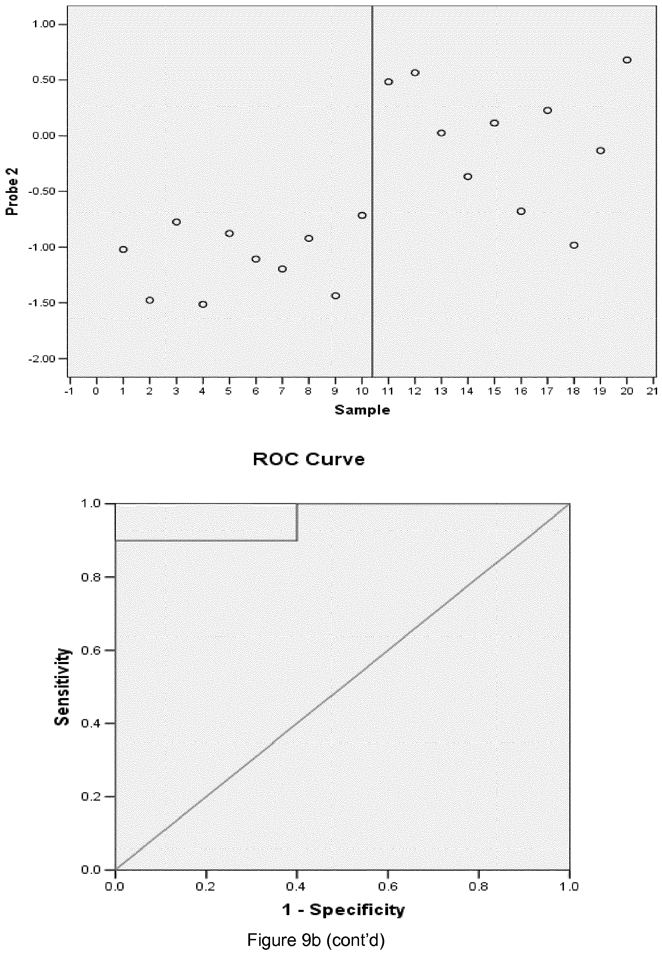

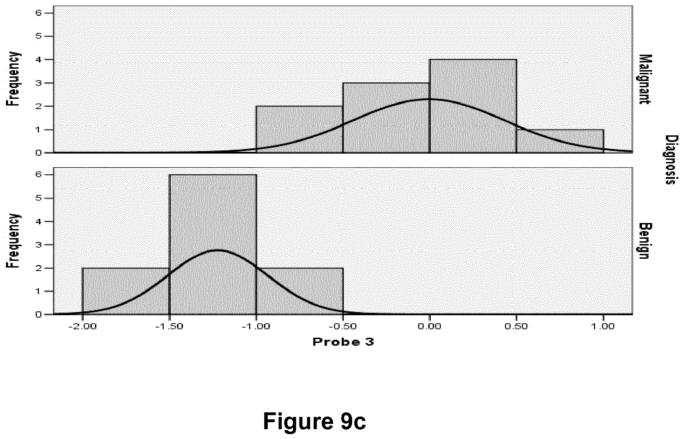

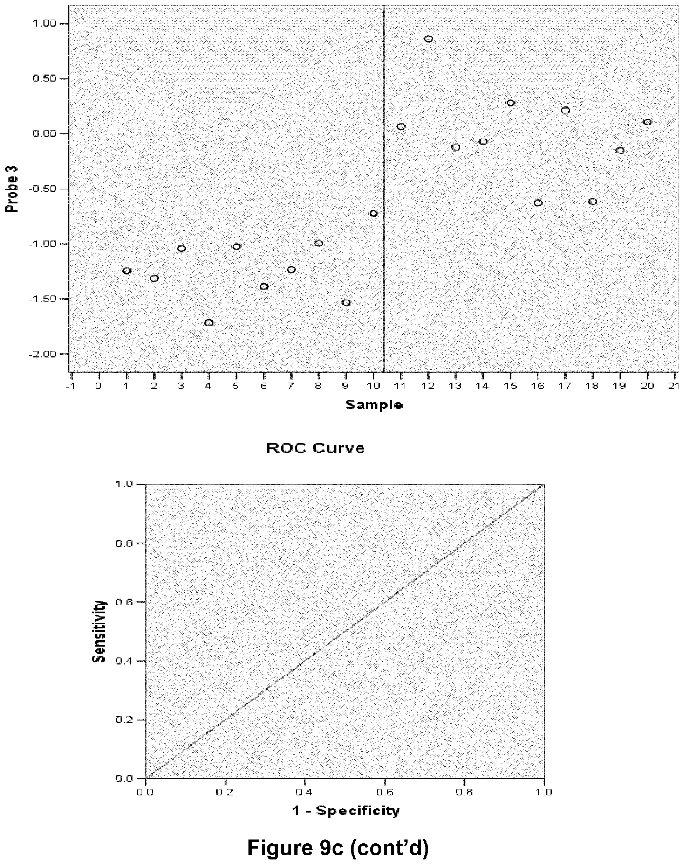

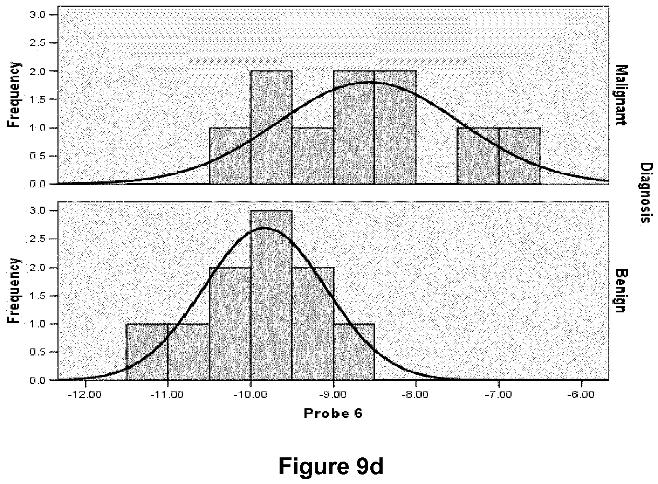

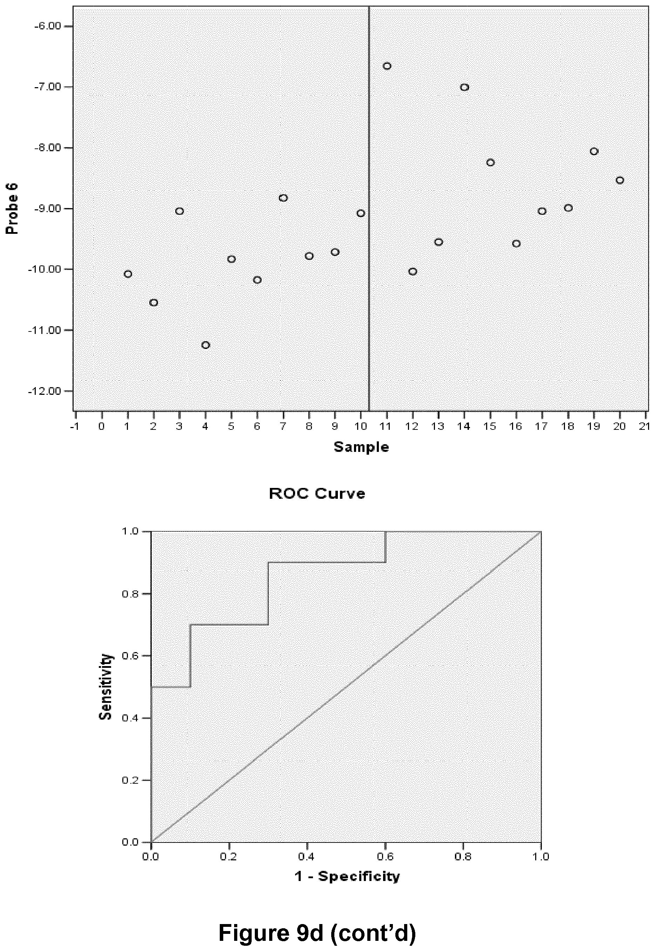

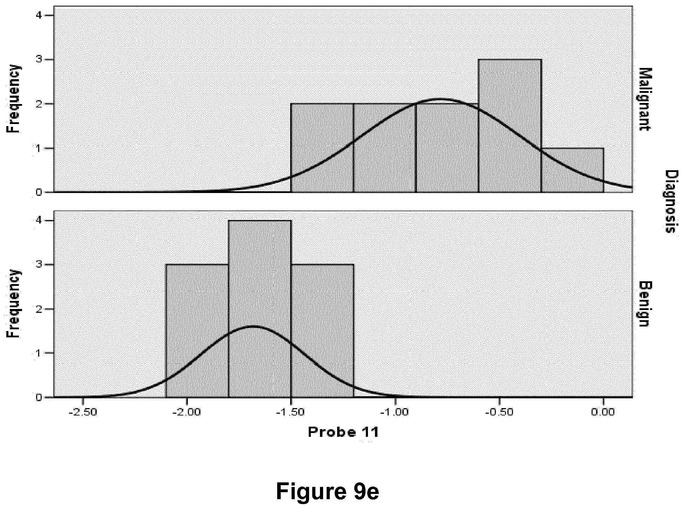

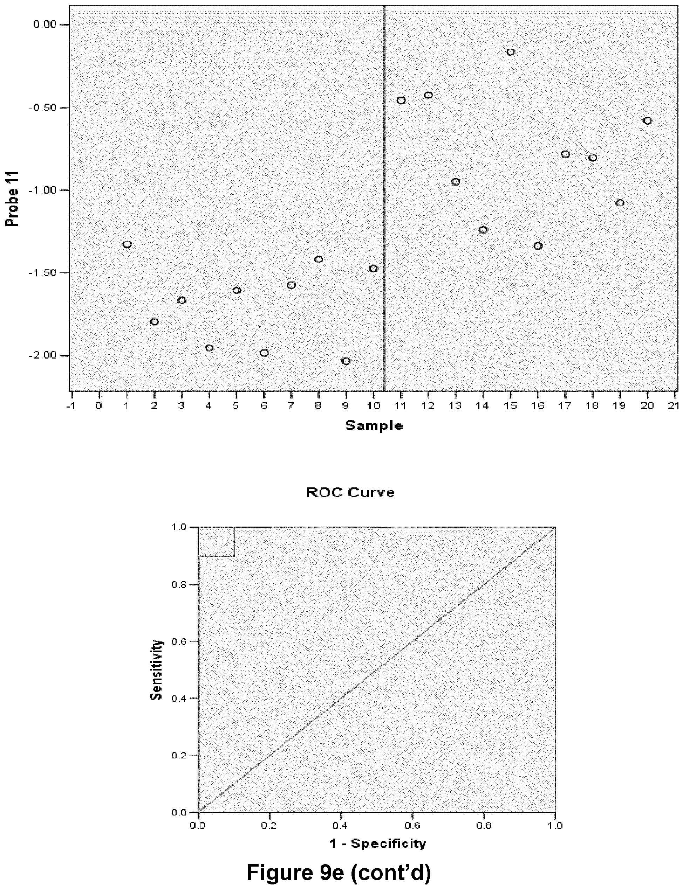

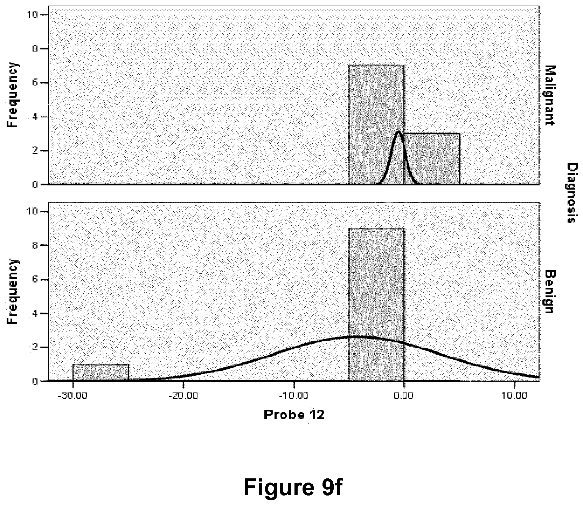

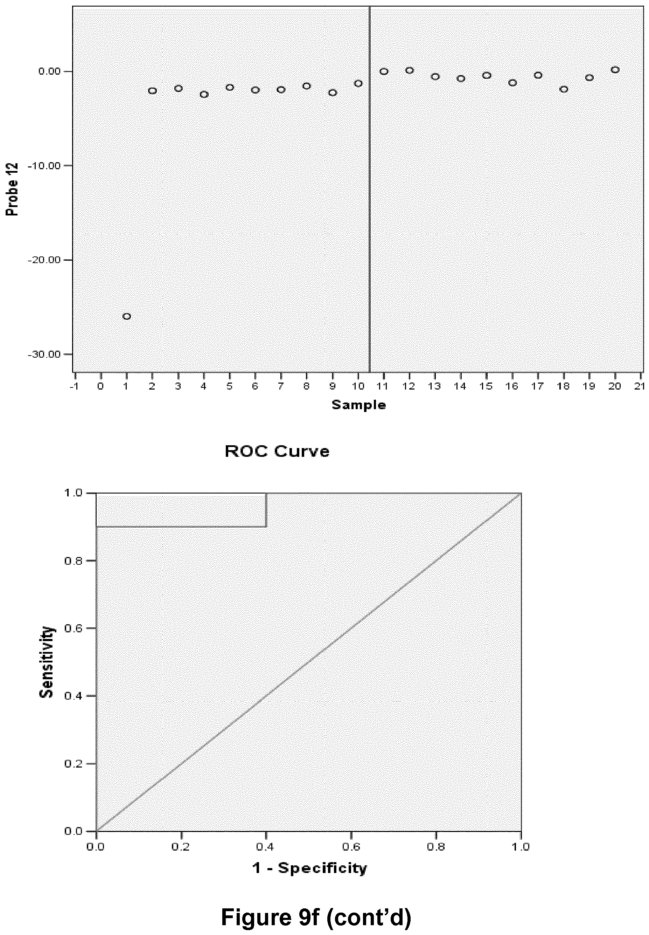

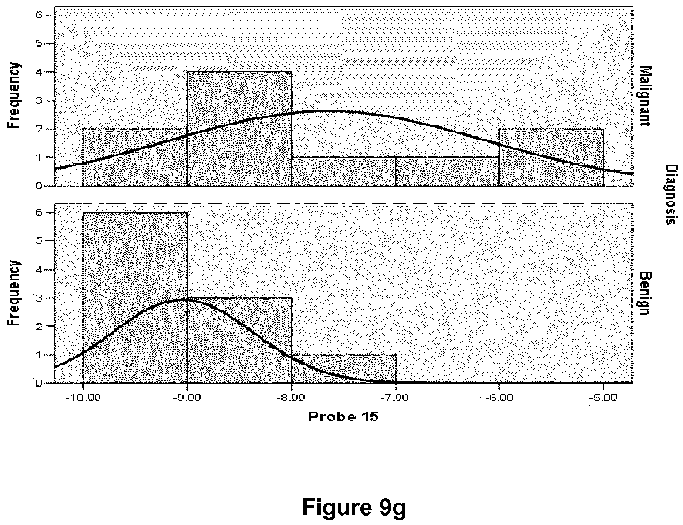

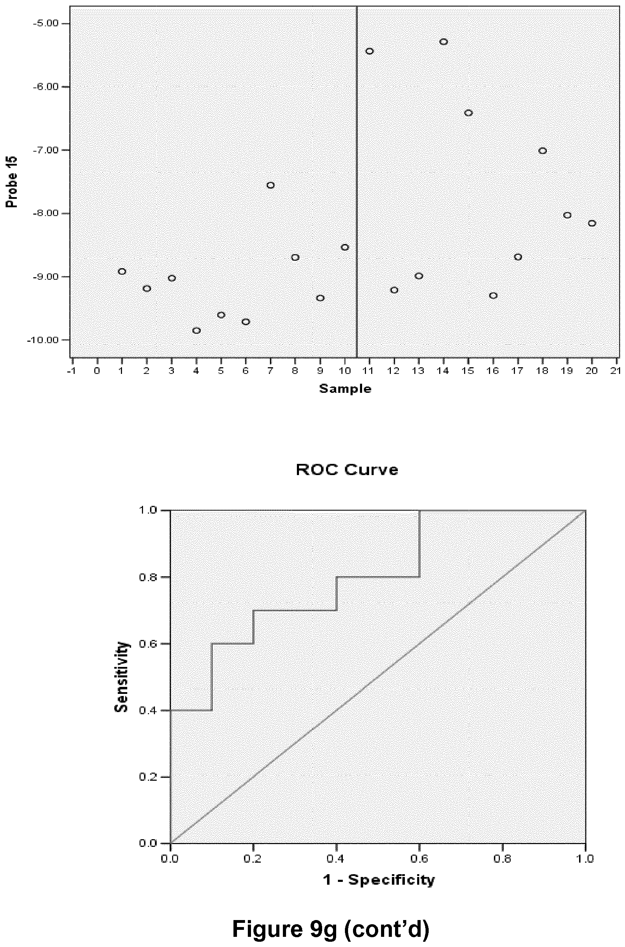

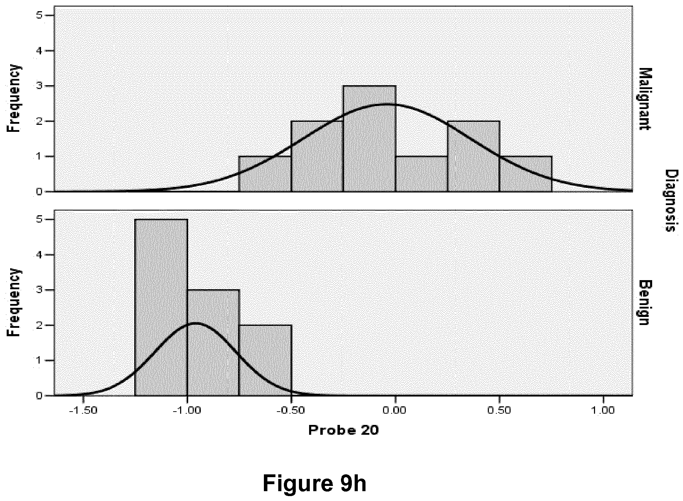

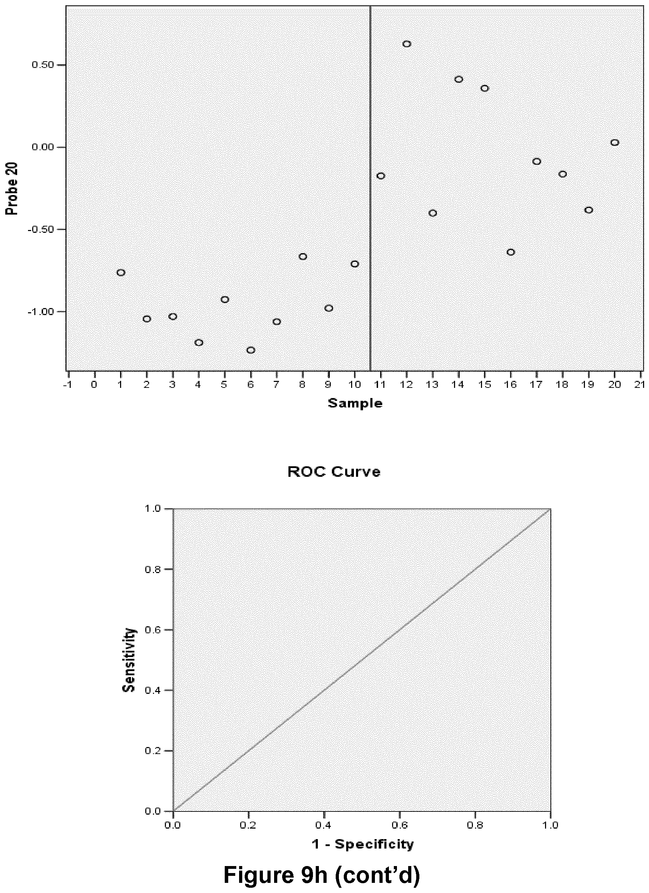

The results of the above analysis are illustrated in Figures 9a to 9h, which comprise plots of the log₂ a RLU - log₂ *h* RLU against sample number. Also illustrated are the respective ROC (Receiver Operating Characteristic) curves determined from the results for each transcript.
Transcript 1: There exists a statistically significant difference between the means (p<0.05) of the normal and malignant groups (p=0.002). Using a cutoff value of -11.1503 as demonstrated by the ROC curve results in a sensitivity of 90% and specificity of 80% and the area under the curve is 0.91 indicating very good test accuracy. The threshold value chosen may be adjusted to increase either the specificity or sensitivity of the test for a particular application.
Transcript 2: There exists a statistically significant difference between the means (p<0.01) of the normal and malignant groups (p=0.001). Using a cutoff value of 0.6962 as demonstrated by the ROC curve results in a sensitivity of 90% and specificity of 100% and the area under the curve is 0.96 indicating very good test accuracy. The threshold value chosen may be adjusted to increase either the specificity or sensitivity of the test for a particular application.
Transcript 3: There exists a statistically significant difference between the means (p<0.01) of the normal and malignant groups (p=0.000). Using a cutoff value of 0.6754 as demonstrated by the ROC curve results in a sensitivity of 100% and specificity of 100% and the area under the curve is 1.00 indicating excellent test accuracy. The threshold value chosen may be adjusted to increase either the specificity or sensitivity of the test for a particular application.
Transcript 6: There exists a statistically significant difference between the means (p<0.01) of the normal and malignant groups (p=0.007). Using a cutoff value of -9.6479 as demonstrated by the ROC curve results in a sensitivity of 90% and specificity of 70% and the area under the curve is 0.86 indicating good test accuracy. The threshold value chosen may be adjusted to increase either the specificity or sensitivity of the test for a particular application.
Transcript 11: There is a statistically significant difference between the means (p<0.01) of the normal and malignant groups (p=0.000). Using a cutoff value of -1.3794 demonstrated by the ROC curve results in a sensitivity of 100% and specificity of 90% and the area under the curve is 0.99, indicating excellent test accuracy. The threshold value chosen may be adjusted to increase either the specificity or sensitivity of the test for a particular application.
Transcript 12: There exists a statistically significant difference between the means (p<0.01) of the normal and malignant groups (p=0.001). Using a cutoff value of -1.2379 as demonstrated by the ROC curve results in a sensitivity of 90% and specificity of 100% and the area under the curve is 0.96 indicating excellent test accuracy. The threshold value chosen may be adjusted to increase either the specificity or sensitivity of the test for a particular application.
Transcript 15: There exists a statistically significant difference between the means (p<0.05) of the normal and malignant groups (p=0.023). Using a cut-off value of -8.6926 as demonstrated by the ROC curve results in a sensitivity of 70% and specificity of 80% and the area under the curve is 0.80 indicating good test accuracy. The threshold value chosen may be adjusted to increase either the specificity or sensitivity of the test for a particular application.
Transcript 20: There exists a statistically significant difference between the means (p<0.01) of the normal and malignant groups (p=0.000). Using a cut-off value of 0.6521 as demonstrated by the ROC curve results in a sensitivity of 100% and specificity of 100% and the area under the curve is 0.76 indicating fair to good test accuracy. The threshold value chosen may be adjusted to increase either the specificity or sensitivity of the test for a particular application.

### Conclusions:

The above results illustrate the utility of transcripts 1, 2, 3, 6, 11, 12, 15, and 20 in the detection of ovarian cancer and in distinguishing malignant from normal ovarian tissue. Transcripts 1, 2 and 3 were also found to have utility in the detection of prostate cancer. Transcript 6 was also found to have utility in the detection of melanoma and lung cancer. Transcript 11 was also found to have utility in the detection of melanoma skin cancer, colorectal cancer and testicular cancer. Transcript 12 was also found to have utility in the detection of colorectal cancer and testicular cancer. Transcript 15 was also found to have utility in the detection of melanoma and testicular cancer. Transcript 20 was also found to have utility in the detection of colorectal cancer, melanoma, and testicular cancer. Any of the 8 transcripts listed may be used individually or in combination as a tool for the detection or characterization of ovarian cancer in a clinical setting.

### Example 9: Application to Testicular Cancer

This study sought to determine the effectiveness of several transcripts of the invention in detecting testicular cancer. A total of 17 samples were prepared comprising eight control (benign) tissue samples (samples 1 to 8) and 9 tumour (malignant) tissue samples (samples 9 to 17), 5 of the malignant samples were non-seminomas (samples 9-13)and 4 were seminomas (samples 14-17). The samples were homogenized according to the manufacturer's recommendations (Quantigene® Sample Processing Kit for Fresh or Frozen Animal Tissues; and Quantigene 2.0 Reagent System User Manual). 10 target transcripts and one housekeeper transcript were prepared in the manner as outlined above in previous examples.

The 17 tissue samples used in this example had the following characteristics:

**Table 14: Characteristics of Testicular Cancer Samples**

| Sample | General Diagnosis | Stratified Malignant Diagnosis |
|---|---|---|
| 1 | Benign | Benign |
| 2 | Benign | Benign |
| 3 | Benign | Benign |
| 4 | Benign | Benign |
| 5 | Benign | Benign |
| 6 | Benign | Benign |
| 7 | Benign | Benign |
| 8 | Benign | Benign |
| 9 | Malignant | Non-Seminoma |
| 10 | Malignant | Non-Seminoma |
| 11 | Malignant | Non-Seminoma |
| 12 | Malignant | Non-Seminoma |
| 13 | Malignant | Non-Seminoma |
| 14 | Malignant | Seminoma |
| 15 | Malignant | Seminoma |
| 16 | Malignant | Seminoma |
| 17 | Malignant | Seminoma |

The characteristics of the transcripts are summarized as follows:

**Table 15: Characteristics of Testicular Cancer Transcripts**

| **Transcript ID** | **Junction Site** | **Gene Junction** |
|---|---|---|
| 2 | 10744:14124 | ND4L:ND5 |
| 3 | 7974:15496 | COII:Cytb |
| 4 | 7992:15730 | COII:Cytb |
| 11 | 7775:13532 | COII:ND5 |
| 12 | 8213:13991 | COII:ND5 |
| 13 | 9144:13816 | ATPase6:ND5 |
| 15 | 9574:12972 | COIII:ND5 |
| 16 | 10367:12829 | ND3:ND5 |
| 20 | 8469:13447 | ATPase8:ND5 |
| Peptidylpropyl isomerase B (PPIB) | N/A | N/A |

It is noted that transcripts 2, 3, 4, 11, 12, 15, 16 and 20 are the same as those discussed above with respect to Examples 3-8.

Homogenates were prepared using approximately 25mg of frozen tissue and diluted 1:4. The quantity of the transcripts was measured in Relative Luminenscence Units RLU on a Glomax™ Multi Detection System (Promega). All samples were assayed in triplicate for each transcript. Background measurements (no template) were done in triplicate as well. The analysis accounted for background by subtracting the lower limit from the RLU values for the samples. Input RNA was accounted for by using the formula log₂ a RLU - log₂ *h* RLU where a is the target fusion transcript and h is the housekeeper transcript.

The analysis of the data comprised the following steps:
a) Establish CV's (coefficients of variation) for triplicate assays; acceptable if ≤ 15%.
b) Establish average RLU value for triplicate assays of target fusion transcript(a) and housekeeper transcript (h).
c) Establish lower limit from triplicate value of background RLU (I).
d) Subtract lower limit (I) from (a).
e) Calculate log₂ a RLU - log₂ *h* RLU.

### Summary of Results:

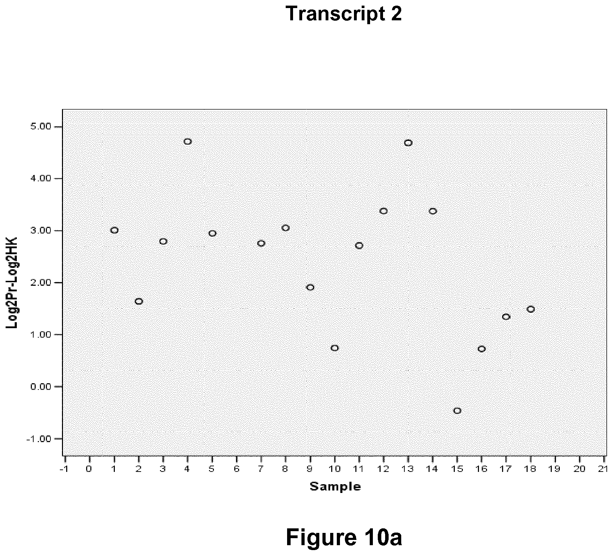

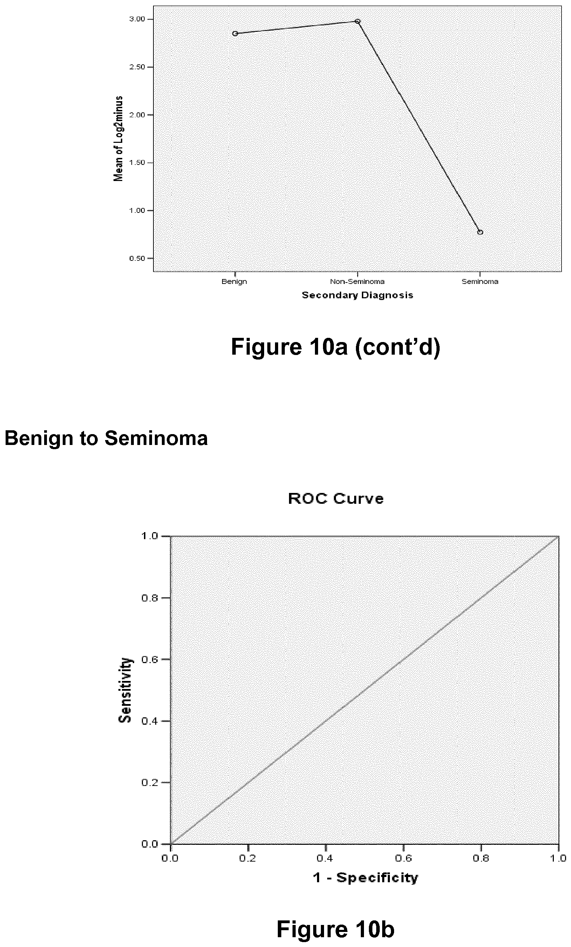

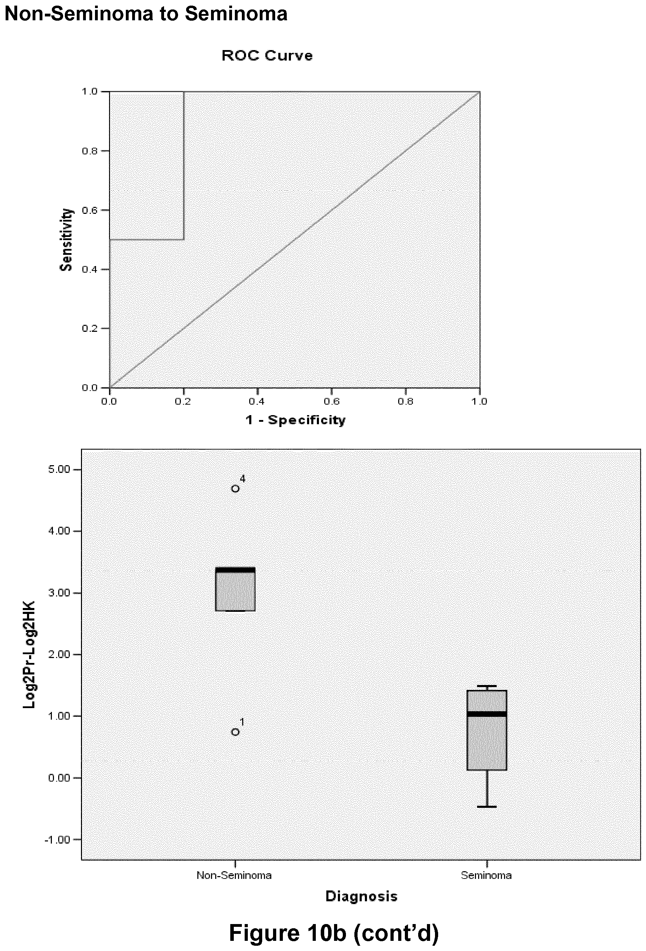

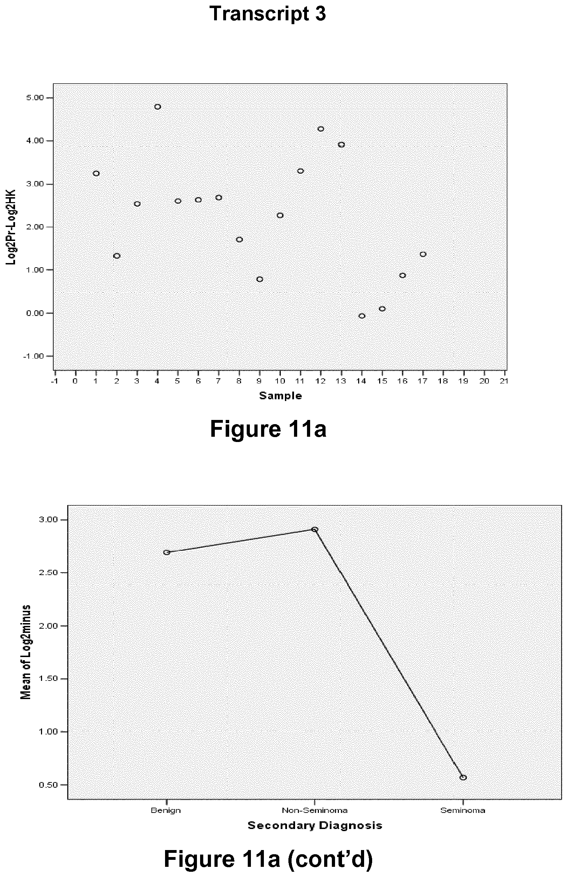

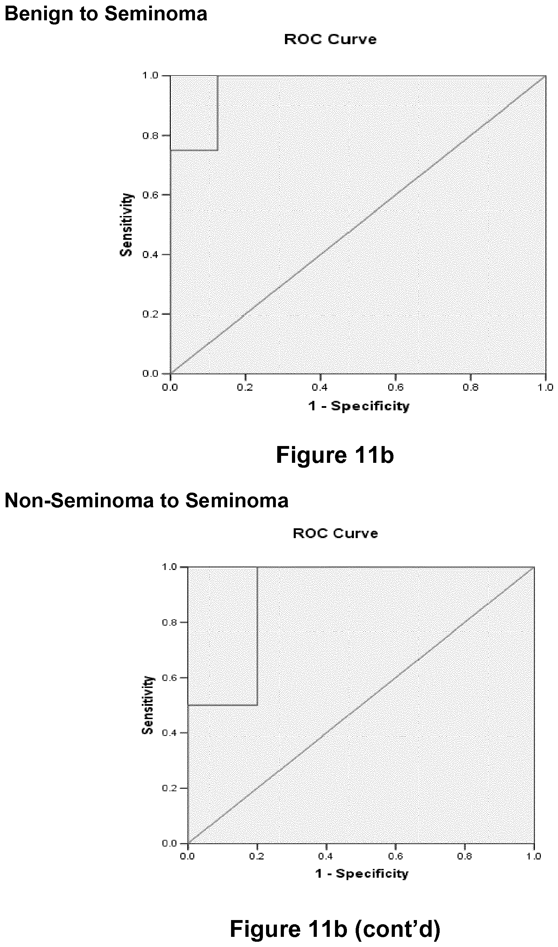

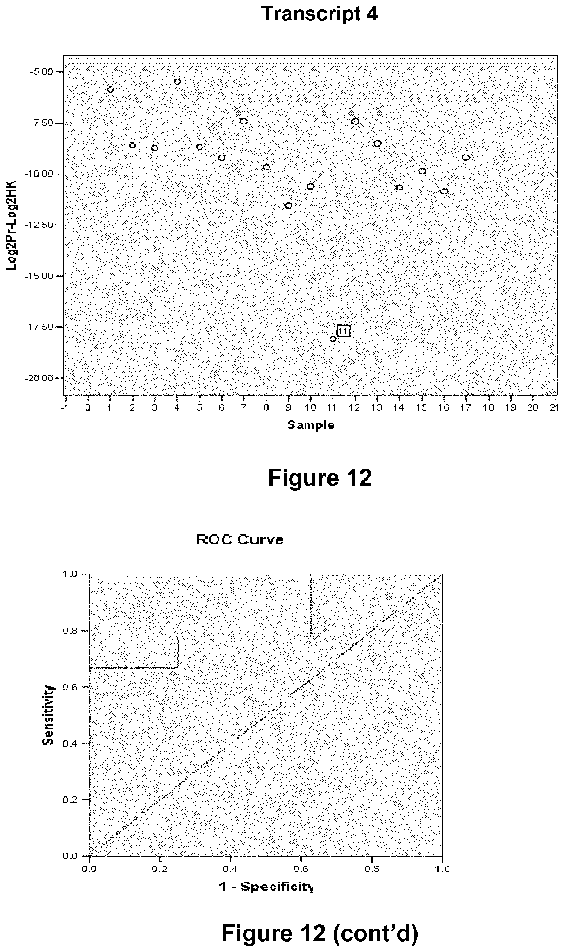

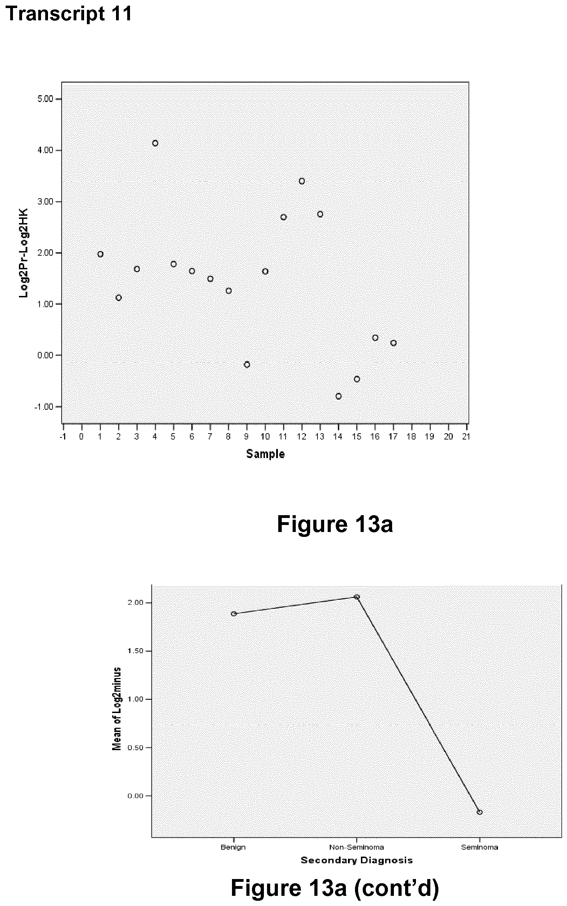

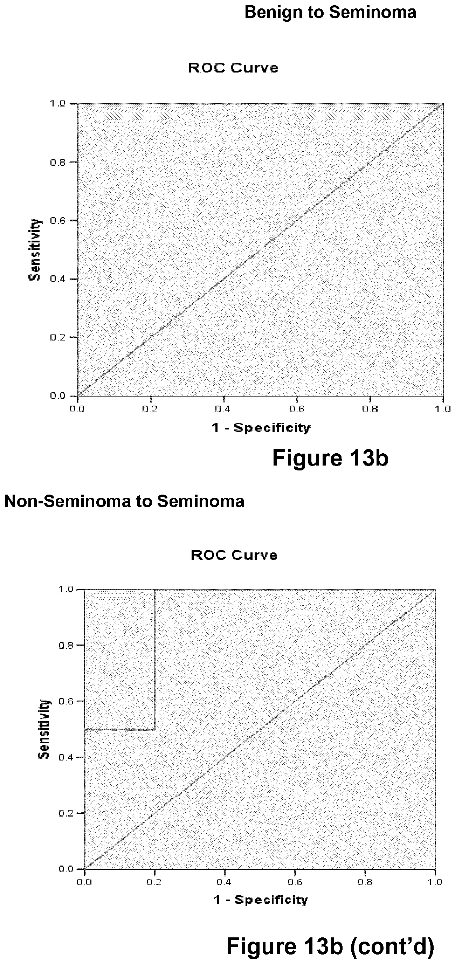

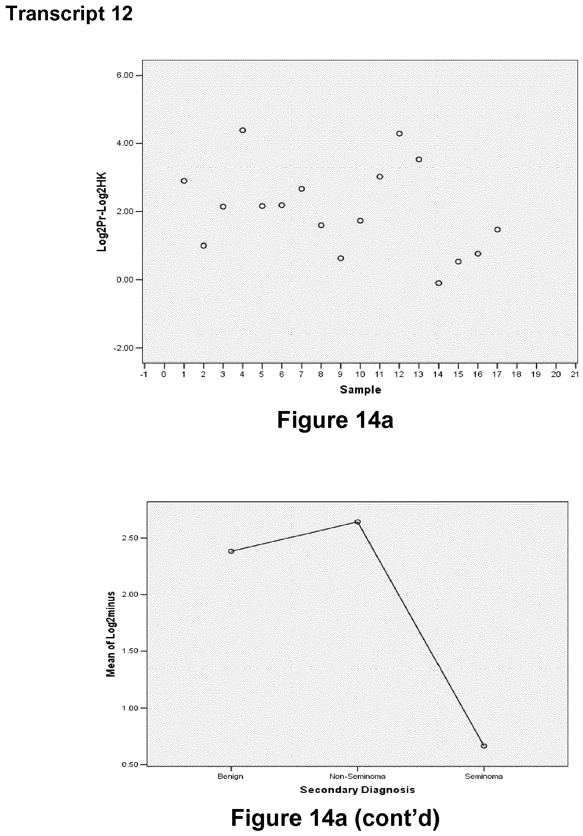

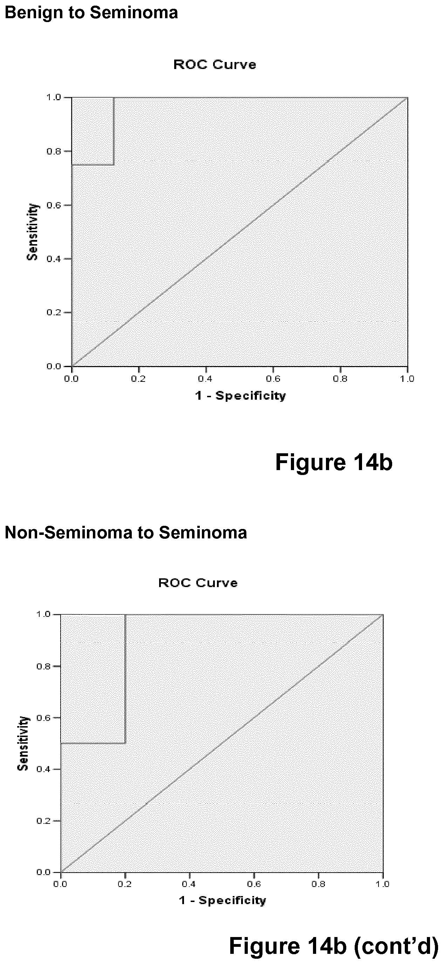

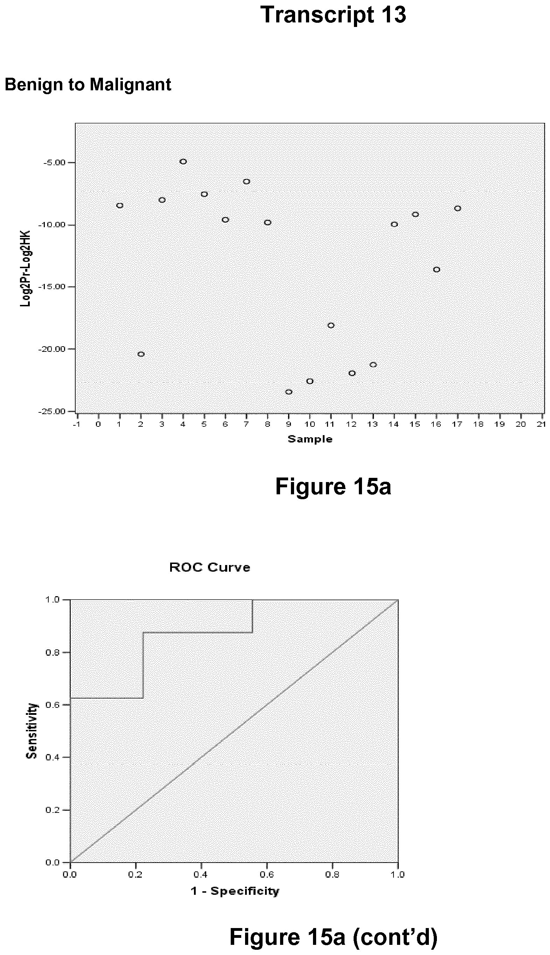

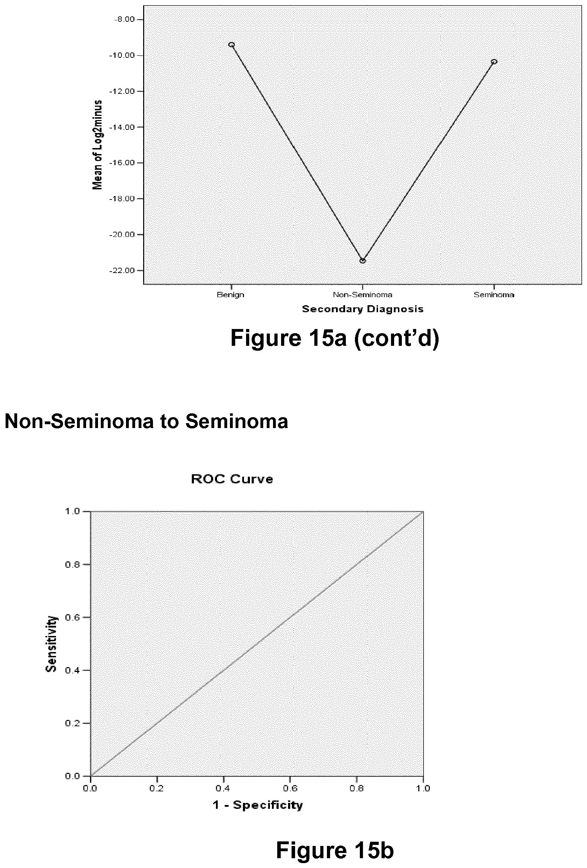

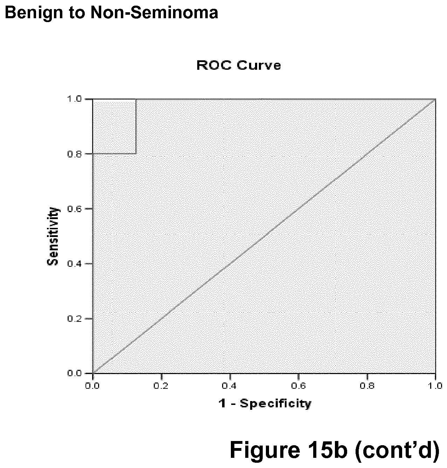

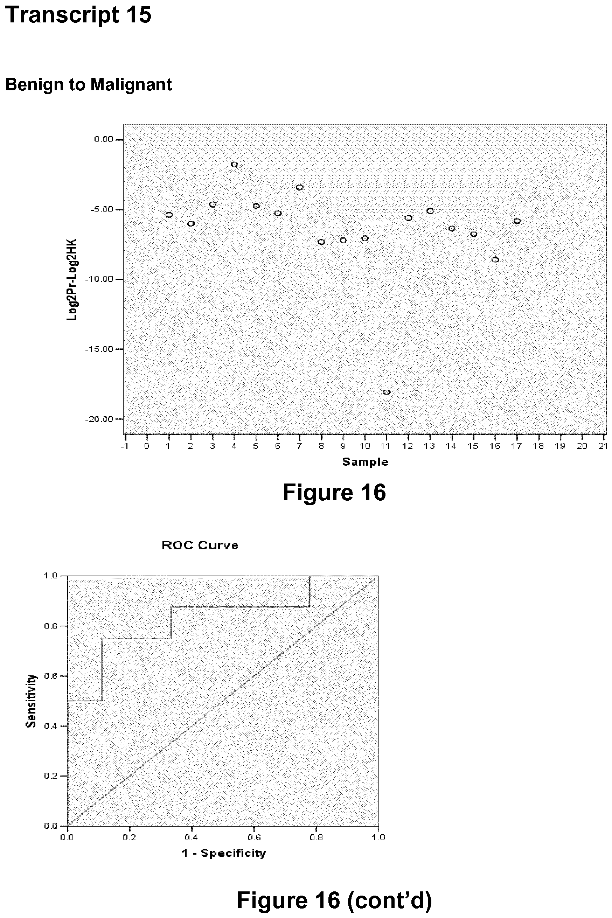

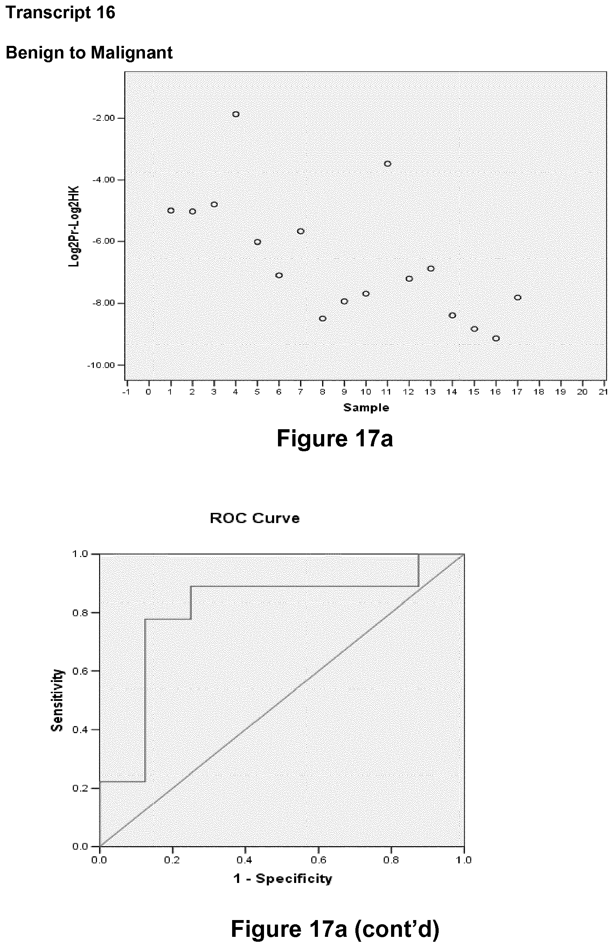

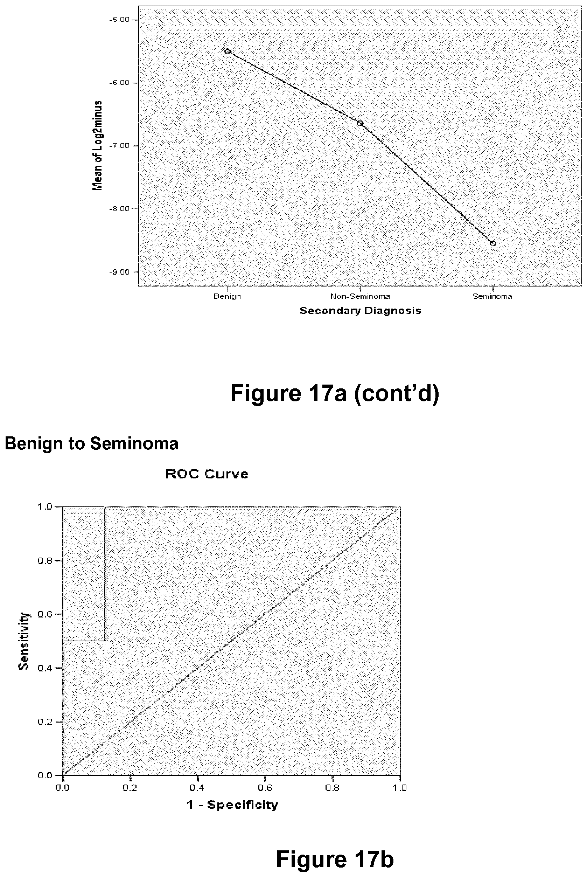

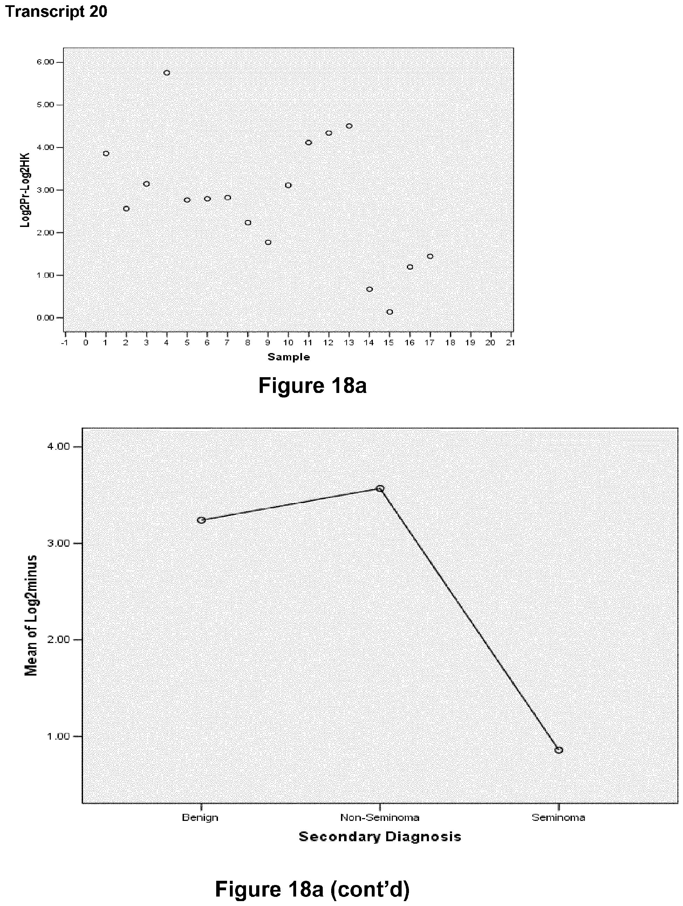

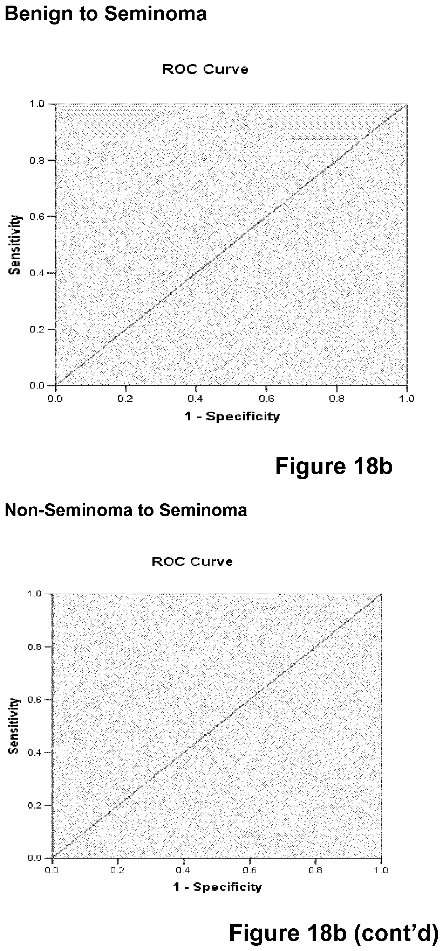

The results of the above analysis are illustrated in Figures 10 to 18, which comprise plots of the log₂ *a* RLU - log₂ *h* RLU against sample number. Also illustrated are the respective ROC (Receiver Operating Characteristic) curves determined from the results for each transcript.

While some transcripts distinguish between benign and malignant testicular tissue, others demonstrate distinction between the tumour subtypes of seminoma and non-seminoma and/or benign testicular tissue. It is therefore anticipated that combining transcripts from each class will facilitate not only detection of testicular cancer but also classification into subtype of seminoma or non-seminomas.
Transcript 2: There exists a statistically significant difference between the means (p<0.05) of the normal group and the malignant seminomas (p=0.02). Using a cutoff value of 1.5621 as demonstrated by the ROC curve results in a sensitivity of 100% and specificity of 100% and the area under the curve is 1.00 indicating excellent test accuracy. There also exists a statistically significant difference between the means (p<0.05) of the malignant seminomas and the malignant non-seminomas (p=0.024). Using a cutoff value of 2.1006 as demonstrated by the ROC curve results in a sensitivity of 100% and specificity of 80% and the area under the curve is 0.90 indicating excellent test accuracy. The threshold value chosen may be adjusted to increase either the specificity or sensitivity of the test for a particular application.
Transcript 3: There exists a statistically significant difference between the means (p<0.05) of the normal group and the malignant seminomas (p=0.018). Using a cutoff value of 0.969 as demonstrated by the ROC curve results in a sensitivity of 100% and specificity of 87.5% and the area under the curve is 0.969 indicating excellent accuracy. There also exists a statistically significant difference between the means (p<0.05) of the malignant seminomas and the malignant non-seminomas (p=0.017). Using a cutoff value of 1.8181 as demonstrated by the ROC curve results in a sensitivity of 100% and specificity of 80% and the area under the curve is 0.9 indicating excellent test accuracy. The threshold value chosen may be adjusted to increase either the specificity or sensitivity of the test for a particular application.
Transcript 4: There exists a statistically significant difference between the means (p<0.05) of the normal and malignant groups (p=0.034). Using a cutoff value of -9.7628 as demonstrated by the ROC curve results in a sensitivity of 67% and specificity of 100% and the area under the curve is 0.833 indicating good test accuracy. The threshold value chosen may be adjusted to increase either the specificity or sensitivity of the test for a particular application.
Transcript 11: There exists a statistically significant difference between the means (p<0.05) of the normal group and the malignant seminomas (p=0.016). Using a cutoff value of 0.732 as demonstrated by the ROC curve results in a sensitivity of 100% and specificity of 100% and the area under the curve is 1.00 indicating excellent test accuracy. There also exists a statistically significant difference between the means (p<0.05) of the malignant seminomas and the malignant non-seminomas (p=0.016). Using a cutoff value of 0.9884 as demonstrated by the ROC curve results in a sensitivity of 100% and specificity of 80% and the area under the curve is 0.90 indicating excellent test accuracy. The threshold value chosen may be adjusted to increase either the specificity or sensitivity of the test for a particular application.
Transcript 12: There exists a statistically significant difference between the means (p<0.1) of the normal group and the malignant seminomas (p=0.056). Using a cutoff value of 1.5361 as demonstrated by the ROC curve results in a sensitivity of 100% and specificity of 87.5% and the area under the curve is 0.969 indicating excellent test accuracy. There also exists a statistically significant difference between the means (p<0.05) of the malignant seminomas and the malignant non-seminomas (p=0.044). Using a cutoff value of 1.6039 as demonstrated by the ROC curve results in a sensitivity of 100% and specificity of 80% and the area under the curve is 0.9 indicating excellent test accuracy. The threshold value chosen may be adjusted to increase either the specificity or sensitivity of the test for a particular application.
Transcript 13: There exists a statistically significant difference between the means (p<0.05) of the normal group and the malignant group (p=0.019). Using a cutoff value of -9.8751 as demonstrated by the ROC curve results in a sensitivity of 87.5% and specificity of 78% and the area under the curve is 0.875 indicating very good test accuracy. There also exists a statistically significant difference between the means (p<0.01) of the malignant non-seminomas and the benign group (p=0.000). Using a cutoff value of-13.9519 as demonstrated by the ROC curve results in a sensitivity of 100% and specificity of 87.5% and the area under the curve is 0.975 indicating excellent test accuracy. There also exists a statistically significant difference between the means (p<0.01) of the malignant seminomas and the malignant non-seminomas (p=0.001). Using a cutoff value of -15.8501 as demonstrated by the ROC curve results in a sensitivity of 100% and specificity of 100% and the area under the curve is 1.00 indicating excellent test accuracy. The threshold value chosen may be adjusted to increase either the specificity or sensitivity of the test for a particular application.
Transcript 15: There exists a statistically significant difference between the means (p<0.1) of the normal and malignant groups (p=0.065). Using a cut-off value of -5.4916 as demonstrated by the ROC curve results in a sensitivity of 75% and specificity of 89% and the area under the curve is 0.833 indicating good test accuracy. The threshold value chosen may be adjusted to increase either the specificity or sensitivity of the test for a particular application.
Transcript 16: There exists a statistically significant difference between the means (p<0.05) of the normal and malignant groups including both seminomas and non-seminomas (p=0.037). Using a cut-off value of -6.448 as demonstrated by the ROC curve results in a sensitivity of 89% and specificity of 75% and the area under the curve is 0.806 indicating good test accuracy. There also exists a statistically significant difference between the means (p<0.05) of the normal and malignant seminomas (p=0.037). Using a cut-off value of -7.4575 as demonstrated by the ROC curve results in a sensitivity of 100% and specificity of 87.5% and the area under the curve is 0.938 indicating excellent test accuracy. The threshold value chosen may be adjusted to increase either the specificity or sensitivity of the test for a particular application.
Transcript 20: There exists a statistically significant difference between the means (p<0.01) of the normal group and the malignant seminomas (p=0.006). Using a cutoff value of 1.8364 as demonstrated by the ROC curve results in a sensitivity of 100% and specificity of 100% and the area under the curve is 1.00 indicating excellent test accuracy. There also exists a statistically significant difference between the means (p<0.01) of the malignant seminomas and the malignant non-seminomas (p=0.004). Using a cutoff value of 1.6065 as demonstrated by the ROC curve results in a sensitivity of 100% and specificity of 100% and the area under the curve is 1.00 indicating excellent test accuracy. The threshold value chosen may be adjusted to increase either the specificity or sensitivity of the test for a particular application.

### Conclusions:

The above results illustrate the utility of transcripts 2, 3, 4, 11, 12, 13, 15, 16, and 20 in the detection of testicular cancer, and testicular cancer subtypes, and in distinguishing malignant from normal testicular tissue. Transcript 2 was also found to have utility in the detection of prostate, breast, colorectal and ovarian cancer. Transcript 3 was also found to have utility in the detection of prostate, breast, melanoma, colorectal, and ovarian cancers. Transcript 4 was also found to have utility in the detection of prostate and colorectal cancers. Transcript 11 was also found to have utility in the detection of colorectal, melanoma, and ovarian cancers. Transcript 12 was also found to have utility in the detection of colorectal and ovarian cancers. Transcript 15 was also found to have utility in the detection of melanoma and ovarian cancers. Transcript 16 was also found to have utility in the detection of melanoma skin cancer. Transcript 20 was also found to have utility in the detection of colorectal cancer, melanoma, and ovarian cancer. Any of the 9 transcripts listed may be used individually or in combination as a tool for the detection or characterization of ovarian cancer in a clinical setting.

In one aspect, the invention provides a kit for conducting an assay for determining the presence of cancer in a tissue sample. The kit includes the required reagents for conducting the assay as described above. In particular, the kit includes one or more containers containing one or more hybridization probes corresponding to transcripts 1 to 17, and 20 described above. As will be understood, the reagents for conducting the assay may include any necessary buffers, salts, detection reagents etc. Further, the kit may include any necessary sample collection devices, containers etc. for obtaining the needed tissue samples, reagents or materials to prepare the tissue samples for example by homogenization or nucleic acid extraction, and for conducting the subject assay or assays. The kit may also include control tissues or samples to establish or validate acceptable values for diseased or non-diseased tissues.

### Example 10: Detection of Fusion Protein

### Cell lines

The presence of fusion proteins was investigated in two human prostate cell lines. Firstly the normal prostate cell line RWPE-1 (ATCC Cat# CRL-11609), these cells are non tumourigenic in nude mice and were established by infection with human papilloma virus 18 of histologically normal adult human prostate cells. Secondly a tumorigenic cell line WPE1-NA22 were examined (ATCC Cat# CRL-2849). These cells were derived from the RWPE-1 cells following exposure to N-methyl-N-nitrosourea. These cells are tumourogenic in nude mice unlike it's parent cell line RWPE-1.

Both cell lines were grown in Keratinicyte Serum Free Medium (Invitrogen Cat#17005-042), medium is supplemented with bovine pituitary extract and human recombinant epidermal growth factor. Cells were grown to 90% confluence then trypsinised using TrypLE Select (Invitrogen Cat#12563029). Cells were then counted using an automated counting system (Invitrogen Countess Cat#C10227), aliquots were then snap frozen and stored at -80°C.

### Protein Extraction

Cell fractions were extracted from both RWPE1 and WPE1-NA22 cell lines using the Qproteome Mitochondria Isolation Kit (Qiagen Cat#37612). Both mitochondrial and cytoplasmic fractions were extracted from 1x10⁷ cells. Protein concentration was then calculated using a fluorescent protein assay (Quant-IT Protein, Invitrogen Cat#Q33211) measured on a Qubit fluorometer (Invitrogen Cat#Q32857).

### SDS-Page Gel electrophoresis

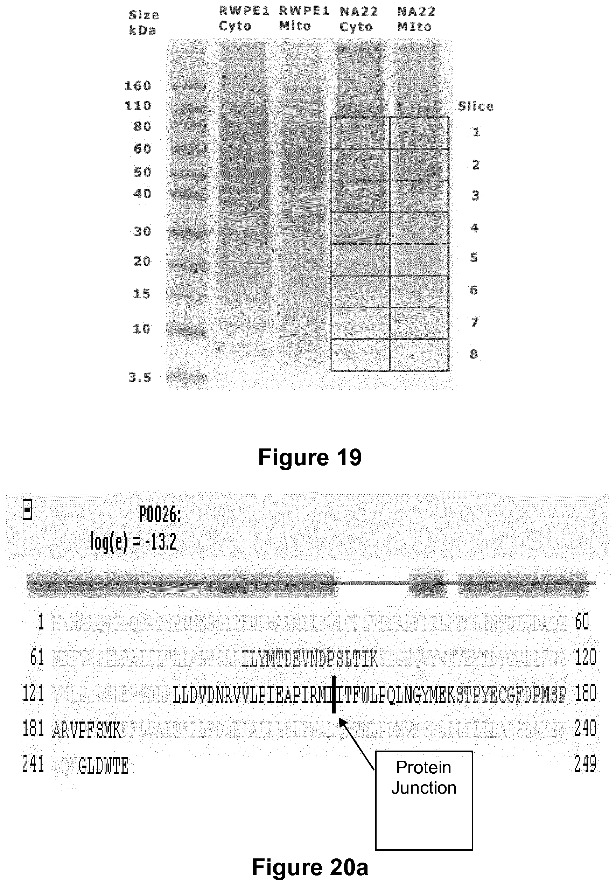

SDS-Page electrophoresis was carried out on mitochondrial and cytosolic fractions prepared using the Qproteome mitochondrial isolation kit. 20µg of protein was run in each lane on a 4-12% precast (invitrogen Nupage Cat#NP0321) bis-tris gel reducing gel, using a MES running buffer (Invitrogen Cat# NP00020). The gel was stained overnight with colloidal blue gel stain (Invitrogen Cat# LC6025). The results are illustrated in Figure 19. The approximate size (kD) range of proteins predicted to be contained in each of the 8 gel slices illustrated in Figure 19 are as follows:

| | |
|---|---|
| 1 | 60-80 |
| 2 | 50-60 |
| 3 | 40-50 |
| 4 | 30-40 |
| 5 | 20-30 |
| 6 | 15-20 |
| 7 | 10-15 |
| 8 | 3.5-10 |

### LCMS

Eight gel slices were cut out from each lane of a colloidal blue stained 1D SDS-PAGE (Figure 19) and in gel digested with trypsin following standard procedures.

The digestion products were eluted form the gel and evaporated. An aliquot was injected onto an LCMS system (Dionex / LC Packings Ultimate3000 coupled online to a Thermo LTQ XL orbitrap) and separated on a 25 cm (75 um ID) PepMap (Dionex) column at a flow rate of 300 ml/min with formic acid as a ion pairing agent and a linear gradient starting at 5% MeCN going to 40% MeCN over 110 min. MS spectra were collected in the orbitrap at a resolution of 60000 (400 Da) and MSMS spectra in the linear ion trap at low resolution.

Data were processed using Thermo Proteome Discoverer to generate .mgf (mascot generic format) peak list files, which were submitted in house to X!Tandem, searching a custom database comprised of the human proteome (ensembl) and predicted fusion proteins based on the fusion transcripts described previously. To calculate a false discovery rate (FDR), the searched database also included the reverse sequence of all proteins.

### Protein Complexes Analysis

Upon completion of the X!Tandem custom database search all identified proteins and fusion transcripts were returned. The proteins were scored by their log(e)⁺ values and classified as significant when the log(e)⁺ was less than negative one, with preference given to proteins with a log(e)⁺ less than negative three. Fusion proteins were identified by the presence of at least one peptide from each of the contributing genes of the fusion transcript present in the same gel slice. Protein sequence coverage from the identified peptides by the LC/MS-MS are displayed in red. The sequence of the protein which may be difficult to observe a peptide due to experimental conditions are indicated in green. Finally, protein sequence that is displayed in black represents a neutral possibility of identifying a peptide.

### Examples of Identified Fusion Proteins

Many mitochondrial fusion proteins were identified using this methodology. Four of such fusion proteins are described below as representative examples.

### Example Fusion Protein 1

Figures 20a illustrates the amino acid sequence of the fusion protein corresponding to the fusion transcript identified as P0026, which was identified (log(e)⁺ = -13.2) in slice 7 of the mitochondrial NA22 cell line (Figure 19) from the presence of the Cytochrome c oxidase subunit 2 (CO2) N-terminus peptide ILYMTDEVNDPSLTIK and the NADH-ubiquinone oxidoreductase chain 3 (ND3) C-terminus peptide STPYECGFDPMSP (Figure 20a).

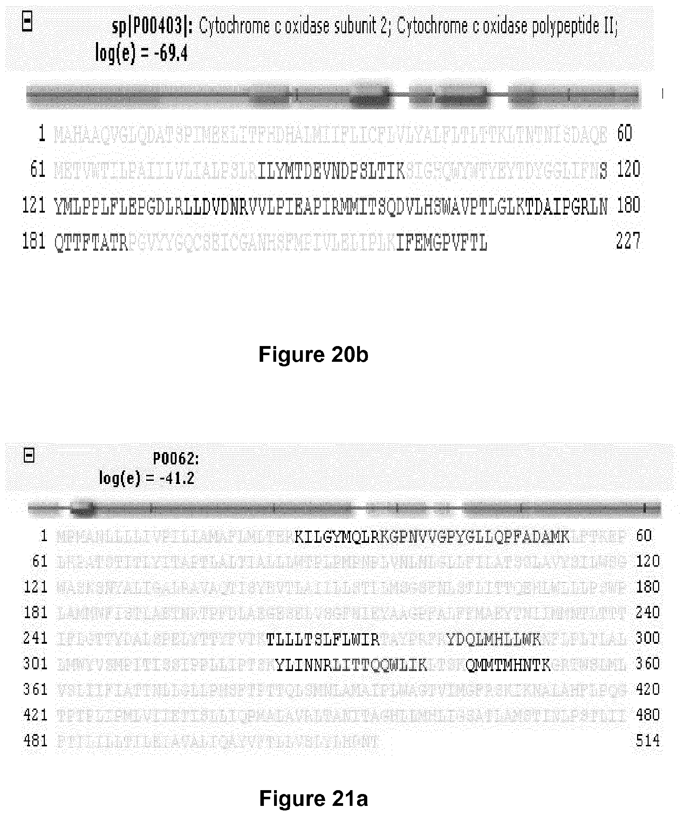

The most C-terminus tryptic peptide of wild-type CO2, IFEMGPVFTL, was searched against all mitochondrial NA22 cell line gel slices .xml data. This peptide was only observed in gel slice 5 (Figure 19). This was further confirmed by identifying CO2 wild-type (log(e)⁺ = -42.9) (Figure 20b) in mitochondrial NA22 cell line gel slice 5 only after searching the Human (SwissProt) database (no fusion transcripts) with all gel slices.

Cytochrome c oxidase subunit 2 peptide ILYMTDEVNDPSLTIK was observed in gel slices 5 and 7. This indicates that wild type CO2, with a molecular weight of ∼25kDa is present in the 20-30kDa gel slice 5, and a fragment of CO2 N-terminus exists in gel slice 7. The tryptic peptide STPYECGFDPMSP from ND3 is only identified in gel slice 7 (10-15kDa), which identifies the wild-type gene (13kDa) and the C-terminus of P0026.

The sequences for the fusion transcript P0026, the mutant DNA from which it is derived and the resulting protein are provided herein, respectively, as SEQ ID NO: 56, SEQ ID NO: 57 and SEQ ID NO: 58.

### Example Fusion Protein 2

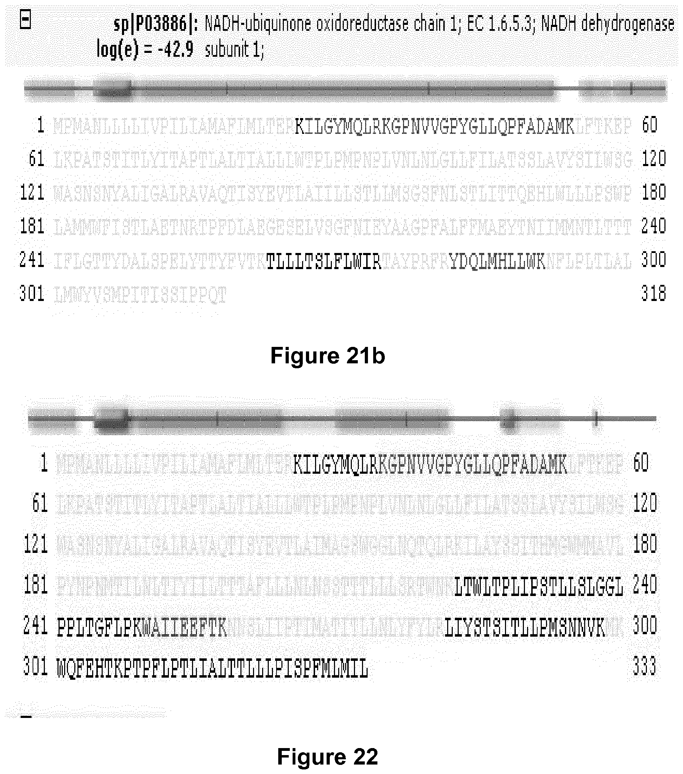

Figure 21a illustrates the amino acid sequence of the fusion protein corresponding to fusion transcript P0062, which was identified (log(e)⁺ = -41.2) in slice 5 (20-30kDa), shown in Figure 19, of the mitochondrial NA22 cell line from the presence of the NADH dehydrogenase subunit 1 (ND1) N-terminus peptides KGPNVVGPYGLLQPFADAMK and YDQLMHLLWK and the ATP synthase subunit 6 C-terminus peptide LITTQQWLIK. All three peptides were identified in the mitochondrial NA22 cell line gel slice 5 (Figure 19) but due to the most C-terminus peptide of ND1 (YDQLMHLLWK) being present only in gel slice 5, the presence of both wild-type (Figure 21b) and the fusion protein corresponding to fusion transcript P0062 is possible.

The sequences for the fusion transcript P0062, the mutant DNA from which it is derived and the resulting protein are provided herein, respectively, as SEQ ID NO: 59, SEQ ID NO: 60 and SEQ ID NO: 61.

### Example Fusion Protein 3

Figure 22 illustrates the amino acid sequence of the fusion protein corresponding to fusion transcript P0064, which was identified in slice 4 (log(e)+ = -22), shown in Figure 19, of the mitochondrial NA22 cell line with the peptide KGPNVVGPYGLLQPFADAMK from the N-terminus of ND1 and the NADH dehydrogenase subunit 2 (ND2) C-terminus peptide WAIIEEFTK. The ND1 C-terminus peptide YDQLMHLLWK was not observed in gel slice 4, and based on the expected sizes of P0064 and ND2 it is suggested that gel slice 4 contains P0064 and ND2.

The sequences for the fusion transcript P0064, the mutant DNA from which it is derived and the resulting protein are provided herein, respectively, as SEQ ID NO: 62, SEQ ID NO: 63 and SEQ ID NO: 64.

### Example Fusion Protein 4

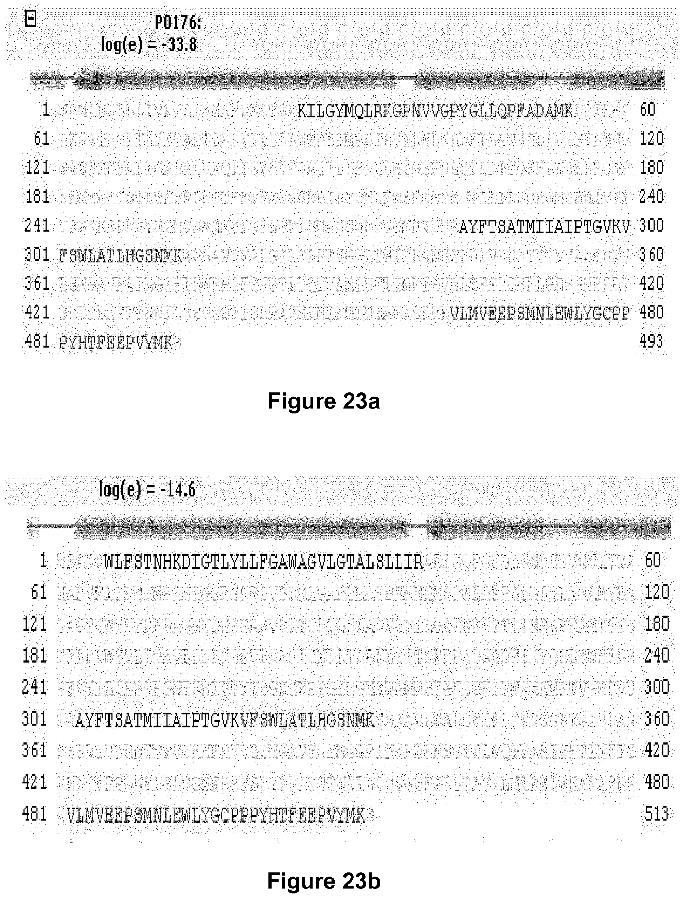

Figure 23a illustrates the amino acid sequence of the fusion protein corresponding to fusion transcript P0176, which was identified in slice 4 (log(e)+ = -33.8), shown in Figure 19, of the mitochondrial NA22 cell line with the peptide KGPNVVGPYGLLQPFADAMK from the N-terminus of ND1 and the Cytochrome c oxidase subunit 1 (CO1) C-terminus peptides VFSWLATLHGSNMK and VLMVEEPSMNLEWLYGCPPPYHTFEEPVYMK. Both of the CO1 peptides were only observed together in gel slice 4 (30-40kDa) of the mitochondrial NA22 cell line despite an expected size of 55 kDa. This was further confirmed by identifying CO1 wild-type (log(e)⁺ = -14.6) (Figure 23b) in mitochondrial NA22 cell line gel slice 4 only after searching the Human (SwissProt) database (no fusion transcripts) with all gel slices.

The only ND1 peptide observed in gel slice 4 was KGPNVVGPYGLLQPFADAMK. Since the ND1 C-terminus peptide YDQLMHLLWK was not present, wild-type ND1 is not present in the slice, which supports the presence of P0176.

The sequences for the fusion transcript P0176, the mutant DNA from which it is derived and the resulting protein are provided herein, respectively, as SEQ ID NO: 65, SEQ ID NO: 66 and SEQ ID NO: 67.

### Corresponding Fusion Transcripts

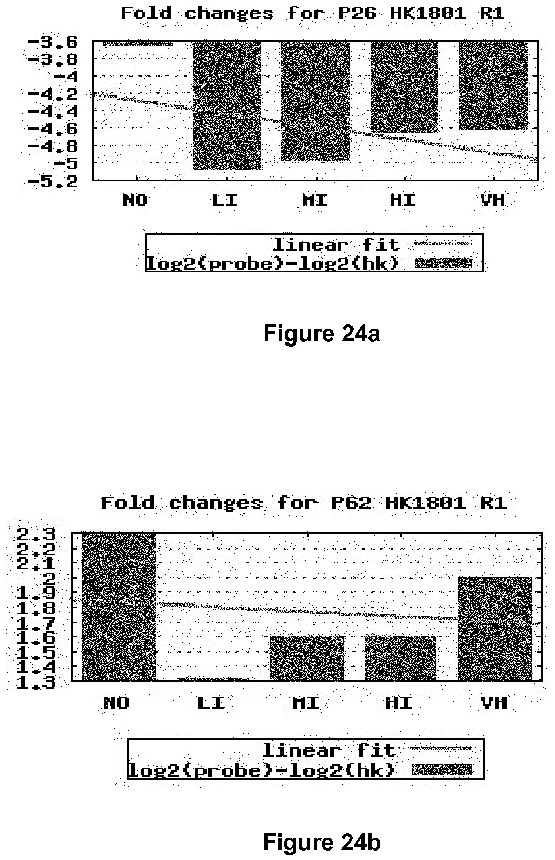

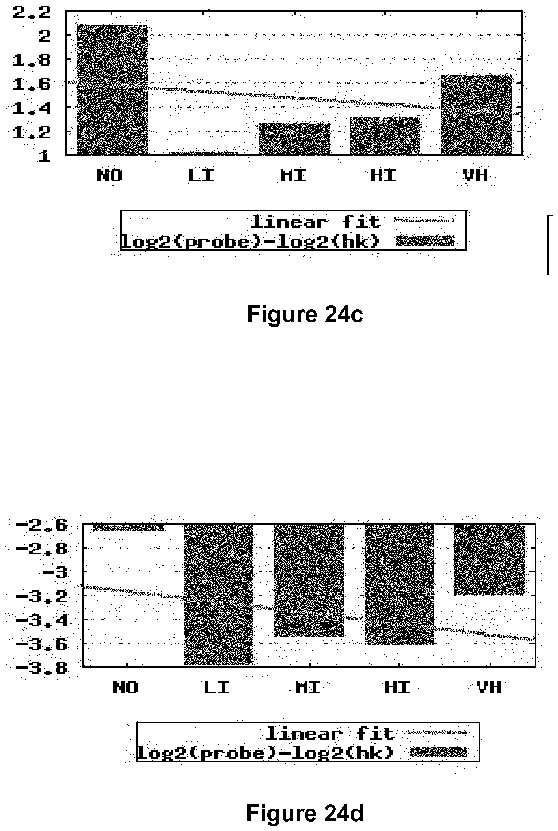

Quantitative measurements of the fusion transcripts associated with each of these four fusion proteins were conducted in a series of cell lines of which two were those used in the LC-MS/MS experiment, specifically RWPE-1 and WPE1-NA22, which is a malignant cell line with low invasive potential. The results of these measurements is illustrated in Figures 24a-24d, corresponding to the four proteins discussed above. In Figures 24a-d, cell line RWPE-1 is indicated as NO and cell line WPE1-NA22 is indicated as LI. The additional cell lines included in this experiment represent a continued progression of malignancy with moderate invasive potential (MI), high invasive potential (HI), and very high invasive potential (VH).

The cells were lysed and assayed using custom probes specific to each of the fusion transcripts on the branching DNA platform as described herein or previously in PCT application no. PCT/CA2009/000351 (published under number WO 2009/117811). Results indicated high levels of expression (with RLU values ranging from 10⁶⁻10⁸). A general trend was observed in the quantity of each fusion transcript in that the initial transformation from normal cells to malignant cells (NO-LI) was punctuated by a marked change in quantity of the transcript, followed by either a continued increase or continued decrease in the quantity as malignant progression proceeds from LI to VH).

Any examples provided herein are included solely for the purpose of illustrating the invention and are not intended to limit the invention in any way. Any drawings provided herein are solely for the purpose of illustrating various aspects of the invention and are not intended to be drawn to scale or to limit the invention in any way.

### Bibliography

The following references, amongst others, were cited in the foregoing description.

| **Author** | **Journal** | **Title** | **Volume** | **Date** |
|---|---|---|---|---|
| Anderson et al | Nature | Sequence and Organization of the Human Mitochondrial Genome | 290(5806):457-65 | 1981 |
| Andrews et al | Nat Genet | Reanalysis and revision of the Cambridge reference sequence for human mitochondrial DNA. | 23(2):147 | 1999 |
| Modica-Napolitano et al | Expert Rev Mol Med | Mitochondria as targets for detection and treatment of cancer | 4:1-19 | 2002 |
| Sherratt et al | Clin Sci (Lond) | Mitochondrial DNA defects: a widening clinical spectrum of disorders. | 92(3):225-35 | 1997 |
| Croteau et al | Mutat Res | Mitochondrial DNA repair pathways. | 434(3):137-48 | 1999 |
| Green and Kroemer | J Clin Invest | Pharmacological manipulation of cell death: clinical applications in sight? | 115(10): 2610-2617 | 2005 |
| Dai et al | Acta Otolaryngol | Correlation of cochlear blood supply with mitochondrial DNA common deletion in presbyacusis. | 24(2): 130-6 | 2004 |
| Ro et al | Muscle Nerve | Deleted 4977-bp mitochondrial DNA mutation is associated with sporadic amyotrophic lateral sclerosis: a hospital-based case-control study. | 28(6):737-43 | 2003 |
| Barron et al | Invest Ophthalmol Vis Sci | Mitochondrial abnormalities in ageing macular photoreceptors. | 42(12):3016-22 | 2001 |
| Lewis et al | J Pathol | Detection of damage to the mitochondrial genome in the oncocytic cells of Warthin's tumour. | 191(3):274-81 | 2000 |
| Muller-Hocker et al | Mod Pathol | The common 4977 base pair deletion of mitochondrial DNA preferentially accumulates in the cardiac conduction system of patients with Kearns-Sayre syndrome. | 11(3):295-301. | 1998 |
| Porteous et al | Eur J Biochem | Bioenergetic consequences of accumulating the common 4977-bp mitochondrial DNA deletion. | 257(1): 192-201 | 1998 |
| Parr et al | J Mol Diagn | Somatic mitochondrial DNA mutations in prostate cancer and normal appearing adjacent glands in comparison to age-matched prostate samples without malignant histology. | 8(3):312-9. | 2006 |
| Maki et al | Am J Clin Pathol | Mitochondrial genome deletion aids in the identification of false- and true-negative prostate needle core biopsy specimens. | 129(1):57-66 | 2008 |
| Nakase et al | Am J Hum Genet | Transcription and translation of deleted mitochondrial genomes in Kearns-Sayre syndrome: implications for pathogenesis. | 46(3):418-27. | 1990 |
| Libura et al | Blood | Therapy-related acute myeloid leukemia-like MLL rearrangements are induced by etoposide in primary human CD34+ cells and remain stable after clonal expansion. | 105(5):2124-31 | 2005 |
| Meyer et al | Proc Natl Acad Sci U S A | Diagnostic tool for the identification of MLL rearrangements including unknown partner genes. | 102(2):449-54 | 2005 |
| Eguchi et al | Genes Chromosomes Cancer | MLL chimeric protein activation renders cells vulnerable to chromosomal damage: an explanation for the very short latency of infant leukemia. | 45(8):754-60 | 2006 |
| Hayashi et al | Proc Natl Acad Sci U S A | Introduction of disease-related mitochondrial DNA deletions into HeLa cells lacking mitochondrial DNA results in mitochondrial dysfunction | 88: 10614-10618 | 1991 |

**Table 1: Known mitochondrial deletions having an ORF**

| Deletion Junction (nt:nt) | Deletion Size (bp) | Repeat Location (nt/nt) | Number of Repeats | References |
|---|---|---|---|---|
| COX I - ND5 | | | | |
| 6075:13799 | -7723 | 6076-6084/13799-13807 | D, 9/9 | ▪Mita, S., Rizzuto, R., Moraes, C.T., Shanske, S., Arnaudo, E., Fabrizi, G.M., Koga, Y., DiMauro, S., Schon, E.A. (1990) "Recombination via flanking direct repeats is a major cause of large-scale deletions of human mitochondrial DNA" Nucleic Acids Research 18(3): 561-567 |
| 6238:14103 | -7864 | 6235-6238/1 4099-14102 | D,4/4 | ▪Blok, R.B., Thorburn, D.R., Thompson, G.N., Dahl, H.H. (1995) "A topoisomerase II cleavage site is associated with a novel mitochondrial DNA deletion" Human Genetics 95 (1): 75-81 |
| 6325:13989 | -7663 | 6326-6341/13889-14004 | D,16/17 | ▪Larsson, N.G., Holme, E., Kristiansson, B., Oldfors, A., Tulinius, M. (1990) "Progressive increase of the mutated mitochondrial DNA fraction in Kearns-Sayre syndrome " Pediatric Research 28 (2): 131-136 ▪Larsson, N.G., Holme, E. (1992) "Multiple short direct repeats associated with single mtDNA deletions " Biochimica et Biophysica Acta 1139 (4): 311-314 |
| 6330:13994 | -7663 | 6331-6341/13994-14004 | D, 11/11 | ▪Mita, S., Rizzuto, R., Moraes, C.T., Shanske, S., Arnaudo, E., Fabrizi, G.M., Koga, Y., DiMauro, S., Schon, E.A. (1990) "Recombination via flanking direct repeats is a major cause of large-scale deletions of human mitochondrial DNA" Nucleic Acids Research 18(3): 561 -567 |

| COX II - ND5 | | | | |
|---|---|---|---|---|
| 7829:14135 | -6305 | 7824-7829/14129-14134 | D, 6/6 | ▪Bet, L., Moggio, M., Comi, G.P., Mariani, C., Prelle, A., Checcarelli, N., Bordoni, A., Bresolin, N., Scarpini, E., Scarlato, G. (1994) "Multiple sclerosis and mitochondrial myopathy: an unusual combination of diseases" Journal of Neurology 241 (8): 511-516 |
| 8213:13991 | -5777 | 8214-8220/13991-13997 | D,7/7 | ▪Hinokio, Y., Suzuki, S., Komatu, K., Ohtomo, M., Onoda, M., Matsumoto, M., Hirai, S., Sato, Y., Akai, H., Abe, K.. Toyota, T. (1995) "A new mitochondrial DNA deletion associated with diabetic amyotrophy, diabetic myoatrophy and diabetic fatty liver" Muscle and Nerve 3 (9): S1 42-149 |

| ATPase - ND5 | | | | |
|---|---|---|---|---|
| 8631:13513 | -4881 | 8625-8631 /1 3506-13512 | D, 7/7 | ▪Zhang, C., Baumer, A., Mackay, I.R., Linnane, A.W., Nagley, P. (1995) "Unusual pattern of mitochondrial DNA deletions in skeletal muscle of an adult human with chronic fatigue syndrome" Human Molecular Genetics 4 (4): 751-754 |
| 9144:13816 | -4671 | 9137-91 44/13808-13815 | D, 8/8 | ▪Ota, Y., Tanaka, M., Sato, W., Ohno, K., Yamamoto, T., Maehara, M., Negoro, T., Watanabe, K., Awaya, S., Ozawa, T. (1991) "Detection of platelet mitochondrial DNA deletions in Kearns-Sayre syndrome" Investiqative Ophthalmology and Visual Science 32 (10): 2667-2675 |
| 9191:12909 | -3717 | 9189-91 91/12906-12908 | D, 3/3 | ▪Tanaka, M., Sato, W., Ohno, K., Yamamoto, T., Ozawa, T. (1989) "Direct sequencing of mitochondrial DNA in myopathic patients" Biochemical and Biophysical Research Communications 164 (): 156-163 |

| COX III - ND5 | | | | |
|---|---|---|---|---|
| 10190:13753 | -3562 | 10191-10198/13753-13760 | D, 8/8 | ▪Rotig, A., Bourgeron, T., Chretien, D., Rustin, P., Munnich, A. (1995) "Spectrum of mitochondrial DNA rearrangements in the Pearson marrow-pancreas syndrome" Human Molecular Genetics 4 (8): 1327-1330 ▪Rotig, A., Cormier, V., Koll, F., Mize, C. E., Saudubray, J.-M., Veerman, A., Pearson, H. A., Munnich, A. (1991) "Site-specific deletions of the mitochondrial genome in Pearson marrow-pancreas syndrome" Genomics 10 (2): 502-504 |
| 10367:12829 | -2461 | 10365-10367/12826-12828 | D, 3/3 | ▪Kapsa, R., Thompson, G.N., Thorburn, D.R., Dahl, H.H., Marzuki, S., Byrne, E., Blok, R.B. (1994) "A novel mtDNA deletion in an infant with Pearson syndrome" Journal of Inherited Metabolic Disease 17 (5): 521-526 |

| ND4L - ND5 | | | | |
|---|---|---|---|---|
| 10744:14124 | -3379 | 10745-10754/14124-14133 | D, 9/10 | ▪Cormier-Daire, V., Bonnefont, J.P., Rustin, P., Maurage, C., Ogler, H., Schmitz, J., Ricour, C., Saudubray, J.M., Munnich, A., Rotig, A. (1994) "Mitochondrial DNA rearrangements with onset as chronic diarrhea with villous atrophy" Journal of Pediatrics 124 (1): 63-70 |

| WD4 - ND5 | | | | |
|---|---|---|---|---|
| 11232:13980 | -2747 | 11234-11242/13981-13989 | D. 9/9 | ▪Rotig, A., Cormier, V., Koll, F., Mize, C. E., Saudubray, J.-M., Veerman, A., Pearson, H. A., Munnich, A. (1991) "Site-specific deletions of the mitochondrial genome in Pearson marrow-pancreas syndrome" Genomics 10 (2): 502-504 ▪Rotig, A., Cormier, V., Blanche, S., Bonnefont, J.P., Ledeist, F., Romero, N., Schmitz, J., Rustin, P., Fischer, A., Saudubray, J.M. (1990) "Pearson's marrow-pancreas syndrome. A multi-system mitochondrial disorder in infancy" Journal of Clinical Investigation 86 (): 1601 -1608 ▪Cormier, V., Rotig, A., Quartino, A.R., Forni, G.L., Cerone, R., Maier, M., Saudubray, J.M., Munnich, A. (1990) "Widespread multitissue deletions of the mitochondrial genome in Pearson marrow-pancreas syndrome" Journal of Pediatrics 117 (4): 599-602 ▪Awata, T., Matsumoto, T., Iwamoto, Y., Matsuda, A., Kuzuya, T., Saito, T. (1993) "Japanese case of diabetes mellitus and deafness with mutations in mitochondrial tRNALeu(UUR) gene [letter]" Lancet 341 (8855): 1291-1292 |

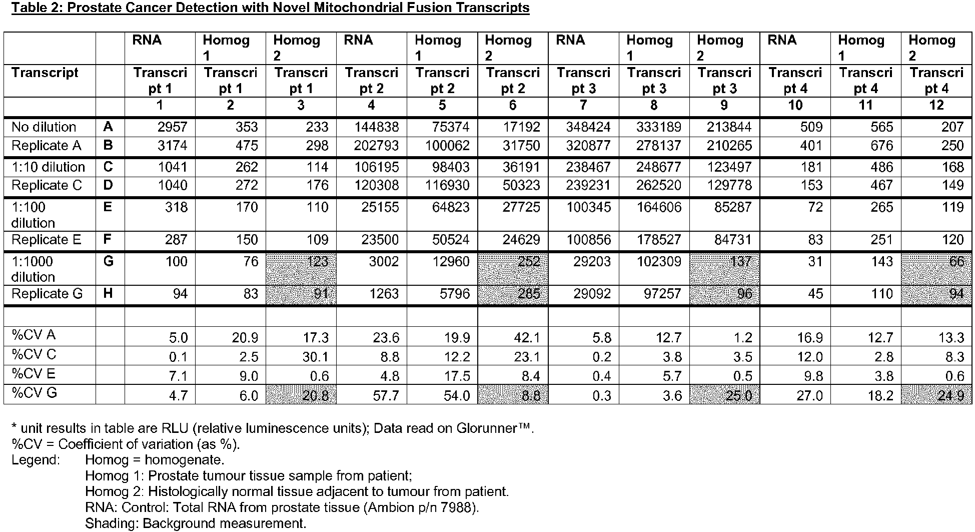

**Table 3: Deletion/Transcript/Hypothetical translation product relationships**

| **Deletion** | **RNA transcript** | **DNA sequence with deletion complementary to RNA transcript** | **Transcript No.** | **Hypothetical Fusion Protein** |
|---|---|---|---|---|
| ATP synthase F0 subunit 8 to NADH dehydrogenase subunit mitochondrial positions 8366-14148 (with reference to SEQ ID NO: 1). Translated sequence begins at position 8389 | SEQ ID NO: 19 | SEQ ID NO: 2 | **1** | SEQ ID NO: 36 |
| NADH dehydrogenase subunit 4L (ND4L) to NADH dehydrogenase subunit 5 (ND5); Mitochondrial positions 10470-14148 (with reference to SEQ ID NO: 1) | SEQ ID NO: 20 | SEQ ID NO: 3 | **2** | SEQ ID NO: 37 |
| Cytochrome c oxidase subunit II (COII) to Cytochrome b (Cytb); Mitochondrial positions 7586-15887 (with reference to SEQ ID NO: 1) | SEQ ID NO: 21 | SEQ ID NO:4 | **3** | SEQ ID NO: 38 |
| Cytochrome c oxidase subunit II (COII) to Cytochrome b (Cytb); Mitochondrial positions 7586-15887 (with reference to SEQ ID NO:1) | SEQ ID NO: 22 | SEQ ID NO:5 | **4** | SEQ ID NO: 39 |

**Table 4: Breast and Prostate Cancer Detection**

| | | **Breast Tumour 1** | **Normal adjacent Breast Tumour 1** | | **Breast Tumour 2** | **Normal Adjacent to Breast Tumour 2** | | **Prostate Tumour 3** | **Prostate Tumour 4** | **Prostate Tumour 5** | **Normal Adjacent to Prostate Tumour 5** |
|---|---|---|---|---|---|---|---|---|---|---|---|
| | | **1** | **2** | | **3** | **4** | | **5** | **6** | **7** | **8** |
| 1:100 dilution | **E** | 68920 | 2971 | | 49108 | 1245 | | 46723 | 56679 | 99836 | 35504 |
| 1:100 dilution replicate | **F** | 92409 | 3017 | | 60637 | 1512 | | 53940 | 56155 | 100582 | 44221 |
| | **G** | 420 | 3 | | 31 | 6 | | 26 | 25 | 44 | 23 |
| | **H** | 518 | 3 | | 4 | 5 | | 5 | 3 | 4 | 2 |
| | | | | | | | | | | | |
| | **%CV** | 20.6 | 1.1 | | 14.9 | 13.7 | | 10.1 | 0.7 | 0.5 | 15.5 |

| | | | | | | | | | | | |
|---|---|---|---|---|---|---|---|---|---|---|---|
| - unit results in table are RLU (relative luminescence units) - background G1, H1 - empty well G2-G8, H2- H8 | | | | | | | | | | | |

**Table 5a: Assay Conditions**

| | **Template for the assay** | | | | | | | | | | | |
|---|---|---|---|---|---|---|---|---|---|---|---|---|
| | **RNA** | **Homogen 1** | **Homogen 2** | **RNA** | **Homogen 1** | **Homogen 2** | **RNA** | **Homogen 1** | **Homogen 2** | **RNA** | **Homogen 1** | **Homogen 2** |
| | **Transcript 1** | **Transcript 1** | **Transcript 1** | **Transcript 2** | **Transcript 2** | **Transcript 2** | **Transcript 3** | **Transcript 3** | **Transcript 3** | **Transcript 4** | **Transcript 4** | **Transcript 4** |
| | **1** | **2** | **3** | **4** | **5** | **6** | **7** | **8** | **9** | **10** | **11** | **12** |
| **A** | RNA | Homog 1 | Homog 2 | RNA | Homog 1 | Homog 2 | RNA | Homog 1 | Homog 2 | RNA | Homog 1 | Homog 2 |
| **B** | Dil 1 | Dil 1 | Dil 1 | Dil 1 | Dil 1 | Dil 1 | Dil 1 | Dil 1 | Dil 1 | Dil 1 | Dil 1 | Dil 1 |
| **C** | RNA | Homog 1 | Homog 2 | RNA | Homog 1 | Homog 2 | RNA | Homog 1 | Homog 2 | RNA | Homog 1 | Homog 2 |
| **D** | Dil 2 | Dil 2 | Dil 2 | Dil 2 | Dil 2 | Dil 2 | Dil 2 | Dil 2 | Dil 2 | Dil 2 | Dil 2 | Dil 2 |
| **E** | RNA | Homog 1 | Homog 2 | RNA | Homog 1 | Homog 2 | RNA | Homog 1 | Homog 2 | RNA | Homog 1 | Homog 2 |
| **F** | Dil 3 | Dil 3 | Dil 3 | Dil 3 | Dil 3 | Dil 3 | Dil 3 | Dil 3 | Dil 3 | Dil 3 | Dil 3 | Dil 3 |
| **G** | RNA | Homog 1 | **Transcript** 1 | RNA | Homog 1 | **Transcript** 1 | RNA | Homog 1 | **Transcript** 1 | RNA | Homog 1 | **Transcript** 1 |
| **H** | Dil 4 | Dil 4 | Background | Dil 4 | Dil 4 | Background | Dil 4 | Dil 4 | Background | Dil 4 | Dil 4 | Background |

| | | | | | | | | | | | | |
|---|---|---|---|---|---|---|---|---|---|---|---|---|
| Homogenate1- Used 26 mg of tissue to homogenize in 700ul H soln with Proteinase K (PK). Used Qiagen TissueRuptor. Used 40ul homogenate supernatant, 20, 10 and 5 ul for dilution Homogenate1 = Tumour tissue from the tumorous Prostate Homogenate2- Used 29 mg of tissue to homogenize in 700ul H soln with PK. Used Qiagen TissueRuptor. Used 40ul homogenate supernatant, 20, 10 and 5 ul for dilution Homogenate2= Normal tissue from the tumorous Prostate RNA dilution was made as below. RNA was from Prostate Normal from Ambion. Assay was done in duplicates. | | | | | | | | | | | | |

**Table 5b: RNA dilution**

| **RNA Dilution** | | ng/ul |
|---|---|---|
| | Dil 1 | 3000 |
| **1:3 dil** | Dil 2 | 1000 |
| **Serial dil** | Dil 3 | 333 |
| | Dil 4 | 111 |

**Table 6: Transcript Summary by Disease**

| Probe | Prostate Cancer | Breast Cancer | Colorectal Cancer | Melanoma Skin Cancer | Lung Cancer | Ovarian Cancer | Testicular Cancer |
|---|---|---|---|---|---|---|---|
| 1 | • | | | | | • | |
| 2 | • | • | • | • | • | • | • |
| 3 | • | | • | • | | • | • |
| 4 | • | | • | | | | • |
| 5 | | | • | • | | | |
| 6 | | | | • | • | • | |
| 7 | | | • | • | | | |
| 8 | | | | | • | | |
| 9 | | | | • | | | |
| 10 | | | • | | • | | |
| 11 | | | • | • | | • | • |
| 12 | | | • | | | • | • |
| 13 | | | | | | | • |
| 14 | | | | • | | | |
| 15 | | | | • | | • | • |
| 16 | | | | • | | | • |
| 17 | | | | • | | | |
| 20 | | | • | • | | • | • |

## Claims

1. An isolated mitochondrial fusion transcript associated with cancer, wherein the transcript comprises a nucleic acid sequence as set forth in SEQ ID NO: 20.

2. A mitochondrial fusion protein corresponding to the fusion transcript of claim 1 or having an amino acid sequence as set forth in SEQ ID NO: 37.

3. An isolated mitochondrial DNA (mtDNA) encoding the fusion transcript of claim 1.

4. An isolated mtDNA according to claim 3 having a nucleic acid sequence as set forth in SEQ ID NO: 3.

5. A hybridization probe having a nucleic acid sequence complementary to the mitochondrial fusion transcript of claim 1 or the mtDNA of claim 3.

6. A method of detecting a cancer in a mammal, the method comprising detecting in a tissue sample from the mammal the presence of the mitochondrial fusion transcript of claim 1 or the presence of the mtDNA of claim 3 or 4.

7. A method of detecting a cancer in a mammal, the method comprising assaying a tissue sample from the mammal for the presence of the mitochondrial fusion transcript of claim 1 by hybridizing the sample with a hybridization probe having a nucleic acid sequence complementary to at least a portion of the mitochondrial fusion transcript.

8. A method of detecting a cancer in a mammal, the method comprising assaying a tissue sample from the mammal for the presence of the mtDNA of claim 3 or 4 by hybridizing the sample with a hybridization probe having a nucleic acid sequence complementary to at least a portion of the mtDNA.

9. A method according to claim 7, wherein said hybridization probe has a nucleic acid sequence complementary to a section of said mitochondrial fusion transcript comprising an expressed section of mtDNA comprising a junction point resulting from a deletion spanning nucleotides 10744 to 14124 of the human mtDNA genome.

10. A method according to claim 8, wherein said hybridization probe has a nucleic acid sequence complementary to a section of said mtDNA comprising a junction point resulting from a deletion spanning nucleotides 10744 to 14124 of the human mtDNA genome.

11. A method according to claim 7 or 9, wherein the method comprises:
a) conducting a hybridization reaction to allow said probe to hybridize to the mitochondrial fusion transcript;
b) quantifying the amount of the mitochondrial fusion transcript in said sample by quantifying the amount of said transcript hybridized to said probe; and,
c) comparing the amount of the mitochondrial fusion transcript in the sample to at least one known reference value.

12. A method according to claim 8 or 10, wherein the method comprises:
a) conducting a hybridization reaction to allow said probe to hybridize to the mtDNA;
b) quantifying the amount of the mtDNA in said sample by quantifying the amount of said mtDNA hybridized to said probe; and,
c) comparing the amount of the mtDNA in the sample to at least one known reference value.

13. A method according to claim 11 or 12, wherein the method is carried out using: diagnostic imaging technology, in particular, high throughput microarray analysis; branched DNA technology; or PCR.

14. A method according to any one of claims 6 to 13, wherein the cancer is one or more of prostate cancer, testicular cancer, ovarian cancer, breast cancer, colorectal cancer, lung cancer, or melanoma skin cancer; preferably wherein the cancer is prostate cancer, colorectal cancer, breast cancer, ovarian cancer or testicular cancer.

15. A kit for conducting an assay for detecting the presence of a cancer in a mammal, said kit comprising:
i) at least one hybridization probe complementary to the fusion transcript of claim 1 or the mtDNA of claim 3 or 4; and
ii) one or more of sampling means, reagents, mixing vessels and instructions for detecting cancer.

## Patentansprüche

1. Isoliertes mitochondriales Fusionstranskript, das mit Krebs assoziiert ist, wobei das Transkript eine Nucleinsäuresequenz umfasst, wie sie in SEQ ID NO: 20 gezeigt ist.

2. Mitochondriales Fusionsprotein, das dem Fusionstranskript gemäß Anspruch 1 entspricht oder eine Aminosäuresequenz aufweist, wie sie in SEQ ID NO: 37 gezeigt ist.

3. Isolierte mitochondriale DNA (mtDNA), die das Fusionstranskript gemäß Anspruch 1 codiert.

4. Isolierte mtDNA gemäß Anspruch 3, die eine Nucleinsäuresequenz aufweist, wie sie in SEQ ID NO: 3 gezeigt ist.

5. Hybridisierungssonde, die eine Nucleinsäuresequenz aufweist, die zu dem mitochondrialen Fusionstranskript gemäß Anspruch 1 oder der mtDNA gemäß Anspruch 3 komplementär ist.

6. Verfahren zum Nachweisen einer Krebserkrankung bei einem Säuger, wobei das Verfahren das Nachweisen der Anwesenheit des mitochondrialen Fusionstranskripts gemäß Anspruch 1 oder der Anwesenheit der mtDNA gemäß Anspruch 3 oder 4 in einer von dem Säuger stammenden Gewebeprobe umfasst.

7. Verfahren zum Nachweisen einer Krebserkrankung bei einem Säuger, wobei das Verfahren das Testen einer von dem Säuger stammenden Gewebeprobe auf die Anwesenheit des mitochondrialen Fusionstranskripts gemäß Anspruch 1 umfasst, indem man die Probe mit einer Hybridisierungssonde, die eine Nucleinsäuresequenz aufweist, die zu wenigstens einem Teil des mitochondrialen Fusionstranskripts komplementär ist, hybridisiert.

8. Verfahren zum Nachweisen einer Krebserkrankung bei einem Säuger, wobei das Verfahren das Testen einer von dem Säuger stammenden Gewebeprobe auf die Anwesenheit der mtDNA gemäß Anspruch 3 oder 4 umfasst, indem man die Probe mit einer Hybridisierungssonde, die eine Nucleinsäuresequenz aufweist, die zu wenigstens einem Teil der mtDNA komplementär ist, hybridisiert.

9. Verfahren gemäß Anspruch 7, wobei die Hybridisierungssonde eine Nucleinsäuresequenz aufweist, die zu einem Abschnitt des mitochondrialen Fusionstranskripts komplementär ist, der einen exprimierten Abschnitt von mtDNA umfasst, der einen Verbindungspunkt umfasst, welcher aus einer Deletion resultiert, die die Nucleotide 10744 bis 14124 des humanen mtDNA-Genoms überspannt.

10. Verfahren gemäß Anspruch 8, wobei die Hybridisierungssonde eine Nucleinsäuresequenz aufweist, die zu einem Abschnitt der mtDNA komplementär ist, der einen Verbindungspunkt umfasst, welcher aus einer Deletion resultiert, die die Nucleotide 10744 bis 14124 des humanen mtDNA-Genoms überspannt.

11. Verfahren gemäß Anspruch 7 oder 9, wobei das Verfahren umfasst:
a) Durchführen einer Hybridisierungsreaktion, damit die Sonde mit dem mitochondrialen Fusionstranskript hybridisieren kann;
b) Quantifizieren der Menge des mitochondrialen Fusionstranskripts in der Probe durch Quantifizieren der Menge des Transkripts, die mit der Sonde hybridisiert ist; und
c) Vergleichen der Menge des mitochondrialen Fusionstranskripts in der Probe mit wenigstens einem bekannten Referenzwert.

12. Verfahren gemäß Anspruch 8 oder 10, wobei das Verfahren umfasst:
a) Durchführen einer Hybridisierungsreaktion, damit die Sonde mit der mtDNA hybridisieren kann;
b) Quantifizieren der Menge der mtDNA in der Probe durch Quantifizieren der Menge der mtDNA, die mit der Sonde hybridisiert ist; und
c) Vergleichen der Menge der mtDNA in der Probe mit wenigstens einem bekannten Referenzwert.

13. Verfahren gemäß Anspruch 11 oder 12, wobei das Verfahren unter Verwendung von diagnostischer Bildgebungstechnik, insbesondere Hochdurchsatz-Mikroarrayanalyse, verzweigte-DNA-Technik oder PCR, durchgeführt wird.

14. Verfahren gemäß einem der Ansprüche 6 bis 13, wobei es sich bei der Krebserkrankung um eine oder mehrere aus Prostatakrebs, Hodenkrebs, Eierstockkrebs, Brustkrebs, Kolorektalkarzinom, Lungenkrebs oder schwarzen Hautkrebs handelt, wobei vorzugsweise es sich bei der Krebserkrankung um Prostatakrebs, Kolorektalkarzinom, Brustkrebs, Eierstockkrebs oder Hodenkrebs handelt.

15. Kit zur Durchführung eines Assays zum Nachweisen der Anwesenheit einer Krebserkrankung bei einem Säuger, wobei der Kit umfasst:
i) wenigstens eine Hybridisierungssonde, die zu dem Fusionstranskript gemäß Anspruch 1 oder der mtDNA gemäß Anspruch 3 oder 4 komplementär ist; und
ii) eine oder mehrere Probenahmeeinrichtungen, Reagentien, Mischgefäße und Anleitungen zum Nachweisen einer Krebserkrankung.

## Revendications

1. Produit de transcription de fusion mitochondriale isolé associé à un cancer, dans lequel le produit de transcription comprend une séquence d'acides nucléiques telle que définie par SEQ ID NO : 20.

2. Protéine de fusion mitochondriale correspondant à un produit de transcription de fusion selon la revendication 1 ou présentant une séquence d'acides nucléiques telle que définie par SEQ ID NO : 37.

3. ADN mitochondrial (ADNmt) isolé codant pour un produit de transcription de fusion selon la revendication 1.

4. ADNmt isolé selon la revendication 3 présentant une séquence d'acides nucléiques telle que définie par SEQ ID NO : 3.

5. Sonde d'hybridation présentant une séquence d'acides nucléiques complémentaire d'un produit de transcription de fusion mitochondriale selon la revendication 1 ou d'un ADNmt selon la revendication 3.

6. Procédé de détection d'un cancer chez un mammifère, le procédé comprenant la détection, dans un échantillon de tissu provenant du mammifère, de la présence d'un produit de transcription de fusion mitochondriale selon la revendication 1 ou de la présence d'un ADNmt selon la revendication 3 ou 4.

7. Procédé de détection d'un cancer chez un mammifère, le procédé comprenant l'analyse d'un échantillon de tissu provenant du mammifère pour y détecter la présence d'un produit de transcription de fusion mitochondriale selon la revendication 1 par hybridation de l'échantillon avec une sonde d'hybridation présentant une séquence d'acides nucléiques complémentaire d'au moins une partie du produit de transcription de fusion mitochondriale.

8. Procédé de détection d'un cancer chez un mammifère, le procédé comprenant l'analyse d'un échantillon de tissu provenant du mammifère pour y détecter la présence d'un ADNmt selon la revendication 3 ou 4 par hybridation de l'échantillon avec une sonde d'hybridation présentant une séquence d'acides nucléiques complémentaire d'au moins une partie de l'ADNmt.

9. Procédé selon la revendication 7, dans lequel ladite sonde d'hybridation présente une séquence d'acides nucléiques complémentaire d'une section dudit produit de transcription de fusion mitochondriale comprenant une section exprimée d'ADNmt comprenant un point de jonction résultant d'une délétion appliquée aux nucléotides 10744 à 14124 du génome d'ADNmt humain.

10. Procédé selon la revendication 8, dans lequel ladite sonde d'hybridation présente une séquence d'acides nucléiques complémentaire d'une section dudit ADNmt comprenant un point de jonction résultant d'une délétion appliquée aux nucléotides 10744 à 14124 du génome d'ADNmt humain.

11. Procédé selon la revendication 7 ou 9, dans lequel ce procédé comprend :
a) la mise en œuvre d'une réaction d'hybridation pour permettre à ladite sonde de s'hybrider au produit de transcription de fusion mitochondriale ;
b) la quantification de la quantité de produit de transcription de fusion mitochondriale dans ledit échantillon par quantification de la quantité dudit produit de transcription hybridé à ladite sonde ; et
c) la comparaison de la quantité de produit de transcription de fusion mitochondriale dans l'échantillon avec au moins une valeur de référence connue.

12. Procédé selon la revendication 8 ou 10, dans lequel ce procédé comprend :
a) la mise en œuvre d'une réaction d'hybridation pour permettre à ladite sonde de s'hybrider à l'ADNmt ;
b) la quantification de la quantité d'ADNmt dans ledit échantillon par quantification de la quantité dudit ADNmt hybridé à ladite sonde ; et
c) la comparaison de la quantité d'ADNmt dans l'échantillon avec au moins une valeur de référence connue.

13. Procédé selon la revendication 11 ou 12, dans lequel le procédé est réalisé en utilisant : une technologie de diagnostic par imagerie, en particulier une analyse par biopuces à haut débit ; une technologie d'ADN ramifié ; ou une technologie d'amplification en chaîne par polymérase (PCR).

14. Procédé selon l'une quelconque des revendications 6 à 13, dans lequel le cancer est un ou plusieurs du cancer de la prostate, du cancer des testicules, du cancer ovarien, du cancer du sein, du cancer colorectal, du cancer du poumon ou du cancer de la peau avec présence de mélanome ; de préférence dans lequel le cancer est le cancer de la prostate, le cancer colorectal, le cancer du sein, le cancer ovarien ou le cancer des testicules.

15. Kit pour réaliser une analyse de détection de la présence d'un cancer chez un mammifère, ledit kit comprenant :
i) au moins une sonde d'hybridation complémentaire d'un produit de transcription de fusion selon la revendication 1 ou d'un ADNmt selon la revendication 3 ou 4 ; et
ii) un ou plusieurs moyens d'échantillonnage, réactifs, récipients de mélange et instructions de détection du cancer.


### SEQUENCE LISTING

<110> MDNA Life Sciences Inc.
<120> Aberrant Mitochondrial DNA, Associated Fusion Transcripts and Translation Products and Hybridization Probes Therefor
<130> P6037457EP1
<140> EP
   <141>
<150> EP 10761136.0
   <151> 2010-03-29
<150> PCT/CA2010/000423
   <151> 2010-03-29
<150> PCT/CA2009/000351
   <151> 2009-03-27
<150> US 61/040616
   <151> 2008-03-28
<160> 67
<170> Patent In version 3.5
<210> 1
   <211> 16568
   <212> DNA
   <213> Human
<400> 1
   gatcacaggt ctatcaccct attaaccact cacgggagct ctccatgcat ttggtatttt      60
   cgtctggggg gtatgcacgc gatagcattg cgagacgctg gagccggagc accctatgtc     120
   gcagtatctg tctttgattc ctgcctcatc ctattattta tcgcacctac gttcaatatt     180
   acaggcgaac atacttacta aagtgtgtta attaattaat gcttgtagga cataataata     240
   acaattgaat gtctgcacag ccactttcca cacagacatc ataacaaaaa atttccacca     300
   aaccccccct cccccgcttc tggccacagc acttaaacac atctctgcca aaccccaaaa     360
   acaaagaacc ctaacaccag cctaaccaga tttcaaattt tatcttttgg cggtatgcac     420
   ttttaacagt caccccccaa ctaacacatt attttcccct cccactccca tactactaat     480
   ctcatcaata caacccccgc ccatcctacc cagcacacac acaccgctgc taaccccata     540
   ccccgaacca accaaacccc aaagacaccc cccacagttt atgtagctta cctcctcaaa     600
   gcaatacact gaaaatgttt agacgggctc acatcacccc ataaacaaat aggtttggtc     660
   ctagcctttc tattagctct tagtaagatt acacatgcaa gcatccccgt tccagtgagt     720
   tcaccctcta aatcaccacg atcaaaagga acaagcatca agcacgcagc aatgcagctc     780
   aaaacgctta gcctagccac acccccacgg gaaacagcag tgattaacct ttagcaataa     840
   acgaaagttt aactaagcta tactaacccc agggttggtc aatttcgtgc cagccaccgc     900
   ggtcacacga ttaacccaag tcaatagaag ccggcgtaaa gagtgtttta gatcaccccc 960
   tccccaataa agctaaaact cacctgagtt gtaaaaaact ccagttgaca caaaatagac 1020
   tacgaaagtg gctttaacat atctgaacac acaatagcta agacccaaac tgggattaga 1080
   taccccacta tgcttagccc taaacctcaa cagttaaatc aacaaaactg ctcgccagaa 1140
   cactacgagc cacagcttaa aactcaaagg acctggcggt gcttcatatc cctctagagg 1200
   agcctgttct gtaatcgata aaccccgatc aacctcacca cctcttgctc agcctatata 1260
   ccgccatctt cagcaaaccc tgatgaaggc tacaaagtaa gcgcaagtac ccacgtaaag 1320
   acgttaggtc aaggtgtagc ccatgaggtg gcaagaaatg ggctacattt tctaccccag 1380
   aaaactacga tagcccttat gaaacttaag ggtcgaaggt ggatttagca gtaaactaag 1440
   agtagagtgc ttagttgaac agggccctga agcgcgtaca caccgcccgt caccctcctc 1500
   aagtatactt caaaggacat ttaactaaaa cccctacgca tttatataga ggagacaagt 1560
   cgtaacatgg taagtgtact ggaaagtgca cttggacgaa ccagagtgta gcttaacaca 1620
   aagcacccaa cttacactta ggagatttca acttaacttg accgctctga gctaaaccta 1680
   gccccaaacc cactccacct tactaccaga caaccttagc caaaccattt acccaaataa 1740
   agtataggcg atagaaattg aaacctggcg caatagatat agtaccgcaa gggaaagatg 1800
   aaaaattata accaagcata atatagcaag gactaacccc tataccttct gcataatgaa 1860
   ttaactagaa ataactttgc aaggagagcc aaagctaaga cccccgaaac cagacgagct 1920
   acctaagaac agctaaaaga gcacacccgt ctatgtagca aaatagtggg aagatttata 1980
   ggtagaggcg acaaacctac cgagcctggt gatagctggt tgtccaagat agaatcttag 2040
   ttcaacttta aatttgccca cagaaccctc taaatcccct tgtaaattta actgttagtc 2100
   caaagaggaa cagctctttg gacactagga aaaaaccttg tagagagagt aaaaaattta 2160
   acacccatag taggcctaaa agcagccacc aattaagaaa gcgttcaagc tcaacaccca 2220
   ctacctaaaa aatcccaaac atataactga actcctcaca cccaattgga ccaatctatc 2280
   accctataga agaactaatg ttagtataag taacatgaaa acattctcct ccgcataagc 2340
   ctgcgtcaga ttaaaacact gaactgacaa ttaacagccc aatatctaca atcaaccaac 2400
   aagtcattat taccctcact gtcaacccaa cacaggcatg ctcataagga aaggttaaaa 2460
   aaagtaaaag gaactcggca aatcttaccc cgcctgttta ccaaaaacat cacctctagc 2520
   atcaccagta ttagaggcac cgcctgccca gtgacacatg tttaacggcc gcggtaccct 2580
   aaccgtgcaa aggtagcata atcacttgtt ccttaaatag ggacctgtat gaatggctcc 2640
   acgagggttc agctgtctct tacttttaac cagtgaaatt gacctgcccg tgaagaggcg 2700
   ggcataacac agcaagacga gaagacccta tggagcttta atttattaat gcaaacagta 2760
   cctaacaaac ccacaggtcc taaactacca aacctgcatt aaaaatttcg gttggggcga 2820
   cctcggagca gaacccaacc tccgagcagt acatgctaag acttcaccag tcaaagcgaa 2880
   ctactatact caattgatcc aataacttga ccaacggaac aagttaccct agggataaca 2940
   gcgcaatcct attctagagt ccatatcaac aatagggttt acgacctcga tgttggatca 3000
   ggacatcccg atggtgcagc cgctattaaa ggttcgtttg ttcaacgatt aaagtcctac 3060
   gtgatctgag ttcagaccgg agtaatccag gtcggtttct atctacttca aattcctccc 3120
   tgtacgaaag gacaagagaa ataaggccta cttcacaaag cgccttcccc cgtaaatgat 3180
   atcatctcaa cttagtatta tacccacacc cacccaagaa cagggtttgt taagatggca 3240
   gagcccggta atcgcataaa acttaaaact ttacagtcag aggttcaatt cctcttctta 3300
   acaacatacc catggccaac ctcctactcc tcattgtacc cattctaatc gcaatggcat 3360
   tcctaatgct taccgaacga aaaattctag gctatataca actacgcaaa ggccccaacg 3420
   ttgtaggccc ctacgggcta ctacaaccct tcgctgacgc cataaaactc ttcaccaaag 3480
   agcccctaaa acccgccaca tctaccatca ccctctacat caccgccccg accttagctc 3540
   tcaccatcgc tcttctacta tgaacccccc tccccatacc caaccccctg gtcaacctca 3600
   acctaggcct cctatttatt ctagccacct ctagcctagc cgtttactca atcctctgat 3660
   cagggtgagc atcaaactca aactacgccc tgatcggcgc actgcgagca gtagcccaaa 3720
   caatctcata tgaagtcacc ctagccatca ttctactatc aacattacta ataagtggct 3780
   cctttaacct ctccaccctt atcacaacac aagaacacct ctgattactc ctgccatcat 3840
   gacccttggc cataatatga tttatctcca cactagcaga gaccaaccga acccccttcg 3900
   accttgccga aggggagtcc gaactagtct caggcttcaa catcgaatac gccgcaggcc 3960
   ccttcgccct attcttcata gccgaataca caaacattat tataataaac accctcacca 4020
   ctacaatctt cctaggaaca acatatgacg cactctcccc tgaactctac acaacatatt 4080
   ttgtcaccaa gaccctactt ctaacctccc tgttcttatg aattcgaaca gcataccccc 4140
   gattccgcta cgaccaactc atacacctcc tatgaaaaaa cttcctacca ctcaccctag 4200
   cattacttat atgatatgtc tccataccca ttacaatctc cagcattccc cctcaaacct 4260
   aagaaatatg tctgataaaa gagttacttt gatagagtaa ataataggag cttaaacccc 4320
   cttatttcta ggactatgag aatcgaaccc atccctgaga atccaaaatt ctccgtgcca 4380
   cctatcacac cccatcctaa agtaaggtca gctaaataag ctatcgggcc cataccccga 4440
   aaatgttggt tatacccttc ccgtactaat taatcccctg gcccaacccg tcatctactc 4500
   taccatcttt gcaggcacac tcatcacagc gctaagctcg cactgatttt ttacctgagt 4560
   aggcctagaa ataaacatgc tagcttttat tccagttcta accaaaaaaa taaaccctcg 4620
   ttccacagaa gctgccatca agtatttcct cacgcaagca accgcatcca taatccttct 4680
   aatagctatc ctcttcaaca atatactctc cggacaatga accataacca atactaccaa 4740
   tcaatactca tcattaataa tcataatagc tatagcaata aaactaggaa tagccccctt 4800
   tcacttctga gtcccagagg ttacccaagg cacccctctg acatccggcc tgcttcttct 4860
   cacatgacaa aaactagccc ccatctcaat catataccaa atctctccct cactaaacgt 4920
   aagccttctc ctcactctct caatcttatc catcatagca ggcagttgag gtggattaaa 4980
   ccaaacccag ctacgcaaaa tcttagcata ctcctcaatt acccacatag gatgaataat 5040
   agcagttcta ccgtacaacc ctaacataac cattcttaat ttaactattt atattatcct 5100
   aactactacc gcattcctac tactcaactt aaactccagc accacgaccc tactactatc 5160
   tcgcacctga aacaagctaa catgactaac acccttaatt ccatccaccc tcctctccct 5220
   aggaggcctg cccccgctaa ccggcttttt gcccaaatgg gccattatcg aagaattcac 5280
   aaaaaacaat agcctcatca tccccaccat catagccacc atcaccctcc ttaacctcta 5340
   cttctaccta cgcctaatct actccacctc aatcacacta ctccccatat ctaacaacgt 5400
   aaaaataaaa tgacagtttg aacatacaaa acccacccca ttcctcccca cactcatcgc 5460
   ccttaccacg ctactcctac ctatctcccc ttttatacta ataatcttat agaaatttag 5520
   gttaaataca gaccaagagc cttcaaagcc ctcagtaagt tgcaatactt aatttctgta 5580
   acagctaagg actgcaaaac cccactctgc atcaactgaa cgcaaatcag ccactttaat 5640
   taagctaagc ccttactaga ccaatgggac ttaaacccac aaacacttag ttaacagcta 5700
   agcaccctaa tcaactggct tcaatctact tctcccgccg ccgggaaaaa aggcgggaga 5760
   agccccggca ggtttgaagc tgcttcttcg aatttgcaat tcaatatgaa aatcacctcg 5820
   gagctggtaa aaagaggcct aacccctgtc tttagattta cagtccaatg cttcactcag 5880
   ccattttacc tcacccccac tgatgttcgc cgaccgttga ctattctcta caaaccacaa 5940
   agacattgga acactatacc tattattcgg cgcatgagct ggagtcctag gcacagctct 6000
   aagcctcctt attcgagccg agctgggcca gccaggcaac cttctaggta acgaccacat 6060
   ctacaacgtt atcgtcacag cccatgcatt tgtaataatc ttcttcatag taatacccat 6120
   cataatcgga ggctttggca actgactagt tcccctaata atcggtgccc ccgatatggc 6180
   gtttccccgc ataaacaaca taagcttctg actcttacct ccctctctcc tactcctgct 6240
   cgcatctgct atagtggagg ccggagcagg aacaggttga acagtctacc ctcccttagc 6300
   agggaactac tcccaccctg gagcctccgt agacctaacc atcttctcct tacacctagc 6360
   aggtgtctcc tctatcttag gggccatcaa tttcatcaca acaattatca atataaaacc 6420
   ccctgccata acccaatacc aaacgcccct cttcgtctga tccgtcctaa tcacagcagt 6480
   cctacttctc ctatctctcc cagtcctagc tgctggcatc actatactac taacagaccg 6540
   caacctcaac accaccttct tcgaccccgc cggaggagga gaccccattc tataccaaca 6600
   cctattctga tttttcggtc accctgaagt ttatattctt atcctaccag gcttcggaat 6660
   aatctcccat attgtaactt actactccgg aaaaaaagaa ccatttggat acataggtat 6720
   ggtctgagct atgatatcaa ttggcttcct agggtttatc gtgtgagcac accatatatt 6780
   tacagtagga atagacgtag acacacgagc atatttcacc tccgctacca taatcatcgc 6840
   tatccccacc ggcgtcaaag tatttagctg actcgccaca ctccacggaa gcaatatgaa 6900
   atgatctgct gcagtgctct gagccctagg attcatcttt cttttcaccg taggtggcct 6960
   gactggcatt gtattagcaa actcatcact agacatcgta ctacacgaca cgtactacgt 7020
   tgtagcccac ttccactatg tcctatcaat aggagctgta tttgccatca taggaggctt 7080
   cattcactga tttcccctat tctcaggcta caccctagac caaacctacg ccaaaatcca 7140
   tttcactatc atattcatcg gcgtaaatct aactttcttc ccacaacact ttctcggcct 7200
   atccggaatg ccccgacgtt actcggacta ccccgatgca tacaccacat gaaacatcct 7260
   atcatctgta ggctcattca tttctctaac agcagtaata ttaataattt tcatgatttg 7320
   agaagccttc gcttcgaagc gaaaagtcct aatagtagaa gaaccctcca taaacctgga 7380
   gtgactatat ggatgccccc caccctacca cacattcgaa gaacccgtat acataaaatc 7440
   tagacaaaaa aggaaggaat cgaacccccc aaagctggtt tcaagccaac cccatggcct 7500
   ccatgacttt ttcaaaaagg tattagaaaa accatttcat aactttgtca aagttaaatt 7560
   ataggctaaa tcctatatat cttaatggca catgcagcgc aagtaggtct acaagacgct 7620
   acttccccta tcatagaaga gcttatcacc tttcatgatc acgccctcat aatcattttc 7680
   cttatctgct tcctagtcct gtatgccctt ttcctaacac tcacaacaaa actaactaat 7740
   actaacatct cagacgctca ggaaatagaa accgtctgaa ctatcctgcc cgccatcatc 7800
   ctagtcctca tcgccctccc atccctacgc atcctttaca taacagacga ggtcaacgat 7860
   ccctccctta ccatcaaatc aattggccac caatggtact gaacctacga gtacaccgac 7920
   tacggcggac taatcttcaa ctcctacata cttcccccat tattcctaga accaggcgac 7980
   ctgcgactcc ttgacgttga caatcgagta gtactcccga ttgaagcccc cattcgtata 8040
   ataattacat cacaagacgt cttgcactca tgagctgtcc ccacattagg cttaaaaaca 8100
   gatgcaattc ccggacgtct aaaccaaacc actttcaccg ctacacgacc gggggtatac 8160
   tacggtcaat gctctgaaat ctgtggagca aaccacagtt tcatgcccat cgtcctagaa 8220
   ttaattcccc taaaaatctt tgaaataggg cccgtattta ccctatagca ccccctctac 8280
   cccctctaga gcccactgta aagctaactt agcattaacc ttttaagtta aagattaaga 8340
   gaaccaacac ctctttacag tgaaatgccc caactaaata ctaccgtatg gcccaccata 8400
   attaccccca tactccttac actattcctc atcacccaac taaaaatatt aaacacaaac 8460
   taccacctac ctccctcacc aaagcccata aaaataaaaa attataacaa accctgagaa 8520
   ccaaaatgaa cgaaaatctg ttcgcttcat tcattgcccc cacaatccta ggcctacccg 8580
   ccgcagtact gatcattcta tttccccctc tattgatccc cacctccaaa tatctcatca 8640
   acaaccgact aatcaccacc caacaatgac taatcaaact aacctcaaaa caaatgataa 8700
   ccatacacaa cactaaagga cgaacctgat ctcttatact agtatcctta atcattttta 8760
   ttgccacaac taacctcctc ggactcctgc ctcactcatt tacaccaacc acccaactat 8820
   ctataaacct agccatggcc atccccttat gagcgggcac agtgattata ggctttcgct 8880
   ctaagattaa aaatgcccta gcccacttct taccacaagg cacacctaca ccccttatcc 8940
   ccatactagt tattatcgaa accatcagcc tactcattca accaatagcc ctggccgtac 9000
   gcctaaccgc taacattact gcaggccacc tactcatgca cctaattgga agcgccaccc 9060
   tagcaatatc aaccattaac cttccctcta cacttatcat cttcacaatt ctaattctac 9120
   tgactatcct agaaatcgct gtcgccttaa tccaagccta cgttttcaca cttctagtaa 9180
   gcctctacct gcacgacaac acataatgac ccaccaatca catgcctatc atatagtaaa 9240
   acccagccca tgacccctaa caggggccct ctcagccctc ctaatgacct ccggcctagc 9300
   catgtgattt cacttccact ccataacgct cctcatacta ggcctactaa ccaacacact 9360
   aaccatatac caatgatggc gcgatgtaac acgagaaagc acataccaag gccaccacac 9420
   accacctgtc caaaaaggcc ttcgatacgg gataatccta tttattacct cagaagtttt 9480
   tttcttcgca ggatttttct gagcctttta ccactccagc ctagccccta ccccccaatt 9540
   aggagggcac tggcccccaa caggcatcac cccgctaaat cccctagaag tcccactcct 9600
   aaacacatcc gtattactcg catcaggagt atcaatcacc tgagctcacc atagtctaat 9660
   agaaaacaac cgaaaccaaa taattcaagc actgcttatt acaattttac tgggtctcta 9720
   ttttaccctc ctacaagcct cagagtactt cgagtctccc ttcaccattt ccgacggcat 9780
   ctacggctca acattttttg tagccacagg cttccacgga cttcacgtca ttattggctc 9840
   aactttcctc actatctgct tcatccgcca actaatattt cactttacat ccaaacatca 9900
   ctttggcttc gaagccgccg cctgatactg gcattttgta gatgtggttt gactatttct 9960
   gtatgtctcc atctattgat gagggtctta ctcttttagt ataaatagta ccgttaactt 10020
   ccaattaact agttttgaca acattcaaaa aagagtaata aacttcgcct taattttaat 10080
   aatcaacacc ctcctagcct tactactaat aattattaca ttttgactac cacaactcaa 10140
   cggctacata gaaaaatcca ccccttacga gtgcggcttc gaccctatat cccccgcccg 10200
   cgtccctttc tccataaaat tcttcttagt agctattacc ttcttattat ttgatctaga 10260
   aattgccctc cttttacccc taccatgagc cctacaaaca actaacctgc cactaatagt 10320
   tatgtcatcc ctcttattaa tcatcatcct agccctaagt ctggcctatg agtgactaca 10380
   aaaaggatta gactgaaccg aattggtata tagtttaaac aaaacgaatg atttcgactc 10440
   attaaattat gataatcata tttaccaaat gcccctcatt tacataaata ttatactagc 10500
   atttaccatc tcacttctag gaatactagt atatcgctca cacctcatat cctccctact 10560
   atgcctagaa ggaataatac tatcgctgtt cattatagct actctcataa ccctcaacac 10620
   ccactccctc ttagccaata ttgtgcctat tgccatacta gtctttgccg cctgcgaagc 10680
   agcggtgggc ctagccctac tagtctcaat ctccaacaca tatggcctag actacgtaca 10740
   taacctaaac ctactccaat gctaaaacta atcgtcccaa caattatatt actaccactg 10800
   acatgacttt ccaaaaaaca cataatttga atcaacacaa ccacccacag cctaattatt 10860
   agcatcatcc ctctactatt ttttaaccaa atcaacaaca acctatttag ctgttcccca 10920
   accttttcct ccgaccccct aacaaccccc ctcctaatac taactacctg actcctaccc 10980
   ctcacaatca tggcaagcca acgccactta tccagtgaac cactatcacg aaaaaaactc 11040
   tacctctcta tactaatctc cctacaaatc tccttaatta taacattcac agccacagaa 11100
   ctaatcatat tttatatctt cttcgaaacc acacttatcc ccaccttggc tatcatcacc 11160
   cgatgaggca accagccaga acgcctgaac gcaggcacat acttcctatt ctacacccta 11220
   gtaggctccc ttcccctact catcgcacta atttacactc acaacaccct aggctcacta 11280
   aacattctac tactcactct cactgcccaa gaactatcaa actcctgagc caacaactta 11340
   atatgactag cttacacaat agcttttata gtaaagatac ctctttacgg actccactta 11400
   tgactcccta aagcccatgt cgaagccccc atcgctgggt caatagtact tgccgcagta 11460
   ctcttaaaac taggcggcta tggtataata cgcctcacac tcattctcaa ccccctgaca 11520
   aaacacatag cctacccctt ccttgtacta tccctatgag gcataattat aacaagctcc 11580
   atctgcctac gacaaacaga cctaaaatcg ctcattgcat actcttcaat cagccacata 11640
   gccctcgtag taacagccat tctcatccaa accccctgaa gcttcaccgg cgcagtcatt 11700
   ctcataatcg cccacgggct tacatcctca ttactattct gcctagcaaa ctcaaactac 11760
   gaacgcactc acagtcgcat cataatcctc tctcaaggac ttcaaactct actcccacta 11820
   atagcttttt gatgacttct agcaagcctc gctaacctcg ccttaccccc cactattaac 11880
   ctactgggag aactctctgt gctagtaacc acgttctcct gatcaaatat cactctccta 11940
   cttacaggac tcaacatact agtcacagcc ctatactccc tctacatatt taccacaaca 12000
   caatggggct cactcaccca ccacattaac aacataaaac cctcattcac acgagaaaac 12060
   accctcatgt tcatacacct atcccccatt ctcctcctat ccctcaaccc cgacatcatt 12120
   accgggtttt cctcttgtaa atatagttta accaaaacat cagattgtga atctgacaac 12180
   agaggcttac gaccccttat ttaccgagaa agctcacaag aactgctaac tcatgccccc 12240
   atgtctaaca acatggcttt ctcaactttt aaaggataac agctatccat tggtcttagg 12300
   ccccaaaaat tttggtgcaa ctccaaataa aagtaataac catgcacact actataacca 12360
   ccctaaccct gacttcccta attcccccca tccttaccac cctcgttaac cctaacaaaa 12420
   aaaactcata cccccattat gtaaaatcca ttgtcgcatc cacctttatt atcagtctct 12480
   tccccacaac aatattcatg tgcctagacc aagaagttat tatctcgaac tgacactgag 12540
   ccacaaccca aacaacccag ctctccctaa gcttcaaact agactacttc tccataatat 12600
   tcatccctgt agcattgttc gttacatggt ccatcataga attctcactg tgatatataa 12660
   actcagaccc aaacattaat cagttcttca aatatctact catcttccta attaccatac 12720
   taatcttagt taccgctaac aacctattcc aactgttcat cggctgagag ggcgtaggaa 12780
   ttatatcctt cttgctcatc agttgatgat acgcccgagc agatgccaac acagcagcca 12840
   ttcaagcaat cctatacaac cgtatcggcg atatcggttt catcctcgcc ttagcatgat 12900
   ttatcctaca ctccaactca tgagacccac aacaaatagc ccttctaaac gctaatccaa 12960
   gcctcacccc actactaggc ctcctcctag cagcagcagg caaatcagcc caattaggtc 13020
   tccacccctg actcccctca gccatagaag gccccacccc agtctcagcc ctactccact 13080
   caagcactat agttgtagca ggaatcttct tactcatccg cttccacccc ctagcagaaa 13140
   atagcccact aatccaaact ctaacactat gcttaggcgc tatcaccact ctgttcgcag 13200
   cagtctgcgc ccttacacaa aatgacatca aaaaaatcgt agccttctcc acttcaagtc 13260
   aactaggact cataatagtt acaatcggca tcaaccaacc acacctagca ttcctgcaca 13320
   tctgtaccca cgccttcttc aaagccatac tatttatgtg ctccgggtcc atcatccaca 13380
   accttaacaa tgaacaagat attcgaaaaa taggaggact actcaaaacc atacctctca 13440
   cttcaacctc cctcaccatt ggcagcctag cattagcagg aatacctttc ctcacaggtt 13500
   tctactccaa agaccacatc atcgaaaccg caaacatatc atacacaaac gcctgagccc 13560
   tatctattac tctcatcgct acctccctga caagcgccta tagcactcga ataattcttc 13620
   tcaccctaac aggtcaacct cgcttcccca cccttactaa cattaacgaa aataacccca 13680
   ccctactaaa ccccattaaa cgcctggcag ccggaagcct attcgcagga tttctcatta 13740
   ctaacaacat ttcccccgca tcccccttcc aaacaacaat ccccctctac ctaaaactca 13800
   cagccctcgc tgtcactttc ctaggacttc taacagccct agacctcaac tacctaacca 13860
   acaaacttaa aataaaatcc ccactatgca cattttattt ctccaacata ctcggattct 13920
   accctagcat cacacaccgc acaatcccct atctaggcct tcttacgagc caaaacctgc 13980
   ccctactcct cctagaccta acctgactag aaaagctatt acctaaaaca atttcacagc 14040
   accaaatctc cacctccatc atcacctcaa cccaaaaagg cataattaaa ctttacttcc 14100
   tctctttctt cttcccactc atcctaaccc tactcctaat cacataacct attcccccga 14160
   gcaatctcaa ttacaatata tacaccaaca aacaatgttc aaccagtaac tactactaat 14220
   caacgcccat aatcatacaa agcccccgca ccaataggat cctcccgaat caaccctgac 14280
   ccctctcctt cataaattat tcagcttcct acactattaa agtttaccac aaccaccacc 14340
   ccatcatact ctttcaccca cagcaccaat cctacctcca tcgctaaccc cactaaaaca 14400
   ctcaccaaga cctcaacccc tgacccccat gcctcaggat actcctcaat agccatcgct 14460
   gtagtatatc caaagacaac catcattccc cctaaataaa ttaaaaaaac tattaaaccc 14520
   atataacctc ccccaaaatt cagaataata acacacccga ccacaccgct aacaatcaat 14580
   actaaacccc cataaatagg agaaggctta gaagaaaacc ccacaaaccc cattactaaa 14640
   cccacactca acagaaacaa agcatacatc attattctcg cacggactac aaccacgacc 14700
   aatgatatga aaaaccatcg ttgtatttca actacaagaa caccaatgac cccaatacgc 14760
   aaaactaacc ccctaataaa attaattaac cactcattca tcgacctccc caccccatcc 14820
   aacatctccg catgatgaaa cttcggctca ctccttggcg cctgcctgat cctccaaatc 14880
   accacaggac tattcctagc catgcactac tcaccagacg cctcaaccgc cttttcatca 14940
   atcgcccaca tcactcgaga cgtaaattat ggctgaatca tccgctacct tcacgccaat 15000
   ggcgcctcaa tattctttat ctgcctcttc ctacacatcg ggcgaggcct atattacgga 15060
   tcatttctct actcagaaac ctgaaacatc ggcattatcc tcctgcttgc aactatagca 15120
   acagccttca taggctatgt cctcccgtga ggccaaatat cattctgagg ggccacagta 15180
   attacaaact tactatccgc catcccatac attgggacag acctagttca atgaatctga 15240
   ggaggctact cagtagacag tcccaccctc acacgattct ttacctttca cttcatcttg 15300
   cccttcatta ttgcagccct agcaacactc cacctcctat tcttgcacga aacgggatca 15360
   aacaaccccc taggaatcac ctcccattcc gataaaatca ccttccaccc ttactacaca 15420
   atcaaagacg ccctcggctt acttctcttc cttctctcct taatgacatt aacactattc 15480
   tcaccagacc tcctaggcga cccagacaat tataccctag ccaacccctt aaacacccct 15540
   ccccacatca agcccgaatg atatttccta ttcgcctaca caattctccg atccgtccct 15600
   aacaaactag gaggcgtcct tgccctatta ctatccatcc tcatcctagc aataatcccc 15660
   atcctccata tatccaaaca acaaagcata atatttcgcc cactaagcca atcactttat 15720
   tgactcctag ccgcagacct cctcattcta acctgaatcg gaggacaacc agtaagctac 15780
   ccttttacca tcattggaca agtagcatcc gtactatact tcacaacaat cctaatccta 15840
   ataccaacta tctccctaat tgaaaacaaa atactcaaat gggcctgtcc ttgtagtata 15900
   aactaataca ccagtcttgt aaaccggaga tgaaaacctt tttccaagga caaatcagag 15960
   aaaaagtctt taactccacc attagcaccc aaagctaaga ttctaattta aactattctc 16020
   tgttctttca tggggaagca gatttgggta ccacccaagt attgactcac ccatcaacaa    16080
   ccgctatgta tttcgtacat tactgccagc caccatgaat attgtacggt accataaata    16140
   cttgaccacc tgtagtacat aaaaacccaa tccacatcaa aaccccctcc ccatgcttac    16200
   aagcaagtac agcaatcaac cctcaactat cacacatcaa ctgcaactcc aaagccaccc    16260
   ctcacccact aggataccaa caaacctacc cacccttaac agtacatagt acataaagcc    16320
   atttaccgta catagcacat tacagtcaaa tcccttctcg tccccatgga tgacccccct    16380
   cagatagggg tcccttgacc accatcctcc gtgaaatcaa tatcccgcac aagagtgcta    16440
   ctctcctcgc tccgggccca taacacttgg gggtagctaa agtgaactgt atccgacatc    16500
   tggttcctac ttcagggtca taaagcctaa atagcccaca cgttcccctt aaataagaca    16560
   tcacgatg                                                              16568
<210> 2
   <211> 783
   <212> DNA
   <213> Artificial
<220>
   <223> cDNA
<400> 2
   atggcccacc ataattaccc ccatactcct tacactattc ctcatcaccc aactaaaaat       60
   attaaacaca aactaccacc tacctccctc accattggca gcctagcatt agcaggaata      120
   cctttcctca caggtttcta ctccaaagac cacatcatcg aaaccgcaaa catatcatac      180
   acaaacgcct gagccctatc tattactctc atcgctacct ccctgacaag cgcctatagc      240
   actcgaataa ttcttctcac cctaacaggt caacctcgct tccccaccct tactaacatt      300
   aacgaaaata accccaccct actaaacccc attaaacgcc tggcagccgg aagcctattc      360
   gcaggatttc tcattactaa caacatttcc cccgcatccc ccttccaaac aacaatcccc      420
   ctctacctaa aactcacagc cctcgctgtc actttcctag gacttctaac agccctagac      480
   ctcaactacc taaccaacaa acttaaaata aaatccccac tatgcacatt ttatttctcc      540
   aacatactcg gattctaccc tagcatcaca caccgcacaa tcccctatct aggccttctt      600
   acgagccaaa acctgcccct actcctccta gacctaacct gactagaaaa gctattacct      660
   aaaacaattt cacagcacca aatctccacc tccatcatca cctcaaccca aaaaggcata      720
   attaaacttt acttcctctc tttcttcttc ccactcatcc taaccctact cctaatcaca      780
   taa                                                                   783
<210> 3
   <211> 300
   <212> DNA
   <213> Artificial
<220>
   <223> cDNA
<400> 3
   atgcccctca tttacataaa tattatacta gcatttacca tctcacttct aggaatacta      60
   gtatatcgct cacacctcat atcctcccta ctatgcctag aaggaataat actatcgctg     120
   ttcattatag ctactctcat aaccctcaac acccactccc tcttagccaa tattgtgcct     180
   attgccatac tagtctttgc cgcctgcgaa gcagcggtgg gcctagccct actagtctca     240
   atctccaaca catatggcct agactacgta cataacctaa ccctactcct aatcacataa     300
<210> 4
   <211> 781
   <212> DNA
   <213> Artificial
<220>
   <223> cDNA
<400> 4
   atggcacatg cagcgcaagt aggtctacaa gacgctactt cccctatcat agaagagctt      60
   atcacctttc atgatcacgc cctcataatc attttcctta tctgcttcct agtcctgtat     120
   gcccttttcc taacactcac aacaaaacta actaatacta acatctcaga cgctcaggaa     180
   atagaaaccg tctgaactat cctgcccgcc atcatcctag tcctcatcgc cctcccatcc     240
   ctacgcatcc tttacataac agacgaggtc aacgatccct cccttaccat caaatcaatt     300
   ggccaccaat ggtactgaac ctacgagtac accgactacg gcggactaat cttcaactcc     360
   tacatacttc ccccattatt cctagaacca ggcgacccag acaattatac cctagccaac     420
   cccttaaaca cccctcccca catcaagccc gaatgatatt tcctattcgc ctacacaatt     480
   ctccgatccg tccctaacaa actaggaggc gtccttgccc tattactatc catcctcatc     540
   ctagcaataa tccccatcct ccatatatcc aaacaacaaa gcataatatt tcgcccacta     600
   agccaatcac tttattgact cctagccgca gacctcctca ttctaacctg aatcggagga     660
   caaccagtaa gctacccttt taccatcatt ggacaagtag catccgtact atacttcaca     720
   acaatcctaa tcctaatacc aactatctcc ctaattgaaa acaaaatact caaatgggcc     780
   t                                                                     781
<210> 5
   <211> 565
   <212> DNA
   <213> Artificial
<220>
   <223> cDNA
<400> 5
   atggcacatg cagcgcaagt aggtctacaa gacgctactt cccctatcat agaagagctt      60
   atcacctttc atgatcacgc cctcataatc attttcctta tctgcttcct agtcctgtat     120
   gcccttttcc taacactcac aacaaaacta actaatacta acatctcaga cgctcaggaa      180
   atagaaaccg tctgaactat cctgcccgcc atcatcctag tcctcatcgc cctcccatcc     240
   ctacgcatcc tttacataac agacgaggtc aacgatccct cccttaccat caaatcaatt     300
   ggccaccaat ggtactgaac ctacgagtac accgactacg gcggactaat cttcaactcc     360
   tacatacttc ccccattatt cctagaacca ggcgacctgc gactcctagc cgcagacctc     420
   ctcattctaa cctgaatcgg aggacaacca gtaagctacc cttttaccat cattggacaa     480
   gtagcatccg tactatactt cacaacaatc ctaatcctaa taccaactat ctccctaatt     540
   gaaaacaaaa tactcaaatg ggcct                                           565
<210> 6
   <211> 1174
   <212> DNA
   <213> Artificial
<220>
   <223> cDNA
<400> 6
   atggcacatg cagcgcaagt aggtctacaa gacgctactt cccctatcat agaagagctt 60
   atcacctttc atgatcacgc cctcataatc attttcctta tctgcttcct agtcctgtat 120
   gcccttttcc taacactcac aacaaaacta actaatacta acatctcaga cgctcaggaa 180
   atagaaaccg tctgaactat cctgcccgcc atcatcctag tcctcatcgc cctcccatcc 240
   ctacgcatcc tttacataac agacgaggtc aacgatccct cccttaccat caaatcaatt 300
   ggccaccaat ggtactgaac ctacgagtac accgactacg gcggactaat cttcaactcc 360
   tacatacttc ccccattatt cctagaacca ggcgacctgc gactccttga cgttgacaat 420
   cgagtagtac tcccgattga agcccccatt cgtataataa ttacatcaca agacgtcttg 480
   cactcatgag ctgtccccac attaggctta aaaacagatg caattcccgg acgtctaaac 540
   caaaccactt tcaccgctac acgaccgggg gtatactacg gtcaatgctc tgaaatctgt 600
   ggagcaaacc acagtttcat gcccatattc ttgcacgaaa cgggatcaaa caacccccta 660
   ggaatcacct cccattccga taaaatcacc ttccaccctt actacacaat caaagacgcc 720
   ctcggcttac ttctcttcct tctctcctta atgacattaa cactattctc accagacctc     780
   ctaggcgacc cagacaatta taccctagcc aaccccttaa acacccctcc ccacatcaag     840
   cccgaatgat atttcctatt cgcctacaca attctccgat ccgtccctaa caaactagga 900
   ggcgtccttg ccctattact atccatcctc atcctagcaa taatccccat cctccatata 960
   tccaaacaac aaagcataat atttcgccca ctaagccaat cactttattg actcctagcc 1020
   gcagacctcc tcattctaac ctgaatcgga ggacaaccag taagctaccc ttttaccatc 1080
   attggacaag tagcatccgt actatacttc acaacaatcc taatcctaat accaactatc 1140
   tccctaattg aaaacaaaat actcaaatgg gcct 1174
<210> 7
   <211> 1294
   <212> DNA
   <213> Artificial
<220>
   <223> cDNA
<400> 7
   atgaacgaaa atctgttcgc ttcattcatt gcccccacaa tcctaggcct acccgccgca      60
   gtactgatca ttctatttcc ccctctattg atccccacct ccaaatatct catcaacaac     120
   cgactaatca ccacccaaca atgactaatc aaactaacct caaaacaaat gataaccata     180
   cacaacacta aaggacgaac ctgatctctt atactagtat ccttaatcat ttttattgcc     240
   acaactaacc tcctcggact cctgcctcac tcatttacac caaccaccca actatctata     300
   aacctagcca tgcactactc accagacgcc tcaaccgcct tttcatcaat cgcccacatc     360
   actcgagacg taaattatgg ctgaatcatc cgctaccttc acgccaatgg cgcctcaata     420
   ttctttatct gcctcttcct acacatcggg cgaggcctat attacggatc atttctctac     480
   tcagaaacct gaaacatcgg cattatcctc ctgcttgcaa ctatagcaac agccttcata     540
   ggctatgtcc tcccgtgagg ccaaatatca ttctgagggg ccacagtaat tacaaactta     600
   ctatccgcca tcccatacat tgggacagac ctagttcaat gaatctgagg aggctactca     660
   gtagacagtc ccaccctcac acgattcttt acctttcact tcatcttgcc cttcattatt     720
   gcagccctag caacactcca cctcctattc ttgcacgaaa cgggatcaaa caacccccta     780
   ggaatcacct cccattccga taaaatcacc ttccaccctt actacacaat caaagacgcc     840
   ctcggcttac ttctcttcct tctctcctta atgacattaa cactattctc accagacctc     900
   ctaggcgacc cagacaatta taccctagcc aaccccttaa acacccctcc ccacatcaag     960
   cccgaatgat atttcctatt cgcctacaca attctccgat ccgtccctaa caaactagga    1020
   ggcgtccttg ccctattact atccatcctc atcctagcaa taatccccat cctccatata    1080
   tccaaacaac aaagcataat atttcgccca ctaagccaat cactttattg actcctagcc    1140
   gcagacctcc tcattctaac ctgaatcgga ggacaaccag taagctaccc ttttaccatc    1200
   attggacaag tagcatccgt actatacttc acaacaatcc taatcctaat accaactatc    1260
   tccctaattg aaaacaaaat actcaaatgg gcct                                1294
<210> 8
   <211> 1228
   <212> DNA
   <213> Artificial
<220>
   <223> cDNA
<400> 8
   atgcccctca tttacataaa tattatacta gcatttacca tctcacttct aggaatacta 60
   gtatatcgct cacacctcat atcctcccta ctatgcctag aaggaataat actatcgctg 120
   ttcattatag ctactctcat aaccctcaac acccactccc tcttagccaa tattgtgcct 180
   attgccatac tagtctttgg cgcctgcctg atcctccaaa tcaccacagg actattccta 240
   gccatgcact actcaccaga cgcctcaacc gccttttcat caatcgccca catcactcga 300
   gacgtaaatt atggctgaat catccgctac cttcacgcca atggcgcctc aatattcttt 360
   atctgcctct tcctacacat cgggcgaggc ctatattacg gatcatttct ctactcagaa 420
   acctgaaaca tcggcattat cctcctgctt gcaactatag caacagcctt cataggctat 480
   gtcctcccgt gaggccaaat atcattctga ggggccacag taattacaaa cttactatcc 540
   gccatcccat acattgggac agacctagtt caatgaatct gaggaggcta ctcagtagac 600
   agtcccaccc tcacacgatt ctttaccttt cacttcatct tgcccttcat tattgcagcc 660
   ctagcaacac tccacctcct attcttgcac gaaacgggat caaacaaccc cctaggaatc 720
   acctcccatt ccgataaaat caccttccac ccttactaca caatcaaaga cgccctcggc 780
   ttacttctct tccttctctc cttaatgaca ttaacactat tctcaccaga cctcctaggc 840
   gacccagaca attataccct agccaacccc ttaaacaccc ctccccacat caagcccgaa 900
   tgatatttcc tattcgccta cacaattctc cgatccgtcc ctaacaaact aggaggcgtc 960
   cttgccctat tactatccat cctcatccta gcaataatcc ccatcctcca tatatccaaa 1020
   caacaaagca taatatttcg cccactaagc caatcacttt attgactcct agccgcagac 1080
   ctcctcattc taacctgaat cggaggacaa ccagtaagct acccttttac catcattgga 1140
   caagtagcat ccgtactata cttcacaaca atcctaatcc taataccaac tatctcccta 1200
   attgaaaaca aaatactcaa atgggcct 1228
<210> 9
   <211> 522
   <212> DNA
   <213> Artificial
<220>
   <223> cDNA
<400> 9
<210> 10
   <211> 582
   <212> DNA
   <213> Artificial
<220>
   <223> cDNA
<400> 10
   atgttcgccg accgttgact attctctaca aaccacaaag acattggaac actataccta 60
   ttattcggcg catgagctgg agtcctaggc acagctctaa gcctccttat tcgagccgag 120
   ctgggccagc caggcaacct tctaggtaac gaccacatct acaacgttat cgtcacagcc 180
   catgcatttg taataatctt cttcatagta atacccatca taatcggagg ctttggcaac 240
   tgactagttc ccctaataat cggtgccccc gatatggcgt ttccccgcat aaacaacata 300
   agcttctgac tcttacctcc ctctctccta ctcctgctcg catctgctat agtggaggcc 360
   ggagcaggaa caggttgaac agtctaccct cccttagcag ggaactactc ccaccctgga 420
   gccctcctag acctaacctg actagaaaag ctattaccta aaacaatttc acagcaccaa 480
   atctccacct ccatcatcac ctcaacccaa aaaggcataa ttaaacttta cttcctctct 540
   ttcttcttcc cactcatcct aaccctactc ctaatcacat aa 582
<210> 11
   <211> 2208
   <212> DNA
   <213> Artificial
<220>
   <223> cDNA
<400> 11
   atgttcgccg accgttgact attctctaca aaccacaaag acattggaac actataccta 60
   ttattcggcg catgagctgg agtcctaggc acagctctaa gcctccttat tcgagccgag 120
   ctgggccagc caggcaacct tctaggtaac gaccacatct acaacgttat cgtcacagcc 180
   catgcatttg taataatctt cttcatagta atacccatca taatcggagg ctttggcaac 240
   tgactagttc ccctaataat cggtgccccc gatatggcgt ttccccgcat aaacaacata 300
   agcttctgac tcttacctcc ctctctccta ctcctgctcg catctgctat agtggaggcc 360
   ggagcaggaa caggttgaac agtctaccct cccttagcag ggaactactc ccaccctgga 420
   gcctccgtag acctaaccat cttctcctta cacctagcag gtgtctcctc tatcttaggg 480
   gccatcaatt tcatcacaac aattatcaat ataaaacccc ctgccataac ccaataccaa 540
   acgcccctct tcgtctgatc cgtcctaatc acagcagtcc tacttctcct atctctccca 600
   gtcctagctg ctggcatcac tatactacta acagaccgca acctcaacac caccttcttc 660
   gaccccgccg gaggaggaga ccccattcta taccaacacc tattctgatt tttcggtcac 720
   cctgaagttt atattcttat cctaccaggc ttcggaataa tctcccatat tgtaacttac 780
   tactccggaa aaaaagaacc atttggatac ataggtatgg tctgagctat gatatcaatt 840
   ggcttcctag ggtttatcgt gtgagcacac catatattta cagtaggaat agacgtagac 900
   acacgagcat atttcacctc cgctaccata atcatcgcta tccccaccgg cgtcaaagta 960
   tttagctgac tcgccacact ccacggaagc aatatgaaat gatctgctgc agtgctctga 1020
   gccctaggat tcatctttct tttcaccgta ggtggcctga ctggcattgt attagcaaac 1080
   tcatcactag acatcgtact acacgacacg tactacgttg tagcccactt ccactatgtc 1140
   ctatcaatag gagctgtatt tgccatcata ggaggcttca ttcactgatt tcccctattc 1200
   tcaggctaca ccctagacca aacctacgcc aaaatccatt tcactatcat attcatcggc 1260
   gtaaatctaa ctttcttccc acaacacttt ctcggcctat ccggaatgcc ccgacgttac 1320
   tcggactacc ccgatgcata caccacatga aacatcctat catctgtagg ctcattcatt 1380
   tctctaacag cagtaatatt aataattttc atgatttgag aagccttcgc ttcgaagcga 1440
   aaagtcctaa tagtagaaga accctccata aacctggagt gactatatgg atgcccccca 1500
   ccctaccaca cattcgaaga acccgtatac ataaaagcag gaatacctttcctcacaggt 1560
   ttctactcca aagaccacat catcgaaacc gcaaacatat catacacaaa cgcctgagcc 1620
   ctatctatta ctctcatcgc tacctccctg acaagcgcct atagcactcg aataattctt 1680
   ctcaccctaa caggtcaacc tcgcttcccc acccttacta acattaacga aaataacccc 1740
   accctactaa accccattaa acgcctggca gccggaagcc tattcgcagg atttctcatt 1800
   actaacaaca tttcccccgc atcccccttc caaacaacaa tccccctcta cctaaaactc 1860
   acagccctcg ctgtcacttt cctaggactt ctaacagccc tagacctcaa ctacctaacc 1920
   aacaaactta aaataaaatc cccactatgc acattttatt tctccaacat actcggattc 1980
   taccctagca tcacacaccg cacaatcccc tatctaggcc ttcttacgag ccaaaacctg 2040
   cccctactcc tcctagacct aacctgacta gaaaagctat tacctaaaac aatttcacag 2100
   caccaaatct ccacctccat catcacctca acccaaaaag gcataattaa actttacttc 2160
   ctctctttct tcttcccact catcctaacc ctactcctaa tcacataa 2208
<210> 12
   <211> 807
   <212> DNA
   <213> Artificial
<220>
   <223> cDNA
<400> 12
   atggcacatg cagcgcaagt aggtctacaa gacgctactt cccctatcat agaagagctt 60
   atcacctttc atgatcacgc cctcataatc attttcctta tctgcttcct agtcctgtat 120
   gcccttttcc taacactcac aacaaaacta actaatacta acatctcaga cgctcaggaa 180
   atagaaaccg caaacatatc atacacaaac gcctgagccc tatctattac tctcatcgct 240
   acctccctga caagcgccta tagcactcga ataattcttc tcaccctaac aggtcaacct 300
   cgcttcccca cccttactaa cattaacgaa aataacccca ccctactaaa ccccattaaa 360
   cgcctggcag ccggaagcct attcgcagga tttctcatta ctaacaacat ttcccccgca 420
   tcccccttcc aaacaacaat ccccctctac ctaaaactca cagccctcgc tgtcactttc 480
   ctaggacttc taacagccct agacctcaac tacctaacca acaaacttaa aataaaatcc 540
   ccactatgca cattttattt ctccaacata ctcggattct accctagcat cacacaccgc 600
   acaatcccct atctaggcct tcttacgagc caaaacctgc ccctactcct cctagaccta 660
   acctgactag aaaagctatt acctaaaaca atttcacagc accaaatctc cacctccatc 720
   atcacctcaa cccaaaaagg cataattaaa ctttacttcc tctctttctt cttcccactc 780
   atcctaaccc tactcctaat cacataa 807
<210> 13
   <211> 786
   <212> DNA
   <213> Artificial
<220>
   <223> cDNA
<400> 13
   atggcacatg cagcgcaagt aggtctacaa gacgctactt cccctatcat agaagagctt        60
   atcacctttc atgatcacgc cctcataatc attttcctta tctgcttcct agtcctgtat       120
   gcccttttcc taacactcac aacaaaacta actaatacta acatctcaga cgctcaggaa       180
   atagaaaccg tctgaactat cctgcccgcc atcatcctag tcctcatcgc cctcccatcc       240
   ctacgcatcc tttacataac agacgaggtc aacgatccct cccttaccat caaatcaatt       300
   ggccaccaat ggtactgaac ctacgagtac accgactacg gcggactaat cttcaactcc       360
   tacatacttc ccccattatt cctagaacca ggcgacctgc gactccttga cgttgacaat       420
   cgagtagtac tcccgattga agcccccatt cgtataataa ttacatcaca agacgtcttg       480
   cactcatgag ctgtccccac attaggctta aaaacagatg caattcccgg acgtctaaac       540
   caaaccactt tcaccgctac acgaccgggg gtatactacg gtcaatgctc tgaaatctgt       600
   ggagcaaacc acagtttcat gcccatcgtc ctagacctaa cctgactaga aaagctatta       660
   cctaaaacaa tttcacagca ccaaatctcc acctccatca tcacctcaac ccaaaaaggc       720
   ataattaaac tttacttcct ctctttcttc ttcccactca tcctaaccct actcctaatc       780
   acataa                                                                  786
<210> 14
   <211> 1905
   <212> DNA
   <213> Artificial
<220>
   <223> cDNA
<400> 14
   atgaacgaaa atctgttcgc ttcattcatt gcccccacaa tcctaggcct acccgccgca      60
   gtactgatca ttctatttcc ccctctattg atccccacct ccaaatatct catcaacaac     120
   cgactaatca ccacccaaca atgactaatc aaactaacct caaaacaaat gataaccata     180
   cacaacacta aaggacgaac ctgatctctt atactagtat ccttaatcat ttttattgcc     240
   acaactaacc tcctcggact cctgcctcac tcatttacac caaccaccca actatctata     300
   aacctagcca tggccatccc cttatgagcg ggcacagtga ttataggctt tcgctctaag     360
   attaaaaatg ccctagccca cttcttacca caaggcacac ctacacccct tatccccata     420
   ctagttatta tcgaaaccat cagcctactc attcaaccaa tagccctggc cgtacgccta     480
   accgctaaca ttactgcagg ccacctactc atgcacctaa ttggaagcgc caccctagca     540
   atatcaacca ttaaccttcc ctctacactt atcatcttca caattctaat tctactgact     600
   atcctagaaa tcgctgtcgc cttaatccaa gcctacgttt tcacacttct agtaagcctc     660
   tacctacact ccaactcatg agacccacaa caaatagccc ttctaaacgc taatccaagc     720
   ctcaccccac tactaggcct cctcctagca gcagcaggca aatcagccca attaggtctc     780
   cacccctgac tcccctcagc catagaaggc cccaccccag tctcagccct actccactca     840
   agcactatag ttgtagcagg aatcttctta ctcatccgct tccaccccct agcagaaaat     900
   agcccactaa tccaaactct aacactatgc ttaggcgcta tcaccactct gttcgcagca     960
   gtctgcgccc ttacacaaaa tgacatcaaa aaaatcgtag ccttctccac ttcaagtcaa    1020
   ctaggactca taatagttac aatcggcatc aaccaaccac acctagcatt cctgcacatc    1080
   tgtacccacg ccttcttcaa agccatacta tttatgtgct ccgggtccat catccacaac 1140
   cttaacaatg aacaagatat tcgaaaaata ggaggactac tcaaaaccat acctctcact 1200
   tcaacctccc tcaccattgg cagcctagca ttagcaggaa tacctttcct cacaggtttc 1260
   tactccaaag accacatcat cgaaaccgca aacatatcat acacaaacgc ctgagcccta 1320
   tctattactc tcatcgctac ctccctgaca agcgcctata gcactcgaat aattcttctc 1380
   accctaacag gtcaacctcg cttccccacc cttactaaca ttaacgaaaa taaccccacc 1440
   ctactaaacc ccattaaacg cctggcagcc ggaagcctat tcgcaggatt tctcattact 1500
   aacaacattt cccccgcatc ccccttccaa acaacaatcc ccctctacct aaaactcaca 1560
   gccctcgctg tcactttcct aggacttcta acagccctag acctcaacta cctaaccaac 1620
   aaacttaaaa taaaatcccc actatgcaca ttttatttct ccaacatact cggattctac 1680
   cctagcatca cacaccgcac aatcccctat ctaggccttc ttacgagcca aaacctgccc 1740
   ctactcctcc tagacctaac ctgactagaa aagctattac ctaaaacaat ttcacagcac 1800
   caaatctcca cctccatcat cacctcaacc caaaaaggca taattaaact ttacttcctc 1860
   tctttcttct tcccactcat cctaacccta ctcctaatca cataa 1905
<210> 15
   <211> 1545
   <212> DNA
   <213> Artificial
<220>
   <223> cDNA
<400> 15
   atgacccacc aatcacatgc ctatcatata gtaaaaccca gcccatgacc cctaacaggg      60
   gccctctcag ccctcctaat gacctccggc ctagccatgt gatttcactt ccactccata     120
   acgctcctca tactaggcct actaaccaac acactaacca tataccaatg atggcgcgat     180
   gtaacacgag aaagcacata ccaaggccac cacacaccac ctgtccaaaa aggccttcga     240
   tacgggataa tcctatttat tacctcagaa gtttttttct tcgcaggatt tttctgagcc     300
   ttttaccact ccagcctagc ccctaccccc caattaggag ggcactggcc cccaacaggc     360
   atcaccccac tactaggcct cctcctagca gcagcaggca aatcagccca attaggtctc     420
   cacccctgac tcccctcagc catagaaggc cccaccccag tctcagccct actccactca     480
   agcactatag ttgtagcagg aatcttctta ctcatccgct tccaccccct agcagaaaat      540
   agcccactaa tccaaactct aacactatgc ttaggcgcta tcaccactct gttcgcagca     600
   gtctgcgccc ttacacaaaa tgacatcaaa aaaatcgtag ccttctccac ttcaagtcaa     660
   ctaggactca taatagttac aatcggcatc aaccaaccac acctagcatt cctgcacatc     720
   tgtacccacg ccttcttcaa agccatacta tttatgtgct ccgggtccat catccacaac     780
   cttaacaatg aacaagatat tcgaaaaata ggaggactac tcaaaaccat acctctcact     840
   tcaacctccc tcaccattgg cagcctagca ttagcaggaa tacctttcct cacaggtttc     900
   tactccaaag accacatcat cgaaaccgca aacatatcat acacaaacgc ctgagcccta     960
   tctattactc tcatcgctac ctccctgaca agcgcctata gcactcgaat aattcttctc    1020
   accctaacag gtcaacctcg cttccccacc cttactaaca ttaacgaaaa taaccccacc    1080
   ctactaaacc ccattaaacg cctggcagcc ggaagcctat tcgcaggatt tctcattact    1140
   aacaacattt cccccgcatc ccccttccaa acaacaatcc ccctctacct aaaactcaca     1200
   gccctcgctg tcactttcct aggacttcta acagccctag acctcaacta cctaaccaac    1260
   aaacttaaaa taaaatcccc actatgcaca ttttatttct ccaacatact cggattctac    1320
   cctagcatca cacaccgcac aatcccctat ctaggccttc ttacgagcca aaacctgccc    1380
   ctactcctcc tagacctaac ctgactagaa aagctattac ctaaaacaat ttcacagcac    1440
   caaatctcca cctccatcat cacctcaacc caaaaaggca taattaaact ttacttcctc    1500
   tctttcttct tcccactcat cctaacccta ctcctaatca cataa                    1545
<210> 16
   <211> 1629
   <212> DNA
   <213> Artificial
<220>
   <223> cDNA
<400> 16
   ataaacttcg ccttaatttt aataatcaac accctcctag ccttactact aataattatt 60
   acattttgac taccacaact caacggctac atagaaaaat ccacccctta cgagtgcggc 120
   ttcgacccta tatcccccgc ccgcgtccct ttctccataa aattcttctt agtagctatt 180
   accttcttat tatttgatct agaaattgcc ctccttttac ccctaccatg agccctacaa 240
   acaactaacc tgccactaat agttatgtca tccctcttat taatcatcat cctagcccta 300
   agtctggcca acacagcagc cattcaagca atcctataca accgtatcgg cgatatcggt 360
   ttcatcctcg ccttagcatg atttatccta cactccaact catgagaccc acaacaaata 420
   gcccttctaa acgctaatcc aagcctcacc ccactactag gcctcctcct agcagcagca 480
   ggcaaatcag cccaattagg tctccacccc tgactcccct cagccataga aggccccacc 540
   ccagtctcag ccctactcca ctcaagcact atagttgtag caggaatctt cttactcatc 600
   cgcttccacc ccctagcaga aaatagccca ctaatccaaa ctctaacact atgcttaggc 660
   gctatcacca ctctgttcgc agcagtctgc gcccttacac aaaatgacat caaaaaaatc 720
   gtagccttct ccacttcaag tcaactagga ctcataatag ttacaatcgg catcaaccaa 780
   ccacacctag cattcctgca catctgtacc cacgccttct tcaaagccat actatttatg 840
   tgctccgggt ccatcatcca caaccttaac aatgaacaag atattcgaaa aataggagga 900
   ctactcaaaa ccatacctct cacttcaacc tccctcacca ttggcagcct agcattagca 960
   ggaatacctt tcctcacagg tttctactcc aaagaccaca tcatcgaaac cgcaaacata 1020
   tcatacacaa acgcctgagc cctatctatt actctcatcg ctacctccct gacaagcgcc 1080
   tatagcactc gaataattct tctcacccta acaggtcaac ctcgcttccc cacccttact 1140
   aacattaacg aaaataaccc caccctacta aaccccatta aacgcctggc agccggaagc 1200
   ctattcgcag gatttctcat tactaacaac atttcccccg catccccctt ccaaacaaca 1260
   atccccctct acctaaaact cacagccctc gctgtcactt tcctaggact tctaacagcc 1320
   ctagacctca actacctaac caacaaactt aaaataaaat ccccactatg cacattttat 1380
   ttctccaaca tactcggatt ctaccctagc atcacacacc gcacaatccc ctatctaggc 1440
   cttcttacga gccaaaacct gcccctactc ctcctagacc taacctgact agaaaagcta 1500
   ttacctaaaa caatttcaca gcaccaaatc tccacctcca tcatcacctc aacccaaaaa 1560
   ggcataatta aactttactt cctctctttc ttcttcccac tcatcctaac cctactccta 1620
   atcacataa 1629
<210> 17
   <211> 642
   <212> DNA
   <213> Artificial
<220>
   <223> cDNA
<400> 17
   atgctaaaac taatcgtccc aacaattata ttactaccac tgacatgact ttccaaaaaa      60
   cacataattt gaatcaacac aaccacccac agcctaatta ttagcatcat ccctctacta     120
   ttttttaacc aaatcaacaa caacctattt agctgttccc caaccttttc ctccgacccc     180
   ctaacaaccc ccctcctaat actaactacc tgactcctac ccctcacaat catggcaagc     240
   caacgccact tatccagtga accactatca cgaaaaaaac tctacctctc tatactaatc     300
   tccctacaaa tctccttaat tataacattc acagccacag aactaatcat attttatatc     360
   ttcttcgaaa ccacacttat ccccaccttg gctatcatca cccgatgagg caaccagcca     420
   gaacgcctga acgcaggcac atacttccta ttctacaccc tagtaggctc cctgccccta     480
   ctcctcctag acctaacctg actagaaaag ctattaccta aaacaatttc acagcaccaa     540
   atctccacct ccatcatcac ctcaacccaa aaaggcataa ttaaacttta cttcctctct     600
   ttcttcttcc cactcatcct aaccctactc ctaatcacat aa                        642
<210> 18
   <211> 129
   <212> DNA
   <213> Artificial
<220>
   <223> cDNA
<400> 18
   atgccccaac taaatactac cgtatggccc accataatta cccccatact ccttacacta      60
   ttcctcatca cccaactaaa aatattaaac acaaactacc acctacctcc ctcaccattg     120
   gcagcctag                                                             129
<210> 19
   <211> 783
   <212> RNA
   <213> Human
<400> 19
   auggcccacc auaauuaccc ccauacuccu uacacuauuc cucaucaccc aacuaaaaau        60
   auuaaacaca aacuaccacc uaccucccuc accauuggca gccuagcauu agcaggaaua       120
   ccuuuccuca cagguuucua cuccaaagac cacaucaucg aaaccgcaaa cauaucauac       180
   acaaacgccu gagcccuauc uauuacucuc aucgcuaccu cccugacaag cgccuauagc       240
   acucgaauaa uucuucucac ccuaacaggu caaccucgcu uccccacccu uacuaacauu       300
   aacgaaaaua accccacccu acuaaacccc auuaaacgcc uggcagccgg aagccuauuc       360
   gcaggauuuc ucauuacuaa caacauuucc cccgcauccc ccuuccaaac aacaaucccc       420
   cucuaccuaa aacucacagc ccucgcuguc acuuuccuag gacuucuaac agcccuagac       480
   cucaacuacc uaaccaacaa acuuaaaaua aaauccccac uaugcacauu uuauuucucc       540
   aacauacucg gauucuaccc uagcaucaca caccgcacaa uccccuaucu aggccuucuu       600
   acgagccaaa accugccccu acuccuccua gaccuaaccu gacuagaaaa gcuauuaccu       660
   aaaacaauuu cacagcacca aaucuccacc uccaucauca ccucaaccca aaaaggcaua       720
   auuaaacuuu acuuccucuc uuucuucuuc ccacucaucc uaacccuacu ccuaaucaca       780
   uaa                                                                     783
<210> 20
   <211> 300
   <212> RNA
   <213> Human
<400> 20
   augccccuca uuuacauaaa uauuauacua gcauuuacca ucucacuucu aggaauacua        60
   guauaucgcu cacaccucau auccucccua cuaugccuag aaggaauaau acuaucgcug       120
   uucauuauag cuacucucau aacccucaac acccacuccc ucuuagccaa uauugugccu       180
   auugccauac uagucuuugc cgccugcgaa gcagcggugg gccuagcccu acuagucuca       240
   aucuccaaca cauauggccu agacuacgua cauaaccuaa cccuacuccu aaucacauaa       300
<210> 21
   <211> 781
   <212> RNA
   <213> Human
<400> 21
   auggcacaug cagcgcaagu aggucuacaa gacgcuacuu ccccuaucau agaagagcuu 60
   aucaccuuuc augaucacgc ccucauaauc auuuuccuua ucugcuuccu aguccuguau 120
   gcccuuuucc uaacacucac aacaaaacua acuaauacua acaucucaga cgcucaggaa 180
   auagaaaccg ucugaacuau ccugcccgcc aucauccuag uccucaucgc ccucccaucc 240
   cuacgcaucc uuuacauaac agacgagguc aacgaucccu cccuuaccau caaaucaauu 300
   ggccaccaau gguacugaac cuacgaguac accgacuacg gcggacuaau cuucaacucc 360
   uacauacuuc ccccauuauu ccuagaacca ggcgacccag acaauuauac ccuagccaac 420
   cccuuaaaca ccccucccca caucaagccc gaaugauauu uccuauucgc cuacacaauu 480
   cuccgauccg ucccuaacaa acuaggaggc guccuugccc uauuacuauc cauccucauc 540
   cuagcaauaa uccccauccu ccauauaucc aaacaacaaa gcauaauauu ucgcccacua 600
   agccaaucac uuuauugacu ccuagccgca gaccuccuca uucuaaccug aaucggagga 660
   caaccaguaa gcuacccuuu uaccaucauu ggacaaguag cauccguacu auacuucaca 720
   acaauccuaa uccuaauacc aacuaucucc cuaauugaaa acaaaauacu caaaugggcc 780
   u 781
<210> 22
   <211> 565
   <212> RNA
   <213> Human
<400> 22
   auggcacaug cagcgcaagu aggucuacaa gacgcuacuu ccccuaucau agaagagcuu 60
   aucaccuuuc augaucacgc ccucauaauc auuuuccuua ucugcuuccu aguccuguau 120
   gcccuuuucc uaacacucac aacaaaacua acuaauacua acaucucaga cgcucaggaa 180
   auagaaaccg ucugaacuau ccugcccgcc aucauccuag uccucaucgc ccucccaucc 240
   cuacgcaucc uuuacauaac agacgagguc aacgaucccu cccuuaccau caaaucaauu 300
   ggccaccaau gguacugaac cuacgaguac accgacuacg gcggacuaau cuucaacucc 360
   uacauacuuc ccccauuauu ccuagaacca ggcgaccugc gacuccuagc cgcagaccuc 420
   cucauucuaa ccugaaucgg aggacaacca guaagcuacc cuuuuaccau cauuggacaa 480
   guagcauccg uacuauacuu cacaacaauc cuaauccuaa uaccaacuau cucccuaauu 540
   gaaaacaaaa uacucaaaug ggccu 565
<210> 23
   <211> 1174
   <212> RNA
   <213> Human
<400> 23
   auggcacaug cagcgcaagu aggucuacaa gacgcuacuu ccccuaucau agaagagcuu      60
   aucaccuuuc augaucacgc ccucauaauc auuuuccuua ucugcuuccu aguccuguau     120
   gcccuuuucc uaacacucac aacaaaacua acuaauacua acaucucaga cgcucaggaa     180
   auagaaaccg ucugaacuau ccugcccgcc aucauccuag uccucaucgc ccucccaucc     240
   cuacgcaucc uuuacauaac agacgagguc aacgaucccu cccuuaccau caaaucaauu     300
   ggccaccaau gguacugaac cuacgaguac accgacuacg gcggacuaau cuucaacucc     360
   uacauacuuc ccccauuauu ccuagaacca ggcgaccugc gacuccuuga cguugacaau     420
   cgaguaguac ucccgauuga agcccccauu cguauaauaa uuacaucaca agacgucuug     480
   cacucaugag cuguccccac auuaggcuua aaaacagaug caauucccgg acgucuaaac     540
   caaaccacuu ucaccgcuac acgaccgggg guauacuacg gucaaugcuc ugaaaucugu     600
   ggagcaaacc acaguuucau gcccauauuc uugcacgaaa cgggaucaaa caacccccua     660
   ggaaucaccu cccauuccga uaaaaucacc uuccacccuu acuacacaau caaagacgcc     720
   cucggcuuac uucucuuccu ucucuccuua augacauuaa cacuauucuc accagaccuc     780
   cuaggcgacc cagacaauua uacccuagcc aaccccuuaa acaccccucc ccacaucaag     840
   cccgaaugau auuuccuauu cgccuacaca auucuccgau ccgucccuaa caaacuagga     900
   ggcguccuug cccuauuacu auccauccuc auccuagcaa uaauccccau ccuccauaua     960
   uccaaacaac aaagcauaau auuucgccca cuaagccaau cacuuuauug acuccuagcc    1020
   gcagaccucc ucauucuaac cugaaucgga ggacaaccag uaagcuaccc uuuuaccauc    1080
   auuggacaag uagcauccgu acuauacuuc acaacaaucc uaauccuaau accaacuauc    1140
   ucccuaauug aaaacaaaau acucaaaugg gccu                                1174
<210> 24
   <211> 1294
   <212> RNA
   <213> Human
<400> 24
   augaacgaaa aucuguucgc uucauucauu gcccccacaa uccuaggccu acccgccgca      60
   guacugauca uucuauuucc cccucuauug auccccaccu ccaaauaucu caucaacaac     120
   cgacuaauca ccacccaaca augacuaauc aaacuaaccu caaaacaaau gauaaccaua     180
   cacaacacua aaggacgaac cugaucucuu auacuaguau ccuuaaucau uuuuauugcc     240
   acaacuaacc uccucggacu ccugccucac ucauuuacac caaccaccca acuaucuaua     300
   aaccuagcca ugcacuacuc accagacgcc ucaaccgccu uuucaucaau cgcccacauc     360
   acucgagacg uaaauuaugg cugaaucauc cgcuaccuuc acgccaaugg cgccucaaua     420
   uucuuuaucu gccucuuccu acacaucggg cgaggccuau auuacggauc auuucucuac     480
   ucagaaaccu gaaacaucgg cauuauccuc cugcuugcaa cuauagcaac agccuucaua     540
   ggcuaugucc ucccgugagg ccaaauauca uucugagggg ccacaguaau uacaaacuua     600
   cuauccgcca ucccauacau ugggacagac cuaguucaau gaaucugagg aggcuacuca     660
   guagacaguc ccacccucac acgauucuuu accuuucacu ucaucuugcc cuucauuauu     720
   gcagcccuag caacacucca ccuccuauuc uugcacgaaa cgggaucaaa caacccccua     780
   ggaaucaccu cccauuccga uaaaaucacc uuccacccuu acuacacaau caaagacgcc     840
   cucggcuuac uucucuuccu ucucuccuua augacauuaa cacuauucuc accagaccuc     900
   cuaggcgacc cagacaauua uacccuagcc aaccccuuaa acaccccucc ccacaucaag     960
   cccgaaugau auuuccuauu cgccuacaca auucuccgau ccgucccuaa caaacuagga    1020
   ggcguccuug cccuauuacu auccauccuc auccuagcaa uaauccccau ccuccauaua    1080
   uccaaacaac aaagcauaau auuucgccca cuaagccaau cacuuuauug acuccuagcc    1140
   gcagaccucc ucauucuaac cugaaucgga ggacaaccag uaagcuaccc uuuuaccauc    1200
   auuggacaag uagcauccgu acuauacuuc acaacaaucc uaauccuaau accaacuauc    1260
   ucccuaauug aaaacaaaau acucaaaugg gccu                                1294
<210> 25
   <211> 1228
   <212> RNA
   <213> Human
<400> 25
   augccccuca uuuacauaaa uauuauacua gcauuuacca ucucacuucu aggaauacua 60
   guauaucgcu cacaccucau auccucccua cuaugccuag aaggaauaau acuaucgcug 120
   uucauuauag cuacucucau aacccucaac acccacuccc ucuuagccaa uauugugccu 180
   auugccauac uagucuuugg cgccugccug auccuccaaa ucaccacagg acuauuccua 240
   gccaugcacu acucaccaga cgccucaacc gccuuuucau caaucgccca caucacucga 300
   gacguaaauu auggcugaau cauccgcuac cuucacgcca auggcgccuc aauauucuuu 360
   aucugccucu uccuacacau cgggcgaggc cuauauuacg gaucauuucu cuacucagaa 420
   accugaaaca ucggcauuau ccuccugcuu gcaacuauag caacagccuu cauaggcuau 480
   guccucccgu gaggccaaau aucauucuga ggggccacag uaauuacaaa cuuacuaucc 540
   gccaucccau acauugggac agaccuaguu caaugaaucu gaggaggcua cucaguagac 600
   agucccaccc ucacacgauu cuuuaccuuu cacuucaucu ugcccuucau uauugcagcc 660
   cuagcaacac uccaccuccu auucuugcac gaaacgggau caaacaaccc ccuaggaauc 720
   accucccauu ccgauaaaau caccuuccac ccuuacuaca caaucaaaga cgcccucggc 780
   uuacuucucu uccuucucuc cuuaaugaca uuaacacuau ucucaccaga ccuccuaggc 840
   gacccagaca auuauacccu agccaacccc uuaaacaccc cuccccacau caagcccgaa 900
   ugauauuucc uauucgccua cacaauucuc cgauccgucc cuaacaaacu aggaggcguc 960
   cuugcccuau uacuauccau ccucauccua gcaauaaucc ccauccucca uauauccaaa 1020
   caacaaagca uaauauuucg cccacuaagc caaucacuuu auugacuccu agccgcagac 1080
   cuccucauuc uaaccugaau cggaggacaa ccaguaagcu acccuuuuac caucauugga 1140
   caaguagcau ccguacuaua cuucacaaca auccuaaucc uaauaccaac uaucucccua 1200
   auugaaaaca aaauacucaa augggccu 1228
<210> 26
   <211> 522
   <212> RNA
   <213> Human
<400> 26
   auguucgccg accguugacu auucucuaca aaccacaaag acauuggaac acuauaccua 60
   uuauucggcg caugagcugg aguccuaggc acagcucuaa gccuccuuau ucgagccgag 120
   cugggccagc caggcaaccu ucuagguaac gaccacaucu acaacguuau cgucacagcc 180
   cucgcuguca cuuuccuagg acuucuaaca gcccuagacc ucaacuaccu aaccaacaaa 240
   cuuaaaauaa aauccccacu augcacauuu uauuucucca acauacucgg auucuacccu 300
   agcaucacac accgcacaau ccccuaucua ggccuucuua cgagccaaaa ccugccccua 360
   cuccuccuag accuaaccug acuagaaaag cuauuaccua aaacaauuuc acagcaccaa 420
   aucuccaccu ccaucaucac cucaacccaa aaaggcauaa uuaaacuuua cuuccucucu 480
   uucuucuucc cacucauccu aacccuacuc cuaaucacau aa 522
<210> 27
   <211> 582
   <212> RNA
   <213> Human
<400> 27
   auguucgccg accguugacu auucucuaca aaccacaaag acauuggaac acuauaccua 60
   uuauucggcg caugagcugg aguccuaggc acagcucuaa gccuccuuau ucgagccgag 120
   cugggccagc caggcaaccu ucuagguaac gaccacaucu acaacguuau cgucacagcc 180
   caugcauuug uaauaaucuu cuucauagua auacccauca uaaucggagg cuuuggcaac 240
   ugacuaguuc cccuaauaau cggugccccc gauauggcgu uuccccgcau aaacaacaua 300
   agcuucugac ucuuaccucc cucucuccua cuccugcucg caucugcuau aguggaggcc 360
   ggagcaggaa cagguugaac agucuacccu cccuuagcag ggaacuacuc ccacccugga 420
   gcccuccuag accuaaccug acuagaaaag cuauuaccua aaacaauuuc acagcaccaa 480
   aucuccaccu ccaucaucac cucaacccaa aaaggcauaa uuaaacuuua cuuccucucu 540
   uucuucuucc cacucauccu aacccuacuc cuaaucacau aa 582
<210> 28
   <211> 2208
   <212> RNA
   <213> Human
<400> 28
   auguucgccg accguugacu auucucuaca aaccacaaag acauuggaac acuauaccua      60
   uuauucggcg caugagcugg aguccuaggc acagcucuaa gccuccuuau ucgagccgag     120
   cugggccagc caggcaaccu ucuagguaac gaccacaucu acaacguuau cgucacagcc     180
   caugcauuug uaauaaucuu cuucauagua auacccauca uaaucggagg cuuuggcaac     240
   ugacuaguuc cccuaauaau cggugccccc gauauggcgu uuccccgcau aaacaacaua     300
   agcuucugac ucuuaccucc cucucuccua cuccugcucg caucugcuau aguggaggcc     360
   ggagcaggaa cagguugaac agucuacccu cccuuagcag ggaacuacuc ccacccugga     420
   gccuccguag accuaaccau cuucuccuua caccuagcag gugucuccuc uaucuuaggg     480
   gccaucaauu ucaucacaac aauuaucaau auaaaacccc cugccauaac ccaauaccaa     540
   acgccccucu ucgucugauc cguccuaauc acagcagucc uacuucuccu aucucuccca     600
   guccuagcug cuggcaucac uauacuacua acagaccgca accucaacac caccuucuuc     660
   gaccccgccg gaggaggaga ccccauucua uaccaacacc uauucugauu uuucggucac     720
   ccugaaguuu auauucuuau ccuaccaggc uucggaauaa ucucccauau uguaacuuac     780
   uacuccggaa aaaaagaacc auuuggauac auagguaugg ucugagcuau gauaucaauu     840
   ggcuuccuag gguuuaucgu gugagcacac cauauauuua caguaggaau agacguagac     900
   acacgagcau auuucaccuc cgcuaccaua aucaucgcua uccccaccgg cgucaaagua     960
   uuuagcugac ucgccacacu ccacggaagc aauaugaaau gaucugcugc agugcucuga    1020
   gcccuaggau ucaucuuucu uuucaccgua gguggccuga cuggcauugu auuagcaaac    1080
   ucaucacuag acaucguacu acacgacacg uacuacguug uagcccacuu ccacuauguc    1140
   cuaucaauag gagcuguauu ugccaucaua ggaggcuuca uucacugauu uccccuauuc    1200
   ucaggcuaca cccuagacca aaccuacgcc aaaauccauu ucacuaucau auucaucggc    1260
   guaaaucuaa cuuucuuccc acaacacuuu cucggccuau ccggaaugcc ccgacguuac    1320
   ucggacuacc ccgaugcaua caccacauga aacauccuau caucuguagg cucauucauu 1380
   ucucuaacag caguaauauu aauaauuuuc augauuugag aagccuucgc uucgaagcga 1440
   aaaguccuaa uaguagaaga acccuccaua aaccuggagu gacuauaugg augcccccca 1500
   cccuaccaca cauucgaaga acccguauac auaaaagcag gaauaccuuu ccucacaggu 1560
   uucuacucca aagaccacau caucgaaacc gcaaacauau cauacacaaa cgccugagcc 1620
   cuaucuauua cucucaucgc uaccucccug acaagcgccu auagcacucg aauaauucuu 1680
   cucacccuaa caggucaacc ucgcuucccc acccuuacua acauuaacga aaauaacccc 1740
   acccuacuaa accccauuaa acgccuggca gccggaagcc uauucgcagg auuucucauu 1800
   acuaacaaca uuucccccgc aucccccuuc caaacaacaa ucccccucua ccuaaaacuc 1860
   acagcccucg cugucacuuu ccuaggacuu cuaacagccc uagaccucaa cuaccuaacc 1920
   aacaaacuua aaauaaaauc cccacuaugc acauuuuauu ucuccaacau acucggauuc 1980
   uacccuagca ucacacaccg cacaaucccc uaucuaggcc uucuuacgag ccaaaaccug 2040
   ccccuacucc uccuagaccu aaccugacua gaaaagcuau uaccuaaaac aauuucacag 2100
   caccaaaucu ccaccuccau caucaccuca acccaaaaag gcauaauuaa acuuuacuuc 2160
   cucucuuucu ucuucccacu cauccuaacc cuacuccuaa ucacauaa 2208
<210> 29
   <211> 807
   <212> RNA
   <213> Human
<400> 29
   auggcacaug cagcgcaagu aggucuacaa gacgcuacuu ccccuaucau agaagagcuu 60
   aucaccuuuc augaucacgc ccucauaauc auuuuccuua ucugcuuccu aguccuguau 120
   gcccuuuucc uaacacucac aacaaaacua acuaauacua acaucucaga cgcucaggaa 180
   auagaaaccg caaacauauc auacacaaac gccugagccc uaucuauuac ucucaucgcu 240
   accucccuga caagcgccua uagcacucga auaauucuuc ucacccuaac aggucaaccu 300
   cgcuucccca cccuuacuaa cauuaacgaa aauaacccca cccuacuaaa ccccauuaaa 360
   cgccuggcag ccggaagccu auucgcagga uuucucauua cuaacaacau uucccccgca 420
   ucccccuucc aaacaacaau cccccucuac cuaaaacuca cagcccucgc ugucacuuuc 480
   cuaggacuuc uaacagcccu agaccucaac uaccuaacca acaaacuuaa aauaaaaucc 540
   ccacuaugca cauuuuauuu cuccaacaua cucggauucu acccuagcau cacacaccgc 600
   acaauccccu aucuaggccu ucuuacgagc caaaaccugc cccuacuccu ccuagaccua 660
   accugacuag aaaagcuauu accuaaaaca auuucacagc accaaaucuc caccuccauc 720
   aucaccucaa cccaaaaagg cauaauuaaa cuuuacuucc ucucuuucuu cuucccacuc 780
   auccuaaccc uacuccuaau cacauaa 807
<210> 30
   <211> 786
   <212> RNA
   <213> Human
<400> 30
   auggcacaug cagcgcaagu aggucuacaa gacgcuacuu ccccuaucau agaagagcuu 60
   aucaccuuuc augaucacgc ccucauaauc auuuuccuua ucugcuuccu aguccuguau 120
   gcccuuuucc uaacacucac aacaaaacua acuaauacua acaucucaga cgcucaggaa 180
   auagaaaccg ucugaacuau ccugcccgcc aucauccuag uccucaucgc ccucccaucc 240
   cuacgcaucc uuuacauaac agacgagguc aacgaucccu cccuuaccau caaaucaauu 300
   ggccaccaau gguacugaac cuacgaguac accgacuacg gcggacuaau cuucaacucc 360
   uacauacuuc ccccauuauu ccuagaacca ggcgaccugc gacuccuuga cguugacaau 420
   cgaguaguac ucccgauuga agcccccauu cguauaauaa uuacaucaca agacgucuug 480
   cacucaugag cuguccccac auuaggcuua aaaacagaug caauucccgg acgucuaaac 540
   caaaccacuu ucaccgcuac acgaccgggg guauacuacg gucaaugcuc ugaaaucugu 600
   ggagcaaacc acaguuucau gcccaucguc cuagaccuaa ccugacuaga aaagcuauua 660
   ccuaaaacaa uuucacagca ccaaaucucc accuccauca ucaccucaac ccaaaaaggc 720
   auaauuaaac uuuacuuccu cucuuucuuc uucccacuca uccuaacccu acuccuaauc 780
   acauaa 786
<210> 31
   <211> 1905
   <212> RNA
   <213> Human
<400> 31
   augaacgaaa aucuguucgc uucauucauu gcccccacaa uccuaggccu acccgccgca 60
   guacugauca uucuauuucc cccucuauug auccccaccu ccaaauaucu caucaacaac 120
   cgacuaauca ccacccaaca augacuaauc aaacuaaccu caaaacaaau gauaaccaua 180
   cacaacacua aaggacgaac cugaucucuu auacuaguau ccuuaaucau uuuuauugcc 240
   acaacuaacc uccucggacu ccugccucac ucauuuacac caaccaccca acuaucuaua 300
   aaccuagcca uggccauccc cuuaugagcg ggcacaguga uuauaggcuu ucgcucuaag 360
   auuaaaaaug cccuagccca cuucuuacca caaggcacac cuacaccccu uauccccaua 420
   cuaguuauua ucgaaaccau cagccuacuc auucaaccaa uagcccuggc cguacgccua 480
   accgcuaaca uuacugcagg ccaccuacuc augcaccuaa uuggaagcgc cacccuagca 540
   auaucaacca uuaaccuucc cucuacacuu aucaucuuca caauucuaau ucuacugacu 600
   auccuagaaa ucgcugucgc cuuaauccaa gccuacguuu ucacacuucu aguaagccuc 660
   uaccuacacu ccaacucaug agacccacaa caaauagccc uucuaaacgc uaauccaagc 720
   cucaccccac uacuaggccu ccuccuagca gcagcaggca aaucagccca auuaggucuc 780
   caccccugac uccccucagc cauagaaggc cccaccccag ucucagcccu acuccacuca 840
   agcacuauag uuguagcagg aaucuucuua cucauccgcu uccacccccu agcagaaaau 900
   agcccacuaa uccaaacucu aacacuaugc uuaggcgcua ucaccacucu guucgcagca 960
   gucugcgccc uuacacaaaa ugacaucaaa aaaaucguag ccuucuccac uucaagucaa 1020
   cuaggacuca uaauaguuac aaucggcauc aaccaaccac accuagcauu ccugcacauc 1080
   uguacccacg ccuucuucaa agccauacua uuuaugugcu ccggguccau cauccacaac 1140
   cuuaacaaug aacaagauau ucgaaaaaua ggaggacuac ucaaaaccau accucucacu 1200
   ucaaccuccc ucaccauugg cagccuagca uuagcaggaa uaccuuuccu cacagguuuc 1260
   uacuccaaag accacaucau cgaaaccgca aacauaucau acacaaacgc cugagcccua 1320
   ucuauuacuc ucaucgcuac cucccugaca agcgccuaua gcacucgaau aauucuucuc 1380
   acccuaacag gucaaccucg cuuccccacc cuuacuaaca uuaacgaaaa uaaccccacc 1440
   cuacuaaacc ccauuaaacg ccuggcagcc ggaagccuau ucgcaggauu ucucauuacu 1500
   aacaacauuu cccccgcauc ccccuuccaa acaacaaucc cccucuaccu aaaacucaca 1560
   gcccucgcug ucacuuuccu aggacuucua acagcccuag accucaacua ccuaaccaac 1620
   aaacuuaaaa uaaaaucccc acuaugcaca uuuuauuucu ccaacauacu cggauucuac 1680
   ccuagcauca cacaccgcac aauccccuau cuaggccuuc uuacgagcca aaaccugccc 1740
   cuacuccucc uagaccuaac cugacuagaa aagcuauuac cuaaaacaau uucacagcac 1800
   caaaucucca ccuccaucau caccucaacc caaaaaggca uaauuaaacu uuacuuccuc 1860
   ucuuucuucu ucccacucau ccuaacccua cuccuaauca cauaa 1905
<210> 32
   <211> 1545
   <212> RNA
   <213> Human
<400> 32
   augacccacc aaucacaugc cuaucauaua guaaaaccca gcccaugacc ccuaacaggg      60
   gcccucucag cccuccuaau gaccuccggc cuagccaugu gauuucacuu ccacuccaua     120
   acgcuccuca uacuaggccu acuaaccaac acacuaacca uauaccaaug auggcgcgau     180
   guaacacgag aaagcacaua ccaaggccac cacacaccac cuguccaaaa aggccuucga     240
   uacgggauaa uccuauuuau uaccucagaa guuuuuuucu ucgcaggauu uuucugagcc     300
   uuuuaccacu ccagccuagc cccuaccccc caauuaggag ggcacuggcc cccaacaggc     360
   aucaccccac uacuaggccu ccuccuagca gcagcaggca aaucagccca auuaggucuc     420
   caccccugac uccccucagc cauagaaggc cccaccccag ucucagcccu acuccacuca     480
   agcacuauag uuguagcagg aaucuucuua cucauccgcu uccacccccu agcagaaaau     540
   agcccacuaa uccaaacucu aacacuaugc uuaggcgcua ucaccacucu guucgcagca     600
   gucugcgccc uuacacaaaa ugacaucaaa aaaaucguag ccuucuccac uucaagucaa     660
   cuaggacuca uaauaguuac aaucggcauc aaccaaccac accuagcauu ccugcacauc     720
   uguacccacg ccuucuucaa agccauacua uuuaugugcu ccggguccau cauccacaac     780
   cuuaacaaug aacaagauau ucgaaaaaua ggaggacuac ucaaaaccau accucucacu     840
   ucaaccuccc ucaccauugg cagccuagca uuagcaggaa uaccuuuccu cacagguuuc     900
   uacuccaaag accacaucau cgaaaccgca aacauaucau acacaaacgc cugagcccua     960
   ucuauuacuc ucaucgcuac cucccugaca agcgccuaua gcacucgaau aauucuucuc    1020
   acccuaacag gucaaccucg cuuccccacc cuuacuaaca uuaacgaaaa uaaccccacc    1080
   cuacuaaacc ccauuaaacg ccuggcagcc ggaagccuau ucgcaggauu ucucauuacu    1140
   aacaacauuu cccccgcauc ccccuuccaa acaacaaucc cccucuaccu aaaacucaca    1200
   gcccucgcug ucacuuuccu aggacuucua acagcccuag accucaacua ccuaaccaac    1260
   aaacuuaaaa uaaaaucccc acuaugcaca uuuuauuucu ccaacauacu cggauucuac    1320
   ccuagcauca cacaccgcac aauccccuau cuaggccuuc uuacgagcca aaaccugccc    1380
   cuacuccucc uagaccuaac cugacuagaa aagcuauuac cuaaaacaau uucacagcac    1440
   caaaucucca ccuccaucau caccucaacc caaaaaggca uaauuaaacu uuacuuccuc    1500
   ucuuucuucu ucccacucau ccuaacccua cuccuaauca cauaa                    1545
<210> 33
   <211> 1629
   <212> RNA
   <213> Human
<400> 33
   auaaacuucg ccuuaauuuu aauaaucaac acccuccuag ccuuacuacu aauaauuauu 60
   acauuuugac uaccacaacu caacggcuac auagaaaaau ccaccccuua cgagugcggc 120
   uucgacccua uaucccccgc ccgcgucccu uucuccauaa aauucuucuu aguagcuauu 180
   accuucuuau uauuugaucu agaaauugcc cuccuuuuac cccuaccaug agcccuacaa 240
   acaacuaacc ugccacuaau aguuauguca ucccucuuau uaaucaucau ccuagcccua 300
   agucuggcca acacagcagc cauucaagca auccuauaca accguaucgg cgauaucggu 360
   uucauccucg ccuuagcaug auuuauccua cacuccaacu caugagaccc acaacaaaua 420
   gcccuucuaa acgcuaaucc aagccucacc ccacuacuag gccuccuccu agcagcagca 480
   ggcaaaucag cccaauuagg ucuccacccc ugacuccccu cagccauaga aggccccacc 540
   ccagucucag cccuacucca cucaagcacu auaguuguag caggaaucuu cuuacucauc 600
   cgcuuccacc cccuagcaga aaauagccca cuaauccaaa cucuaacacu augcuuaggc 660
   gcuaucacca cucuguucgc agcagucugc gcccuuacac aaaaugacau caaaaaaauc 720
   guagccuucu ccacuucaag ucaacuagga cucauaauag uuacaaucgg caucaaccaa 780
   ccacaccuag cauuccugca caucuguacc cacgccuucu ucaaagccau acuauuuaug 840
   ugcuccgggu ccaucaucca caaccuuaac aaugaacaag auauucgaaa aauaggagga 900
   cuacucaaaa ccauaccucu cacuucaacc ucccucacca uuggcagccu agcauuagca 960
   ggaauaccuu uccucacagg uuucuacucc aaagaccaca ucaucgaaac cgcaaacaua 1020
   ucauacacaa acgccugagc ccuaucuauu acucucaucg cuaccucccu gacaagcgcc 1080
   uauagcacuc gaauaauucu ucucacccua acaggucaac cucgcuuccc cacccuuacu 1140
   aacauuaacg aaaauaaccc cacccuacua aaccccauua aacgccuggc agccggaagc 1200
   cuauucgcag gauuucucau uacuaacaac auuucccccg caucccccuu ccaaacaaca 1260
   aucccccucu accuaaaacu cacagcccuc gcugucacuu uccuaggacu ucuaacagcc 1320
   cuagaccuca acuaccuaac caacaaacuu aaaauaaaau ccccacuaug cacauuuuau 1380
   uucuccaaca uacucggauu cuacccuagc aucacacacc gcacaauccc cuaucuaggc 1440
   cuucuuacga gccaaaaccu gccccuacuc cuccuagacc uaaccugacu agaaaagcua 1500
   uuaccuaaaa caauuucaca gcaccaaauc uccaccucca ucaucaccuc aacccaaaaa 1560
   ggcauaauua aacuuuacuu ccucucuuuc uucuucccac ucauccuaac ccuacuccua 1620
   aucacauaa 1629
<210> 34
   <211> 642
   <212> RNA
   <213> Human
<400> 34
   augcuaaaac uaaucguccc aacaauuaua uuacuaccac ugacaugacu uuccaaaaaa      60
   cacauaauuu gaaucaacac aaccacccac agccuaauua uuagcaucau cccucuacua     120
   uuuuuuaacc aaaucaacaa caaccuauuu agcuguuccc caaccuuuuc cuccgacccc     180
   cuaacaaccc cccuccuaau acuaacuacc ugacuccuac cccucacaau cauggcaagc     240
   caacgccacu uauccaguga accacuauca cgaaaaaaac ucuaccucuc uauacuaauc     300
   ucccuacaaa ucuccuuaau uauaacauuc acagccacag aacuaaucau auuuuauauc     360
   uucuucgaaa ccacacuuau ccccaccuug gcuaucauca cccgaugagg caaccagcca     420
   gaacgccuga acgcaggcac auacuuccua uucuacaccc uaguaggcuc ccugccccua     480
   cuccuccuag accuaaccug acuagaaaag cuauuaccua aaacaauuuc acagcaccaa     540
   aucuccaccu ccaucaucac cucaacccaa aaaggcauaa uuaaacuuua cuuccucucu     600
   uucuucuucc cacucauccu aacccuacuc cuaaucacau aa                         642
<210> 35
   <211> 129
   <212> RNA
   <213> Human
<400> 35
   augccccaac uaaauacuac cguauggccc accauaauua cccccauacu ccuuacacua      60
   uuccucauca cccaacuaaa aauauuaaac acaaacuacc accuaccucc cucaccauug     120
   gcagccuag                                                             129
<210> 36
   <211> 261
   <212> PRT
   <213> Artificial
<220>
   <223> putative protein sequence
<220>
   <221> misc_feature
   <222> (261)..(261)
   <223> Xaa can be any naturally occurring amino acid
<400> 36
   Met Ala His His Asn Tyr Pro His Thr Pro Tyr Thr Ile Pro His His
   1               5                   10                  15
   Pro Thr Lys Asn Ile Lys His Lys Leu Pro Pro Thr Ser Leu Thr Ile
               20                  25                  30
   Gly Ser Leu Ala Leu Ala Gly Met Pro Phe Leu Thr Gly Phe Tyr Ser
           35                  40                  45
   Lys Asp His Ile Ile Glu Thr Ala Asn Met Ser Tyr Thr Asn Ala Trp
       50                  55                  60
   Ala Leu Ser Ile Thr Leu Ile Ala Thr Ser Leu Thr Ser Ala Tyr Ser
   65                  70                  75                  80
   Thr Arg Met Ile Leu Leu Thr Leu Thr Gly Gln Pro Arg Phe Pro Thr
                   85                  90                  95
   Leu Thr Asn Ile Asn Glu Asn Asn Pro Thr Leu Leu Asn Pro Ile Lys
   100 105 110
   Arg Leu Ala Ala Gly Ser Leu Phe Ala Gly Phe Leu Ile Thr Asn Asn
   115 120 125
   Ile Ser Pro Ala Ser Pro Phe Gln Thr Thr Ile Pro Leu Tyr Leu Lys
   130 135 140
   Leu Thr Ala Leu Ala Val Thr Phe Leu Gly Leu Leu Thr Ala Leu Asp
   145 150 155 160
   Leu Asn Tyr Leu Thr Asn Lys Leu Lys Met Lys Ser Pro Leu Cys Thr
   165 170 175
   Phe Tyr Phe Ser Asn Met Leu Gly Phe Tyr Pro Ser Ile Thr His Arg
   180 185 190
   Thr Ile Pro Tyr Leu Gly Leu Leu Thr Ser Gln Asn Leu Pro Leu Leu
   195 200 205
   Leu Leu Asp Leu Thr Trp Leu Glu Lys Leu Leu Pro Lys Thr Ile Ser
   210 215 220
   Gln His Gln Ile Ser Thr Ser Ile Ile Thr Ser Thr Gln Lys Gly Met
   225 230 235 240
   Ile Lys Leu Tyr Phe Leu Ser Phe Phe Phe Pro Leu Ile Leu Thr Leu
   245 250 255
   Leu Leu Ile Thr Xaa
   260
<210> 37
   <211> 100
   <212> PRT
   <213> Artificial
<220>
   <223> putative protein sequence
<220>
   <221> misc_feature
   <222> (100)..(100)
   <223> Xaa can be any naturally occurring amino acid
<400> 37
   Met Pro Leu Ile Tyr Met Asn Ile Met Leu Ala Phe Thr Ile Ser Leu
   1 5 10 15
   Leu Gly Met Leu Val Tyr Arg Ser His Leu Met Ser Ser Leu Leu Cys
   20 25 30
   Leu Glu Gly Met Met Leu Ser Leu Phe Ile Met Ala Thr Leu Met Thr
   35 40 45
   Leu Asn Thr His Ser Leu Leu Ala Asn Ile Val Pro Ile Ala Met Leu
   50 55 60
   Val Phe Ala Ala Cys Glu Ala Ala Val Gly Leu Ala Leu Leu Val Ser
   65 70 75 80
   Ile Ser Asn Thr Tyr Gly Leu Asp Tyr Val His Asn Leu Thr Leu Leu
   85 90 95
   Leu Ile Thr Xaa
   100
<210> 38
   <211> 261
   <212> PRT
   <213> Artificial
<220>
   <223> putative protein sequence
<220>
   <221> misc_feature
   <222> (261) .. (261)
   <223> Xaa can be any naturally occurring amino acid
<400> 38
   Met Ala His Ala Ala Gln Val Gly Leu Gln Asp Ala Thr Ser Pro Ile
   1 5 10 15
   Met Glu Glu Leu Ile Thr Phe His Asp His Ala Leu Met Ile Ile Phe
   20 25 30
   Leu Ile Cys Phe Leu Val Leu Tyr Ala Leu Phe Leu Thr Leu Thr Thr
   35 40 45
   Lys Leu Thr Asn Thr Asn Ile Ser Asp Ala Gln Glu Met Glu Thr Val
   50 55 60
   Trp Thr Ile Leu Pro Ala Ile Ile Leu Val Leu Ile Ala Leu Pro Ser
   65 70 75 80
   Leu Arg Ile Leu Tyr Met Thr Asp Glu Val Asn Asp Pro Ser Leu Thr
   85 90 95
   Ile Lys Ser Ile Gly His Gln Trp Tyr Trp Thr Tyr Glu Tyr Thr Asp
   100 105 110
   Tyr Gly Gly Leu Ile Phe Asn Ser Tyr Met Leu Pro Pro Leu Phe Leu
   115 120 125
   Glu Pro Gly Asp Pro Asp Asn Tyr Thr Leu Ala Asn Pro Leu Asn Thr
   130 135 140
   Pro Pro His Ile Lys Pro Glu Trp Tyr Phe Leu Phe Ala Tyr Thr Ile
   145 150 155 160
   Leu Arg Ser Val Pro Asn Lys Leu Gly Gly Val Leu Ala Leu Leu Leu
   165 170 175
   Ser Ile Leu Ile Leu Ala Met Ile Pro Ile Leu His Met Ser Lys Gln
   180 185 190
   Gln Ser Met Met Phe Arg Pro Leu Ser Gln Ser Leu Tyr Trp Leu Leu
   195 200 205
   Ala Ala Asp Leu Leu Ile Leu Thr Trp Ile Gly Gly Gln Pro Val Ser
   210 215 220
   Tyr Pro Phe Thr Ile Ile Gly Gln Val Ala Ser Val Leu Tyr Phe Thr
   225 230 235 240
   Thr Ile Leu Ile Leu Met Pro Thr Ile Ser Leu Ile Glu Asn Lys Met
   245 250 255
   Leu Lys Trp Ala Xaa
   260
<210> 39
   <211> 189
   <212> PRT
   <213> Artificial
<220>
   <223> putative protein sequence
<220>
   <221> misc_feature
   <222> (189)..(189)
   <223> Xaa can be any naturally occurring amino acid
<400> 39
   Met Ala His Ala Ala Gln Val Gly Leu Gln Asp Ala Thr Ser Pro Ile
   1 5 10 15
   Met Glu Glu Leu Ile Thr Phe His Asp His Ala Leu Met Ile Ile Phe
   20 25 30
   Leu Ile Cys Phe Leu Val Leu Tyr Ala Leu Phe Leu Thr Leu Thr Thr
   35 40 45
   Lys Leu Thr Asn Thr Asn Ile Ser Asp Ala Gln Glu Met Glu Thr Val
   50 55 60
   Trp Thr Ile Leu Pro Ala Ile Ile Leu Val Leu Ile Ala Leu Pro Ser
   65 70 75 80
   Leu Arg Ile Leu Tyr Met Thr Asp Glu Val Asn Asp Pro Ser Leu Thr
   85 90 95
   Ile Lys Ser Ile Gly His Gln Trp Tyr Trp Thr Tyr Glu Tyr Thr Asp
   100 105 110
   Tyr Gly Gly Leu Ile Phe Asn Ser Tyr Met Leu Pro Pro Leu Phe Leu
   115 120 125
   Glu Pro Gly Asp Leu Arg Leu Leu Ala Ala Asp Leu Leu Ile Leu Thr
   130 135 140
   Trp Ile Gly Gly Gln Pro Val Ser Tyr Pro Phe Thr Ile Ile Gly Gln
   145 150 155 160
   Val Ala Ser Val Leu Tyr Phe Thr Thr Ile Leu Ile Leu Met Pro Thr
   165 170 175
   Ile Ser Leu Ile Glu Asn Lys Met Leu Lys Trp Ala Xaa
   180 185
<210> 40
   <211> 392
   <212> PRT
   <213> Artificial
<220>
   <223> putative protein sequence
<220>
   <221> misc_feature
   <222> (392)..(392)
   <223> Xaa can be any naturally occurring amino acid
<400> 40
   Met Ala His Ala Ala Gln Val Gly Leu Gln Asp Ala Thr Ser Pro Ile
   1               5                   10                  15
   Met Glu Glu Leu Ile Thr Phe His Asp His Ala Leu Met Ile Ile Phe
               20                  25                  30
   Leu Ile Cys Phe Leu Val Leu Tyr Ala Leu Phe Leu Thr Leu Thr Thr
           35                  40                  45
   Lys Leu Thr Asn Thr Asn Ile Ser Asp Ala Gln Glu Met Glu Thr Val
       50                  55                  60
   Trp Thr Ile Leu Pro Ala Ile Ile Leu Val Leu Ile Ala Leu Pro Ser
   65                  70                  75                  80
   Leu Arg Ile Leu Tyr Met Thr Asp Glu Val Asn Asp Pro Ser Leu Thr
                   85                  90                  95
   Ile Lys Ser Ile Gly His Gln Trp Tyr Trp Thr Tyr Glu Tyr Thr Asp
               100                 105                 110
   Tyr Gly Gly Leu Ile Phe Asn Ser Tyr Met Leu Pro Pro Leu Phe Leu
           115                 120                 125
   Glu Pro Gly Asp Leu Arg Leu Leu Asp Val Asp Asn Arg Val Val Leu
       130                 135                 140
   Pro Ile Glu Ala Pro Ile Arg Met Met Ile Thr Ser Gln Asp Val Leu
   145                 150                 155                 160
   His Ser Trp Ala Val Pro Thr Leu Gly Leu Lys Thr Asp Ala Ile Pro
                   165                 170                 175
   Gly Arg Leu Asn Gln Thr Thr Phe Thr Ala Thr Arg Pro Gly Val Tyr
               180                 185                 190
   Tyr Gly Gln Cys Ser Glu Ile Cys Gly Ala Asn His Ser Phe Met Pro
           195                 200                 205
   Met Phe Leu His Glu Thr Gly Ser Asn Asn Pro Leu Gly Ile Thr Ser
       210                 215                 220
   His Ser Asp Lys Ile Thr Phe His Pro Tyr Tyr Thr Ile Lys Asp Ala
   225                 230                 235                 240
   Leu Gly Leu Leu Leu Phe Leu Leu Ser Leu Met Thr Leu Thr Leu Phe
                   245                 250                 255
   Ser Pro Asp Leu Leu Gly Asp Pro Asp Asn Tyr Thr Leu Ala Asn Pro
   260 265 270
   Leu Asn Thr Pro Pro His Ile Lys Pro Glu Trp Tyr Phe Leu Phe Ala
   275 280 285
   Tyr Thr Ile Leu Arg Ser Val Pro Asn Lys Leu Gly Gly Val Leu Ala
   290 295 300
   Leu Leu Leu Ser Ile Leu Ile Leu Ala Met Ile Pro Ile Leu His Met
   305 310 315 320
   Ser Lys Gln Gln Ser Met Met Phe Arg Pro Leu Ser Gln Ser Leu Tyr
   325 330 335
   Trp Leu Leu Ala Ala Asp Leu Leu Ile Leu Thr Trp Ile Gly Gly Gln
   340 345 350
   Pro Val Ser Tyr Pro Phe Thr Ile Ile Gly Gln Val Ala Ser Val Leu
   355 360 365
   Tyr Phe Thr Thr Ile Leu Ile Leu Met Pro Thr Ile Ser Leu Ile Glu
   370 375 380
   Asn Lys Met Leu Lys Trp Ala Xaa
   385 390
<210> 41
   <211> 432
   <212> PRT
   <213> Artificial
<220>
   <223> putative protein sequence
<220>
   <221> misc_feature
   <222> (432)..(432)
   <223> Xaa can be any naturally occurring amino acid
<400> 41
   Met Asn Glu Asn Leu Phe Ala Ser Phe Ile Ala Pro Thr Ile Leu Gly
   1 5 10 15
   Leu Pro Ala Ala Val Leu Ile Ile Leu Phe Pro Pro Leu Leu Ile Pro
   20 25 30
   Thr Ser Lys Tyr Leu Ile Asn Asn Arg Leu Ile Thr Thr Gln Gln Trp
   35 40 45
   Leu Ile Lys Leu Thr Ser Lys Gln Met Met Thr Met His Asn Thr Lys
       50                  55                  60
   Gly Arg Thr Trp Ser Leu Met Leu Val Ser Leu Ile Ile Phe Ile Ala
   65                  70                  75                  80
   Thr Thr Asn Leu Leu Gly Leu Leu Pro His Ser Phe Thr Pro Thr Thr
                   85                  90                  95
   Gln Leu Ser Met Asn Leu Ala Met His Tyr Ser Pro Asp Ala Ser Thr
               100                 105                 110
   Ala Phe Ser Ser Ile Ala His Ile Thr Arg Asp Val Asn Tyr Gly Trp
           115                 120                 125
   Ile Ile Arg Tyr Leu His Ala Asn Gly Ala Ser Met Phe Phe Ile Cys
       130                 135                 140
   Leu Phe Leu His Ile Gly Arg Gly Leu Tyr Tyr Gly Ser Phe Leu Tyr
   145                 150                 155                 160
   Ser Glu Thr Trp Asn Ile Gly Ile Ile Leu Leu Leu Ala Thr Met Ala
                   165                 170                 175
   Thr Ala Phe Met Gly Tyr Val Leu Pro Trp Gly Gln Met Ser Phe Trp
               180                 185                 190
   Gly Ala Thr Val Ile Thr Asn Leu Leu Ser Ala Ile Pro Tyr Ile Gly
           195                 200                 205
   Thr Asp Leu Val Gln Trp Ile Trp Gly Gly Tyr Ser Val Asp Ser Pro
       210                 215                 220
   Thr Leu Thr Arg Phe Phe Thr Phe His Phe Ile Leu Pro Phe Ile Ile
   225                 230                 235                 240
   Ala Ala Leu Ala Thr Leu His Leu Leu Phe Leu His Glu Thr Gly Ser
                   245                 250                 255
   Asn Asn Pro Leu Gly Ile Thr Ser His Ser Asp Lys Ile Thr Phe His
               260                 265                 270
   Pro Tyr Tyr Thr Ile Lys Asp Ala Leu Gly Leu Leu Leu Phe Leu Leu
           275                 280                 285
   Ser Leu Met Thr Leu Thr Leu Phe Ser Pro Asp Leu Leu Gly Asp Pro
       290                 295                 300
   Asp Asn Tyr Thr Leu Ala Asn Pro Leu Asn Thr Pro Pro His Ile Lys
   305 310 315 320
   Pro Glu Trp Tyr Phe Leu Phe Ala Tyr Thr Ile Leu Arg Ser Val Pro
   325 330 335
   Asn Lys Leu Gly Gly Val Leu Ala Leu Leu Leu Ser Ile Leu Ile Leu
   340 345 350
   Ala Met Ile Pro Ile Leu His Met Ser Lys Gln Gln Ser Met Met Phe
   355 360 365
   Arg Pro Leu Ser Gln Ser Leu Tyr Trp Leu Leu Ala Ala Asp Leu Leu
   370 375 380
   Ile Leu Thr Trp Ile Gly Gly Gln Pro Val Ser Tyr Pro Phe Thr Ile
   385 390 395 400
   Ile Gly Gln Val Ala Ser Val Leu Tyr Phe Thr Thr Ile Leu Ile Leu
   405 410 415
   Met Pro Thr Ile Ser Leu Ile Glu Asn Lys Met Leu Lys Trp Ala Xaa
   420 425 430
<210> 42
   <211> 410
   <212> PRT
   <213> Artificial
<220>
   <223> putative protein sequence
<220>
   <221> misc_feature
   <222> (410)..(410)
   <223> Xaa can be any naturally occurring amino acid
<400> 42
   Met Pro Leu Ile Tyr Met Asn Ile Met Leu Ala Phe Thr Ile Ser Leu
   1 5 10 15
   Leu Gly Met Leu Val Tyr Arg Ser His Leu Met Ser Ser Leu Leu Cys
   20 25 30
   Leu Glu Gly Met Met Leu Ser Leu Phe Ile Met Ala Thr Leu Met Thr
   35 40 45
   Leu Asn Thr His Ser Leu Leu Ala Asn Ile Val Pro Ile Ala Met Leu
       50                  55                  60
   Val Phe Gly Ala Cys Leu Ile Leu Gln Ile Thr Thr Gly Leu Phe Leu
   65                  70                  75                  80
   Ala Met His Tyr Ser Pro Asp Ala Ser Thr Ala Phe Ser Ser Ile Ala
                   85                  90                  95
   His Ile Thr Arg Asp Val Asn Tyr Gly Trp Ile Ile Arg Tyr Leu His
               100                 105                 110
   Ala Asn Gly Ala Ser Met Phe Phe Ile Cys Leu Phe Leu His Ile Gly
           115                 120                 125
   Arg Gly Leu Tyr Tyr Gly Ser Phe Leu Tyr Ser Glu Thr Trp Asn Ile
       130                 135                 140
   Gly Ile Ile Leu Leu Leu Ala Thr Met Ala Thr Ala Phe Met Gly Tyr
   145                 150                 155                 160
   Val Leu Pro Trp Gly Gln Met Ser Phe Trp Gly Ala Thr Val Ile Thr
                   165                 170                 175
   Asn Leu Leu Ser Ala Ile Pro Tyr Ile Gly Thr Asp Leu Val Gln Trp
               180                 185                 190
   Ile Trp Gly Gly Tyr Ser Val Asp Ser Pro Thr Leu Thr Arg Phe Phe
           195                 200                 205
   Thr Phe His Phe Ile Leu Pro Phe Ile Ile Ala Ala Leu Ala Thr Leu
       210                 215                 220
   His Leu Leu Phe Leu His Glu Thr Gly Ser Asn Asn Pro Leu Gly Ile
   225                 230                 235                 240
   Thr Ser His Ser Asp Lys Ile Thr Phe His Pro Tyr Tyr Thr Ile Lys
                   245                 250                 255
   Asp Ala Leu Gly Leu Leu Leu Phe Leu Leu Ser Leu Met Thr Leu Thr
               260                 265                 270
   Leu Phe Ser Pro Asp Leu Leu Gly Asp Pro Asp Asn Tyr Thr Leu Ala
           275                 280                 285
   Asn Pro Leu Asn Thr Pro Pro His Ile Lys Pro Glu Trp Tyr Phe Leu
       290                 295                 300
   Phe Ala Tyr Thr Ile Leu Arg Ser Val Pro Asn Lys Leu Gly Gly Val
   305 310 315 320
   Leu Ala Leu Leu Leu Ser Ile Leu Ile Leu Ala Met Ile Pro Ile Leu
   325 330 335
   His Met Ser Lys Gln Gln Ser Met Met Phe Arg Pro Leu Ser Gln Ser
   340 345 350
   Leu Tyr Trp Leu Leu Ala Ala Asp Leu Leu Ile Leu Thr Trp Ile Gly
   355 360 365
   Gly Gln Pro Val Ser Tyr Pro Phe Thr Ile Ile Gly Gln Val Ala Ser
   370 375 380
   Val Leu Tyr Phe Thr Thr Ile Leu Ile Leu Met Pro Thr Ile Ser Leu
   385 390 395 400
   Ile Glu Asn Lys Met Leu Lys Trp Ala Xaa
   405 410
<210> 43
   <211> 174
   <212> PRT
   <213> Artificial
<220>
   <223> putative protein sequence
<220>
   <221> misc_feature
   <222> (174)..(174)
   <223> Xaa can be any naturally occurring amino acid
<400> 43
   Met Phe Ala Asp Arg Trp Leu Phe Ser Thr Asn His Lys Asp Ile Gly
   1 5 10 15
   Thr Leu Tyr Leu Leu Phe Gly Ala Trp Ala Gly Val Leu Gly Thr Ala
   20 25 30
   Leu Ser Leu Leu Ile Arg Ala Glu Leu Gly Gln Pro Gly Asn Leu Leu
   35 40 45
   Gly Asn Asp His Ile Tyr Asn Val Ile Val Thr Ala Leu Ala Val Thr
   50 55 60
   Phe Leu Gly Leu Leu Thr Ala Leu Asp Leu Asn Tyr Leu Thr Asn Lys
   65 70 75 80
   Leu Lys Met Lys Ser Pro Leu Cys Thr Phe Tyr Phe Ser Asn Met Leu
   85 90 95
   Gly Phe Tyr Pro Ser Ile Thr His Arg Thr Ile Pro Tyr Leu Gly Leu
   100 105 110
   Leu Thr Ser Gln Asn Leu Pro Leu Leu Leu Leu Asp Leu Thr Trp Leu
   115 120 125
   Glu Lys Leu Leu Pro Lys Thr Ile Ser Gln His Gln Ile Ser Thr Ser
   130 135 140
   Ile Ile Thr Ser Thr Gln Lys Gly Met Ile Lys Leu Tyr Phe Leu Ser
   145 150 155 160
   Phe Phe Phe Pro Leu Ile Leu Thr Leu Leu Leu Ile Thr Xaa
   165 170
<210> 44
   <211> 194
   <212> PRT
   <213> Artificial
<220>
   <223> putative protein sequence
<220>
   <221> misc_feature
   <222> (194)..(194)
   <223> Xaa can be any naturally occurring amino acid
<400> 44
   Met Phe Ala Asp Arg Trp Leu Phe Ser Thr Asn His Lys Asp Ile Gly
   1 5 10 15
   Thr Leu Tyr Leu Leu Phe Gly Ala Trp Ala Gly Val Leu Gly Thr Ala
   20 25 30
   Leu Ser Leu Leu Ile Arg Ala Glu Leu Gly Gln Pro Gly Asn Leu Leu
   35 40 45
   Gly Asn Asp His Ile Tyr Asn Val Ile Val Thr Ala His Ala Phe Val
   50 55 60
   Met Ile Phe Phe Met Val Met Pro Ile Met Ile Gly Gly Phe Gly Asn
   65 70 75 80
   Trp Leu Val Pro Leu Met Ile Gly Ala Pro Asp Met Ala Phe Pro Arg
   85 90 95
   Met Asn Asn Met Ser Phe Trp Leu Leu Pro Pro Ser Leu Leu Leu Leu
   100 105 110
   Leu Ala Ser Ala Met Val Glu Ala Gly Ala Gly Thr Gly Trp Thr Val
   115 120 125
   Tyr Pro Pro Leu Ala Gly Asn Tyr Ser His Pro Gly Ala Leu Leu Asp
   130 135 140
   Leu Thr Trp Leu Glu Lys Leu Leu Pro Lys Thr Ile Ser Gln His Gln
   145 150 155 160
   Ile Ser Thr Ser Ile Ile Thr Ser Thr Gln Lys Gly Met Ile Lys Leu
   165 170 175
   Tyr Phe Leu Ser Phe Phe Phe Pro Leu Ile Leu Thr Leu Leu Leu Ile
   180 185 190
   Thr Xaa
<210> 45
   <211> 736
   <212> PRT
   <213> Artificial
<220>
   <223> putative protein sequence
<220>
   <221> misc_feature
   <222> (736)..(736)
   <223> Xaa can be any naturally occurring amino acid
<400> 45
   Met Phe Ala Asp Arg Trp Leu Phe Ser Thr Asn His Lys Asp Ile Gly
   1 5 10 15
   Thr Leu Tyr Leu Leu Phe Gly Ala Trp Ala Gly Val Leu Gly Thr Ala
   20 25 30
   Leu Ser Leu Leu Ile Arg Ala Glu Leu Gly Gln Pro Gly Asn Leu Leu
   35 40 45
   Gly Asn Asp His Ile Tyr Asn Val Ile Val Thr Ala His Ala Phe Val
   50 55 60
   Met Ile Phe Phe Met Val Met Pro Ile Met Ile Gly Gly Phe Gly Asn
   65 70 75 80
   Trp Leu Val Pro Leu Met Ile Gly Ala Pro Asp Met Ala Phe Pro Arg
   85 90 95
   Met Asn Asn Met Ser Phe Trp Leu Leu Pro Pro Ser Leu Leu Leu Leu
   100 105 110
   Leu Ala Ser Ala Met Val Glu Ala Gly Ala Gly Thr Gly Trp Thr Val
   115 120 125
   Tyr Pro Pro Leu Ala Gly Asn Tyr Ser His Pro Gly Ala Ser Val Asp
   130 135 140
   Leu Thr Ile Phe Ser Leu His Leu Ala Gly Val Ser Ser Ile Leu Gly
   145 150 155 160
   Ala Ile Asn Phe Ile Thr Thr Ile Ile Asn Met Lys Pro Pro Ala Met
   165 170 175
   Thr Gln Tyr Gln Thr Pro Leu Phe Val Trp Ser Val Leu Ile Thr Ala
   180 185 190
   Val Leu Leu Leu Leu Ser Leu Pro Val Leu Ala Ala Gly Ile Thr Met
   195 200 205
   Leu Leu Thr Asp Arg Asn Leu Asn Thr Thr Phe Phe Asp Pro Ala Gly
   210 215 220
   Gly Gly Asp Pro Ile Leu Tyr Gln His Leu Phe Trp Phe Phe Gly His
   225 230 235 240
   Pro Glu Val Tyr Ile Leu Ile Leu Pro Gly Phe Gly Met Ile Ser His
   245 250 255
   Ile Val Thr Tyr Tyr Ser Gly Lys Lys Glu Pro Phe Gly Tyr Met Gly
   260 265 270
   Met Val Trp Ala Met Met Ser Ile Gly Phe Leu Gly Phe Ile Val Trp
   275 280 285
   Ala His His Met Phe Thr Val Gly Met Asp Val Asp Thr Arg Ala Tyr
   290 295 300
   Phe Thr Ser Ala Thr Met Ile Ile Ala Ile Pro Thr Gly Val Lys Val
   305 310 315 320
   Phe Ser Trp Leu Ala Thr Leu His Gly Ser Asn Met Lys Trp Ser Ala
                   325                 330                 335
   Ala Val Leu Trp Ala Leu Gly Phe Ile Phe Leu Phe Thr Val Gly Gly
               340                 345                 350
   Leu Thr Gly Ile Val Leu Ala Asn Ser Ser Leu Asp Ile Val Leu His
           355                 360                 365
   Asp Thr Tyr Tyr Val Val Ala His Phe His Tyr Val Leu Ser Met Gly
       370                 375                 380
   Ala Val Phe Ala Ile Met Gly Gly Phe Ile His Trp Phe Pro Leu Phe
   385                 390                 395                 400
   Ser Gly Tyr Thr Leu Asp Gln Thr Tyr Ala Lys Ile His Phe Thr Ile
                   405                 410                 415
   Met Phe Ile Gly Val Asn Leu Thr Phe Phe Pro Gln His Phe Leu Gly
               420                 425                 430
   Leu Ser Gly Met Pro Arg Arg Tyr Ser Asp Tyr Pro Asp Ala Tyr Thr
           435                 440                 445
   Thr Trp Asn Ile Leu Ser Ser Val Gly Ser Phe Ile Ser Leu Thr Ala
       450                 455                 460
   Val Met Leu Met Ile Phe Met Ile Trp Glu Ala Phe Ala Ser Lys Arg
   465                 470                 475                 480
   Lys Val Leu Met Val Glu Glu Pro Ser Met Asn Leu Glu Trp Leu Tyr
                   485                 490                 495
   Gly Cys Pro Pro Pro Tyr His Thr Phe Glu Glu Pro Val Tyr Met Lys
               500                 505                 510
   Ala Gly Met Pro Phe Leu Thr Gly Phe Tyr Ser Lys Asp His Ile Ile
           515                 520                 525
   Glu Thr Ala Asn Met Ser Tyr Thr Asn Ala Trp Ala Leu Ser Ile Thr
       530                 535                 540
   Leu Ile Ala Thr Ser Leu Thr Ser Ala Tyr Ser Thr Arg Met Ile Leu
   545                 550                 555                 560
   Leu Thr Leu Thr Gly Gln Pro Arg Phe Pro Thr Leu Thr Asn Ile Asn
                   565                 570                 575
   Glu Asn Asn Pro Thr Leu Leu Asn Pro Ile Lys Arg Leu Ala Ala Gly
   580 585 590
   Ser Leu Phe Ala Gly Phe Leu Ile Thr Asn Asn Ile Ser Pro Ala Ser
   595 600 605
   Pro Phe Gln Thr Thr Ile Pro Leu Tyr Leu Lys Leu Thr Ala Leu Ala
   610 615 620
   Val Thr Phe Leu Gly Leu Leu Thr Ala Leu Asp Leu Asn Tyr Leu Thr
   625 630 635 640
   Asn Lys Leu Lys Met Lys Ser Pro Leu Cys Thr Phe Tyr Phe Ser Asn
   645 650 655
   Met Leu Gly Phe Tyr Pro Ser Ile Thr His Arg Thr Ile Pro Tyr Leu
   660 665 670
   Gly Leu Leu Thr Ser Gln Asn Leu Pro Leu Leu Leu Leu Asp Leu Thr
   675 680 685
   Trp Leu Glu Lys Leu Leu Pro Lys Thr Ile Ser Gln His Gln Ile Ser
   690 695 700
   Thr Ser Ile Ile Thr Ser Thr Gln Lys Gly Met Ile Lys Leu Tyr Phe
   705 710 715 720
   Leu Ser Phe Phe Phe Pro Leu Ile Leu Thr Leu Leu Leu Ile Thr Xaa
   725 730 735
<210> 46
   <211> 269
   <212> PRT
   <213> Artificial
<220>
   <223> putative protein sequence
<220>
   <221> misc_feature
   <222> (269)..(269)
   <223> Xaa can be any naturally occurring amino acid
<400> 46
   Met Ala His Ala Ala Gln Val Gly Leu Gln Asp Ala Thr Ser Pro Ile
   1 5 10 15
   Met Glu Glu Leu Ile Thr Phe His Asp His Ala Leu Met Ile Ile Phe
   20 25 30
   Leu Ile Cys Phe Leu Val Leu Tyr Ala Leu Phe Leu Thr Leu Thr Thr
           35                  40                  45
   Lys Leu Thr Asn Thr Asn Ile Ser Asp Ala Gln Glu Met Glu Thr Ala
       50                  55                  60
   Asn Met Ser Tyr Thr Asn Ala Trp Ala Leu Ser Ile Thr Leu Ile Ala
   65                  70                  75                  80
   Thr Ser Leu Thr Ser Ala Tyr Ser Thr Arg Met Ile Leu Leu Thr Leu
                   85                  90                  95
   Thr Gly Gln Pro Arg Phe Pro Thr Leu Thr Asn Ile Asn Glu Asn Asn
               100                 105                 110
   Pro Thr Leu Leu Asn Pro Ile Lys Arg Leu Ala Ala Gly Ser Leu Phe
           115                 120                 125
   Ala Gly Phe Leu Ile Thr Asn Asn Ile Ser Pro Ala Ser Pro Phe Gln
       130                 135                 140
   Thr Thr Ile Pro Leu Tyr Leu Lys Leu Thr Ala Leu Ala Val Thr Phe
   145                 150                 155                 160
   Leu Gly Leu Leu Thr Ala Leu Asp Leu Asn Tyr Leu Thr Asn Lys Leu
                   165                 170                 175
   Lys Met Lys Ser Pro Leu Cys Thr Phe Tyr Phe Ser Asn Met Leu Gly
               180                 185                 190
   Phe Tyr Pro Ser Ile Thr His Arg Thr Ile Pro Tyr Leu Gly Leu Leu
           195                 200                 205
   Thr Ser Gln Asn Leu Pro Leu Leu Leu Leu Asp Leu Thr Trp Leu Glu
       210                 215                 220
   Lys Leu Leu Pro Lys Thr Ile Ser Gln His Gln Ile Ser Thr Ser Ile
   225                 230                 235                 240
   Ile Thr Ser Thr Gln Lys Gly Met Ile Lys Leu Tyr Phe Leu Ser Phe
                   245                 250                 255
   Phe Phe Pro Leu Ile Leu Thr Leu Leu Leu Ile Thr Xaa
               260                 265
<210> 47
   <211> 262
   <212> PRT
   <213> Artificial
<220>
   <223> putative protein sequence
<220>
   <221> misc_feature
   <222> (262)..(262)
   <223> Xaa can be any naturally occurring amino acid
<400> 47
   Met Ala His Ala Ala Gln Val Gly Leu Gln Asp Ala Thr Ser Pro Ile
   1               5                   10                  15
   Met Glu Glu Leu Ile Thr Phe His Asp His Ala Leu Met Ile Ile Phe
               20                  25                  30
   Leu Ile Cys Phe Leu Val Leu Tyr Ala Leu Phe Leu Thr Leu Thr Thr
           35                  40                  45
   Lys Leu Thr Asn Thr Asn Ile Ser Asp Ala Gln Glu Met Glu Thr Val
       50                  55                  60
   Trp Thr Ile Leu Pro Ala Ile Ile Leu Val Leu Ile Ala Leu Pro Ser
   65                  70                  75                  80
   Leu Arg Ile Leu Tyr Met Thr Asp Glu Val Asn Asp Pro Ser Leu Thr
                   85                  90                  95
   Ile Lys Ser Ile Gly His Gln Trp Tyr Trp Thr Tyr Glu Tyr Thr Asp
               100                 105                 110
   Tyr Gly Gly Leu Ile Phe Asn Ser Tyr Met Leu Pro Pro Leu Phe Leu
           115                 120                 125
   Glu Pro Gly Asp Leu Arg Leu Leu Asp Val Asp Asn Arg Val Val Leu
       130                 135                 140
   Pro Ile Glu Ala Pro Ile Arg Met Met Ile Thr Ser Gln Asp Val Leu
   145                 150                 155                 160
   His Ser Trp Ala Val Pro Thr Leu Gly Leu Lys Thr Asp Ala Ile Pro
                   165                 170                 175
   Gly Arg Leu Asn Gln Thr Thr Phe Thr Ala Thr Arg Pro Gly Val Tyr
               180                 185                 190
   Tyr Gly Gln Cys Ser Glu Ile Cys Gly Ala Asn His Ser Phe Met Pro
   195 200 205
   Ile Val Leu Asp Leu Thr Trp Leu Glu Lys Leu Leu Pro Lys Thr Ile
   210 215 220
   Ser Gln His Gln Ile Ser Thr Ser Ile Ile Thr Ser Thr Gln Lys Gly
   225 230 235 240
   Met Ile Lys Leu Tyr Phe Leu Ser Phe Phe Phe Pro Leu Ile Leu Thr
   245 250 255
   Leu Leu Leu Ile Thr Xaa
   260
<210> 48
   <211> 635
   <212> PRT
   <213> Artificial
<220>
   <223> putative protein sequence
<220>
   <221> misc_feature
   <222> (635) .. (635)
   <223> Xaa can be any naturally occurring amino acid
<400> 48
   Met Asn Glu Asn Leu Phe Ala Ser Phe Ile Ala Pro Thr Ile Leu Gly
   1 5 10 15
   Leu Pro Ala Ala Val Leu Ile Ile Leu Phe Pro Pro Leu Leu Ile Pro
   20 25 30
   Thr Ser Lys Tyr Leu Ile Asn Asn Arg Leu Ile Thr Thr Gln Gln Trp
   35 40 45
   Leu Ile Lys Leu Thr Ser Lys Gln Met Met Thr Met His Asn Thr Lys
   50 55 60
   Gly Arg Thr Trp Ser Leu Met Leu Val Ser Leu Ile Ile Phe Ile Ala
   65 70 75 80
   Thr Thr Asn Leu Leu Gly Leu Leu Pro His Ser Phe Thr Pro Thr Thr
   85 90 95
   Gln Leu Ser Met Asn Leu Ala Met Ala Ile Pro Leu Trp Ala Gly Thr
   100 105 110
   Val Ile Met Gly Phe Arg Ser Lys Ile Lys Asn Ala Leu Ala His Phe
           115                 120                 125
   Leu Pro Gln Gly Thr Pro Thr Pro Leu Ile Pro Met Leu Val Ile Ile
       130                 135                 140
   Glu Thr Ile Ser Leu Leu Ile Gln Pro Met Ala Leu Ala Val Arg Leu
   145                 150                 155                 160
   Thr Ala Asn Ile Thr Ala Gly His Leu Leu Met His Leu Ile Gly Ser
                   165                 170                 175
   Ala Thr Leu Ala Met Ser Thr Ile Asn Leu Pro Ser Thr Leu Ile Ile
               180                 185                 190
   Phe Thr Ile Leu Ile Leu Leu Thr Ile Leu Glu Ile Ala Val Ala Leu
           195                 200                 205
   Ile Gln Ala Tyr Val Phe Thr Leu Leu Val Ser Leu Tyr Leu His Ser
       210                 215                 220
   Asn Ser Trp Asp Pro Gln Gln Met Ala Leu Leu Asn Ala Asn Pro Ser
   225                 230                 235                 240
   Leu Thr Pro Leu Leu Gly Leu Leu Leu Ala Ala Ala Gly Lys Ser Ala
                   245                 250                 255
   Gln Leu Gly Leu His Pro Trp Leu Pro Ser Ala Met Glu Gly Pro Thr
               260                 265                 270
   Pro Val Ser Ala Leu Leu His Ser Ser Thr Met Val Val Ala Gly Ile
           275                 280                 285
   Phe Leu Leu Ile Arg Phe His Pro Leu Ala Glu Asn Ser Pro Leu Ile
       290                 295                 300
   Gln Thr Leu Thr Leu Cys Leu Gly Ala Ile Thr Thr Leu Phe Ala Ala
   305                 310                 315                 320
   Val Cys Ala Leu Thr Gln Asn Asp Ile Lys Lys Ile Val Ala Phe Ser
                   325                 330                 335
   Thr Ser Ser Gln Leu Gly Leu Met Met Val Thr Ile Gly Ile Asn Gln
               340                 345                 350
   Pro His Leu Ala Phe Leu His Ile Cys Thr His Ala Phe Phe Lys Ala
           355                 360                 365
   Met Leu Phe Met Cys Ser Gly Ser Ile Ile His Asn Leu Asn Asn Glu
       370                 375                 380
   Gln Asp Ile Arg Lys Met Gly Gly Leu Leu Lys Thr Met Pro Leu Thr
   385                 390                 395                 400
   Ser Thr Ser Leu Thr Ile Gly Ser Leu Ala Leu Ala Gly Met Pro Phe
                   405                 410                 415
   Leu Thr Gly Phe Tyr Ser Lys Asp His Ile Ile Glu Thr Ala Asn Met
               420                 425                 430
   Ser Tyr Thr Asn Ala Trp Ala Leu Ser Ile Thr Leu Ile Ala Thr Ser
           435                 440                 445
   Leu Thr Ser Ala Tyr Ser Thr Arg Met Ile Leu Leu Thr Leu Thr Gly
       450                 455                 460
   Gln Pro Arg Phe Pro Thr Leu Thr Asn Ile Asn Glu Asn Asn Pro Thr
   465                 470                 475                 480
   Leu Leu Asn Pro Ile Lys Arg Leu Ala Ala Gly Ser Leu Phe Ala Gly
                   485                 490                 495
   Phe Leu Ile Thr Asn Asn Ile Ser Pro Ala Ser Pro Phe Gln Thr Thr
               500                 505                 510
   Ile Pro Leu Tyr Leu Lys Leu Thr Ala Leu Ala Val Thr Phe Leu Gly
           515                 520                 525
   Leu Leu Thr Ala Leu Asp Leu Asn Tyr Leu Thr Asn Lys Leu Lys Met
       530                 535                 540
   Lys Ser Pro Leu Cys Thr Phe Tyr Phe Ser Asn Met Leu Gly Phe Tyr
   545                 550                 555                 560
   Pro Ser Ile Thr His Arg Thr Ile Pro Tyr Leu Gly Leu Leu Thr Ser
                   565                 570                 575
   Gln Asn Leu Pro Leu Leu Leu Leu Asp Leu Thr Trp Leu Glu Lys Leu
               580                 585                 590
   Leu Pro Lys Thr Ile Ser Gln His Gln Ile Ser Thr Ser Ile Ile Thr
           595                 600                 605
   Ser Thr Gln Lys Gly Met Ile Lys Leu Tyr Phe Leu Ser Phe Phe Phe
   610 615 620
   Pro Leu Ile Leu Thr Leu Leu Leu Ile Thr Xaa
   625 630 635
<210> 49
   <211> 515
   <212> PRT
   <213> Artificial
<220>
   <223> putative protein sequence
<220>
   <221> misc_feature
   <222> (515) .. (515)
   <223> Xaa can be any naturally occurring amino acid
<400> 49
   Met Thr His Gln Ser His Ala Tyr His Met Val Lys Pro Ser Pro Trp
   1 5 10 15
   Pro Leu Thr Gly Ala Leu Ser Ala Leu Leu Met Thr Ser Gly Leu Ala
   20 25 30
   Met Trp Phe His Phe His Ser Met Thr Leu Leu Met Leu Gly Leu Leu
   35 40 45
   Thr Asn Thr Leu Thr Met Tyr Gln Trp Trp Arg Asp Val Thr Arg Glu
   50 55 60
   Ser Thr Tyr Gln Gly His His Thr Pro Pro Val Gln Lys Gly Leu Arg
   65 70 75 80
   Tyr Gly Met Ile Leu Phe Ile Thr Ser Glu Val Phe Phe Phe Ala Gly
   85 90 95
   Phe Phe Trp Ala Phe Tyr His Ser Ser Leu Ala Pro Thr Pro Gln Leu
   100 105 110
   Gly Gly His Trp Pro Pro Thr Gly Ile Thr Pro Leu Leu Gly Leu Leu
   115 120 125
   Leu Ala Ala Ala Gly Lys Ser Ala Gln Leu Gly Leu His Pro Trp Leu
   130 135 140
   Pro Ser Ala Met Glu Gly Pro Thr Pro Val Ser Ala Leu Leu His Ser
   145 150 155 160
   Ser Thr Met Val Val Ala Gly Ile Phe Leu Leu Ile Arg Phe His Pro
                   165                 170                 175
   Leu Ala Glu Asn Ser Pro Leu Ile Gln Thr Leu Thr Leu Cys Leu Gly
               180                 185                 190
   Ala Ile Thr Thr Leu Phe Ala Ala Val Cys Ala Leu Thr Gln Asn Asp
           195                 200                 205
   Ile Lys Lys Ile Val Ala Phe Ser Thr Ser Ser Gln Leu Gly Leu Met
       210                 215                 220
   Met Val Thr Ile Gly Ile Asn Gln Pro His Leu Ala Phe Leu His Ile
   225                 230                 235                 240
   Cys Thr His Ala Phe Phe Lys Ala Met Leu Phe Met Cys Ser Gly Ser
                   245                 250                 255
   Ile Ile His Asn Leu Asn Asn Glu Gln Asp Ile Arg Lys Met Gly Gly
               260                 265                 270
   Leu Leu Lys Thr Met Pro Leu Thr Ser Thr Ser Leu Thr Ile Gly Ser
           275                 280                 285
   Leu Ala Leu Ala Gly Met Pro Phe Leu Thr Gly Phe Tyr Ser Lys Asp
       290                 295                 300
   His Ile Ile Glu Thr Ala Asn Met Ser Tyr Thr Asn Ala Trp Ala Leu
   305                 310                 315                 320
   Ser Ile Thr Leu Ile Ala Thr Ser Leu Thr Ser Ala Tyr Ser Thr Arg
                   325                 330                 335
   Met Ile Leu Leu Thr Leu Thr Gly Gln Pro Arg Phe Pro Thr Leu Thr
               340                 345                 350
   Asn Ile Asn Glu Asn Asn Pro Thr Leu Leu Asn Pro Ile Lys Arg Leu
           355                 360                 365
   Ala Ala Gly Ser Leu Phe Ala Gly Phe Leu Ile Thr Asn Asn Ile Ser
       370                 375                 380
   Pro Ala Ser Pro Phe Gln Thr Thr Ile Pro Leu Tyr Leu Lys Leu Thr
   385                 390                 395                 400
   Ala Leu Ala Val Thr Phe Leu Gly Leu Leu Thr Ala Leu Asp Leu Asn
                   405                 410                 415
   Tyr Leu Thr Asn Lys Leu Lys Met Lys Ser Pro Leu Cys Thr Phe Tyr
   420 425 430
   Phe Ser Asn Met Leu Gly Phe Tyr Pro Ser Ile Thr His Arg Thr Ile
   435 440 445
   Pro Tyr Leu Gly Leu Leu Thr Ser Gln Asn Leu Pro Leu Leu Leu Leu
   450 455 460
   Asp Leu Thr Trp Leu Glu Lys Leu Leu Pro Lys Thr Ile Ser Gln His
   465 470 475 480
   Gln Ile Ser Thr Ser Ile Ile Thr Ser Thr Gln Lys Gly Met Ile Lys
   485 490 495
   Leu Tyr Phe Leu Ser Phe Phe Phe Pro Leu Ile Leu Thr Leu Leu Leu
   500 505 510
   Ile Thr Xaa
   515
<210> 50
   <211> 543
   <212> PRT
   <213> Artificial
<220>
   <223> putative protein sequence
<220>
   <221> misc_feature
   <222> (543)..(543)
   <223> Xaa can be any naturally occurring amino acid
<400> 50
   Met Asn Phe Ala Leu Ile Leu Met Ile Asn Thr Leu Leu Ala Leu Leu
   1 5 10 15
   Leu Met Ile Ile Thr Phe Trp Leu Pro Gln Leu Asn Gly Tyr Met Glu
   20 25 30
   Lys Ser Thr Pro Tyr Glu Cys Gly Phe Asp Pro Met Ser Pro Ala Arg
   35 40 45
   Val Pro Phe Ser Met Lys Phe Phe Leu Val Ala Ile Thr Phe Leu Leu
   50 55 60
   Phe Asp Leu Glu Ile Ala Leu Leu Leu Pro Leu Pro Trp Ala Leu Gln
   65 70 75 80
   Thr Thr Asn Leu Pro Leu Met Val Met Ser Ser Leu Leu Leu Ile Ile
   85 90 95
   Ile Leu Ala Leu Ser Leu Ala Asn Thr Ala Ala Ile Gln Ala Ile Leu
   100 105 110
   Tyr Asn Arg Ile Gly Asp Ile Gly Phe Ile Leu Ala Leu Ala Trp Phe
   115 120 125
   Ile Leu His Ser Asn Ser Trp Asp Pro Gln Gln Met Ala Leu Leu Asn
   130 135 140
   Ala Asn Pro Ser Leu Thr Pro Leu Leu Gly Leu Leu Leu Ala Ala Ala
   145 150 155 160
   Gly Lys Ser Ala Gln Leu Gly Leu His Pro Trp Leu Pro Ser Ala Met
   165 170 175
   Glu Gly Pro Thr Pro Val Ser Ala Leu Leu His Ser Ser Thr Met Val
   180 185 190
   Val Ala Gly Ile Phe Leu Leu Ile Arg Phe His Pro Leu Ala Glu Asn
   195 200 205
   Ser Pro Leu Ile Gln Thr Leu Thr Leu Cys Leu Gly Ala Ile Thr Thr
   210 215 220
   Leu Phe Ala Ala Val Cys Ala Leu Thr Gln Asn Asp Ile Lys Lys Ile
   225 230 235 240
   Val Ala Phe Ser Thr Ser Ser Gln Leu Gly Leu Met Met Val Thr Ile
   245 250 255
   Gly Ile Asn Gln Pro His Leu Ala Phe Leu His Ile Cys Thr His Ala
   260 265 270
   Phe Phe Lys Ala Met Leu Phe Met Cys Ser Gly Ser Ile Ile His Asn
   275 280 285
   Leu Asn Asn Glu Gln Asp Ile Arg Lys Met Gly Gly Leu Leu Lys Thr
   290 295 300
   Met Pro Leu Thr Ser Thr Ser Leu Thr Ile Gly Ser Leu Ala Leu Ala
   305 310 315 320
   Gly Met Pro Phe Leu Thr Gly Phe Tyr Ser Lys Asp His Ile Ile Glu
   325 330 335
   Thr Ala Asn Met Ser Tyr Thr Asn Ala Trp Ala Leu Ser Ile Thr Leu
   340 345 350
   Ile Ala Thr Ser Leu Thr Ser Ala Tyr Ser Thr Arg Met Ile Leu Leu
   355 360 365
   Thr Leu Thr Gly Gln Pro Arg Phe Pro Thr Leu Thr Asn Ile Asn Glu
   370 375 380
   Asn Asn Pro Thr Leu Leu Asn Pro Ile Lys Arg Leu Ala Ala Gly Ser
   385 390 395 400
   Leu Phe Ala Gly Phe Leu Ile Thr Asn Asn Ile Ser Pro Ala Ser Pro
   405 410 415
   Phe Gln Thr Thr Ile Pro Leu Tyr Leu Lys Leu Thr Ala Leu Ala Val
   420 425 430
   Thr Phe Leu Gly Leu Leu Thr Ala Leu Asp Leu Asn Tyr Leu Thr Asn
   435 440 445
   Lys Leu Lys Met Lys Ser Pro Leu Cys Thr Phe Tyr Phe Ser Asn Met
   450 455 460
   Leu Gly Phe Tyr Pro Ser Ile Thr His Arg Thr Ile Pro Tyr Leu Gly
   465 470 475 480
   Leu Leu Thr Ser Gln Asn Leu Pro Leu Leu Leu Leu Asp Leu Thr Trp
   485 490 495
   Leu Glu Lys Leu Leu Pro Lys Thr Ile Ser Gln His Gln Ile Ser Thr
   500 505 510
   Ser Ile Ile Thr Ser Thr Gln Lys Gly Met Ile Lys Leu Tyr Phe Leu
   515 520 525
   Ser Phe Phe Phe Pro Leu Ile Leu Thr Leu Leu Leu Ile Thr Xaa
   530 535 540
<210> 51
   <211> 214
   <212> PRT
   <213> Artificial
<220>
   <223> putative protein sequence
<220>
   <221> misc_feature
   <222> (214)..(214)
   <223> Xaa can be any naturally occurring amino acid
<400> 51
   Met Leu Lys Leu Ile Val Pro Thr Ile Met Leu Leu Pro Leu Thr Trp
   1               5                   10                  15
   Leu Ser Lys Lys His Met Ile Trp Ile Asn Thr Thr Thr His Ser Leu
               20                  25                  30
   Ile Ile Ser Ile Ile Pro Leu Leu Phe Phe Asn Gln Ile Asn Asn Asn
           35                  40                  45
   Leu Phe Ser Cys Ser Pro Thr Phe Ser Ser Asp Pro Leu Thr Thr Pro
       50                  55                  60
   Leu Leu Met Leu Thr Thr Trp Leu Leu Pro Leu Thr Ile Met Ala Ser
   65                  70                  75                  80
   Gln Arg His Leu Ser Ser Glu Pro Leu Ser Arg Lys Lys Leu Tyr Leu
                   85                  90                  95
   Ser Met Leu Ile Ser Leu Gln Ile Ser Leu Ile Met Thr Phe Thr Ala
               100                 105                 110
   Thr Glu Leu Ile Met Phe Tyr Ile Phe Phe Glu Thr Thr Leu Ile Pro
           115                 120                 125
   Thr Leu Ala Ile Ile Thr Arg Trp Gly Asn Gln Pro Glu Arg Leu Asn
       130                 135                 140
   Ala Gly Thr Tyr Phe Leu Phe Tyr Thr Leu Val Gly Ser Leu Pro Leu
   145                 150                 155                 160
   Leu Leu Leu Asp Leu Thr Trp Leu Glu Lys Leu Leu Pro Lys Thr Ile
                   165                 170                 175
   Ser Gln His Gln Ile Ser Thr Ser Ile Ile Thr Ser Thr Gln Lys Gly
               180                 185                 190
   Met Ile Lys Leu Tyr Phe Leu Ser Phe Phe Phe Pro Leu Ile Leu Thr
           195                 200                 205
   Leu Leu Leu Ile Thr Xaa
       210
<210> 52
   <211> 43
   <212> PRT
   <213> Artificial
<220>
   <223> putative protein sequence
<220>
   <221> misc_feature
   <222> (43)..(43)
   <223> Xaa can be any naturally occurring amino acid
<400> 52
   Met Pro Gln Leu Asn Thr Thr Val Trp Pro Thr Met Ile Thr Pro Met
   1               5                   10                  15
   Leu Leu Thr Leu Phe Leu Ile Thr Gln Leu Lys Met Leu Asn Thr Asn
               20                  25                  30
   Tyr His Leu Pro Pro Ser Pro Leu Ala Ala Xaa
           35                  40
<210> 53
   <211> 951
   <212> RNA
   <213> Human
<400> 53
   augaacgaaa aucuguucgc uucauucauu gcccccacaa uccuaggccu acccgccgca 60
   guacugauca uucuauuucc cccucuauug auccccaccu ccaaauaucu caucaacaac 120
   cgacuaauca ccacccaaca augacuaauc aaacuaaccu caaaacaaau gauaaccaua 180
   cacaacacua aaggacgaac cugaucucuu auacuaguau ccuuaaucau uuuuauugcc 240
   acaacuaacc uccucggacu ccugccucac ucauuuacac caaccaccca acuaucuaua 300
   aaccuagcca uggccauccc cuuaugagcg ggcacaguga uuauaggcuu ucgcucuaag 360
   auuaaaaaug cccuagccca cuucuuacca caaggcacac cuacaccccu uauccccaua 420
   cuaguuauua ucgaaaccau cagccuacuc auucaaccaa uagcccuggc cguacgccua 480
   accgcuaaca uuacugcagg ccaccuacuc augcaccuaa uuggaagcgc cacccuagca 540
   auaucaacca uuaaccuucc cucuacacuu aucaucuuca caauucuaau ucuacugacu 600
   auccuagaaa ucgcugucac uuuccuagga cuucuaacag cccuagaccu caacuaccua 660
   accaacaaac uuaaaauaaa auccccacua ugcacauuuu auuucuccaa cauacucgga 720
   uucuacccua gcaucacaca ccgcacaauc cccuaucuag gccuucuuac gagccaaaac 780
   cugccccuac uccuccuaga ccuaaccuga cuagaaaagc uauuaccuaa aacaauuuca 840
   cagcaccaaa ucuccaccuc caucaucacc ucaacccaaa aaggcauaau uaaacuuuac 900
   uuccucucuu ucuucuuccc acucauccua acccuacucc uaaucacaua a 951
<210> 54
   <211> 951
   <212> DNA
   <213> Artificial
<220>
   <223> cDNA
<400> 54
   atgaacgaaa atctgttcgc ttcattcatt gcccccacaa tcctaggcct acccgccgca        60
   gtactgatca ttctatttcc ccctctattg atccccacct ccaaatatct catcaacaac       120
   cgactaatca ccacccaaca atgactaatc aaactaacct caaaacaaat gataaccata       180
   cacaacacta aaggacgaac ctgatctctt atactagtat ccttaatcat ttttattgcc       240
   acaactaacc tcctcggact cctgcctcac tcatttacac caaccaccca actatctata       300
   aacctagcca tggccatccc cttatgagcg ggcacagtga ttataggctt tcgctctaag       360
   attaaaaatg ccctagccca cttcttacca caaggcacac ctacacccct tatccccata       420
   ctagttatta tcgaaaccat cagcctactc attcaaccaa tagccctggc cgtacgccta       480
   accgctaaca ttactgcagg ccacctactc atgcacctaa ttggaagcgc caccctagca       540
   atatcaacca ttaaccttcc ctctacactt atcatcttca caattctaat tctactgact       600
   atcctagaaa tcgctgtcac tttcctagga cttctaacag ccctagacct caactaccta       660
   accaacaaac ttaaaataaa atccccacta tgcacatttt atttctccaa catactcgga       720
   ttctacccta gcatcacaca ccgcacaatc ccctatctag gccttcttac gagccaaaac       780
   ctgcccctac tcctcctaga cctaacctga ctagaaaagc tattacctaa aacaatttca       840
   cagcaccaaa tctccacctc catcatcacc tcaacccaaa aaggcataat taaactttac       900
   ttcctctctt tcttcttccc actcatccta accctactcc taatcacata a                951
<210> 55
   <211> 317
   <212> PRT
   <213> Artificial
<220>
   <223> putative protein sequence
<220>
   <221> misc_feature
   <222> (317)..(317)
   <223> Xaa can be any naturally occurring amino acid
<400> 55
   Met Asn Glu Asn Leu Phe Ala Ser Phe Ile Ala Pro Thr Ile Leu Gly
   1               5                   10                  15
   Leu Pro Ala Ala Val Leu Ile Ile Leu Phe Pro Pro Leu Leu Ile Pro
               20                  25                  30
   Thr Ser Lys Tyr Leu Ile Asn Asn Arg Leu Ile Thr Thr Gln Gln Trp
           35                  40                  45
   Leu Ile Lys Leu Thr Ser Lys Gln Met Met Thr Met His Asn Thr Lys
       50                  55                  60
   Gly Arg Thr Trp Ser Leu Met Leu Val Ser Leu Ile Ile Phe Ile Ala
   65                  70                  75                  80
   Thr Thr Asn Leu Leu Gly Leu Leu Pro His Ser Phe Thr Pro Thr Thr
                   85                  90                  95
   Gln Leu Ser Met Asn Leu Ala Met Ala Ile Pro Leu Trp Ala Gly Thr
               100                 105                 110
   Val Ile Met Gly Phe Arg Ser Lys Ile Lys Asn Ala Leu Ala His Phe
           115                 120                 125
   Leu Pro Gln Gly Thr Pro Thr Pro Leu Ile Pro Met Leu Val Ile Ile
       130                 135                 140
   Glu Thr Ile Ser Leu Leu Ile Gln Pro Met Ala Leu Ala Val Arg Leu
   145                 150                 155                 160
   Thr Ala Asn Ile Thr Ala Gly His Leu Leu Met His Leu Ile Gly Ser
                   165                 170                 175
   Ala Thr Leu Ala Met Ser Thr Ile Asn Leu Pro Ser Thr Leu Ile Ile
               180                 185                 190
   Phe Thr Ile Leu Ile Leu Leu Thr Ile Leu Glu Ile Ala Val Thr Phe
           195                 200                 205
   Leu Gly Leu Leu Thr Ala Leu Asp Leu Asn Tyr Leu Thr Asn Lys Leu
       210                 215                 220
   Lys Met Lys Ser Pro Leu Cys Thr Phe Tyr Phe Ser Asn Met Leu Gly
   225                 230                 235                 240
   Phe Tyr Pro Ser Ile Thr His Arg Thr Ile Pro Tyr Leu Gly Leu Leu
                   245                 250                 255
   Thr Ser Gln Asn Leu Pro Leu Leu Leu Leu Asp Leu Thr Trp Leu Glu
   260 265 270
   Lys Leu Leu Pro Lys Thr Ile Ser Gln His Gln Ile Ser Thr Ser Ile
   275 280 285
   Ile Thr Ser Thr Gln Lys Gly Met Ile Lys Leu Tyr Phe Leu Ser Phe
   290 295 300
   Phe Phe Pro Leu Ile Leu Thr Leu Leu Leu Ile Thr Xaa
   305 310 315
<210> 56
   <211> 747
   <212> RNA
   <213> Human
<220>
   <221> misc_feature
   <223> Fusion transcript P0026 (MT-CO2 (7586-8038) + MT-ND3 (10110-10404))
<400> 56
   auggcacaug cagcgcaagu aggucuacaa gacgcuacuu ccccuaucau agaagagcuu 60
   aucaccuuuc augaucacgc ccucauaauc auuuuccuua ucugcuuccu aguccuguau 120
   gcccuuuucc uaacacucac aacaaaacua acuaauacua acaucucaga cgcucaggaa 180
   auagaaaccg ucugaacuau ccugcccgcc aucauccuag uccucaucgc ccucccaucc 240
   cuacgcaucc uuuacauaac agacgagguc aacgaucccu cccuuaccau caaaucaauu 300
   ggccaccaau gguacugaac cuacgaguac accgacuacg gcggacuaau cuucaacucc 360
   uacauacuuc ccccauuauu ccuagaacca ggcgaccugc gacuccuuga cguugacaau 420
   cgaguaguac ucccgauuga agcccccauu cguauaauua uuacauuuug acuaccacaa 480
   cucaacggcu acauagaaaa auccaccccu uacgagugcg gcuucgaccc uauauccccc 540
   gcccgcgucc cuuucuccau aaaauucuuc uuaguagcua uuaccuucuu auuauuugau 600
   cuagaaauug cccuccuuuu accccuacca ugagcccuac aaacaacuaa ccugccacua 660
   auaguuaugu caucccucuu auuaaucauc auccuagccc uaagucuggc cuaugaguga 720
   cuacaaaaag gauuagacug aaccgaa 747
<210> 57
   <211> 747
   <212> DNA
   <213> Human
<220>
   <221> misc_feature
   <223> Mutant DNA for fusion transcript P0026
<400> 57
   atggcacatg cagcgcaagt aggtctacaa gacgctactt cccctatcat agaagagctt      60
   atcacctttc atgatcacgc cctcataatc attttcctta tctgcttcct agtcctgtat     120
   gcccttttcc taacactcac aacaaaacta actaatacta acatctcaga cgctcaggaa     180
   atagaaaccg tctgaactat cctgcccgcc atcatcctag tcctcatcgc cctcccatcc     240
   ctacgcatcc tttacataac agacgaggtc aacgatccct cccttaccat caaatcaatt     300
   ggccaccaat ggtactgaac ctacgagtac accgactacg gcggactaat cttcaactcc     360
   tacatacttc cccattattt cctagaacca ggcgacctgc gactccttga cgttgacaat     420
   cgagtagtac tcccgattga agcccccatt cgtataatta ttacattttg actaccacaa     480
   ctcaacggct acatagaaaa atccacccct tacgagtgcg gcttcgaccc tatatccccc     540
   gcccgcgtcc ctttctccat aaaattcttc ttagtagcta ttaccttctt attatttgat     600
   ctagaaattg ccctcctttt acccctacca tgagccctac aaacaactaa cctgccacta     660
   atagttatgt catccctctt attaatcatc atcctagccc taagtctggc ctatgagtga     720
   ctacaaaaag gattagactg aaccgaa                                         747
<210> 58
   <211> 249
   <212> PRT
   <213> Human
<220>
   <221> misc_feature
   <223> Fusion protein corresponding to P0026
<400> 58
   Met Ala His Ala Ala Gln Val Gly Leu Gln Asp Ala Thr Ser Pro Ile
   1               5                   10                  15
   Met Glu Glu Leu Ile Thr Phe His Asp His Ala Leu Met Ile Ile Phe
               20                  25                  30
   Leu Ile Cys Phe Leu Val Leu Tyr Ala Leu Phe Leu Thr Leu Thr Thr
           35                  40                  45
   Lys Leu Thr Asn Thr Asn Ile Ser Asp Ala Gln Glu Met Glu Thr Val
       50                  55                  60
   Trp Thr Ile Leu Pro Ala Ile Ile Leu Val Leu Ile Ala Leu Pro Ser
   65                  70                  75                  80
   Leu Arg Ile Leu Tyr Met Thr Asp Glu Val Asn Asp Pro Ser Leu Thr
                   85                  90                  95
   Ile Lys Ser Ile Gly His Gln Trp Tyr Trp Thr Tyr Glu Tyr Thr Asp
               100                 105                 110
   Tyr Gly Gly Leu Ile Phe Asn Ser Tyr Met Leu Pro Pro Leu Phe Leu
           115                 120                 125
   Glu Pro Gly Asp Leu Arg Leu Leu Asp Val Asp Asn Arg Val Val Leu
       130                 135                 140
   Pro Ile Glu Ala Pro Ile Arg Met Ile Ile Thr Phe Trp Leu Pro Gln
   145                 150                 155                 160
   Leu Asn Gly Tyr Met Glu Lys Ser Thr Pro Tyr Glu Cys Gly Phe Asp
                   165                 170                 175
   Pro Met Ser Pro Ala Arg Val Pro Phe Ser Met Lys Phe Phe Leu Val
               180                 185                 190
   Ala Ile Thr Phe Leu Leu Phe Asp Leu Glu Ile Ala Leu Leu Leu Pro
           195                 200                 205
   Leu Pro Trp Ala Leu Gln Thr Thr Asn Leu Pro Leu Met Val Met Ser
       210                 215                 220
   Ser Leu Leu Leu Ile Ile Ile Leu Ala Leu Ser Leu Ala Tyr Glu Trp
   225                 230                 235                 240
   Leu Gln Lys Gly Leu Asp Trp Thr Glu
                   245
<210> 59
   <211> 1544
   <212> RNA
   <213> Human
<220>
   <221> misc_feature
   <223> Fusion transcript P0062 (MT-ND1 (3307-4246) + MT-ATP6 (8603-9207))
<400> 59
   auacccaugg ccaaccuccu acuccucauu guacccauuc uaaucgcaau ggcauuccua      60
   augcuuaccg aacgaaaaau ucuaggcuau auacaacuac gcaaaggccc caacguugua     120
   ggccccuacg ggcuacuaca acccuucgcu gacgccauaa aacucuucac caaagagccc     180
   cuaaaacccg ccacaucuac caucacccuc uacaucaccg ccccgaccuu agcucucacc     240
   aucgcucuuc uacuaugaac cccccucccc auacccaacc cccuggucaa ccucaaccua     300
   ggccuccuau uuauucuagc caccucuagc cuagccguuu acucaauccu cugaucaggg     360
   ugagcaucaa acucaaacua cgcccugauc ggcgcacugc gagcaguagc ccaaacaauc     420
   ucauaugaag ucacccuagc caucauucua cuaucaacau uacuaauaag uggcuccuuu     480
   aaccucucca cccuuaucac aacacaagaa caccucugau uacuccugcc aucaugaccc     540
   uuggccauaa uaugauuuau cuccacacua gcagagacca accgaacccc cuucgaccuu     600
   gccgaagggg aguccgaacu agucucaggc uucaacaucg aauacgccgc aggccccuuc     660
   gcccuauucu ucauagccga auacacaaac auuauuauaa uaaacacccu caccacuaca     720
   aucuuccuag gaacaacaua ugacgcacuc uccccugaac ucuacacaac auauuuuguc     780
   accaagaccc uacuucuaac cucccuguuc uuaugaauuc gaacagcaua cccccgauuc     840
   cgcuacgacc aacucauaca ccuccuauga aaaaacuucc uaccacucac ccuagcauua     900
   cuuauaugau augucuccau acccauuaca aucuccagca uucccccucu auugaucccc     960
   accuccaaau aucucaucaa caaccgacua aucaccaccc aacaaugacu aaucaaacua    1020
   accucaaaac aaaugauaac cauacacaac acuaaaggac gaaccugauc ucuuauacua    1080
   guauccuuaa ucauuuuuau ugccacaacu aaccuccucg gacuccugcc ucacucauuu    1140
   acaccaacca cccaacuauc uauaaaccua gccauggcca uccccuuaug agcgggcaca    1200
   gugauuauag gcuuucgcuc uaagauuaaa aaugcccuag cccacuucuu accacaaggc    1260
   acaccuacac cccuuauccc cauacuaguu auuaucgaaa ccaucagccu acucauucaa    1320
   ccaauagccc uggccguacg ccuaaccgcu aacauuacug caggccaccu acucaugcac    1380
   cuaauuggaa gcgccacccu agcaauauca accauuaacc uucccucuac acuuaucauc    1440
   uucacaauuc uaauucuacu gacuauccua gaaaucgcug ucgccuuaau ccaagccuac    1500
   guuuucacac uucuaguaag ccucuaccug cacgacaaca caua                      1544
<210> 60
   <211> 1544
   <212> DNA
   <213> Human
<220>
   <221> misc_feature
   <223> Mutant DNA for fusion transcript P0062
<400> 60
   atacccatgg ccaacctcct actcctcatt gtacccattc taatcgcaat ggcattccta 60
   atgcttaccg aacgaaaaat tctaggctat atacaactac gcaaaggccc caacgttgta 120
   ggcccctacg ggctactaca acccttcgct gacgccataa aactcttcac caaagagccc 180
   ctaaaacccg ccacatctac catcaccctc tacatcaccg ccccgacctt agctctcacc 240
   atcgctcttc tactatgaac ccccctcccc atacccaacc ccctggtcaa cctcaaccta 300
   ggcctcctat ttattctagc cacctctagc ctagccgttt actcaatcct ctgatcaggg 360
   tgagcatcaa actcaaacta cgccctgatc ggcgcactgc gagcagtagc ccaaacaatc 420
   tcatatgaag tcaccctagc catcattcta ctatcaacat tactaataag tggctccttt 480
   aacctctcca cccttatcac aacacaagaa cacctctgat tactcctgcc atcatgaccc 540
   ttggccataa tatgatttat ctccacacta gcagagacca accgaacccc cttcgacctt 600
   gccgaagggg agtccgaact agtctcaggc ttcaacatcg aatacgccgc aggccccttc 660
   gccctattct tcatagccga atacacaaac attattataa taaacaccct caccactaca 720
   atcttcctag gaacaacata tgacgcactc tcccctgaac tctacacaac atattttgtc 780
   accaagaccc tacttctaac ctccctgttc ttatgaattc gaacagcata cccccgattc 840
   cgctacgacc aactcataca cctcctatga aaaaacttcc taccactcac cctagcatta 900
   cttatatgat atgtctccat acccattaca atctccagca ttccccctct attgatcccc 960
   acctccaaat atctcatcaa caaccgacta atcaccaccc aacaatgact aatcaaacta 1020
   acctcaaaac aaatgataac catacacaac actaaaggac gaacctgatc tcttatacta 1080
   gtatccttaa tcatttttat tgccacaact aacctcctcg gactcctgcc tcactcattt 1140
   acaccaacca cccaactatc tataaaccta gccatggcca tccccttatg agcgggcaca 1200
   gtgattatag gctttcgctc taagattaaa aatgccctag cccacttctt accacaaggc 1260
   acacctacac cccttatccc catactagtt attatcgaaa ccatcagcct actcattcaa 1320
   ccaatagccc tggccgtacg cctaaccgct aacattactg caggccacct actcatgcac 1380
   ctaattggaa gcgccaccct agcaatatca accattaacc ttccctctac acttatcatc 1440
   ttcacaattc taattctact gactatccta gaaatcgctg tcgccttaat ccaagcctac 1500
   gttttcacac ttctagtaag cctctacctg cacgacaaca cata 1544
<210> 61
   <211> 515
   <212> PRT
   <213> Human
<220>
   <221> misc_feature
   <223> Fusion protein corresponding to P0062
<220>
   <221> misc_feature
   <222> (515) .. (515)
   <223> Xaa can be any naturally occurring amino acid
<400> 61 Asn Thr Xaa 515
<210> 62
   <211> 999
   <212> RNA
   <213> Human
<220>
   <221> misc_feature
   <223> Fusion transcript P0064 (MT-ND1 (3307-3752) + MT-ND2 (4958-5511))
<400> 62
   auacccaugg ccaaccuccu acuccucauu guacccauuc uaaucgcaau ggcauuccua      60
   augcuuaccg aacgaaaaau ucuaggcuau auacaacuac gcaaaggccc caacguugua     120
   ggccccuacg ggcuacuaca acccuucgcu gacgccauaa aacucuucac caaagagccc     180
   cuaaaacccg ccacaucuac caucacccuc uacaucaccg ccccgaccuu agcucucacc     240
   aucgcucuuc uacuaugaac cccccucccc auacccaacc cccuggucaa ccucaaccua     300
   ggccuccuau uuauucuagc caccucuagc cuagccguuu acucaauccu cugaucaggg     360
   ugagcaucaa acucaaacua cgcccugauc ggcgcacugc gagcaguagc ccaaacaauc     420
   ucauaugaag ucacccuagc caucauagca ggcaguugag guggauuaaa ccaaacccag     480
   cuacgcaaaa ucuuagcaua cuccucaauu acccacauag gaugaauaau agcaguucua     540
   ccguacaacc cuaacauaac cauucuuaau uuaacuauuu auauuauccu aacuacuacc     600
   gcauuccuac uacucaacuu aaacuccagc accacgaccc uacuacuauc ucgcaccuga     660
   aacaagcuaa caugacuaac acccuuaauu ccauccaccc uccucucccu aggaggccug     720
   cccccgcuaa ccggcuuuuu gcccaaaugg gccauuaucg aagaauucac aaaaaacaau     780
   agccucauca uccccaccau cauagccacc aucacccucc uuaaccucua cuucuaccua     840
   cgccuaaucu acuccaccuc aaucacacua cuccccauau cuaacaacgu aaaaauaaaa     900
   ugacaguuug aacauacaaa acccacccca uuccucccca cacucaucgc ccuuaccacg     960
   cuacuccuac cuaucucccc uuuuauacua auaaucuua                            999
<210> 63
   <211> 999
   <212> DNA
   <213> Human
<220>
   <221> misc_feature
   <223> Mutant DNA for fusion transcript P0064
<400> 63
   atacccatgg ccaacctcct actcctcatt gtacccattc taatcgcaat ggcattccta        60
   atgcttaccg aacgaaaaat tctaggctat atacaactac gcaaaggccc caacgttgta       120
   ggcccctacg ggctactaca acccttcgct gacgccataa aactcttcac caaagagccc       180
   ctaaaacccg ccacatctac catcaccctc tacatcaccg ccccgacctt agctctcacc       240
   atcgctcttc tactatgaac ccccctcccc atacccaacc ccctggtcaa cctcaaccta       300
   ggcctcctat ttattctagc cacctctagc ctagccgttt actcaatcct ctgatcaggg       360
   tgagcatcaa actcaaacta cgccctgatc ggcgcactgc gagcagtagc ccaaacaatc       420
   tcatatgaag tcaccctagc catcatagca ggcagttgag gtggattaaa ccaaacccag       480
   ctacgcaaaa tcttagcata ctcctcaatt acccacatag gatgaataat agcagttcta       540
   ccgtacaacc ctaacataac cattcttaat ttaactattt atattatcct aactactacc       600
   gcattcctac tactcaactt aaactccagc accacgaccc tactactatc tcgcacctga       660
   aacaagctaa catgactaac acccttaatt ccatccaccc tcctctccct aggaggcctg       720
   cccccgctaa ccggcttttt gcccaaatgg gccattatcg aagaattcac aaaaaacaat       780
   agcctcatca tccccaccat catagccacc atcaccctcc ttaacctcta cttctaccta       840
   cgcctaatct actccacctc aatcacacta ctccccatat ctaacaacgt aaaaataaaa       900
   tgacagtttg aacatacaaa acccacccca ttcctcccca cactcatcgc ccttaccacg       960
   ctactcctac ctatctcccc ttttatacta ataatctta                              999
<210> 64
   <211> 333
   <212> PRT
   <213> Human
<220>
   <221> misc_feature
   <223> Fusion protein corresponding to P0064
<400> 64
   Met Pro Met Ala Asn Leu Leu Leu Leu Ile Val Pro Ile Leu Ile Ala
   1               5                   10                  15
   Met Ala Phe Leu Met Leu Thr Glu Arg Lys Ile Leu Gly Tyr Met Gln
               20                  25                  30
   Leu Arg Lys Gly Pro Asn Val Val Gly Pro Tyr Gly Leu Leu Gln Pro
           35                  40                  45
   Phe Ala Asp Ala Met Lys Leu Phe Thr Lys Glu Pro Leu Lys Pro Ala
       50                  55                  60
   Thr Ser Thr Ile Thr Leu Tyr Ile Thr Ala Pro Thr Leu Ala Leu Thr
   65 70 75 80
   Ile Ala Leu Leu Leu Trp Thr Pro Leu Pro Met Pro Asn Pro Leu Val
   85 90 95
   Asn Leu Asn Leu Gly Leu Leu Phe Ile Leu Ala Thr Ser Ser Leu Ala
   100 105 110
   Val Tyr Ser Ile Leu Trp Ser Gly Trp Ala Ser Asn Ser Asn Tyr Ala
   115 120 125
   Leu Ile Gly Ala Leu Arg Ala Val Ala Gln Thr Ile Ser Tyr Glu Val
   130 135 140
   Thr Leu Ala Ile Met Ala Gly Ser Trp Gly Gly Leu Asn Gln Thr Gln
   145 150 155 160
   Leu Arg Lys Ile Leu Ala Tyr Ser Ser Ile Thr His Met Gly Trp Met
   165 170 175
   Met Ala Val Leu Pro Tyr Asn Pro Asn Met Thr Ile Leu Asn Leu Thr
   180 185 190
   Ile Tyr Ile Ile Leu Thr Thr Thr Ala Phe Leu Leu Leu Asn Leu Asn
   195 200 205
   Ser Ser Thr Thr Thr Leu Leu Leu Ser Arg Thr Trp Asn Lys Leu Thr
   210 215 220
   Trp Leu Thr Pro Leu Ile Pro Ser Thr Leu Leu Ser Leu Gly Gly Leu
   225 230 235 240
   Pro Pro Leu Thr Gly Phe Leu Pro Lys Trp Ala Ile Ile Glu Glu Phe
   245 250 255
   Thr Lys Asn Asn Ser Leu Ile Ile Pro Thr Ile Met Ala Thr Ile Thr
   260 265 270
   Leu Leu Asn Leu Tyr Phe Tyr Leu Arg Leu Ile Tyr Ser Thr Ser Ile
   275 280 285
   Thr Leu Leu Pro Met Ser Asn Asn Val Lys Met Lys Trp Gln Phe Glu
   290 295 300
   His Thr Lys Pro Thr Pro Phe Leu Pro Thr Leu Ile Ala Leu Thr Thr
   305 310 315 320
   Leu Leu Leu Pro Ile Ser Pro Phe Met Leu Met Ile Leu
   325 330
<210> 65
   <211> 1481
   <212> RNA
   <213> Human
<220>
   <221> misc_feature
   <223> Fusion transcript P0176 (MT-ND1 (3307-3872) + MT-CO1 (6530-7445))
<400> 65
   auacccaugg ccaaccuccu acuccucauu guacccauuc uaaucgcaau ggcauuccua      60
   augcuuaccg aacgaaaaau ucuaggcuau auacaacuac gcaaaggccc caacguugua     120
   ggccccuacg ggcuacuaca acccuucgcu gacgccauaa aacucuucac caaagagccc     180
   cuaaaacccg ccacaucuac caucacccuc uacaucaccg ccccgaccuu agcucucacc     240
   aucgcucuuc uacuaugaac ccccccuccc auacccaacc cccuggucaa ccucaaccua     300
   ggccuccuau uuauucuagc caccucuagc cuagccguuu acucaauccu cugaucaggg     360
   ugagcaucaa acucaaacua cgcccugauc ggcgcacugc gagcaguagc ccaaacaauc     420
   ucauaugaag ucacccuagc caucauucua cuaucaacau uacuaauaag uggcuccuuu     480
   aaccucucca cccuuaucac aacacaagaa caccucugau uacuccugcc aucaugaccc     540
   uuggccauaa uaugauuuau cuccacacua acagaccgca accucaacac caccuucuuc     600
   gaccccgccg gaggaggaga ccccauucua uaccaacacc uauucugauu uuucggucac     660
   ccugaaguuu auauucuuau ccuaccaggc uucggaauaa ucucccauau uguaacuuac     720
   uacuccggaa aaaaagaacc auuuggauac auagguaugg ucugagcuau gauaucaauu     780
   ggcuuccuag gguuuaucgu gugagcacac cauauauuua caguaggaau agacguagac     840
   acacgagcau auuucaccuc cgcuaccaua aucaucgcua uccccaccgg cgucaaagua     900
   uuuagcugac ucgccacacu ccacggaagc aauaugaaau gaucugcugc agugcucuga     960
   gcccuaggau ucaucuuucu uuucaccgua gguggccuga cuggcauugu auuagcaaac    1020
   ucaucacuag acaucguacu acacgacacg uacuacguug uagcccacuu ccacuauguc    1080
   cuaucaauag gagcuguauu ugccaucaua ggaggcuuca uucacugauu uccccuauuc    1140
   ucaggcuaca cccuagacca aaccuacgcc aaaauccauu ucacuaucau auucaucggc    1200
   guaaaucuaa cuuucuuccc acaacacuuu cucggccuau ccggaaugcc ccgacguuac    1260
   ucggacuacc ccgaugcaua caccacauga aacauccuau caucuguagg cucauucauu    1320
   ucucuaacag caguaauauu aauaauuuuc augauuugag aagccuucgc uucgaagcga      1380
   aaaguccuaa uaguagaaga acccuccaua aaccuggagu gacuauaugg augcccccca      1440
   cccuaccaca cauucgaaga acccguauac auaaaaucua g                          1481
<210> 66
   <211> 1481
   <212> DNA
   <213> Human
<220>
   <221> misc_feature
   <223> Mutant DNA for fusion transcript P0176
<400> 66
   atacccatgg ccaacctcct actcctcatt gtacccattc taatcgcaat ggcattccta       60
   atgcttaccg aacgaaaaat tctaggctat atacaactac gcaaaggccc caacgttgta      120
   ggcccctacg ggctactaca acccttcgct gacgccataa aactcttcac caaagagccc      180
   ctaaaacccg ccacatctac catcaccctc tacatcaccg ccccgacctt agctctcacc      240
   atcgctcttc tactatgaac ccccctcccc atacccaacc ccctggtcaa cctcaaccta      300
   ggcctcctat ttattctagc cacctctagc ctagccgttt actcaatcct ctgatcaggg      360
   tgagcatcaa actcaaacta cgccctgatc ggcgcactgc gagcagtagc ccaaacaatc      420
   tcatatgaag tcaccctagc catcattcta ctatcaacat tactaataag tggctccttt      480
   aacctctcca cccttatcac aacacaagaa cacctctgat tactcctgcc atcatgaccc      540
   ttggccataa tatgatttat ctccacacta acagaccgca acctcaacac caccttcttc      600
   gaccccgccg gaggaggaga ccccattcta taccaacacc tattctgatt tttcggtcac      660
   cctgaagttt atattcttat cctaccaggc ttcggaataa tctcccatat tgtaacttac      720
   tactccggaa aaaaagaacc atttggatac ataggtatgg tctgagctat gatatcaatt      780
   ggcttcctag ggtttatcgt gtgagcacac catatattta cagtaggaat agacgtagac      840
   acacgagcat atttcacctc cgctaccata atcatcgcta tccccaccgg cgtcaaagta      900
   tttagctgac tcgccacact ccacggaagc aatatgaaat gatctgctgc agtgctctga      960
   gccctaggat tcatctttct tttcaccgta ggtggcctga ctggcattgt attagcaaac     1020
   tcatcactag acatcgtact acacgacacg tactacgttg tagcccactt ccactatgtc     1080
   ctatcaatag gagctgtatt tgccatcata ggaggcttca ttcactgatt tcccctattc     1140
   tcaggctaca ccctagacca aacctacgcc aaaatccatt tcactatcat attcatcggc     1200
   gtaaatctaa ctttcttccc acaacacttt ctcggcctat ccggaatgcc ccgacgttac     1260
   tcggactacc ccgatgcata caccacatga aacatcctat catctgtagg ctcattcatt     1320
   tctctaacag cagtaatatt aataattttc atgatttgag aagccttcgc ttcgaagcga     1380
   aaagtcctaa tagtagaaga accctccata aacctggagt gactatatgg atgcccccca     1440
   ccctaccaca cattcgaaga acccgtatac ataaaatcta g                         1481
<210> 67
   <211> 494
   <212> PRT
   <213> Human
<220>
   <221> misc_feature
   <223> Fusion protein corresponding to P0176
<220>
   <221> misc_feature
   <222> (494)..(494)
   <223> Xaa can be any naturally occurring amino acid
<400> 67
   Met Pro Met Ala Asn Leu Leu Leu Leu Ile Val Pro Ile Leu Ile Ala
   1               5                   10                  15
   Met Ala Phe Leu Met Leu Thr Glu Arg Lys Ile Leu Gly Tyr Met Gln
               20                  25                  30
   Leu Arg Lys Gly Pro Asn Val Val Gly Pro Tyr Gly Leu Leu Gln Pro
           35                  40                  45
   Phe Ala Asp Ala Met Lys Leu Phe Thr Lys Glu Pro Leu Lys Pro Ala
       50                  55                  60
   Thr Ser Thr Ile Thr Leu Tyr Ile Thr Ala Pro Thr Leu Ala Leu Thr
   65                  70                  75                  80
   Ile Ala Leu Leu Leu Trp Thr Pro Leu Pro Met Pro Asn Pro Leu Val
                   85                  90                  95
   Asn Leu Asn Leu Gly Leu Leu Phe Ile Leu Ala Thr Ser Ser Leu Ala
               100                 105                 110
   Val Tyr Ser Ile Leu Trp Ser Gly Trp Ala Ser Asn Ser Asn Tyr Ala
           115                 120                 125
   Leu Ile Gly Ala Leu Arg Ala Val Ala Gln Thr Ile Ser Tyr Glu Val
       130                 135                 140
   Thr Leu Ala Ile Ile Leu Leu Ser Thr Leu Leu Met Ser Gly Ser Phe
   145                 150                 155                 160
   Asn Leu Ser Thr Leu Ile Thr Thr Gln Glu His Leu Trp Leu Leu Leu
                   165                 170                 175
   Pro Ser Trp Pro Leu Ala Met Met Trp Phe Ile Ser Thr Leu Thr Asp
               180                 185                 190
   Arg Asn Leu Asn Thr Thr Phe Phe Asp Pro Ala Gly Gly Gly Asp Pro
           195                 200                 205
   Ile Leu Tyr Gln His Leu Phe Trp Phe Phe Gly His Pro Glu Val Tyr
       210                 215                 220
   Ile Leu Ile Leu Pro Gly Phe Gly Met Ile Ser His Ile Val Thr Tyr
   225                 230                 235                 240
   Tyr Ser Gly Lys Lys Glu Pro Phe Gly Tyr Met Gly Met Val Trp Ala
                   245                 250                 255
   Met Met Ser Ile Gly Phe Leu Gly Phe Ile Val Trp Ala His His Met
               260                 265                 270
   Phe Thr Val Gly Met Asp Val Asp Thr Arg Ala Tyr Phe Thr Ser Ala
           275                 280                 285
   Thr Met Ile Ile Ala Ile Pro Thr Gly Val Lys Val Phe Ser Trp Leu
       290                 295                 300
   Ala Thr Leu His Gly Ser Asn Met Lys Trp Ser Ala Ala Val Leu Trp
   305                 310                 315                 320
   Ala Leu Gly Phe Ile Phe Leu Phe Thr Val Gly Gly Leu Thr Gly Ile
                   325                 330                 335
   Val Leu Ala Asn Ser Ser Leu Asp Ile Val Leu His Asp Thr Tyr Tyr
               340                 345                 350
   Val Val Ala His Phe His Tyr Val Leu Ser Met Gly Ala Val Phe Ala
           355                 360                 365
   Ile Met Gly Gly Phe Ile His Trp Phe Pro Leu Phe Ser Gly Tyr Thr
       370                 375                 380
   Leu Asp Gln Thr Tyr Ala Lys Ile His Phe Thr Ile Met Phe Ile Gly
   385                 390                 395                 400
   Val Asn Leu Thr Phe Phe Pro Gln His Phe Leu Gly Leu Ser Gly Met
                   405                 410                 415
   Pro Arg Arg Tyr Ser Asp Tyr Pro Asp Ala Tyr Thr Thr Trp Asn Ile
               420                 425                 430
   Leu Ser Ser Val Gly Ser Phe Ile Ser Leu Thr Ala Val Met Leu Met
           435                 440                 445
   Ile Phe Met Ile Trp Glu Ala Phe Ala Ser Lys Arg Lys Val Leu Met
       450                 455                 460
   Val Glu Glu Pro Ser Met Asn Leu Glu Trp Leu Tyr Gly Cys Pro Pro
   465                 470                 475                 480
   Pro Tyr His Thr Phe Glu Glu Pro Val Tyr Met Lys Ser Xaa
                   485                 490